(19) Europäisches Patentamt
European Patent Office
Office européen des brevets

(11) EP 4 379 007 B1

(12) EUROPEAN PATENT SPECIFICATION

(45) Date of publication and mention of the grant of the patent:
**26.11.2025 Bulletin 2025/48**

(21) Application number: **22870268.4**

(22) Date of filing: **14.09.2022**

(51) International Patent Classification (IPC):
***C09D 11/38*** (2014.01) ***C09D 11/03*** (2014.01)
***H10K 50/00*** (2023.01) ***C09K 11/06*** (2006.01)
***C09D 11/101*** (2014.01) ***C09D 11/36*** (2014.01)

(52) Cooperative Patent Classification (CPC):
**C09K 11/06; C09D 11/101; C09D 11/36; C09D 11/38;** C09K 2211/1007; C09K 2211/1011; C09K 2211/1014; C09K 2211/1088

(86) International application number:
**PCT/KR2022/013715**

(87) International publication number:
**WO 2023/043184 (23.03.2023 Gazette 2023/12)**

# (54) INK COMPOSITION, ORGANIC MATERIAL LAYER COMPRISING SAME, AND ORGANIC LIGHT-EMITTING DEVICE COMPRISING SAME

TINTENZUSAMMENSETZUNG, ORGANISCHE MATERIALSCHICHT DAMIT UND ORGANISCHE LICHTEMITTIERENDE VORRICHTUNG DAMIT

COMPOSITION D'ENCRE, COUCHE DE MATÉRIAU ORGANIQUE LA COMPRENANT ET DISPOSITIF ÉLECTROLUMINESCENT ORGANIQUE LA COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.09.2021 KR 20210122564**

(43) Date of publication of application:
**05.06.2024 Bulletin 2024/23**

(73) Proprietor: **LG Chem, Ltd.**
**Yeongdeungpo-gu**
**Seoul 07336 (KR)**

(72) Inventors:
- **LEE, Keunsoo**
  **Daejeon 34122 (KR)**
- **PARK, Jung Ha**
  **Daejeon 34122 (KR)**
- **KANG, Eunhye**
  **Daejeon 34122 (KR)**
- **KIM, Byungjae**
  **Daejeon 34122 (KR)**
- **SEO, Minseob**
  **Daejeon 34122 (KR)**
- **LEE, Jaechol**
  **Daejeon 34122 (KR)**
- **LEE, Jiyoung**
  **Daejeon 34122 (KR)**
- **LEE, Ho Gyu**
  **Daejeon 34122 (KR)**

(74) Representative: **Hoffmann Eitle**
**Patent- und Rechtsanwälte PartmbB**
**Arabellastraße 30**
**81925 München (DE)**

(56) References cited:
**WO-A1-2021/154041**
**JP-A- 2011 029 666**
**JP-A- 2015 050 095**
**KR-A- 20170 084 917**
**KR-A- 20180 137 258**
**KR-A- 20190 081 263**
**KR-A- 20210 030 019**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 379 007 B1

## Description

[Technical Field]

This application claims priority to and the benefit of Korean Patent Application No. 10-2021-0122564 filed in the Korean Intellectual Property Office on September 14, 2021.

The present invention relates to an ink composition, an organic material layer including the same and an organic light emitting device including the same.

[Background Art]

An organic light emission phenomenon is one of the examples of converting an electric current into visible rays through an internal process of a specific organic molecule. The principle of the organic light emission phenomenon is as follows. When an organic material layer is disposed between an anode and a cathode and an electric current is applied between the two electrodes, electrons and holes are injected into the organic material layer from the cathode and the anode, respectively. The electrons and the holes which are injected into the organic material layer are recombined to form an exciton, and the exciton falls down again to the ground state to emit light. An organic light emitting device using the principle may be generally composed of a cathode, an anode, and an organic material layer disposed therebetween, for example, a hole injection layer, a hole transport layer, a light emitting layer, an electron injection layer, an electron transport layer, an electron blocking layer, a hole blocking layer, and the like.

In order to manufacture an organic light emitting device in the related art, a deposition process has been usually used. However, there are problems in that the loss of materials occurs frequently when an organic light emitting device is manufactured by a deposition process and in that it is difficult to manufacture a device having a large area, and to solve these problems, a device using a solution process has been developed.

WO 2021/154041 A1 relates to a compound, a coating composition comprising the compound, an organic light emitting device formed by using the coating composition, and a method of manufacturing the same.

Therefore, there is a need for the development of a material for a solution process.

[Detailed Description of the Invention]

[Technical Problem]

The present invention relates to an ink composition, an organic material layer including the same and an organic light emitting device including the same.

[Technical Solution]

An exemplary embodiment of the present invention provides an ink composition including a compound represented by the following Chemical Formula 1 and a solvent represented by the following Chemical Formula A.

[Chemical Formula 1]

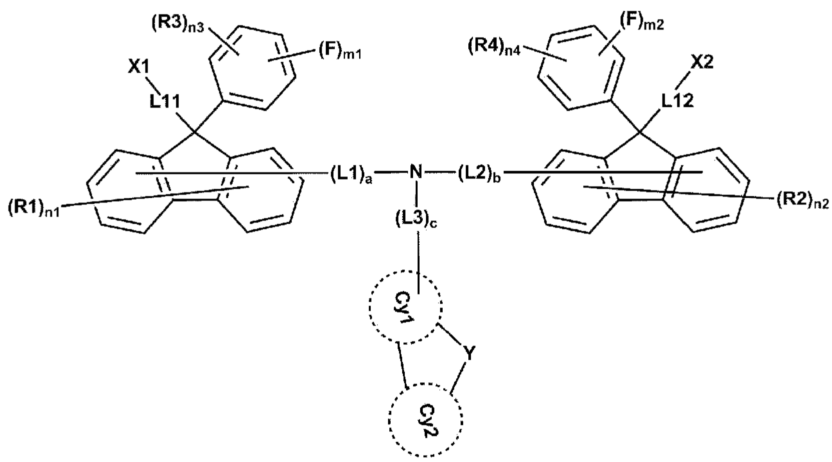

[Chemical Formula A]

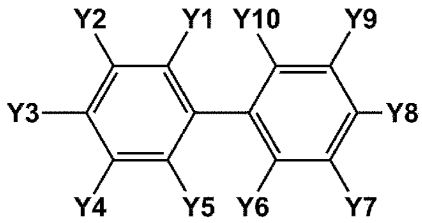

In Chemical Formulae 1 and A,

Y is O, S, CRaRb or SiRcRd,
Cy1 and Cy2 are the same as or different from each other, and are each independently a substituted or unsubstituted benzene ring; or a substituted or unsubstituted naphthalene ring,
Ra, Rb, Rc and Rd are the same as or different from each other, and are each independently hydrogen; deuterium; a substituted or unsubstituted alkyl group; a substituted or unsubstituted aryl group; or a substituted or unsubstituted heteroaryl group, or are bonded to an adjacent group to form a substituted or unsubstituted ring,
L1 to L3 are the same as or different from each other, and are each independently a direct bond; or a substituted or unsubstituted arylene group,
L11 and L12 are the same as or different from each other, and are each independently a direct bond; a substituted or unsubstituted alkylene group; or a substituted or unsubstituted arylene group,
X1 and X2 are the same as or different from each other, and are each independently a curable group,
R1 and R2 are the same as or different from each other, and are each independently deuterium; a halogen group; a substituted or unsubstituted alkyl group; a substituted or unsubstituted alkoxy group; a substituted or unsubstituted aryl group; or a substituted or unsubstituted heteroaryl group,
R3 and R4 are the same as or different from each other, and are each independently deuterium; a substituted or unsubstituted alkyl group; a substituted or unsubstituted alkoxy group; a substituted or unsubstituted aryl group; or a substituted or unsubstituted heteroaryl group,
a, b and c are each 0 or 1,
n1 and n2 are each an integer from 0 to 7, and when n1 and n2 are each 2 or higher, two or more substituents in the parenthesis are the same as or different from each other,
m1 and m2 are each an integer from 1 to 5, n3 and n4 are each an integer from 0 to 4, m1+n3 is 5 or less, and m2+n4 is 5 or less, and
Y1 to Y10 are the same as or different from each other, and are each independently hydrogen; deuterium; a hydroxyl group; an ether group; a carbonyl group; an ester group; a substituted or unsubstituted alkyl group; a substituted or unsubstituted cycloalkyl group; a substituted or unsubstituted cycloalkenyl group; a substituted or unsubstituted alkoxy group; a substituted or unsubstituted aryl group; or a substituted or unsubstituted amine group.

Another exemplary embodiment of the present invention provides a pixel including an ink composition or a cured product thereof.
Still another exemplary embodiment of the present invention provides an organic material layer including the pixel.
Yet another exemplary embodiment of the present invention provides an organic light emitting device including: a first electrode; a second electrode; and an organic material layer having one or more layers provided between the first electrode and the second electrode, in which one or more layers of the organic material layer include the above-described ink composition or a cured product thereof.

[Advantageous Effects]

The ink composition according to an exemplary embodiment of the present invention can be used as a material for an organic material layer of an organic light emitting device, and can obtain a device having low driving voltage, excellent light emitting efficiency and long service life characteristics or can be subjected to a solution process, thereby enabling a large area of the device.
An organic material layer including the ink composition according to an exemplary embodiment of the present invention has excellent flatness characteristics.

[Brief Description of Drawings]

FIG. 1 illustrates an organic light emitting device according to an exemplary embodiment of the present invention.

FIG. 2 illustrates a line bank inkjet substrate to which the ink composition according to an exemplary embodiment of the invention is applied.

FIG. 3 illustrates that the ink composition according to an exemplary embodiment of the present invention is applied to a line bank inkjet substrate.

FIG. 4 illustrates that the ink composition according to an exemplary embodiment of the present specification is formed as a thin film on a line bank inkjet substrate.

FIG. 5 illustrates the thickness of a pixel according to Example 6 of the present invention.

FIG. 6 illustrates the thickness of a pixel according to Example 16 of the present invention.

FIG. 7 illustrates the thickness of a pixel according to Comparative Example 5 of the present invention.

FIG. 8 illustrates the thickness of a pixel according to Comparative Example 10 of the present invention.

FIG. 9 illustrates the thickness of a pixel according to Comparative Example 15 of the present invention.

FIG. 10 illustrates the thickness of a pixel according to Comparative Example 27 of the present invention.

FIG. 11 illustrates the thickness of a pixel according to Example 36 of the present invention.

FIG. 12 illustrates the thickness of a pixel according to Example 40 of the present invention.

FIG. 13 illustrates the thickness of a pixel according to Comparative Example 29 of the present invention.

FIG. 14 illustrates the thickness of a pixel according to Comparative Example 30 of the present invention.

FIG. 15 illustrates the thickness of a pixel according to Comparative Example 31 of the present invention.

FIG. 16 illustrates the thickness of a pixel according to Comparative Example 32 of the present invention.

101: Substrate
201: First electrode
301: Hole injection layer
401: Hole transport layer
501: Light emitting layer
601: Electron injection and transport layer
701: Second electrode

[Best Mode]

Hereinafter, the present invention will be described in detail.

An exemplary embodiment of the present invention provides an ink composition including a compound represented by the following Chemical Formula 1 and a solvent represented by the following Chemical Formula A.

[Chemical Formula 1]

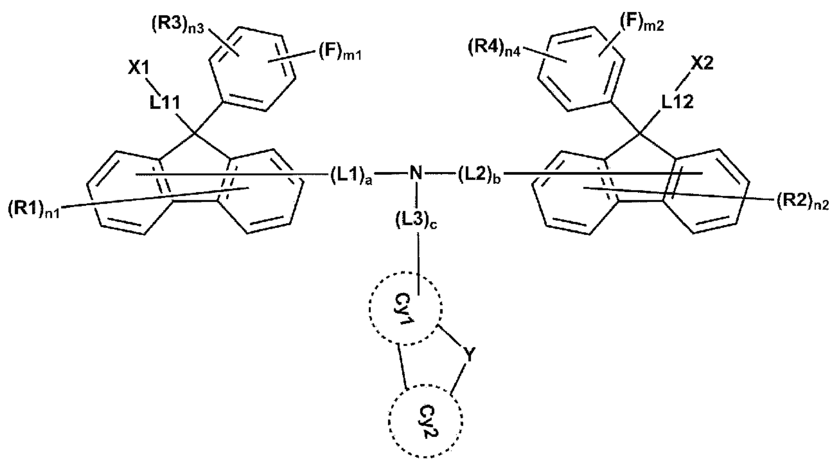

[Chemical Formula A]

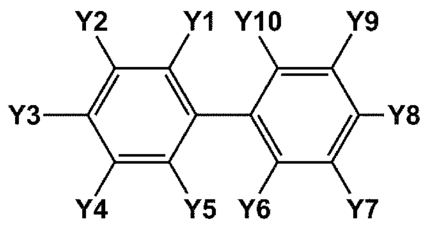

In Chemical Formulae 1 and A,

Y is O, S, CRaRb or SiRcRd,
Cy1 and Cy2 are the same as or different from each other, and are each independently a substituted or unsubstituted benzene ring; or a substituted or unsubstituted naphthalene ring,
Ra, Rb, Rc and Rd are the same as or different from each other, and are each independently hydrogen; deuterium; a substituted or unsubstituted alkyl group; a substituted or unsubstituted aryl group; or a substituted or unsubstituted heteroaryl group, or are bonded to an adjacent group to form a substituted or unsubstituted ring,
L1 to L3 are the same as or different from each other, and are each independently a direct bond; or a substituted or unsubstituted arylene group,
L11 and L12 are the same as or different from each other, and are each independently a direct bond; a substituted or unsubstituted alkylene group; or a substituted or unsubstituted arylene group,
X1 and X2 are the same as or different from each other, and are each independently a curable group,
R1 and R2 are the same as or different from each other, and are each independently deuterium; a halogen group; a substituted or unsubstituted alkyl group; a substituted or unsubstituted alkoxy group; a substituted or unsubstituted aryl group; or a substituted or unsubstituted heteroaryl group,
R3 and R4 are the same as or different from each other, and are each independently deuterium; a substituted or unsubstituted alkyl group; a substituted or unsubstituted alkoxy group; a substituted or unsubstituted aryl group; or a substituted or unsubstituted heteroaryl group,
a, b and c are each 0 or 1,
n1 and n2 are each an integer from 0 to 7, and when n1 and n2 are each 2 or higher, two or more substituents in the parenthesis are the same as or different from each other,
m1 and m2 are each an integer from 1 to 5, n3 and n4 are each an integer from 0 to 4, m1+n3 is 5 or less, and m2+n4 is 5 or less, and
Y1 to Y10 are the same as or different from each other, and are each independently hydrogen; deuterium; a hydroxyl group; an ether group; a carbonyl group; an ester group; a substituted or unsubstituted alkyl group; a substituted or unsubstituted cycloalkyl group; a substituted or unsubstituted cycloalkenyl group; a substituted or unsubstituted alkoxy group; a substituted or unsubstituted aryl group; or a substituted or unsubstituted amine group.

Since the compound represented by Chemical Formula 1 according to an exemplary embodiment of the present invention includes two different types of substituents, specifically a haloaryl group and a curable group and includes an amine group including a tricyclic or higher ring to suppress the formation of radicals at the corresponding position, thereby enhancing the stability of the compound and having high highest occupied molecular orbital (HOMO) energy level values and excellent hole mobility. For this reason, when the compound represented by Chemical Formula 1 is included in a hole injection layer in an organic light emitting device, the compound facilitates the injection of holes from the hole injection layer to a hole transport layer, so that an advantage in that it is possible to obtain an organic light emitting device having long service life characteristics significantly affects the improvement of the service life of the device.

As the solvent represented by Chemical Formula A according to an exemplary embodiment of the present invention includes biphenyl as a core structure, the solvent has excellent solubility in an aromatic solute included in the ink composition. In addition, there is an advantage in that the uniformity of an organic material layer can be improved by reducing the thickness deviation according to the position of each pixel due to the good leveling effect. Specifically, the ink composition may reduce the deviation in thickness between pixels coated, dried and heat-treated. Accordingly, it is possible to expect excellent light emitting and/or coloring effects by reducing factors that affect the interference condition of light emitted from the light emitting device according to the thickness.

When one member (layer) is disposed "on" another member (layer) in the present invention, this includes not only a case where the one member (layer) is brought into contact with another member, but also a case where still another member (layer) is present between the two members (layers).

When one part "includes" one constituent element in the present invention, unless otherwise specifically described, this does not mean that another constituent element is excluded, but means that another constituent element may be further included.

In the present invention, the "layer" has a meaning compatible with a "film" usually used in the art, and means a coating covering a target region. The size of the "layer" is not limited, and the sizes of the respective "layers" may be the same as or different from one another. According to an exemplary embodiment, the size of the "layer" may be the same as that of the entire device, may correspond to the size of a specific functional region, and may also be as small as a single sub-pixel.

Unless otherwise defined in the present invention, all technical and scientific terms used in the present invention have the same meaning as commonly understood by one with ordinary skill in the art to which the present invention pertains. Although methods and materials similar to or equivalent to those described in the present invention may be used in the practice or in the test of exemplary embodiments of the present invention, suitable methods and materials will be described below. In addition, the materials, methods, and examples are illustrative only and are not intended to be limiting.

In the present invention, the term "combination thereof" included in the Markush type expression means a mixture or combination of one or more selected from the group consisting of constituent elements described in the Markush type expression, and means including one or more selected from the group consisting of the above-described constituent elements.

Examples of the substituents in the present invention will be described below, but are not limited thereto.

In the present invention, "------" and

" 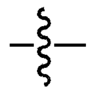 "

each mean a moiety to be linked.

In the present invention, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present invention, the term "substituted or unsubstituted" means being substituted with one or more substituents selected from the group consisting of hydrogen; deuterium; a halogen group; an amine group; an alkyl group; an aryl group; and a heteroaryl group, being substituted with a substituent to which two or more substituents among the exemplified substituents are linked, or having no substituent.

In the present invention, the fact that two or more substituents are linked indicates that hydrogen of any one substituent is linked to another substituent. For example, an isopropyl group and a phenyl group may be linked to each other to become a substituent of

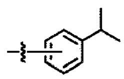 or 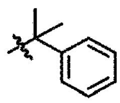 .

In the present invention, the fact that three substituents are linked to one another may include not only a case where (Substituent 1)-(Substituent 2)-(Substituent 3) are consecutively linked to one another, but also a case where (Substituent 2) and (Substituent 3) are linked to (Substituent 1). For example, two phenyl groups and an isopropyl group may be linked to each other to become a substituent of

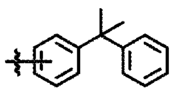 or 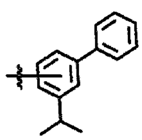 .

The same also applies to the case where four or more substituents are linked to one another.

In the present invention, a halogen group is a fluoro group (-F), a chloro group (-Cl), a bromo group (-Br), or an iodo group (-I).

In the present invention, an alkyl group may be straight-chained or branched, and the number of carbon atoms thereof is not particularly limited, but is preferably 1 to 30; 1 to 20; 1 to 10; or 1 to 5. Specific examples thereof include methyl, ethyl, propyl, n-propyl, isopropyl, butyl, n-butyl, isobutyl, t-butyl, sec-butyl, 1-methylbutyl, 1-ethylbutyl, pentyl, n-pentyl, isopentyl, neopentyl, t-pentyl, hexyl, n-hexyl, 1-methylpentyl, 2-methylpentyl, 3,3-dimethylbutyl, 2-ethylbutyl, heptyl, n-heptyl, 1-methylhexyl, cyclopentylmethyl, cyclohexylmethyl, octyl, n-octyl, t-octyl, 1-methylheptyl, 2-ethylhexyl, 2-propylpentyl, n-nonyl, 2,2-dimethylheptyl, 1-ethylpropyl, 1,1-dimethylpropyl, isohexyl, 4-methylhexyl, 5-methylhexyl, and the like, but are not limited thereto. The alkyl group may include a haloalkyl group which is an alkyl group substituted with a halogen group. Specific examples thereof include a methyl group substituted with three fluoro groups, that is, a trifluoromethyl group, and the like, but are not limited thereto.

In the present specification, a cycloalkyl group is not particularly limited, but has preferably 3 to 30 carbon atoms, and specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present invention, an aryl group means a monovalent aromatic hydrocarbon or a monovalent group of an aromatic hydrocarbon derivative. In the present invention, an aromatic hydrocarbon means a compound in which pi electrons are completely conjugated and which contains a planar ring, and a group derived from an aromatic hydrocarbon means a structure in which an aromatic hydrocarbon or a cyclic aliphatic hydrocarbon is fused with an aromatic hydrocarbon. Further, in the present invention, an aryl group intends to include a monovalent group in which two or more aromatic hydrocarbons or derivatives of an aromatic hydrocarbon are linked to each other. The aryl group is not particularly limited, but preferably has 6 to 60 carbon atoms; 6 to 50 carbon atoms; 6 to 30 carbon atoms; 6 to 25 carbon atoms; 6 to 20 carbon atoms; 6 to 18 carbon atoms; 6 to 15 carbon atoms; 6 to 13 carbon atoms; or 6 to 12 carbon atoms, and may be a monocyclic aryl group or a polycyclic aryl group.

The monocyclic aryl group is not particularly limited, but preferably has 6 to 60 carbon atoms; 6 to 54 carbon atoms; 6 to 48 carbon atoms; 6 to 42 carbon atoms; 6 to 36 carbon atoms; 6 to 30 carbon atoms; 6 to 24 carbon atoms; 6 to 18 carbon atoms; or 6 to 12 carbon atoms, and may be specifically a phenyl group, a biphenyl group, a terphenyl group, and the like, but is not limited thereto.

The polycyclic aryl group is not particularly limited, but preferably has 6 to 60 carbon atoms; 6 to 45 carbon atoms; 6 to 30 carbon atoms; 6 to 22 carbon atoms; 6 to 20 carbon atoms; 6 to 18 carbon atoms; 6 to 16 carbon atoms; 6 to 15 carbon atoms; 6 to 14 carbon atoms; 6 to 13 carbon atoms; 6 to 12 carbon atoms; or 6 to 10 carbon atoms, and may be a naphthyl group, an anthracenyl group, a phenanthrenyl group, a pyrenyl group, a perylenyl group, a triphenylenyl group, a chrysenyl group, a fluorenyl group, and the like, but is not limited thereto.

In the present invention, the aryl group may include a haloaryl group substituted with a halogen group. The haloaryl group means an aryl group substituted with one or more halogen groups, and the haloaryl group may be an aryl group substituted with one or more substituents selected from the group consisting of a fluoro group, a chloro group, a bromo group and an iodo group. In addition, the haloaryl group may have the same two halogen groups or more substituted, and may be, for example, not only an aryl group substituted with one fluoro group, but also an aryl group substituted with two or more fluoro groups.

In the present invention, a heteroaryl group means a monovalent aromatic hetero ring. Here, the aromatic hetero ring is a monovalent group of an aromatic ring or a derivative of the aromatic ring, and means a group including one or more selected from the group consisting of N, O, P, S, Si and Se as a heteroatom. The derivative of the aromatic ring all includes a structure in which an aromatic ring or an aliphatic ring is fused with an aromatic ring. Further, in the present specification, the heteroaryl group intends to include a monovalent group in which two or more aromatic rings including a heteroatom or two or more derivatives of an aromatic ring including a heteroatom are linked to each other. The number of carbon atoms of the heteroaryl group is preferably 2 to 60; 2 to 50; 2 to 30; 2 to 20; 2 to 18; or 2 to 13. Examples of the heteroaryl group include a thiophene group, a furanyl group, a pyrrole group, an imidazole group, a thiazole group, an oxazole group, a pyridine group, a pyrimidine group, a triazine group, a triazole group, an acridine group, a pyridazine group, a pyrazine group, a quinoline group, a quinazoline group, a quinoxaline group, an isoquinoline group, an indole group, a carbazole group, a benzoxazole group, a benzimidazole group, a benzothiazole group, a benzocarbazole group, a benzothiophene group, a dibenzothiophene group, a benzofuran group, a phenanthrolinyl group, a dibenzofuran group, and the like, but are not limited thereto.

In the present invention, the heteroaryl group may be monocyclic or polycyclic, and may be an aromatic ring, an aliphatic ring, or a fused ring of the aromatic ring and the aliphatic ring.

In the present invention, the heterocyclic group is a monovalent group of an aliphatic ring, a derivative of the aliphatic ring, an aromatic ring or a derivative of the aromatic ring, and means a group including one or more selected from the group consisting of N, O, P, S, Si and Se as a heteroatom.

In the present invention, the aliphatic ring is not an aromatic but a hydrocarbon ring, and examples thereof include examples of the above-described cycloalkyl group, an adamantyl group, and the like.

In the present invention, the above-described content on the aryl group may be applied to an aromatic ring.

In the present invention, a hydrocarbon ring may be an aromatic ring, an aliphatic ring, or a fused ring of the aromatic ring and the aliphatic ring. Examples of the fused ring of the aromatic ring and the aliphatic ring include a 1,2,3,4-tetrahydronaphthalene group, a 2,3-dihydro-1H-indene group, and the like, but are not limited thereto.

In the present invention, the description on the heterocyclic group may be applied to a divalent hetero ring except that the hetero ring is a divalent group.

In the present invention, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as "groups which are adjacent" to each other.

In the present invention, a curable group may be subjected to polymerization or cross-linking reaction, and through this, a reaction to increase the molecular weight may be performed, and examples of the curable group include a thermosetting group that is cured by heat, a photocurable group that is cured by light, or the like. The curable group is any one selected from the group consisting of the following structures, but is not limited to the following structures.

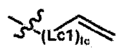 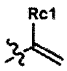 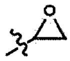 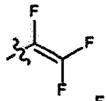 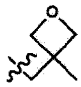

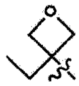 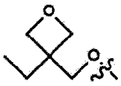 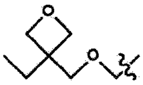 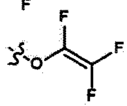 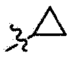

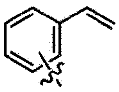 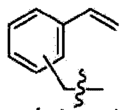 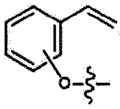 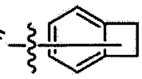 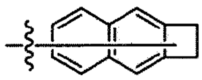

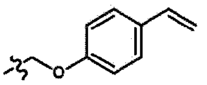 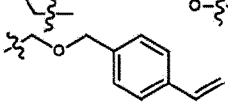 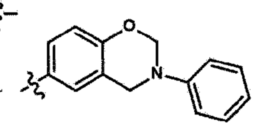

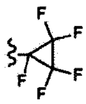 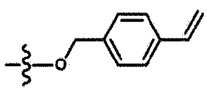 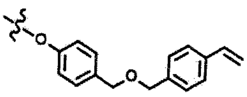

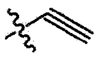 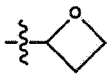 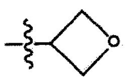 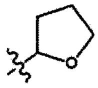 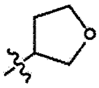

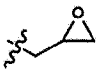 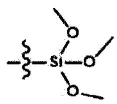 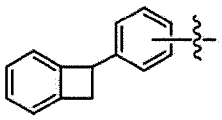 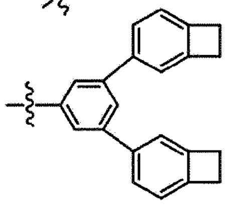

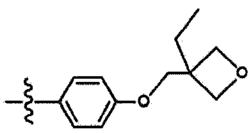 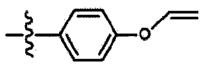 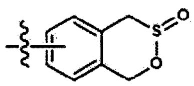

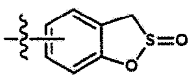

In the structures,

Lc1 is a direct bond; -O-; -S-; a substituted or unsubstituted alkylene group; a substituted or unsubstituted arylene group; or a substituted or unsubstituted heteroarylene group,
lc is 1 or 2,
when lc is 2, Lc1's are the same as or different from each other,
Rc1 is a substituted or unsubstituted alkyl group, and

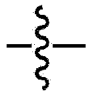

is a moiety bonded to Chemical Formula 1.

According to an exemplary embodiment of the present invention, Lc1 is a direct bond; a methylene group; or an ethylene group.

According to another exemplary embodiment of the present invention, Lc1 is a direct bond.

According to an exemplary embodiment of the present invention, Rc1 is a methyl group; or an ethyl group.

According to another exemplary embodiment of the present invention, Rc1 is a methyl group.

Hereinafter, the compound represented by Chemical Formula 1 will be specifically described.

According to an exemplary embodiment of the present invention, Chemical Formula 1 is represented by the following Chemical Formula 11.

[Chemical Formula 11]

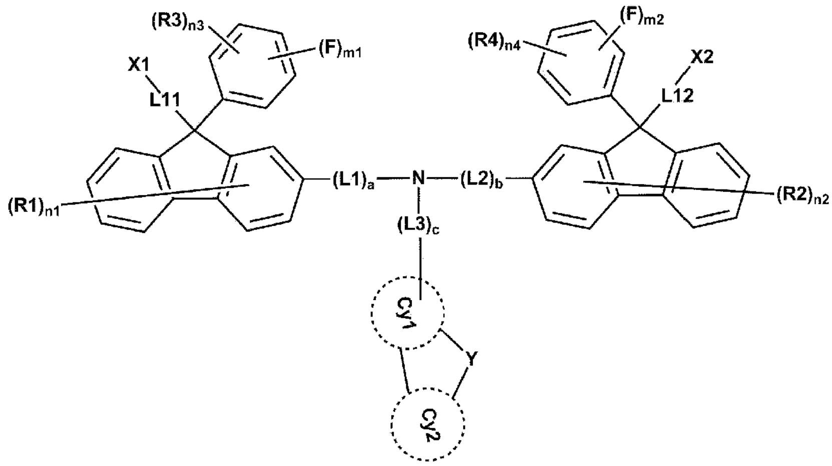

In Chemical Formula 11,
the definitions of L1 to L3, L11, L12, X1, X2, R1 to R4, Y, Cy1, Cy2, a, b, c, n1 to n4, m1 and m2 are the same as those in Chemical Formula 1.

According to an exemplary embodiment of the present invention, L1 to L3 are the same as or different from each other, and are each independently a direct bond; or a substituted or unsubstituted arylene group having 6 to 30 carbon atoms.

According to another exemplary embodiment, L1 to L3 are the same as or different from each other, and are each independently a direct bond; a substituted or unsubstituted phenylene group; or a substituted or unsubstituted naphthylene group.

According to still another exemplary embodiment, L1 and L2 are each a direct bond.

According to yet another exemplary embodiment, L3 is a direct bond; a substituted or unsubstituted phenylene group; or a substituted or unsubstituted naphthylene group.

According to an exemplary embodiment of the present invention, Chemical Formula 1 is represented by any one of the following Chemical Formulae 12 to 14.

[Chemical Formula 12]

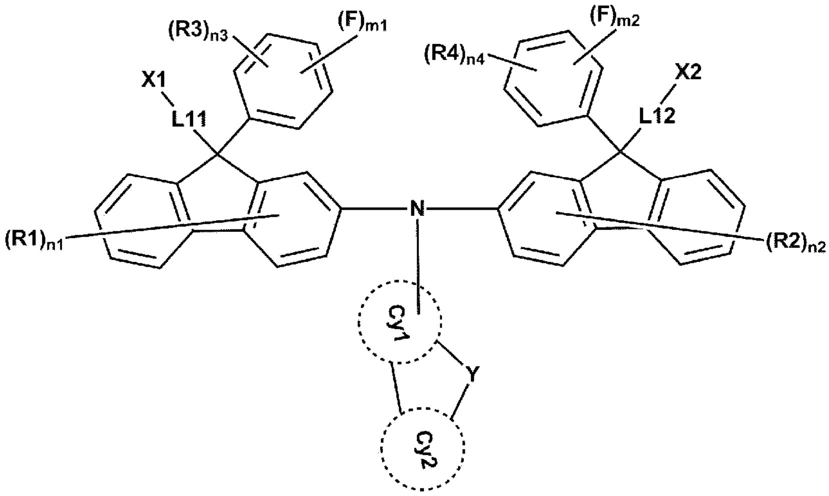

[Chemical Formula 13]

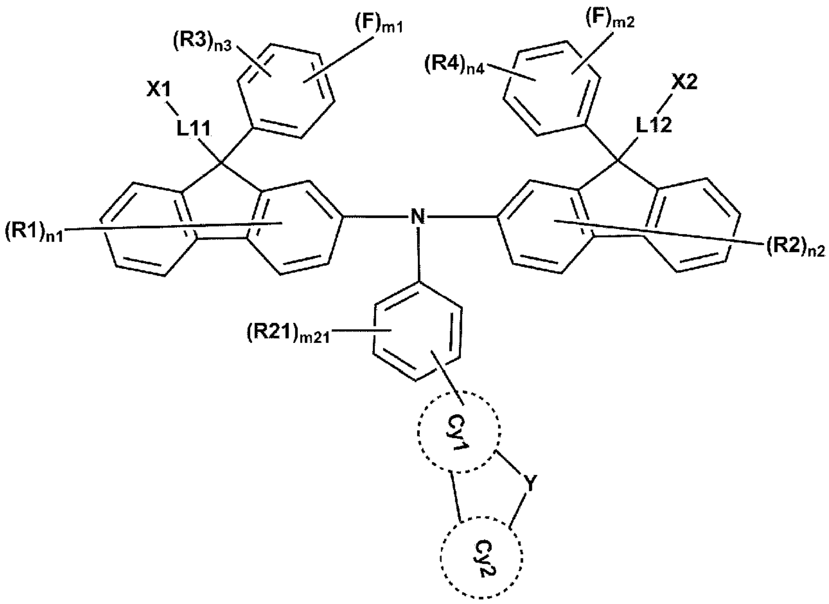

[Chemical Formula 14]

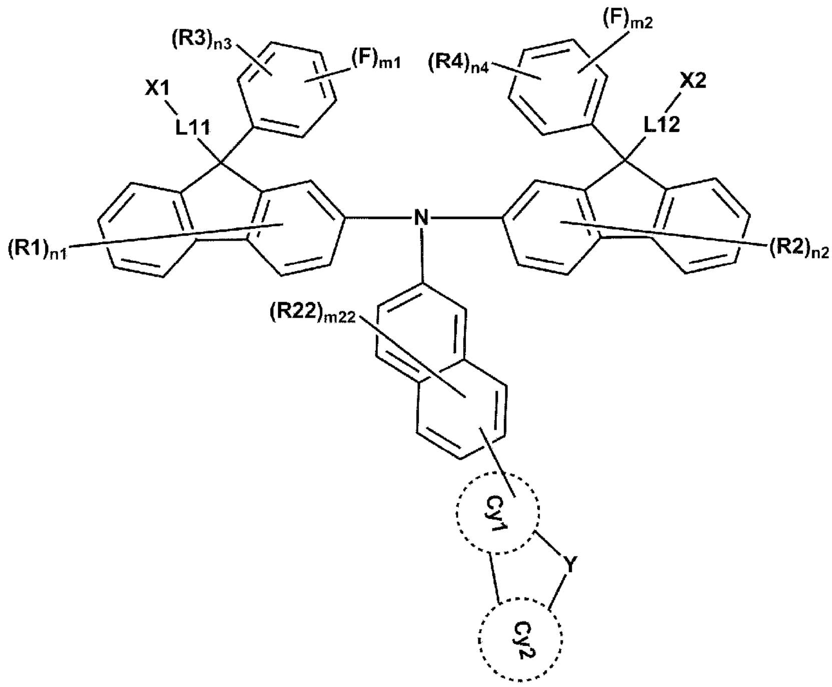

In Chemical Formulae 12 to 14,

the definitions of L11, L12, X1, X2, R1 to R4, Cy1, Cy2, Y, n1 to n4, m1 and m2 are the same as those in Chemical Formula 1,

R21 and R22 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group, and

m21 is an integer from 0 to 4, m22 is an integer from 0 to 6, and when m21 and m22 are each an integer of 2 or higher, two or more substituents in the parenthesis are the same as or different from each other.

According to an exemplary embodiment of the present invention, Y is O, S, CRaRb or SiRcRd.

According to an exemplary embodiment of the present invention, Ra, Rb, Rc and Rd are the same as or different from each other, and are each independently hydrogen; deuterium; a substituted or unsubstituted alkyl group; a substituted or unsubstituted aryl group; or a substituted or unsubstituted heteroaryl group, or are bonded to an adjacent group to form a substituted or unsubstituted ring.

According to another exemplary embodiment, Ra, Rb, Rc and Rd are the same as or different from each other, and are each independently hydrogen; deuterium; a substituted or unsubstituted alkyl group having 1 to 20 carbon atoms; a substituted or unsubstituted aryl group having 6 to 30 carbon atoms; or a substituted or unsubstituted heteroaryl group having 2 to 30 carbon atoms, or are bonded to an adjacent group to form a substituted or unsubstituted ring having 3 to 30 carbon atoms.

According to still another exemplary embodiment, Ra, Rb, Rc and Rd are the same as or different from each other, and are each independently a substituted or unsubstituted methyl group; or a substituted or unsubstituted phenyl group.

According to an exemplary embodiment of the present invention, Cy1 and Cy2 are the same as or different from each other, and are each independently a substituted or unsubstituted benzene ring; or a substituted or unsubstituted naphthalene ring.

In an exemplary embodiment of the present invention, Cy1 and Cy2 are the same as or different from each other, and are each independently any one of the following structures.

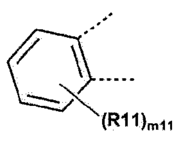

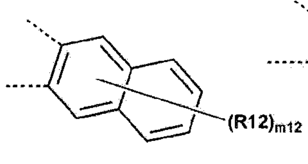

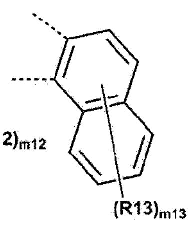

In the structures,

R11 to R13 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group,
m11 is an integer from 0 to 4, m12 and m13 are each an integer from 0 to 6, and when m11 to m13 are each an integer of 2 or higher, two or more substituents in the parenthesis are the same as or different from each other, and
in the structures, **------** denotes a position to be bonded.

According to an exemplary embodiment of the present invention, R11 to R13 are the same as or different from each other, and are each independently hydrogen; deuterium; a halogen group; a substituted or unsubstituted alkyl group having 1 to 20 carbon atoms; a substituted or unsubstituted alkoxy group having 1 to 20 carbon atoms; a substituted or unsubstituted aryl group having 6 to 30 carbon atoms; or a substituted or unsubstituted heteroaryl group having 2 to 30 carbon atoms.
In another exemplary embodiment, R11 to R13 are each hydrogen.
According to an exemplary embodiment of the present invention, m11 to m13 are each 0 or 1.
According to an exemplary embodiment of the present invention, R1 and R2 are the same as or different from each other, and are each independently deuterium; a halogen group; a substituted or unsubstituted alkyl group having 1 to 20 carbon atoms; a substituted or unsubstituted alkoxy group having 1 to 20 carbon atoms; a substituted or unsubstituted aryl group having 6 to 30 carbon atoms; or a substituted or unsubstituted heteroaryl group having 2 to 30 carbon atoms.
According to an exemplary embodiment of the present invention, R3 and R4 are the same as or different from each other, and are each independently deuterium; a substituted or unsubstituted alkyl group having 1 to 20 carbon atoms; a substituted or unsubstituted alkoxy group having 1 to 20 carbon atoms; a substituted or unsubstituted aryl group having 6 to 30 carbon atoms; or a substituted or unsubstituted heteroaryl group having 2 to 30 carbon atoms.
In yet another exemplary embodiment, R3 and R4 are the same as or different from each other, and are each independently an alkyl group having 1 to 20 carbon atoms; or an aryl group having 6 to 30 carbon atoms.
According to another exemplary embodiment, R3 and R4 are the same as or different from each other, and are each independently a substituted or unsubstituted methyl group; or a substituted or unsubstituted phenyl group.
According to still another exemplary embodiment, R3 and R4 are the same as or different from each other, and are each independently a methyl group; or a phenyl group.
According to an exemplary embodiment of the present invention, n1 and n2 are each 0 or 1.
In an exemplary embodiment of the present invention, R21 and R22 are the same as or different from each other, and are each independently hydrogen; deuterium; a halogen group; a substituted or unsubstituted alkyl group having 1 to 20 carbon atoms; a substituted or unsubstituted alkoxy group having 1 to 20 carbon atoms; a substituted or unsubstituted aryl group having 6 to 30 carbon atoms; or a substituted or unsubstituted heteroaryl group having 2 to 30 carbon atoms.
According to another exemplary embodiment, R21 and R22 are each hydrogen.
According to an exemplary embodiment of the present invention, Chemical Formula 1 may be represented by any one of the following compounds.

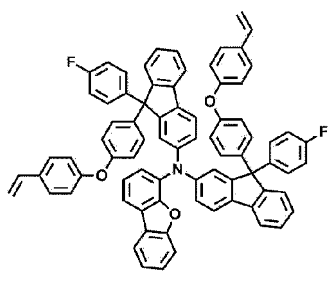
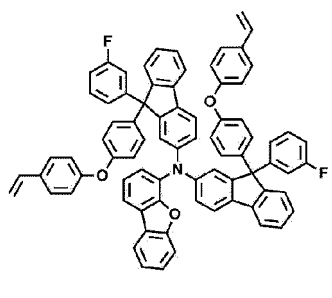
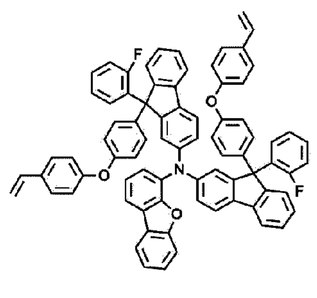

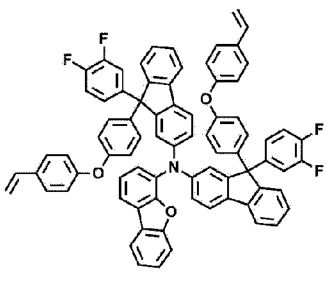
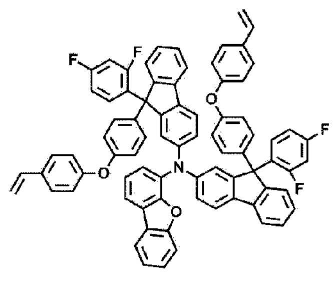
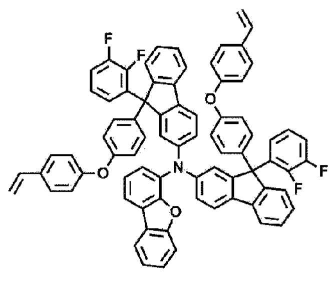

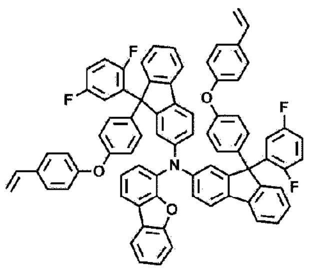
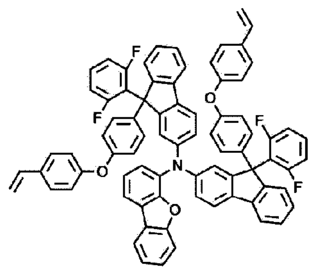
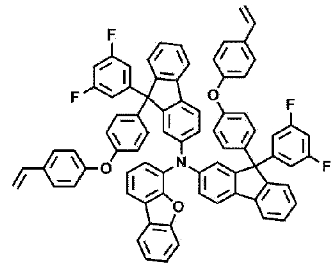

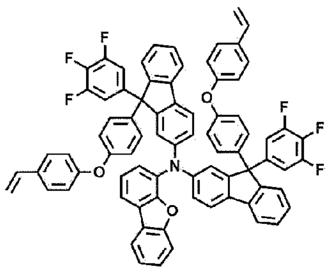
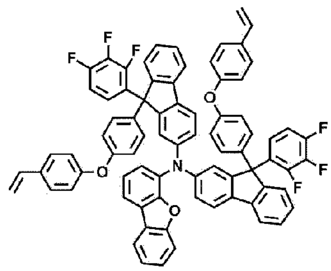
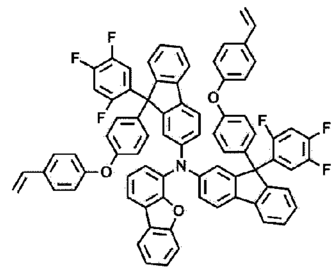

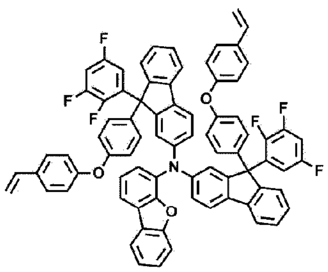
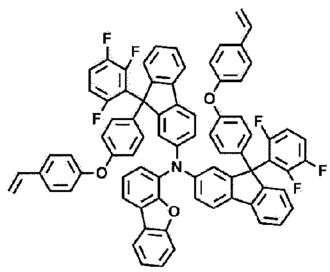
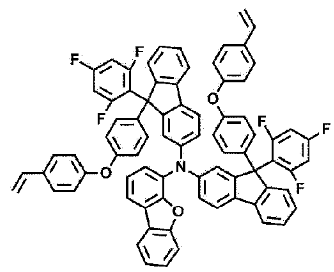

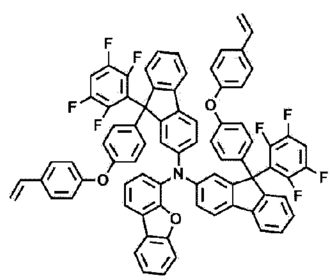
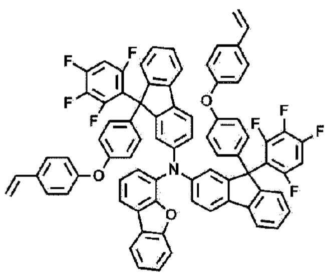
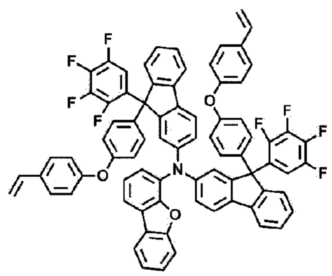

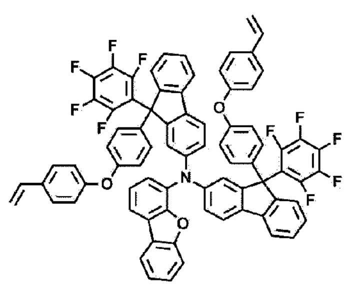
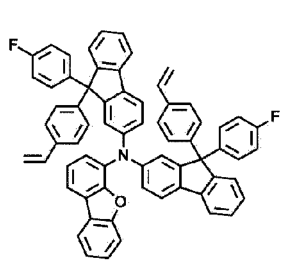
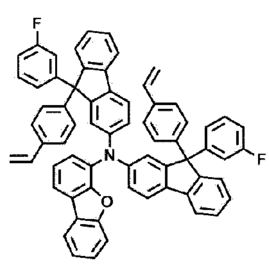

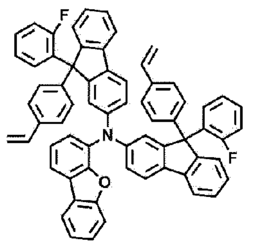
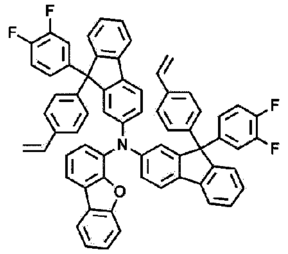
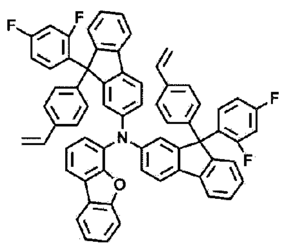

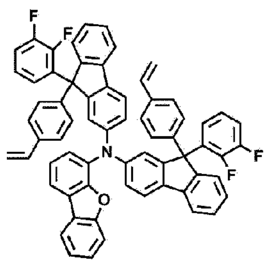
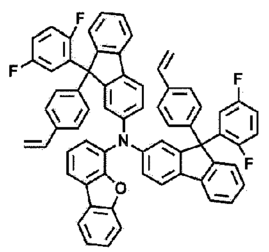
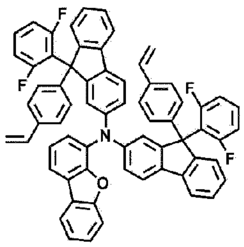

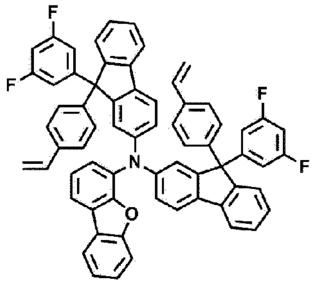
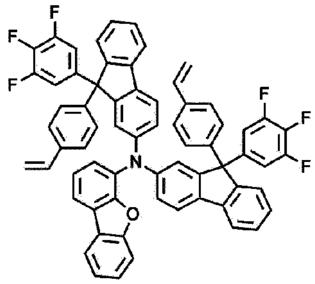
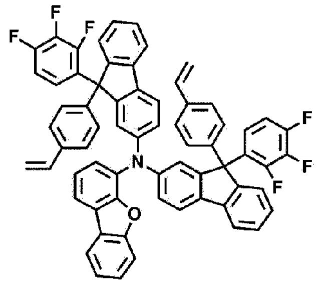

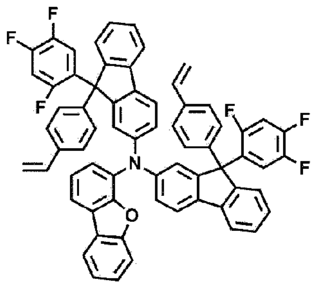
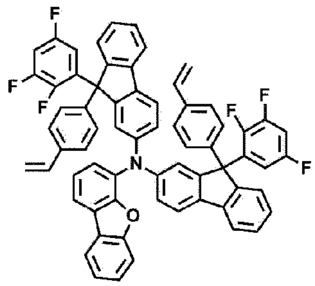
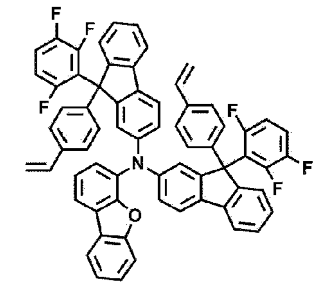

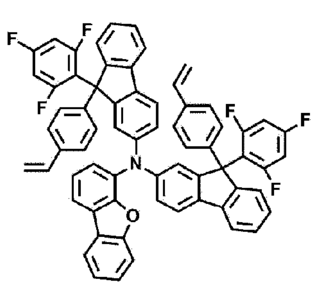
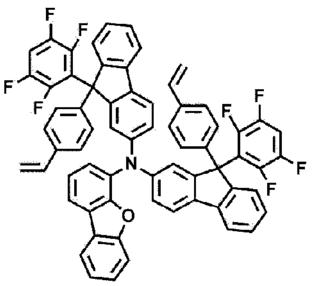
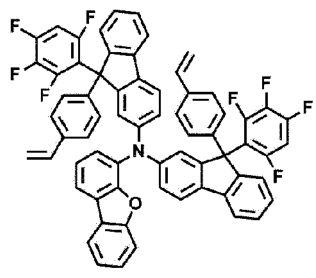

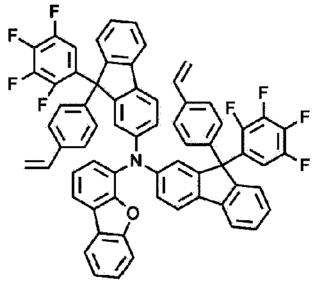

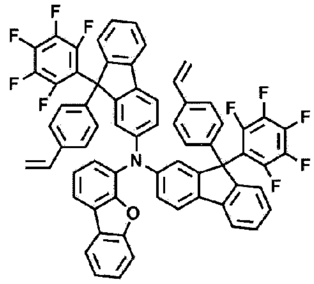

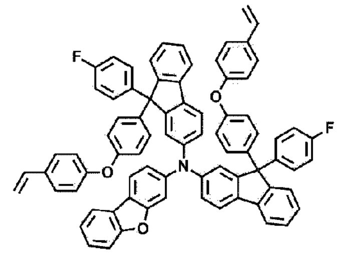

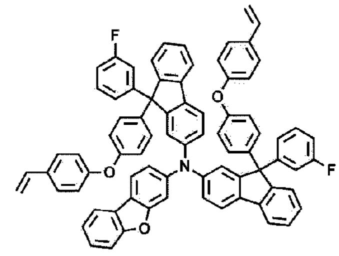

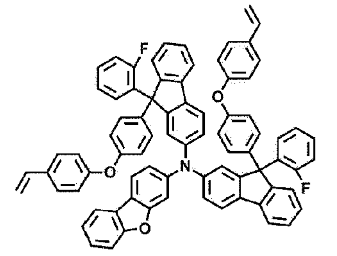

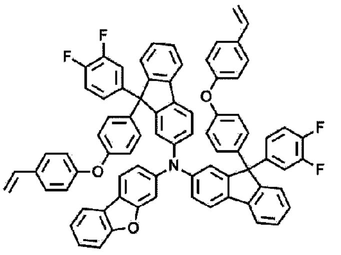

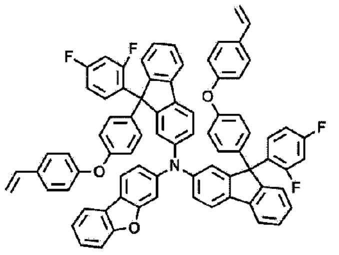

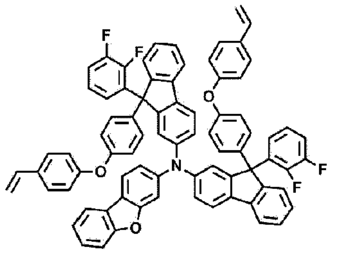

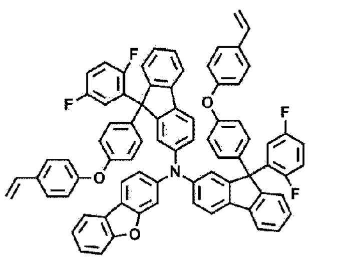

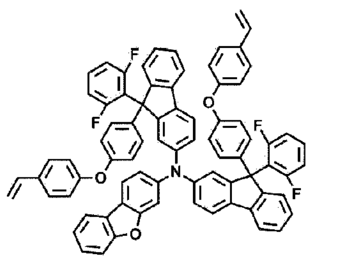

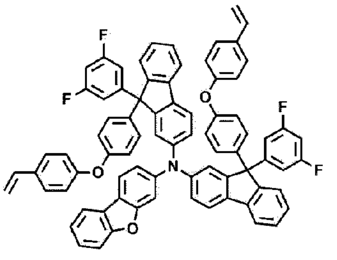

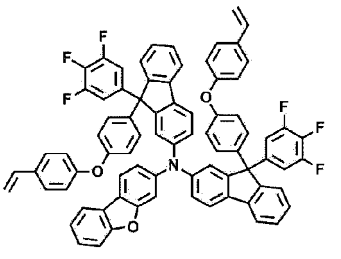

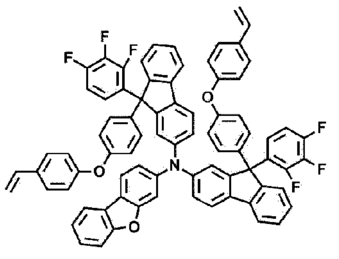

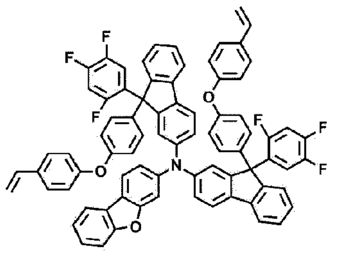

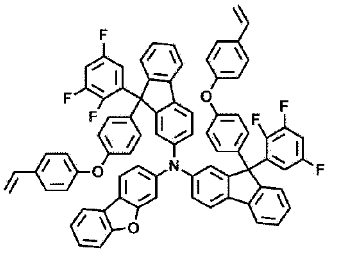

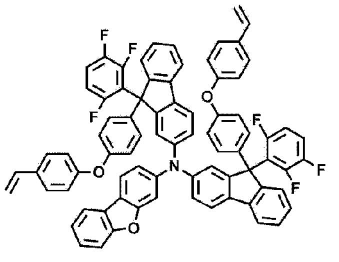

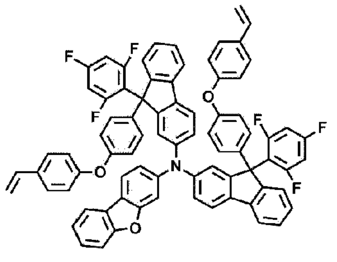

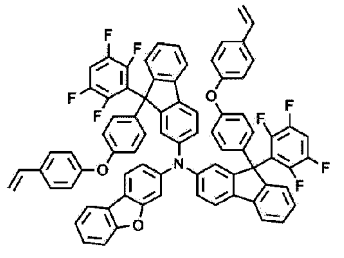
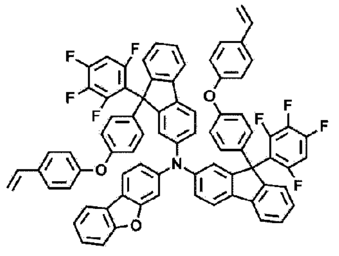
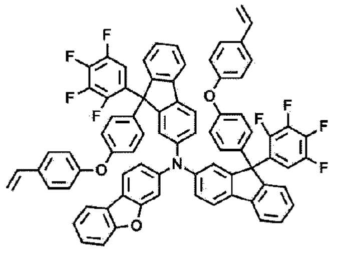

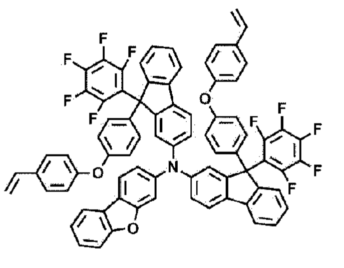
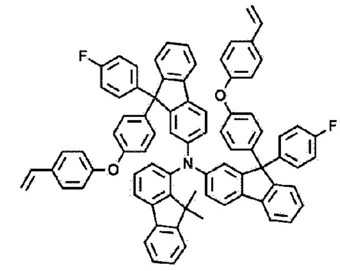
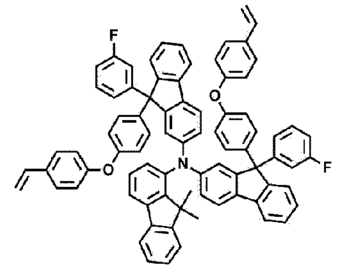

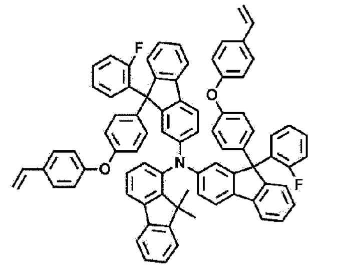
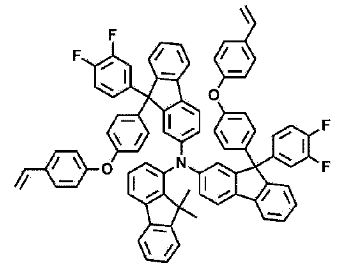
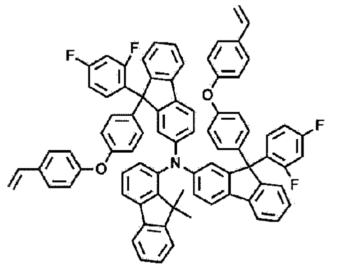

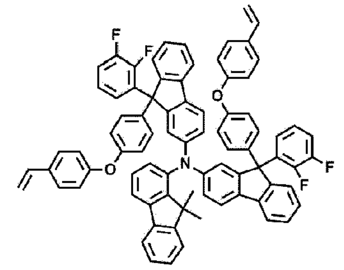
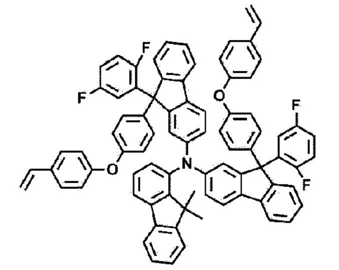
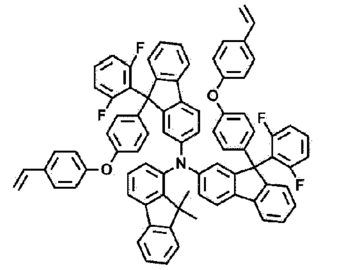

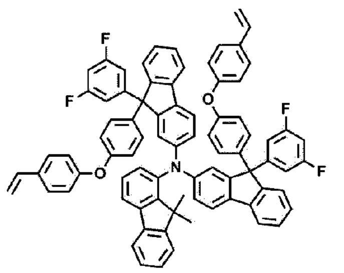
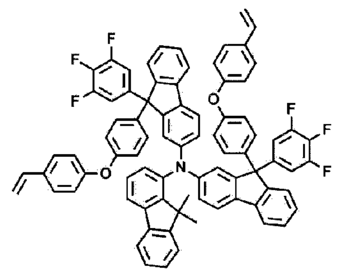
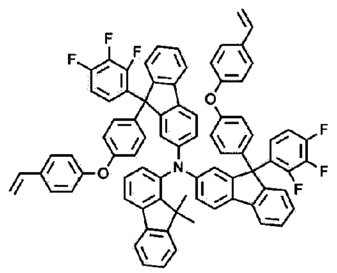

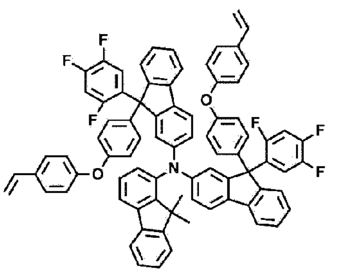
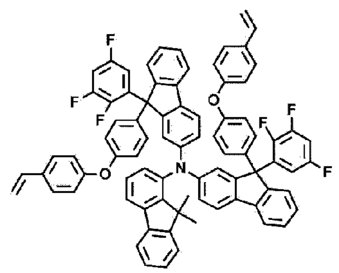
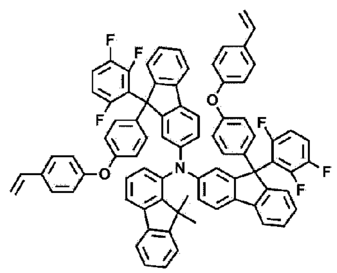

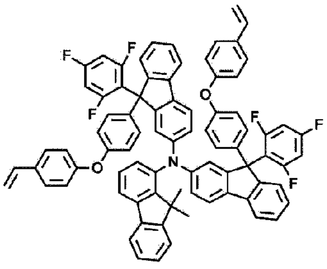
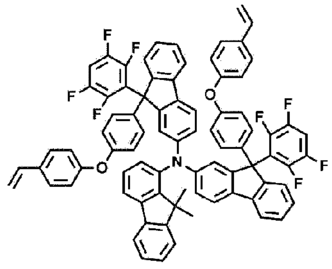
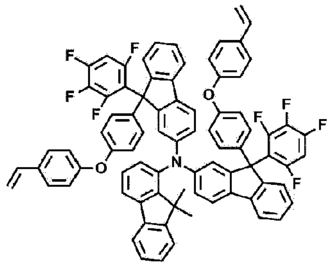

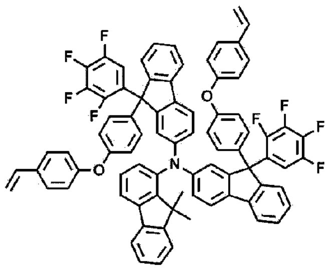
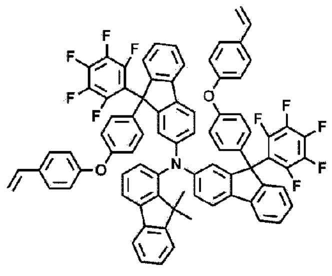

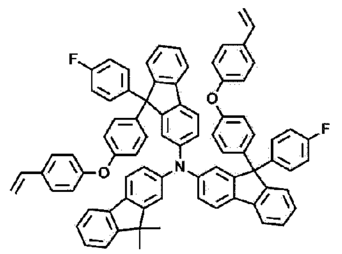
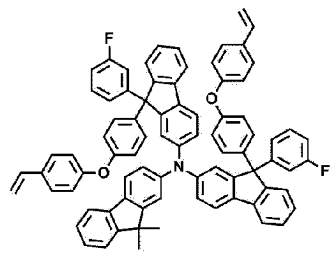
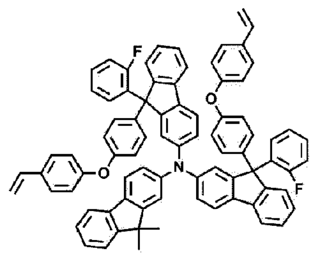

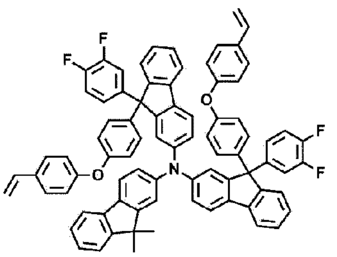
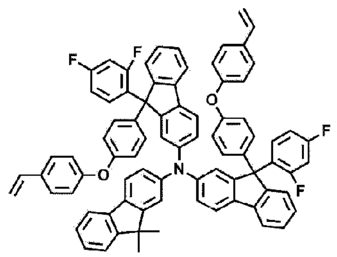
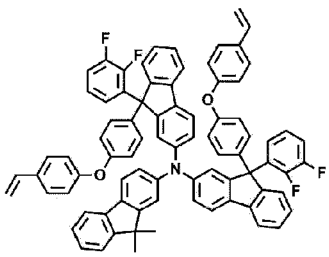

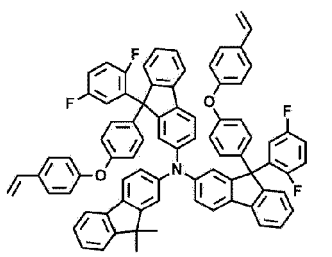
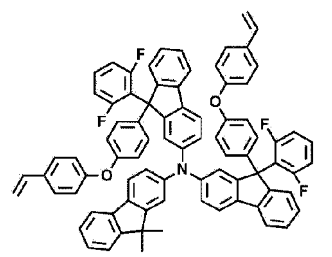
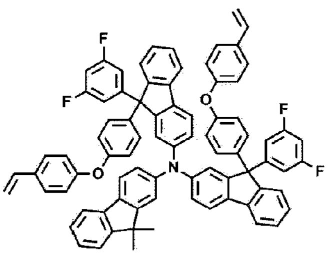

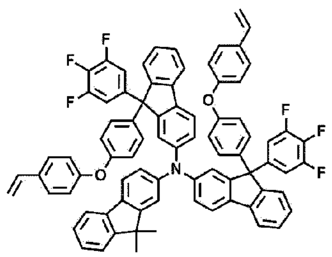
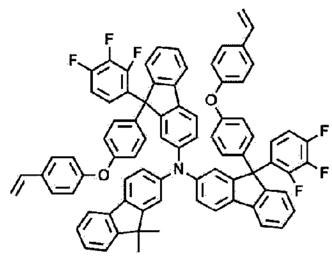
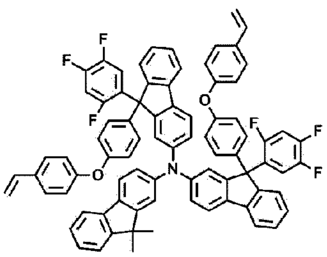

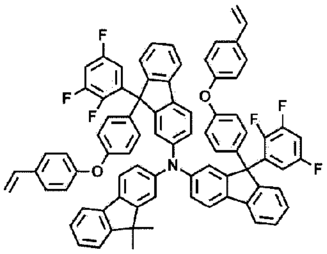
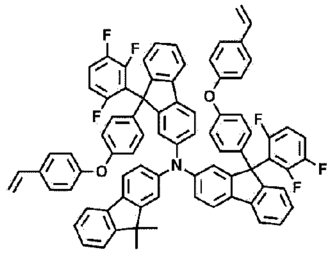
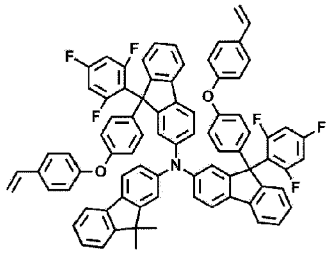

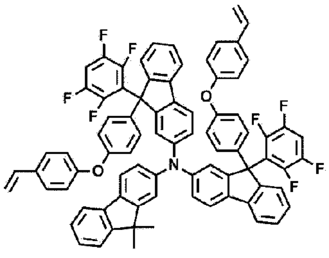
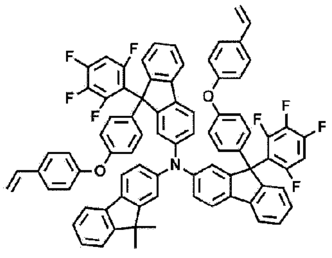
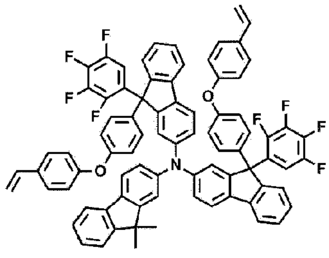

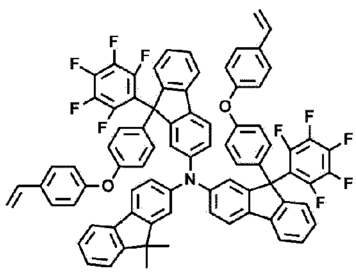
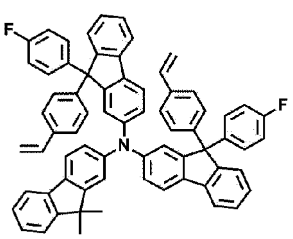
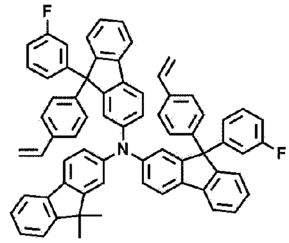

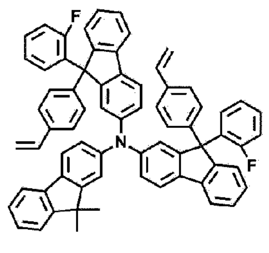
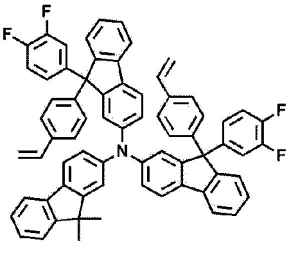
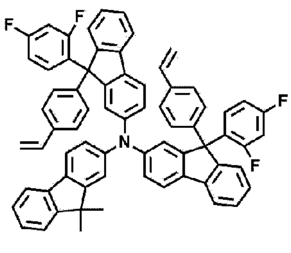

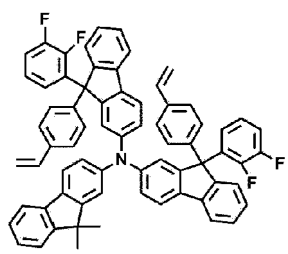
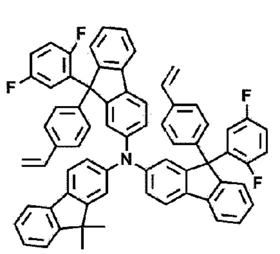
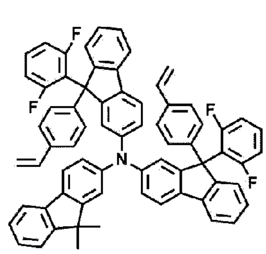

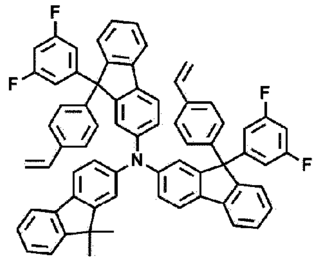
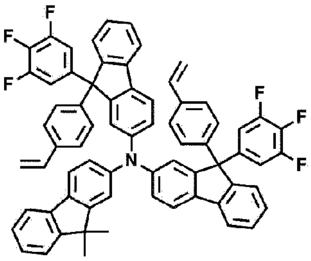
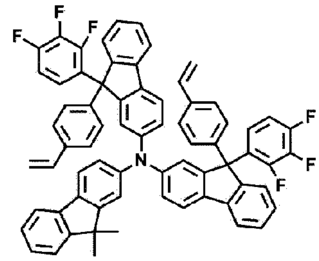

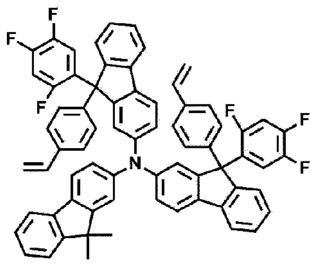
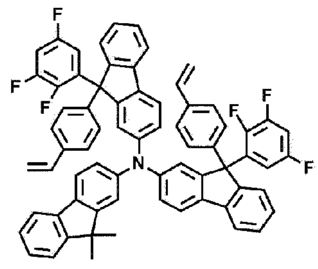
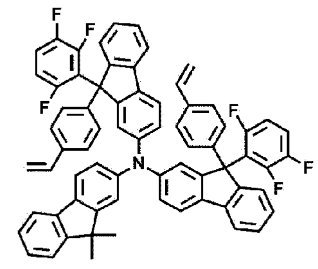

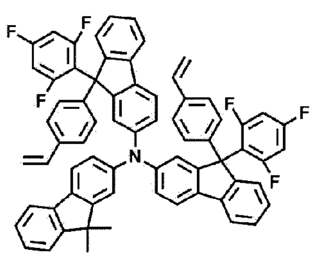
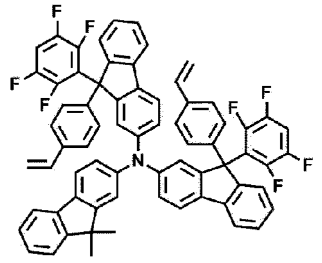
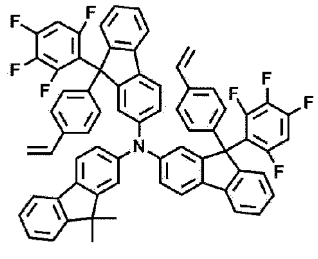

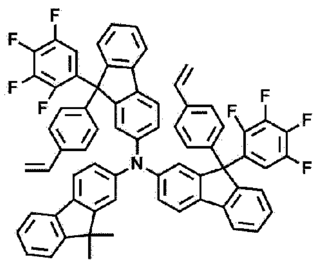
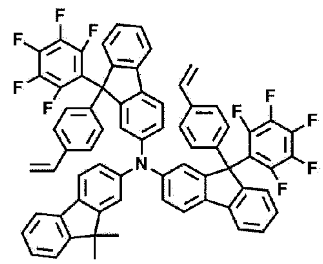

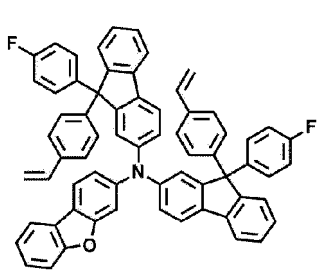
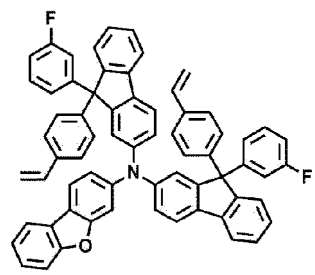
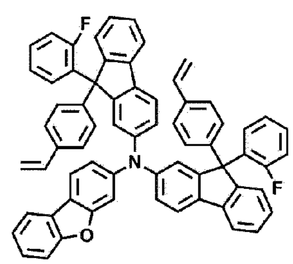

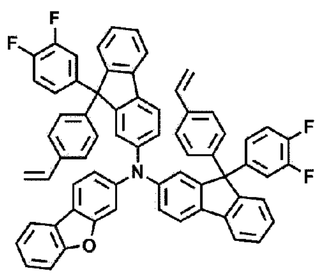
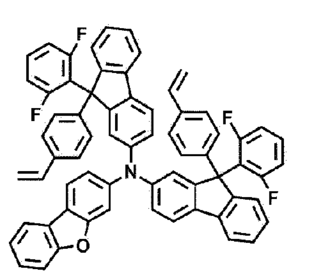
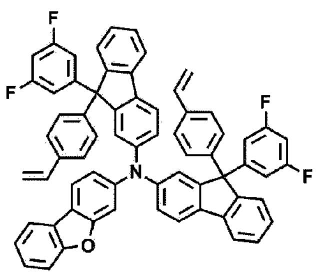

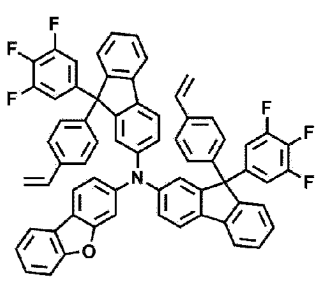
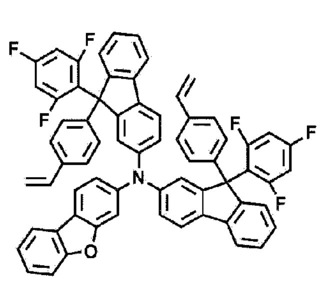
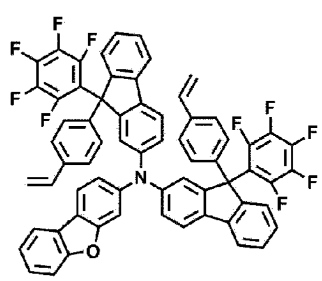

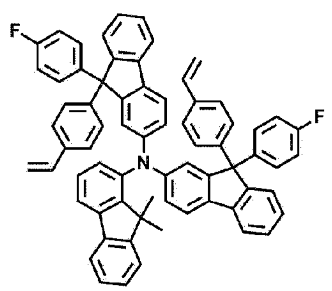
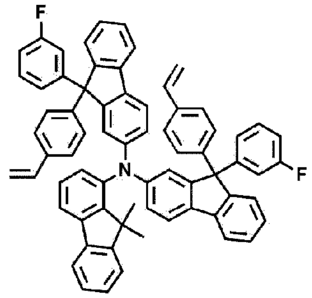
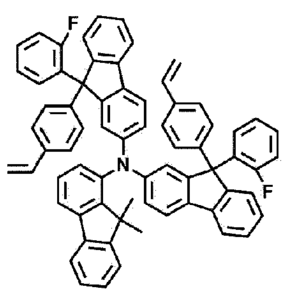

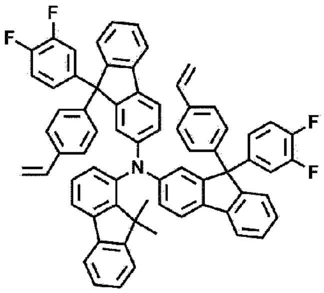
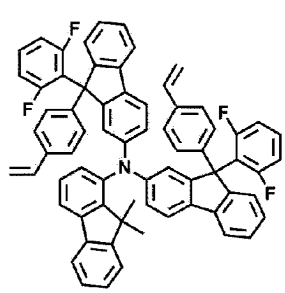
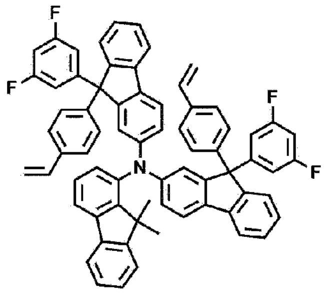

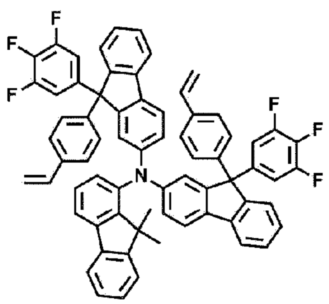
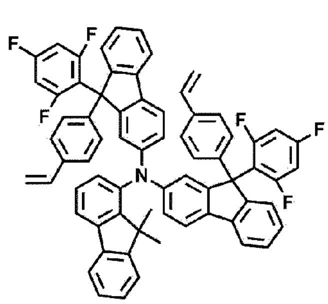
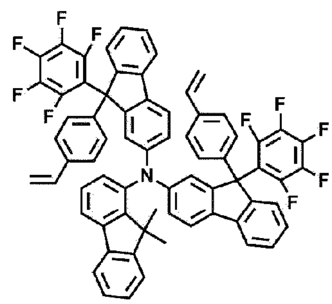

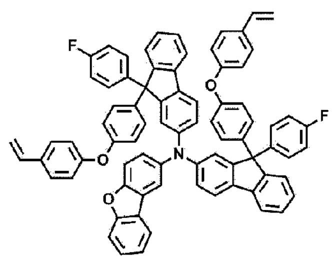
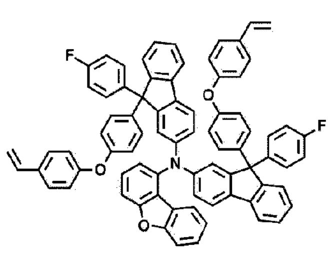
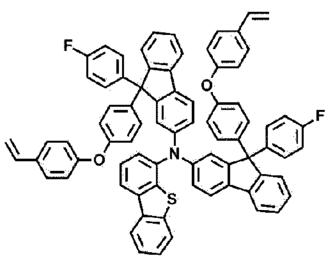

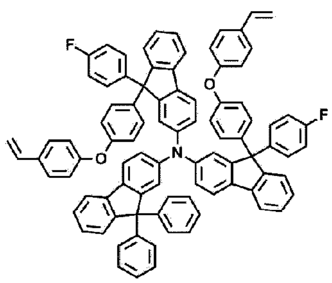
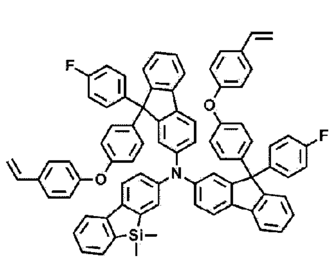
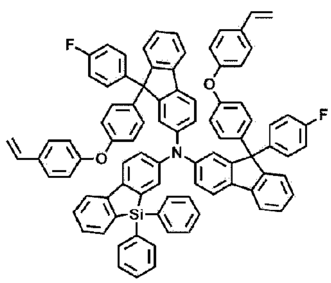

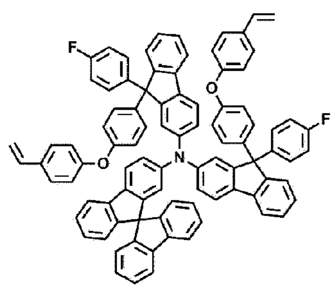
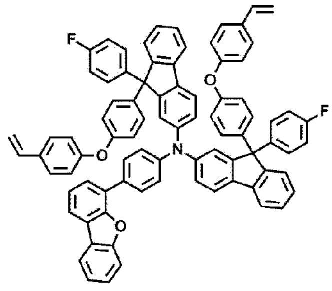
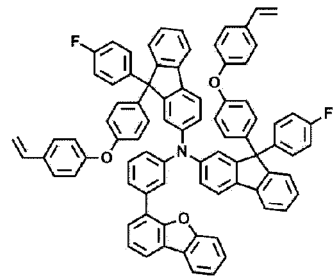

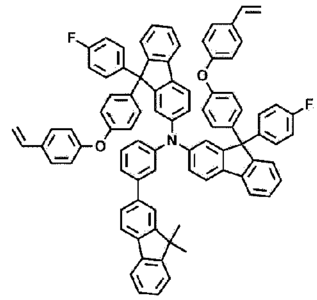
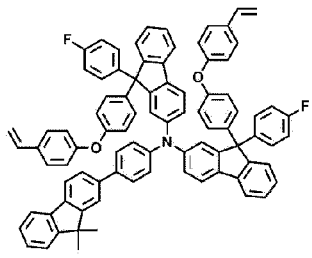
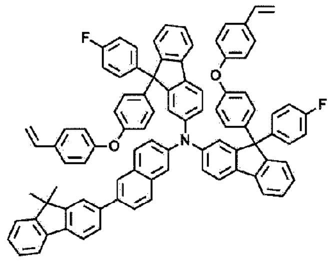

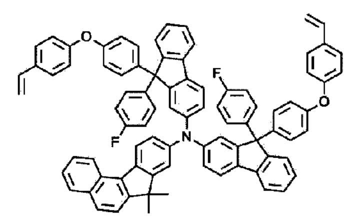
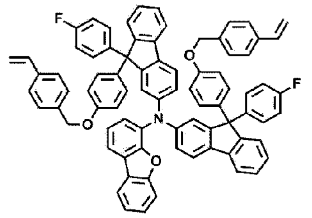
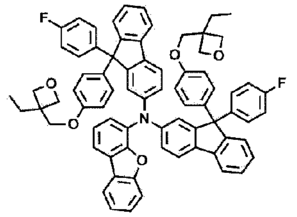

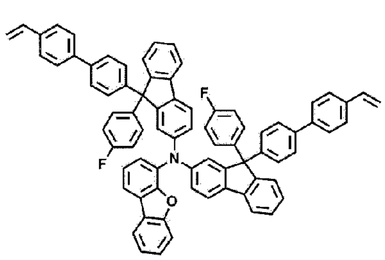
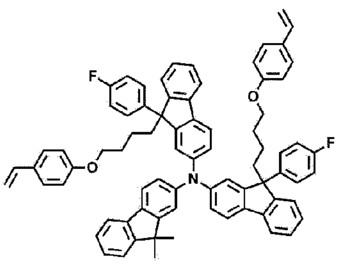
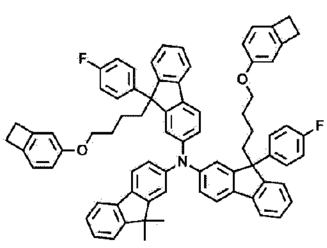

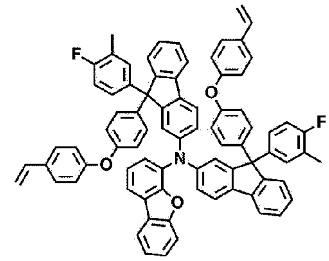
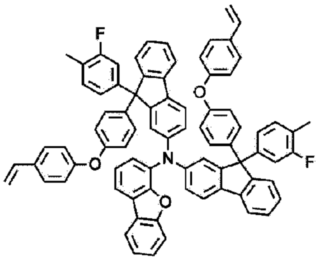
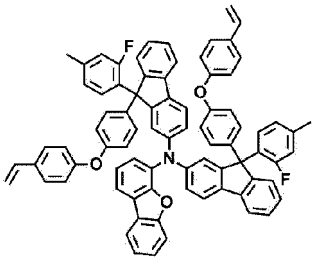

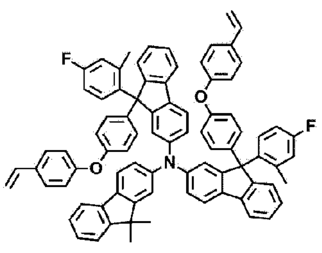
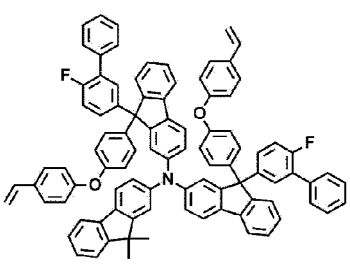
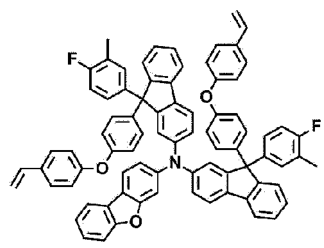

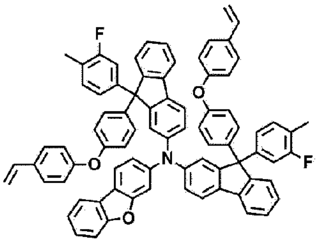
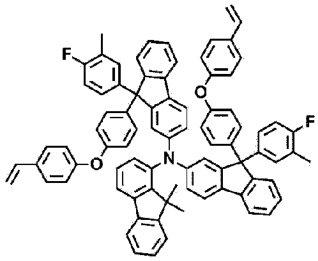
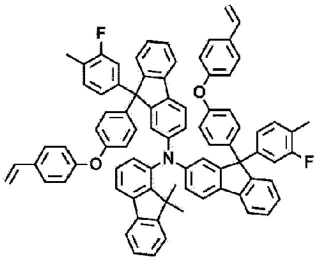
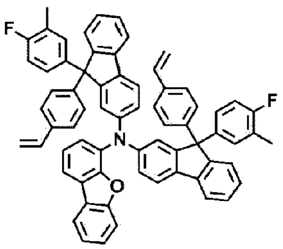
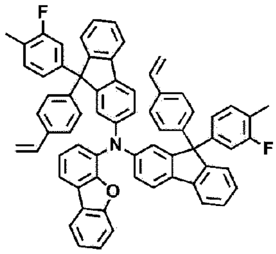
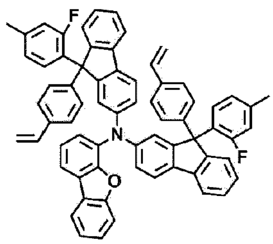
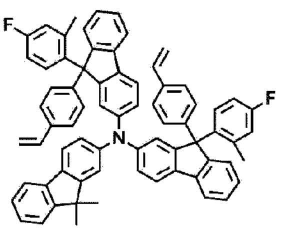
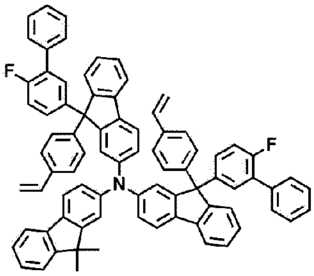
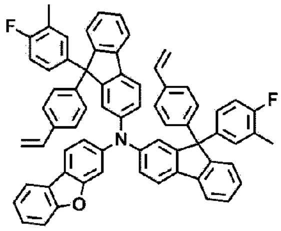
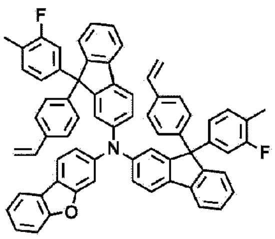
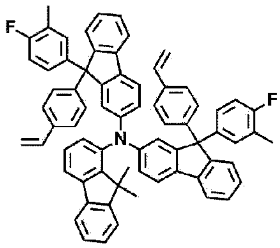
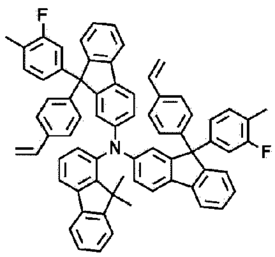

For the compound of Chemical Formula 1 according to an exemplary embodiment of the present invention, a core structure may be prepared according to the following Reaction Scheme 1. In addition, each substituent may be bonded by a method known in the art, and the type and position of the substituent or the number of substituents may be changed according to the technology known in the art.

<Reaction Scheme 1>

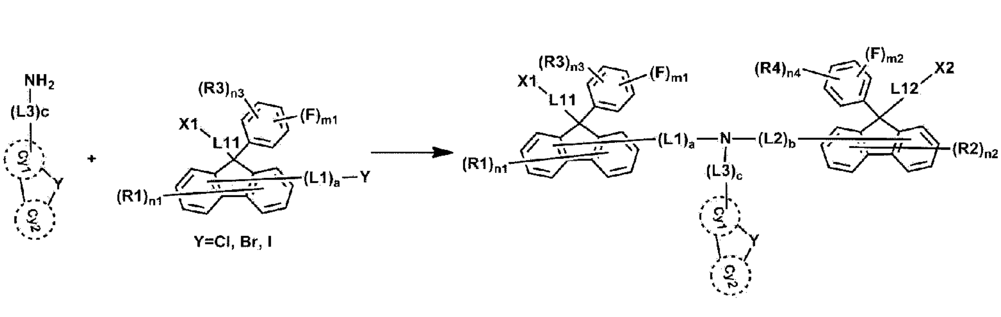


It is preferred that the reaction is carried out as an amine substitution reaction in the presence of a palladium

catalyst and a base, and a reactor for the amine substitution reaction can be changed as known in the art.

Hereinafter, a solvent represented by Chemical Formula A will be specifically described.

According to an exemplary embodiment of the present invention, Y1 to Y10 are the same as or different from each other, and are each independently hydrogen; deuterium; a hydroxyl group; an ether group; a carbonyl group; an ester group; a substituted or unsubstituted alkyl group; a substituted or unsubstituted cycloalkyl group; a substituted or unsubstituted cycloalkenyl group; a substituted or unsubstituted alkoxy group; a substituted or unsubstituted aryl group; or a substituted or unsubstituted amine group.

According to an exemplary embodiment of the present invention, Y1 to Y10 are the same as or different from each other, and are each independently hydrogen; deuterium; a hydroxyl group; a substituted or unsubstituted alkyl group; a substituted or unsubstituted cycloalkyl group; a substituted or unsubstituted cycloalkenyl group; a substituted or unsubstituted alkoxy group; a substituted or unsubstituted aryl group; or a substituted or unsubstituted amine group.

According to an exemplary embodiment of the present invention, Y1 to Y10 are the same as or different from each other, and are each independently hydrogen; deuterium; a substituted or unsubstituted alkyl group; or a substituted or unsubstituted alkoxy group.

According to an exemplary embodiment of the present invention, Y1 to Y10 are the same as or different from each other, and are each independently hydrogen; deuterium; a substituted or unsubstituted alkyl group having 1 to 10 carbon atoms; or a substituted or unsubstituted alkoxy group having 1 to 10 carbon atoms.

According to an exemplary embodiment of the present invention, Y1 to Y10 are the same as or different from each other, and are each independently hydrogen; deuterium; a substituted or unsubstituted alkyl group having 1 to 8 carbon atoms; or a substituted or unsubstituted alkoxy group having 1 to 8 carbon atoms.

According to an exemplary embodiment of the present invention, Y1 to Y10 are the same as or different from each other, and are each independently hydrogen; deuterium; a substituted or unsubstituted alkyl group having 1 to 6 carbon atoms; or a substituted or unsubstituted alkoxy group having 1 to 6 carbon atoms.

According to an exemplary embodiment of the present invention, Y1 to Y10 are the same as or different from each other, and are each independently hydrogen; deuterium; an alkyl group; or an alkoxy group.

According to an exemplary embodiment of the present invention, Y1 to Y10 are the same as or different from each other, and are each independently hydrogen; deuterium; a substituted or unsubstituted methyl group; a substituted or unsubstituted ethyl group; a substituted or unsubstituted propyl group; a substituted or unsubstituted butyl group; a substituted or unsubstituted pentyl group; a substituted or unsubstituted hexyl group; a substituted or unsubstituted isopropyl group; a substituted or unsubstituted isobutyl group; a substituted or unsubstituted tert-butyl group; a substituted or unsubstituted methoxy group; a substituted or unsubstituted ethoxy group; a substituted or unsubstituted propoxy group or a substituted or unsubstituted butoxy group.

According to an exemplary embodiment of the present invention, Y1 to Y10 are the same as or different from each other, and are each independently hydrogen; deuterium; a methyl group; an ethyl group; a propyl group; a butyl group; a pentyl group; a hexyl group; an isopropyl group; an isobutyl group; a tert-butyl group; a methoxy group; an ethoxy group; a propoxy group; or a butoxy group.

According to an exemplary embodiment of the present invention, Y1 to Y10 are the same as or different from each other, and are each independently hydrogen; deuterium; an ethyl group; a propyl group; a butyl group; a pentyl group; an isopropyl group; or a methoxy group.

In an exemplary embodiment of the present invention, the solvent represented by Chemical Formula A is any one of the following structures.

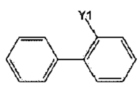 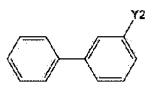 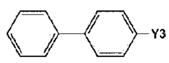 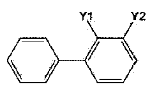 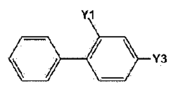

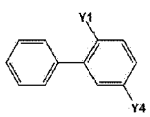 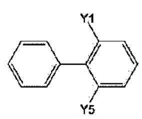 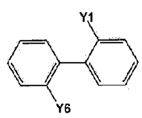 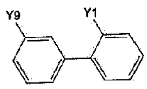 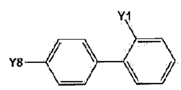

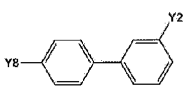 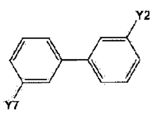 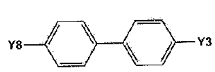 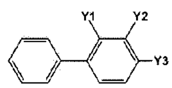 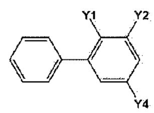

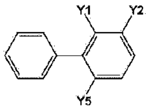 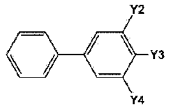 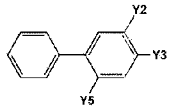 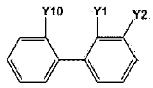 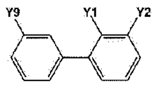

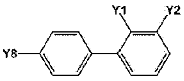 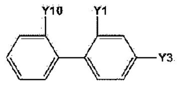 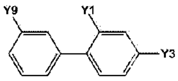 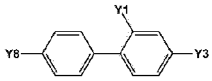 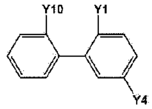

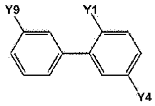 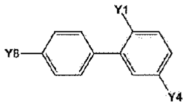 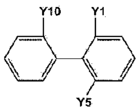 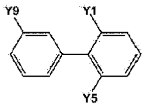 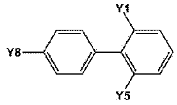

In the structures, Y1 to Y10 are the same as or different from each other, and are each independently a substituted or unsubstituted alkyl group; or a substituted or unsubstituted alkoxy group.

According to a preferred exemplary embodiment of the present invention, the solvent represented by Chemical Formula A is any one or a mixed solution of two or more selected from the group consisting of the following compounds.

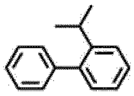 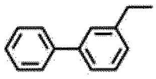 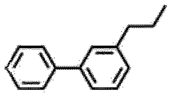 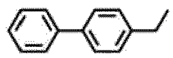

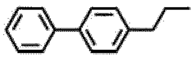 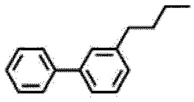 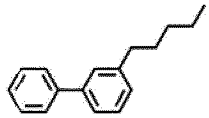 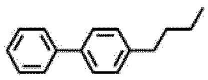

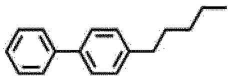 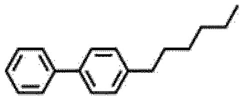 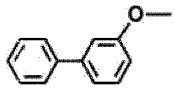 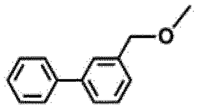

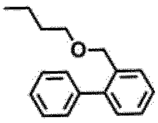 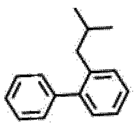 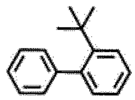

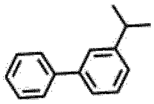 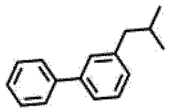 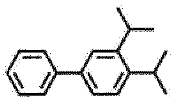 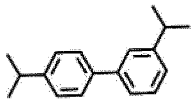

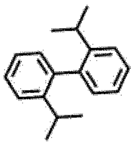 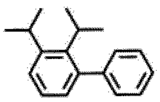 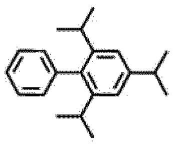 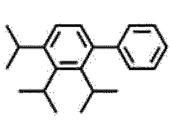

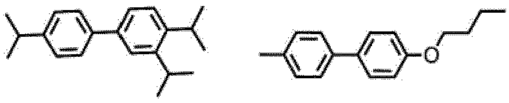

According to a preferred exemplary embodiment of the present invention, the solvent represented by Chemical Formula A is any one selected from the group consisting of the compounds.

According to an exemplary embodiment of the present invention, the solvent represented by Chemical Formula A is any one or a mixed solution of two or more selected from the group consisting of 3-isopropylbiphenyl, 3-methoxybiphenyl, 3-ethylbiphenyl, 4-butylbiphenyl and 4-pentylbiphenyl.

According to an exemplary embodiment of the present invention, the solvent represented by Chemical Formula A has a surface tension of 33 mN/m to 37 mN/m; or 33.5 mN/m to 36.5 mN/m.

As the surface tension of the solvent represented by Chemical Formula A according to an exemplary embodiment of the present invention is limited, the range of surface tension between materials other than the solvent represented by Chemical Formula A may be easily adjusted. Specifically, materials (for example, an additional solvent, other organic solvents, and the like) included in the ink composition used in the solution process generally need to be composed of materials, such that the ink composition has a surface tension in a range of 25 mN/m to 45 mN/m, and further, it is preferred that the difference in surface tension between materials is small. A surface tension in a range of 33 mN/m to 37 mN/m is close to the median value of the preferred surface tension range, and accordingly, when the solvent represented by Chemical Formula A has a surface tension in a range of 33 mN/m to 37 mN/m, the difference in surface tension between materials other than the solvent represented by Chemical Formula A may be adjusted so as to be small. When the difference in surface tension between the materials other than the solvent represented by Chemical Formula A is small, the organic light emitting device manufactured by the solution process has an advantage in that the flatness of the organic material layer and the thickness uniformity of the pixel are excellent.

According to an exemplary embodiment of the present invention, the solvent represented by Chemical Formula A has a boiling point of 220°C to 350°C; 250°C to 330°C; or 265°C to 315°C.

As the solvent represented by Chemical Formula A having a boiling point within the above range is included, it is possible to expect that when an organic material layer of an organic light emitting device is manufactured through a solution process, the flatness of the organic material layer and/or the thickness uniformity of the pixel are excellent. Specifically, since the solvent represented by Chemical Formula A has the lowest vapor pressure among the constituent solvents, and thus, is not removed in the initial drying step, there is an advantage in that it is possible to adjust the difference in surface tension between materials remaining even after the initial drying step. Consequently, the flatness of the organic material layer and/or the thickness uniformity of the pixel of the organic light emitting device are excellent.

The ink composition according to an exemplary embodiment of the present invention further includes an ionic compound including an anionic group represented by the following Chemical Formula 2.

Hereinafter, an ionic compound including an anionic group represented by the following Chemical Formula 2 will be specifically described.

[Chemical Formula 2]

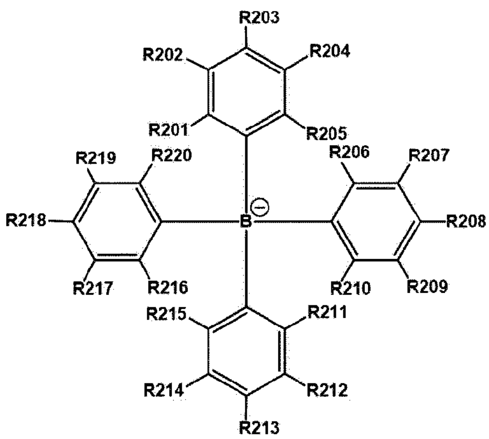

In Chemical Formula 2,

at least one of R201 to R220 is F; a cyano group; or a substituted or unsubstituted fluoroalkyl group,
at least one of the other R201 to R220 is a curable group,
the remaining R201 to R220 are the same as or different from each other, and are each independently hydrogen; deuterium; a nitro group; -C(O)R220'; -OR221; - SR222; $-SO_3R223$; -COOR224; -OC(O)R225; -C(O)NR226R227; a substituted or unsubstituted alkyl group; a substituted or unsubstituted fluoroalkyl group; a substituted or unsubstituted alkenyl group; a substituted or unsubstituted alkynyl group; a substituted or unsubstituted amine group; a substituted or unsubstituted aryl group; or a substituted or unsubstituted heterocyclic group, and

R220' and R221 to R227 are the same as or different from each other, and are each independently hydrogen; deuterium; or a substituted or unsubstituted alkyl group.

According to an exemplary embodiment of the present invention, the above-described ink composition further includes an ionic compound including the anionic group represented by Chemical Formula 2. Hereinafter, the compound of Chemical Formula 2 may be expressed as an anionic group compound.

Unlike an ionic compound (hereinafter, referred to as an anionic group compound) including the anionic group represented by Chemical Formula 2 into which a curable group is introduced according to an exemplary embodiment of the present invention, the ionic compound is not cured when the curable group is not introduced, so the properties of the device are reduced due to migration of the ionic compound between electrodes or layers. In addition, when the number of curable groups increases, the curing rate of the ionic compound or the composition including the same increases, and the film retention rate is improved.

In an exemplary embodiment of the present invention, the number of curable groups of the anionic group compound represented by Chemical Formula 2 is 1.

In an exemplary embodiment of the present invention, the number of curable groups of the anionic group compound represented by Chemical Formula 2 is 2.

In an exemplary embodiment of the present invention, the number of curable groups of the anionic group compound represented by Chemical Formula 2 is 4.

In an exemplary embodiment of the present invention, the number of F's; cyano groups; or substituted or unsubstituted fluoroalkyl groups of the anionic group compound represented by Chemical Formula 2 is 16 to 19.

In an exemplary embodiment of the present invention, based on 100 parts by weight of the anionic group compound, the part by weight of F in the anionic group compound is 15 parts by weight to 50 parts by weight.

In an exemplary embodiment of the present invention, based on 100 parts by weight of the anionic group compound, the part by weight of F in the anionic group compound is 10 parts by weight to 45 parts by weight.

In an exemplary embodiment of the present invention, based on 100 parts by weight of the anionic group compound, the number of F's in the anionic group compound is 8 to 20.

As described above, when the ratio of F, the cyano group, or the fluoroalkyl group in the molecule is increased, the film retention rate during heat treatment is improved.

According to an exemplary embodiment of the present invention, the ionic compound may be used in the hole injection layer of the organic light emitting device, and may be used as a dopant when used in the hole injection layer. In this case, when the content of F of the ionic compound is increased, the force of attracting electrons from another compound (host compound) is increased, and holes are more proficiently produced in the host, so that the performance in the hole injection layer is improved.

According to an exemplary embodiment of the present invention, the content of F may be analyzed using COSA AQF-100 combustion furnace coupled to a Dionex ICS 2000 ion-chromatograph, or may be confirmed through 19F NMR which is a method generally used in the F analysis.

In an exemplary embodiment of the present invention, at least one benzene ring of a benzene ring including R201 to R205, a benzene ring including R206 to R210, a benzene ring including R211 to R215, and a benzene ring including R216 to R220 in Chemical Formula 2 is selected from the following structural formulae.

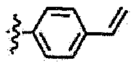 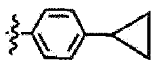 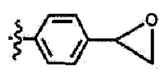 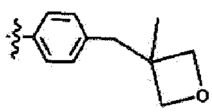

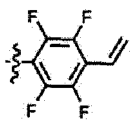 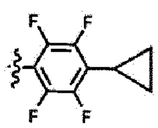 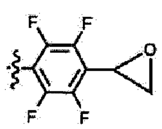 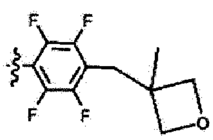

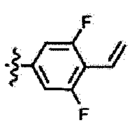 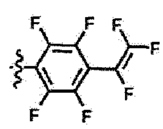 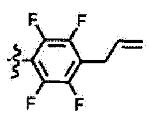 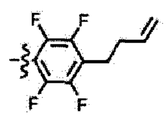

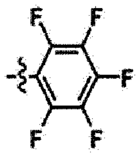
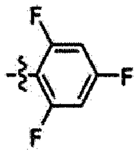
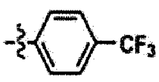
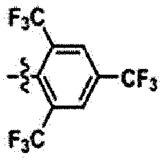
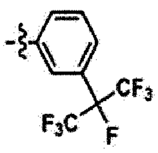
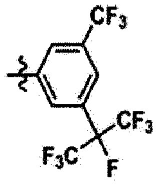

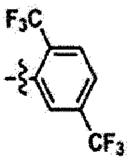
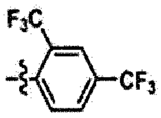
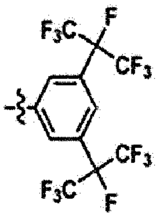
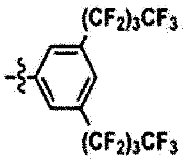

According to an exemplary embodiment of the present invention, Chemical Formula 2 is any one selected from the group consisting of the following compounds.

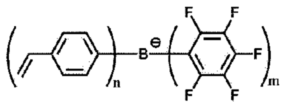
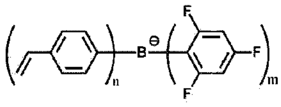
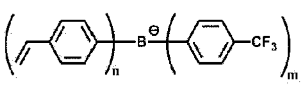

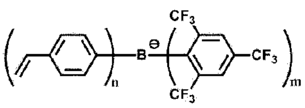
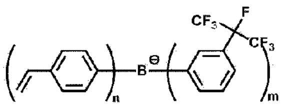
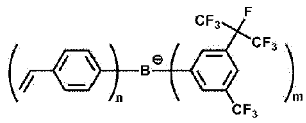

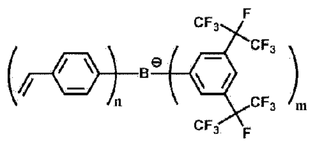
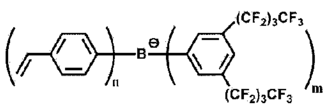
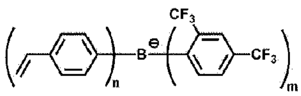

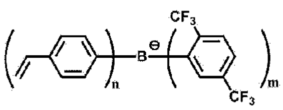
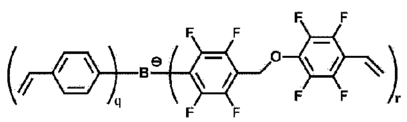

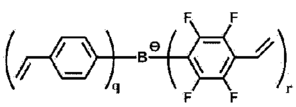
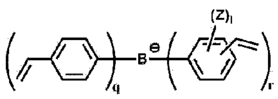
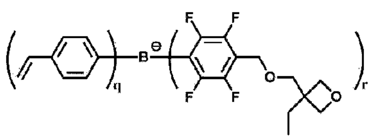

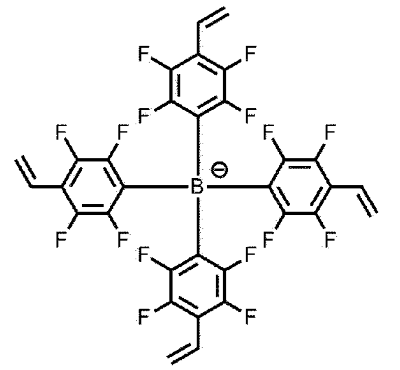

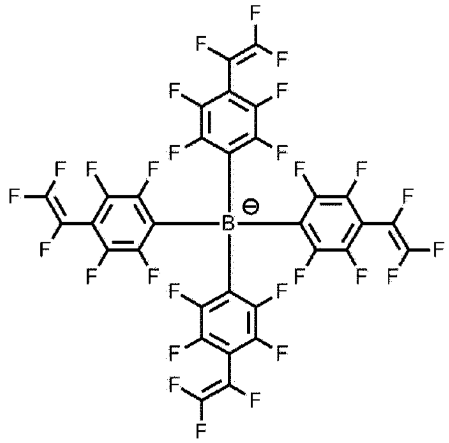

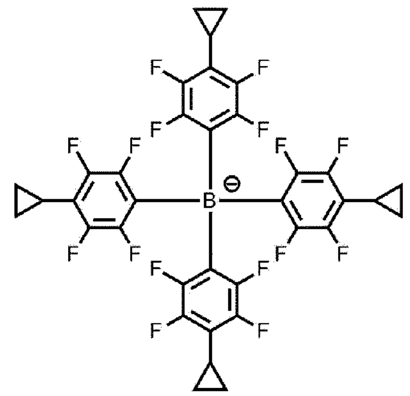

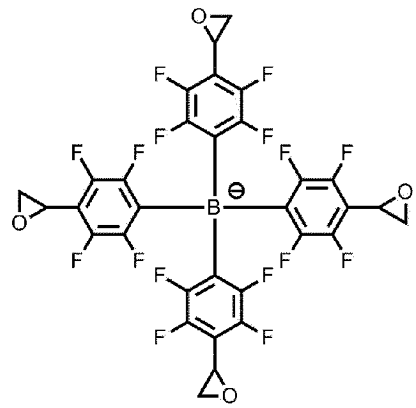

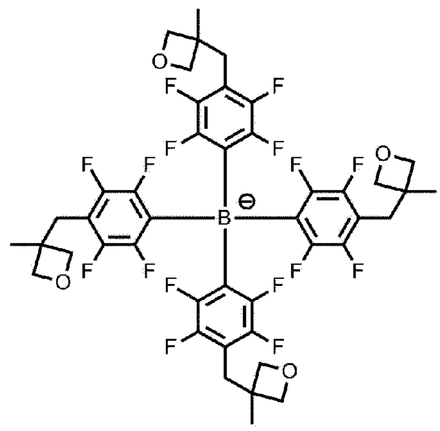

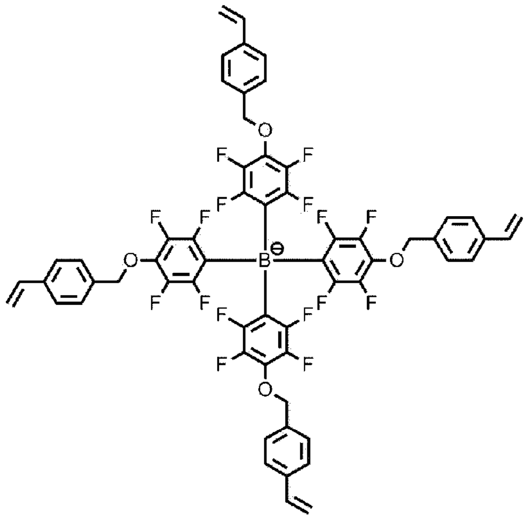

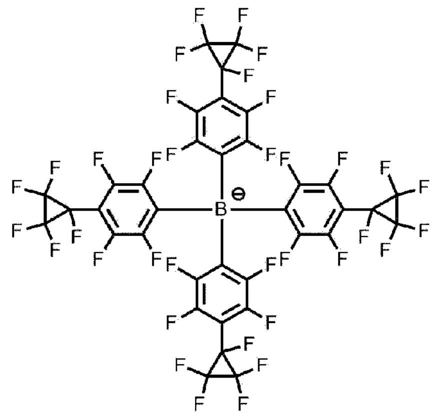

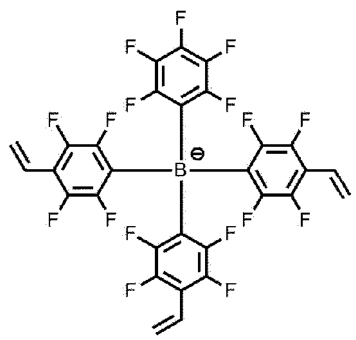

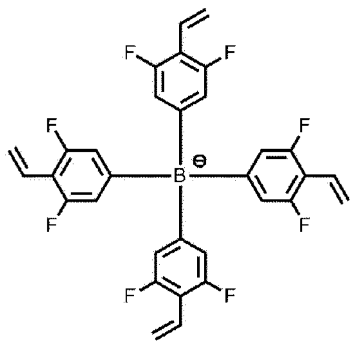

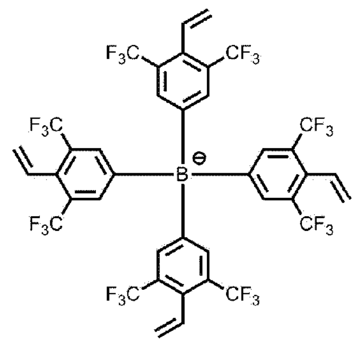

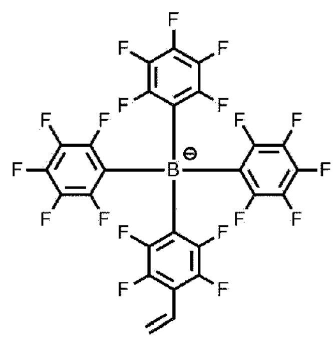

In the compounds,

n is an integer from 1 to 3, m is an integer from 1 to 3, and m+n=4,

q is an integer from 0 to 3, r is an integer from 1 to 4, and q+r=4,

Z is deuterium; a halogen group; a nitro group; a cyano group; an amino group; -C(O)R220'; -OR221; -SR222; -$SO_3$R223; -COOR224; -OC(O)R225; -C(O)NR226R227; a substituted or unsubstituted alkyl group; a substituted or unsubstituted alkenyl group; a substituted or unsubstituted alkynyl group; a substituted or unsubstituted amine group; a substituted or unsubstituted aryl group; or a substituted or unsubstituted heterocyclic group,

l is an integer from 1 to 4, and when l is 2 or higher, Z's are the same as or different from each other, and

R220' and R221 to R227 are the same as or different from each other, and are each independently hydrogen; deuterium; or a substituted or unsubstituted alkyl group.

Hereinafter, an ionic compound including a cationic group will be specifically described.

According to an exemplary embodiment of the present invention, the above-described ink composition further includes a cationic group. Specifically, the above-described ink composition further includes an ionic compound including

the anionic group represented by the above-described Chemical Formula 2 and the cationic group. As another example, the above-described ink composition includes an ionic compound, and the ionic compound includes the anionic group represented by the above-described Chemical Formula 2 and the cationic group.

According to an exemplary embodiment of the present invention, the cationic group is a monovalent cationic group; an onium compound; or any one selected from the following structural formulae.

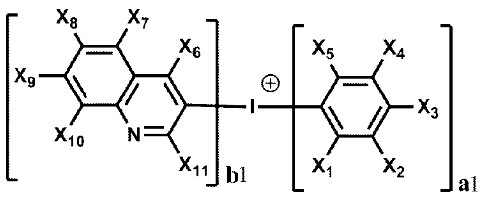
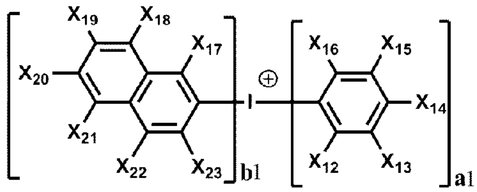
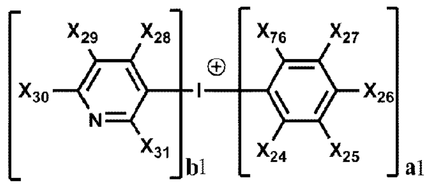
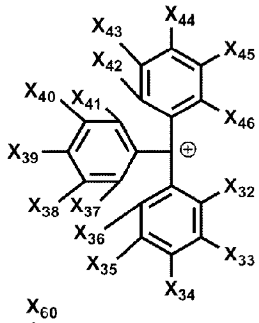
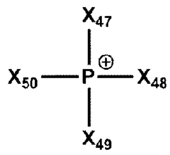
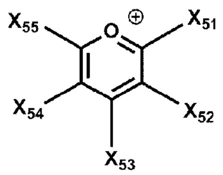
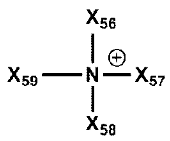
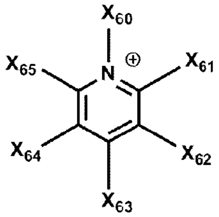
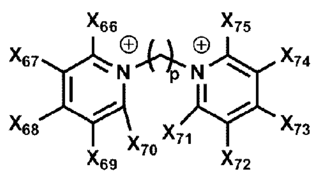

In the structural formulae,

$X_1$ to $X_{76}$ are the same as or different from each other, and are each independently hydrogen; deuterium; a cyano group; a nitro group; a halogen group; -COOR224; a substituted or unsubstituted alkyl group; a substituted or unsubstituted alkoxy group; a substituted or unsubstituted cycloalkyl group; a substituted or unsubstituted fluoroalkyl group; a substituted or unsubstituted aryl group; or a curable group,
R224 is hydrogen; deuterium; or a substituted or unsubstituted alkyl group,
p is an integer from 0 to 10, and
a1 is 1 or 2, b1 is 0 or 1, and a1+b1=2.

According to an exemplary embodiment of the present invention, the monovalent cationic group may be an alkali metal cation, and examples of the alkali metal cation include $Na^+$, $Li^+$, $K^+$, and the like, but are not limited thereto.

According to an exemplary embodiment of the present invention, the cationic group is an onium compound; or any one selected from the above-described structural formulae.

According to an exemplary embodiment of the present invention, the cationic group is represented by any one of the following Chemical Formulae 310 to 315.

[Chemical Formula 310]

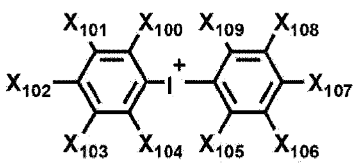

[Chemical Formula 311]

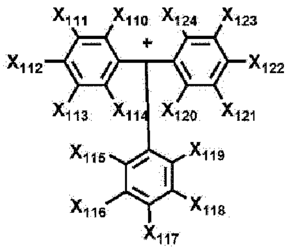

[Chemical Formula 312]

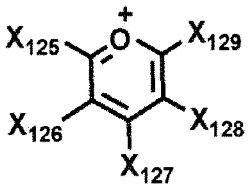

[Chemical Formula 313]

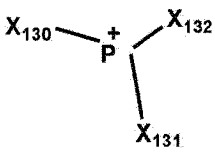

[Chemical Formula 314]

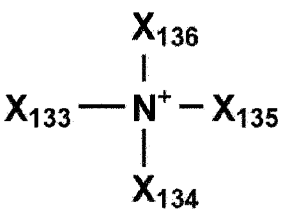

[Chemical Formula 315]

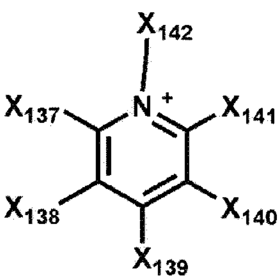

In Chemical Formulae 310 to 315,

$X_{100}$ to $X_{142}$ are the same as or different from each other, and are each independently hydrogen; deuterium; a cyano group; a nitro group; a halogen group; -COOR224; a substituted or unsubstituted alkyl group; a substituted or

unsubstituted alkoxy group; a substituted or unsubstituted cycloalkyl group; a substituted or unsubstituted fluoroalkyl group; a substituted or unsubstituted aryl group; or a curable group, and
R224 is a substituted or unsubstituted alkyl group.

According to an exemplary embodiment of the present invention, the cationic group is any one selected from the following structural formulae.

| | | | |
|---|---|---|---|
| 1-1 | 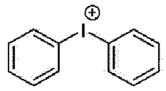 | 1-6 | 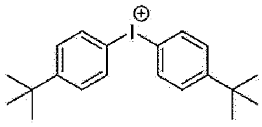 |
| 1-2 | 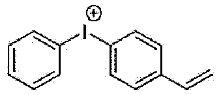 | 1-7 | 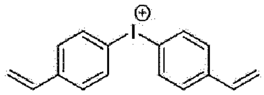 |
| 1-3 | 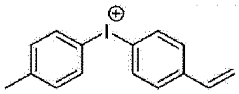 | 1-8 | 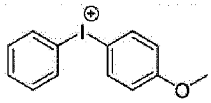 |
| 1-4 | 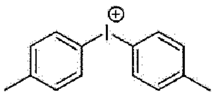 | 1-9 | 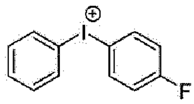 |
| 1-5 | 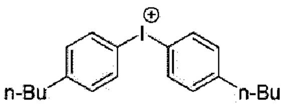 | 1-10 | 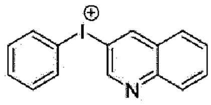 |

| | | | |
|---|---|---|---|
| 1-11 | 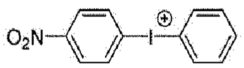 | 1-12 | 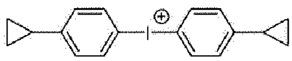 |
| 1-13 | 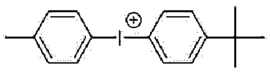 | 1-14 | 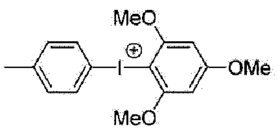 |
| 1-15 | 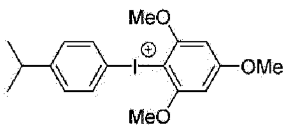 | 1-16 | 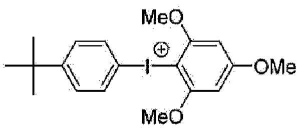 |
| 1-17 | 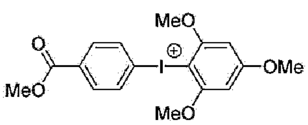 | 1-18 | 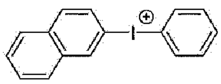 |
| 1-19 | 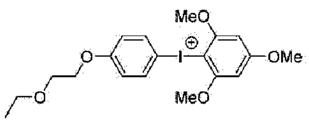 | | |

| | | | |
|---|---|---|---|
| 3-1 | 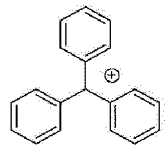 | 3-3 | 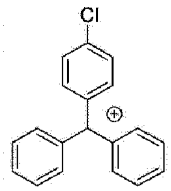 |
| 3-2 | 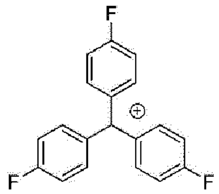 | | |

| | | | |
|---|---|---|---|
| 4-1 | 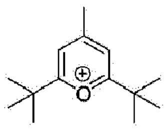 | 4-3 | 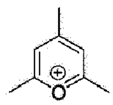 |
| 4-2 | 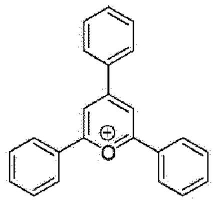 | | |

| | | | |
|---|---|---|---|
| 5-1 | 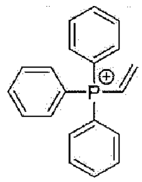 | 5-4 | 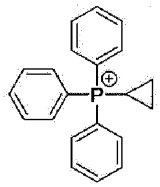 |
| 5-2 | 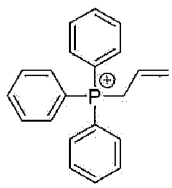 | 5-5 | 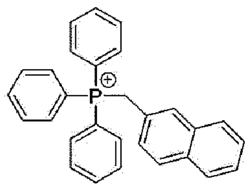 |
| 5-3 | 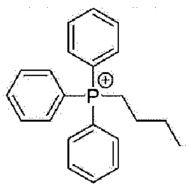 | 5-6 | 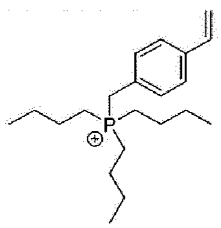 |

| | | | |
|---|---|---|---|
| 6-1 | 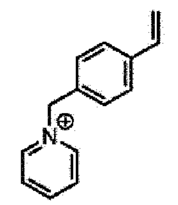 | 6-3 | 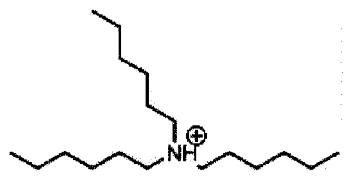 |
| 6-2 | 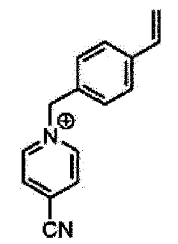 | 6-4 | 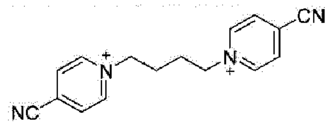 |

According to an exemplary embodiment of the present invention, the ionic compound is any one selected from the group consisting of the following chemical formulae.

[Chemical Formula 1-1-1]

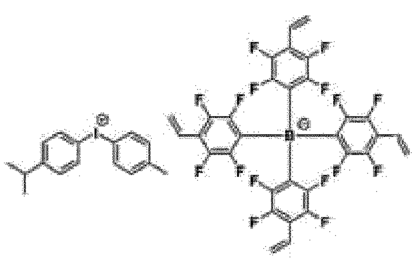

[Chemical Formula 1-1-2]

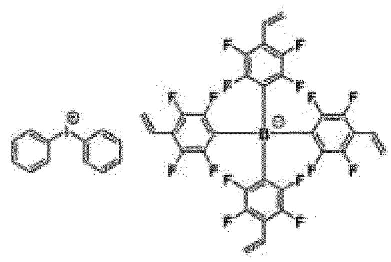

[Chemical Formula 1-1-3]

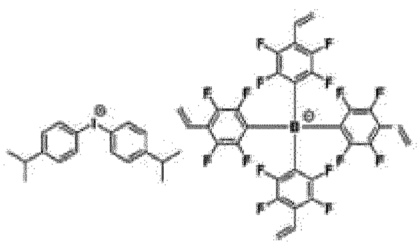

[Chemical Formula 1-1-4]

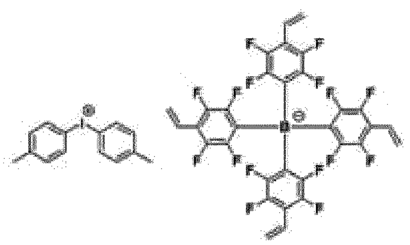

[Chemical Formula 1-1-5]

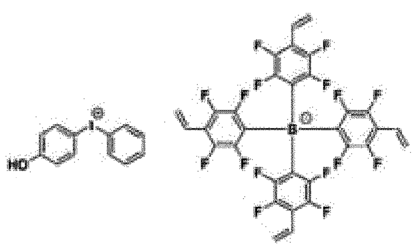

[Chemical Formula 1-1-6]

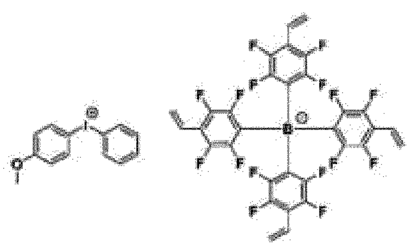

[Chemical Formula 1-1-7]

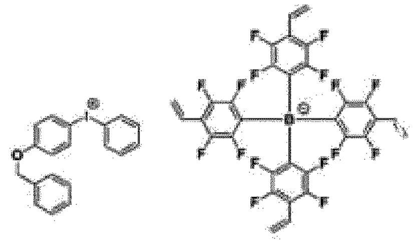

[Chemical Formula 1-1-8]

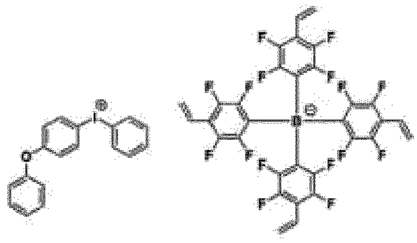

[Chemical Formula 1-1-9]

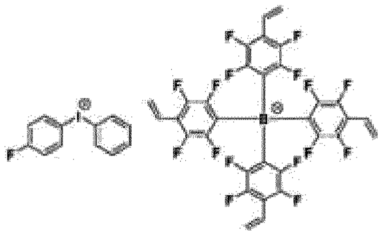

[Chemical Formula 1-1-10]

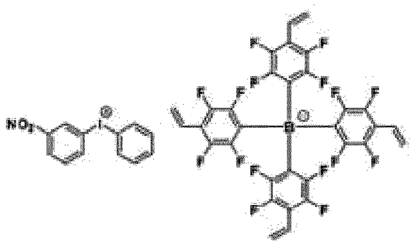

[Chemical Formula 1-1-11]

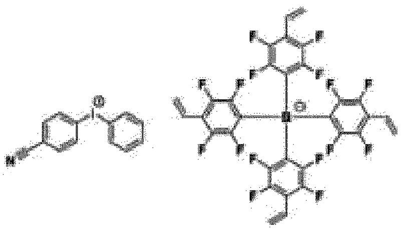

[Chemical Formula 1-1-12]

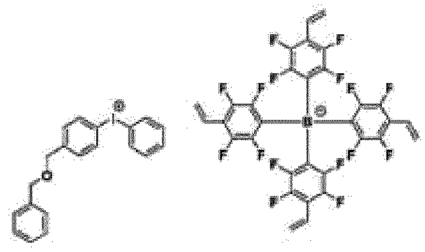

[Chemical Formula 1-1-14]

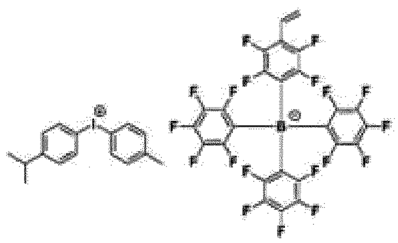

[Chemical Formula 1-1-15]

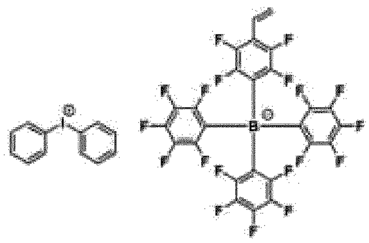

[Chemical Formula 1-1-16]

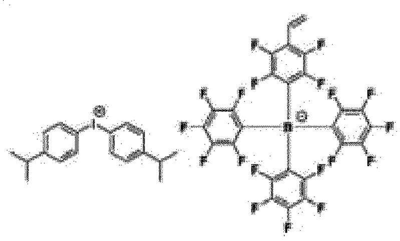

[Chemical Formula 1-1-17]

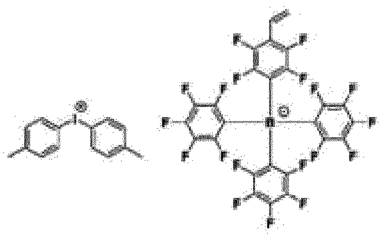

[Chemical Formula 1-1-18]

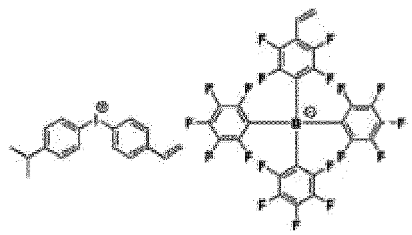

[Chemical Formula 1-1-19]

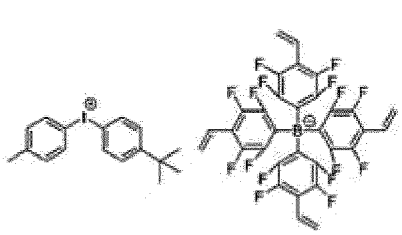

[Chemical Formula 1-1-20]

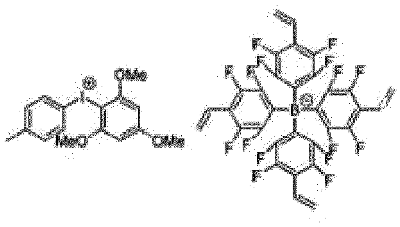

[Chemical Formula 1-1-21]

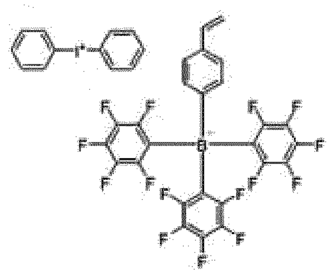

[Chemical Formula 1-1-27]

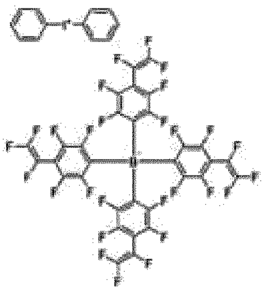

[Chemical Formula 1-1-22]

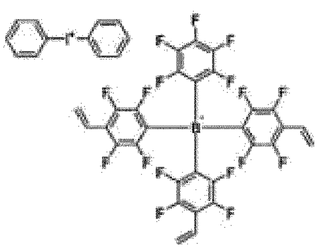

[Chemical Formula 1-1-23]

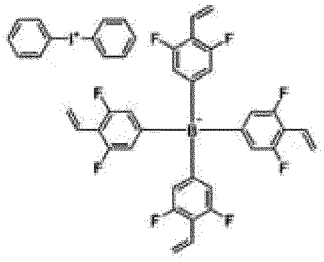

[Chemical Formula 1-1-24]

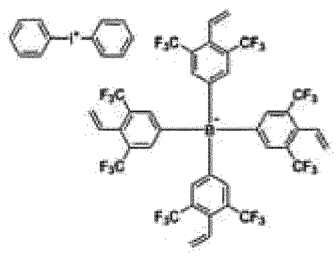

[Chemical Formula 1-1-25]

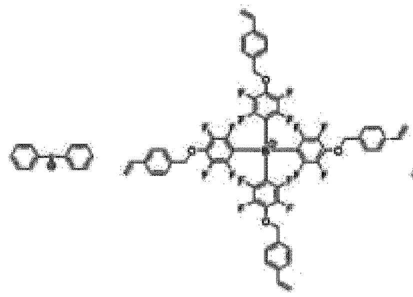

[Chemical Formula 1-1-26]

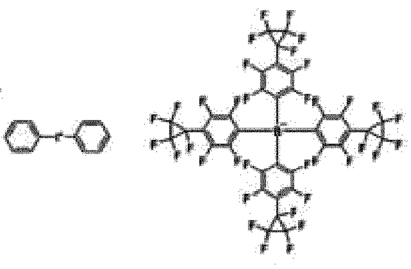

[Chemical Formula 1-2-1]

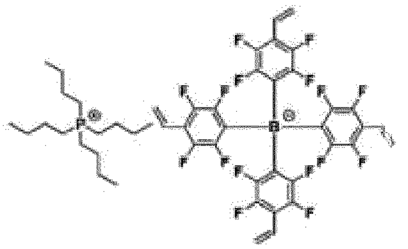

[Chemical Formula 1-2-2]

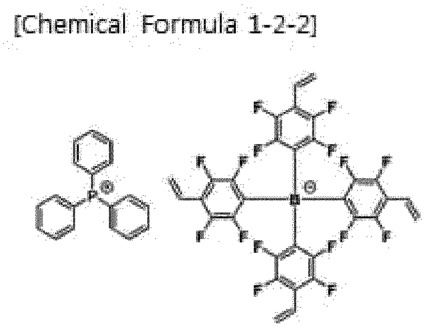

[Chemical Formula 1-2-3]

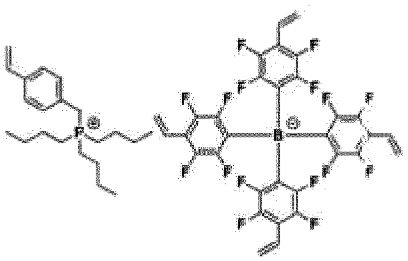

[Chemical Formula 1-2-4]

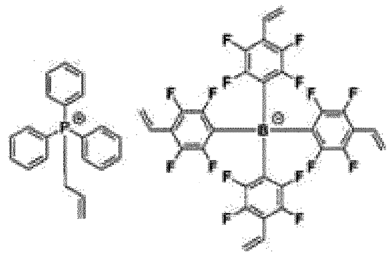

[Chemical Formula 1-2-5]

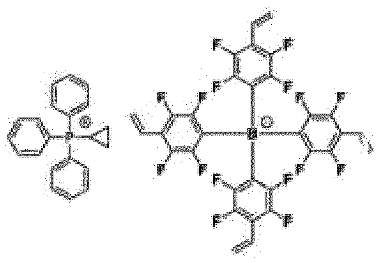

[Chemical Formula 1-3-1]

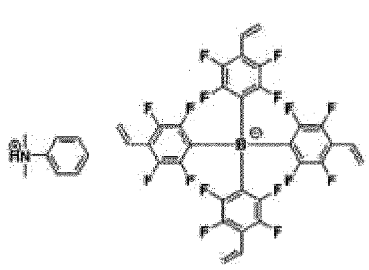

[Chemical Formula 1-3-2]

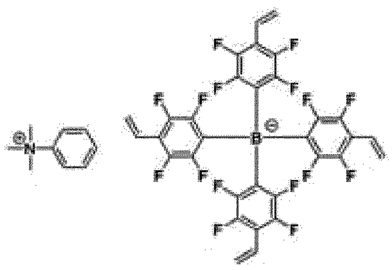

[Chemical Formula 1-3-3]

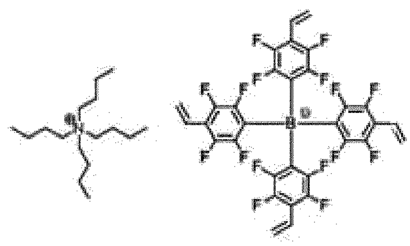

[Chemical Formula 1-3-4]

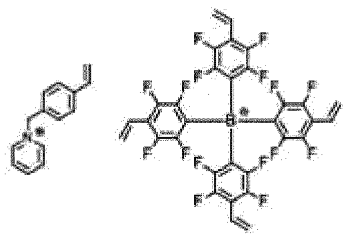

[Chemical Formula 1-4-1]

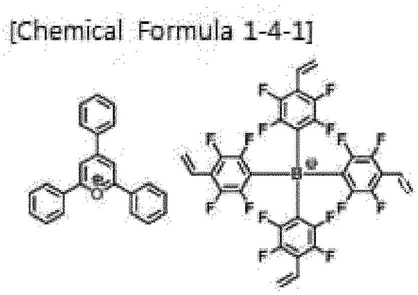

[Chemical Formula 1-5-1]

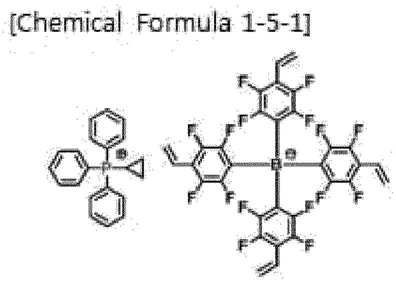

[Chemical Formula 1-6-1]

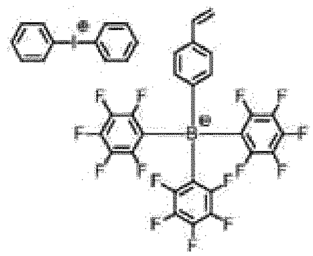

Hereinafter, an ink composition will be specifically described.

An exemplary embodiment of the present invention provides an ink composition including the above-described compound represented by Chemical Formula 1 and the above-described solvent represented by Chemical Formula A.

According to an exemplary embodiment of the present invention, the ink composition further includes an ionic compound including the anionic group represented by the above-described Chemical Formula 2.

According to an exemplary embodiment of the present invention, the ionic compound further includes the above-described cationic group compound. That is, the ink composition further includes an ionic compound including the anionic group represented by the above-described Chemical Formula 2 and the above-described cationic group.

According to a preferred exemplary embodiment of the present invention, the ink composition further includes one or more additional solvents selected from the group consisting of tetralin, 4-methoxytoluene, tetrahydrofuran, dioxane, dipropylene glycol monomethyl ether, tripropylene glycol monomethyl ether, a solvent represented by the following Chemical Formula C-1 and a solvent represented by the following Chemical Formula C-2.

[Chemical Formula C-1]

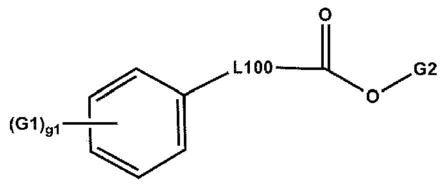

[Chemical Formula C-2]

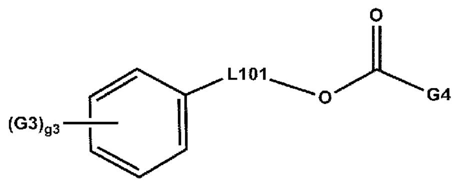

In Chemical Formulae C-1 and C-2,

L100 and L101 are the same as or different from each other, and are each independently a direct bond; or a substituted or unsubstituted alkylene group,
G1 and G3 are the same as or different from each other, and are each independently hydrogen; deuterium; a substituted or unsubstituted alkyl group; a substituted or unsubstituted alkoxy group; a substituted or unsubstituted aryl group; or a substituted or unsubstituted heteroaryl group,
G2 is a straight-chained alkyl group,
G4 is a substituted or unsubstituted alkyl group, and
g1 and g3 are each an integer from 1 to 5, and when g1 and g3 are each 2 or higher, two or more substituents in the parenthesis are the same as or different from each other.

According to an exemplary embodiment of the present specification, the additional solvent is one or more selected from the group consisting of tetralin, 4-methoxytoluene, dipropylene glycol monomethyl ether, tripropylene glycol monomethyl ether, the solvent represented by Chemical Formula C-1 and the solvent represented by Chemical Formula C-2.

According to an exemplary embodiment of the present invention, the additional solvent has a viscosity of 1 cP to 15 cP.

As an additional solvent having a viscosity within the above range is included, there is an advantage in that an ink composition having a viscosity suitable for an inkjet printing process can be prepared.

According to an exemplary embodiment of the present invention, the additional solvent has a boiling point lower than that of the solvent represented by Chemical Formula A.

According to an exemplary embodiment of the present invention, the additional solvent has a boiling point of 70°C to 280°C; 100°C to 280°C; 180°C to 280°C; 180°C to 265°C; or 190°C to 250°C.

As the additional solvent having a boiling point within the above range is included, there is an advantage in that an ink composition having a drying rate suitable for an inkjet printing process can be prepared.

According to an exemplary embodiment of the present invention, the solvent represented by Chemical Formula C-1 is any one selected from the group consisting of the following structures.

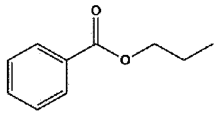
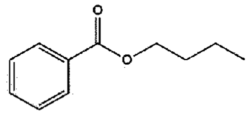
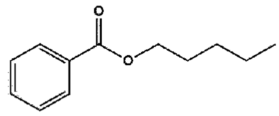

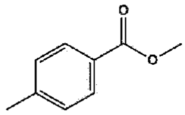
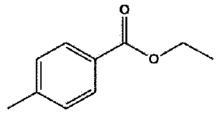
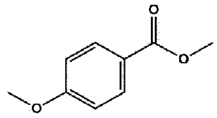
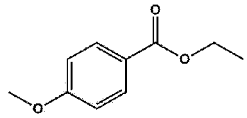

According to an exemplary embodiment of the present invention, the solvent represented by Chemical Formula C-2 is benzyl butyrate.

According to an exemplary embodiment of the present invention, one or two of the solvents of the above-described examples are used as the additional solvent.

According to an exemplary embodiment of the present invention, the ink composition includes a first additional solvent selected from the group consisting of tetralin, 4-methoxytoluene, tetrahydrofuran, dioxane, dipropylene glycol monomethyl ether, tripropylene glycol monomethyl ether, the solvent represented by Chemical Formula C-1 and the solvent represented by Chemical Formula C-2.

According to an exemplary embodiment of the present invention, the ink composition includes a first additional solvent and a second additional solvent selected from the group consisting of tetralin, 4-methoxytoluene, tetrahydrofuran, dioxane, dipropylene glycol monomethyl ether, tripropylene glycol monomethyl ether, the solvent represented by Chemical Formula C-1 and the solvent represented by Chemical Formula C-2, and the first additional solvent and the second additional solvent are different from each other.

According to an exemplary embodiment of the present invention, the ink composition includes a first additional solvent selected from the group consisting of the solvent represented by Chemical Formula C-1 and the solvent represented by Chemical Formula C-2.

According to an exemplary embodiment of the present invention, the ink composition includes a second additional solvent selected from the group consisting of tetralin, 4-methoxytoluene, tetrahydrofuran, dioxane, dipropylene glycol monomethyl ether and tripropylene glycol monomethyl ether.

An exemplary embodiment of the present invention provides an ink composition including: the compound represented by the above-described Chemical Formula 1; the solvent represented by the above-described Chemical Formula A; the ionic compound including the anionic group represented by the above-described Chemical Formula 2; and the above-described additional solvent.

An exemplary embodiment of the present invention provides an ink composition including: the compound represented by the above-described Chemical Formula 1; the solvent represented by the above-described Chemical Formula A; the above-described ionic compound; and the above-described additional solvent, and the ionic compound includes the anionic group represented by the described-above Chemical Formula 2 and the above-described cationic group.

According to an exemplary embodiment of the present invention, based on 100 wt% of the ink composition, the compound represented by Chemical Formula 1 is included in an amount of 0.1 wt% to 15 wt%; 0.1 wt% to 10 wt%; or 0.1 wt% to 5 wt%.

According to an exemplary embodiment of the present invention, based on 100 wt% of the ink composition, the solvent represented by Chemical Formula A is included in an amount of 1 wt% to 70 wt%; 1 wt% to 50 wt%; or 1 wt% to 30 wt%.

According to an exemplary embodiment of the present invention, based on 100 wt% of the ink composition, the ionic compound including the anionic group represented by Chemical Formula 2 is included in an amount of 0.1 wt% to 15 wt%; 0.1 wt% to 10 wt%; or 0.1 wt% to 5 wt%.

According to an exemplary embodiment of the present invention, based on 100 wt% of the ink composition, the additional solvent is included in an amount of 10 wt% to 90 wt%; 10 wt% to 85 wt%; 10 wt% to 80 wt%; or 10 wt% to 70 wt%. However, the amount is not limited to the above examples, and an amount typically used by those skilled in the art to which the present invention pertains may be used as the content of the additional solvent.

According to an exemplary embodiment of the present invention, based on 100 wt% of the ink composition, the compound represented by Chemical Formula 1 is included in an amount of 0.1 wt% to 15 wt%; the solvent represented by Chemical Formula A is included in an amount of 1 wt% to 70 wt%; the ionic compound including the anionic group represented by Chemical Formula 2 is included in an amount of 0.1 wt% to 15 wt; and the additional solvent is included in an amount of 10 wt% to 85 wt%.

According to an exemplary embodiment of the present invention, the ink composition has a viscosity of 2 cP to 15

cP at room temperature. When the above viscosity is satisfied, a device is easily manufactured. Specifically, a uniform film may be formed when an organic material layer in an organic light emitting device is formed.

The above viscosity is a value measured at room temperature using a viscometer manufactured by Brookfield Engineering Labs Inc. after dissolving an object to be measured at any one concentration of 1 wt% to 3 wt% in a solvent. In this case, the object to be measured is a compound represented by Chemical Formula 1; or a mixture of the compound represented by Chemical Formula 1 and the above-described ionic compound. Further, the solvent is a solvent represented by Chemical Formula A; or a mixed solvent of a solvent represented by Chemical Formula A and an additional solvent.

According to an exemplary embodiment of the present invention, the ink composition is a liquid phase. The "liquid phase" means that the composition is in a liquid state at room temperature under atmospheric pressure.

In an exemplary embodiment of the present specification, the ink composition can be cured by a heat treatment or a light treatment. When the ink composition is cured, this may be expressed as a cured product of the ink composition.

According to an exemplary embodiment of the present invention, the ink composition further includes: a single molecule including a photocurable group and/or a thermosetting group; or a single molecule including an end group capable of forming a polymer by heat. As described above, the single molecule including a photocurable group and/or a thermosetting group; or the single molecule including an end group capable of forming a polymer by heat may be a compound having a molecular weight of 3,000 g/mol or less, but the molecular weight is not limited to the exemplified molecular weight.

The single molecule including a photocurable group and/or a thermosetting group; or the single molecule including an end group capable of forming a polymer by heat may mean aryl such as phenyl, biphenyl, fluorene, and naphthalene; arylamine; or a single molecule in which a fluorene is substituted with photocurable group and/or a thermosetting group or an end group capable of forming a polymer by heat.

According to an exemplary embodiment of the present invention, the ink composition may further include another additional solvent (hereinafter, expressed as a third solvent) other than the above-described additional solvent. The third solvent may be one or more selected from the group consisting of a chlorine-based solvent such as chloroform, methylene chloride, 1,2-dichloroethane, 1,1,2-trichloroethane, chlorobenzene, and o-dichlorobenzene; an ether-based solvent such as tetrahydrofuran, dioxane, and dipropylene glycol monomethyl ether; an aromatic hydrocarbon-based solvent such as toluene, xylene, trimethylbenzene, and mesitylene; an aliphatic hydrocarbon-based solvent such as cyclohexane, methylcyclohexane, n-pentane, n-hexane, n-heptane, n-octane, n-nonane, and n-decane; a ketone-based solvent such as acetone, methyl ethyl ketone, cyclohexanone, isophorone, tetralone, decalone, and acetylacetone; an ester-based solvent such as ethyl acetate, butyl acetate, ethyl cellosolve acetate, and benzyl butyrate; a polyhydric alcohol such as ethylene glycol, ethylene glycol monobutyl ether, ethylene glycol monoethyl ether, ethylene glycol monomethyl ether, dimethoxy ethane, propylene glycol, diethoxymethane, triethylene glycol monoethyl ether, glycerin, and 1,2-hexanediol, and derivatives thereof; an alcohol-based solvent such as methanol, ethanol, propanol, isopropanol, and cyclohexanol; a sulfoxide-based solvent such as dimethyl sulfoxide; an amide-based solvent such as N-methyl-2-pyrrolidone and N,N-dimethylformamide; and tetralin. However, the third solvent is not limited to the above example, and is sufficient as long as the third solvent can dissolve or disperse the compound represented by the above-described Chemical Formula 1.

According to an exemplary embodiment of the present invention, the ink composition is a composition for forming one or more layers selected from the group consisting of a hole injection layer, a hole transport layer, a hole injection and transport layer, and an electron blocking layer.

Hereinafter, a pixel including the above-described ink composition or a curred product thereof will be specifically described.

An exemplary embodiment of the present invention provides a pixel including an ink composition or a cured product thereof. The pixel may be included in an organic material layer, an organic light emitting diode, or an organic light emitting display device. Details of the organic material layer will be described below. Specifically, the organic light emitting display device includes a plurality of pixels composed of red (R), green (G), and blue (B) sub-pixels, and an organic light emitting diode (OLED) and a pixel circuit are located for each sub-pixel. An organic light emitting diode includes two electrodes (anode and cathode) and an organic light emitting layer located therebetween, and a pixel circuit includes at least two thin film transistors and at least one capacitor. The organic material layer, the organic light emitting diode, or the organic light emitting display device may include a bank. Preferably, the organic material layer, the organic light emitting diode, or the organic light emitting display device may include a line-shaped bank. The line-shaped bank may define a line-shaped pixel region and/or a sub-pixel region. In a normal case, the light emitting layer among the organic material layers is composed of a red light emitting layer, a green light emitting layer and a blue light emitting layer located in a red sub-pixel, a green sub-pixel and a blue sub-pixel, respectively. In this case, the above-described pixel may correspond to the pixel or sub-pixel. As a preferred exemplary embodiment, the pixel corresponds to the sub-pixel.

According to an exemplary embodiment of the present invention, one or more of the pixels may be included in the above-described organic material layer, organic light emitting diode or organic light emitting display device.

According to an exemplary embodiment of the present invention, the thickness uniformity V of the one or more

pixels is 0.4 or less, and the thickness uniformity V satisfies the following Equation 1.

According to an exemplary embodiment of the present invention, the thickness uniformity V satisfies the following Equation 1.

[Equation 1]

$$V = \frac{\sum_{i=2}^{n'} \left(X(i) - Xm'\right)^2}{n' \times Xm'}$$

In Equation 1,

X(i) is the thickness of the i-th pixel,
Xm' is the average value of X(i) in which i is 2 to n', and
n' is an integer from 2 to $10^4$.

In the present invention, when the thickness uniformity V is 0.4 or lower and the thickness uniformity V satisfies Equation 1, it means that the thickness uniformity V calculated using Equation 1 is 0.4 or lower.

According to an exemplary embodiment of the present invention, the thickness uniformity is 0.01 to 0.4, or 0.02 to 0.4.

In the present invention, a low thickness uniformity value means that the thicknesses of the pixels are uniformly formed. That is, it means that the lower the thickness uniformity value, the more uniformly the pixels are formed.

According to an exemplary embodiment of the present invention, the thickness of the pixel may mean the thickness of the central portion of the pixel, and may be measured by an optical thickness measuring device. As the optical thickness measuring device, an optical profiler manufactured by Bruker or the like may be used, but the optical thickness measuring device is not limited thereto.

An exemplary embodiment of the present invention provides an organic material layer including the above-described pixel. That is, an exemplary embodiment of the present invention provides an organic material layer including the above-described ink composition or a cured product thereof.

Hereinafter, an organic material layer including the above-described pixel will be specifically described.

According to an exemplary embodiment of the present invention, the organic material layer includes a plurality of the pixels. For example, the organic material layer includes an x-axis on which na pixels are aligned and a y-axis that is perpendicular to the x-axis and on which nb pixels are aligned, and na and nb are each an integer from 2 to $10^4$.

According to an exemplary embodiment of the present invention, na and nb are each 2 or higher; 5 or higher; 10 or higher; 20 or higher; or 40 or higher, $10^4$ or lower; $10^3$ or lower; or $10^2$ or lower.

According to an exemplary embodiment of the present invention, the organic material layer includes 2 or more; 4 or more; 10 or more; 20 or more; 40 or more; 80 or more; or 100 or more and $10^8$ or less; $10^6$ or less; $10^4$ or less; or $10^3$ or less pixels.

According to an exemplary embodiment of the present invention, pixels included in the organic material layer are aligned in the form of a line. For example, as shown in FIG. 2, a plurality of pixels are aligned in a row to form a line.

According to an exemplary embodiment of the present invention, the organic material layer includes a plurality of lines in which the plurality of pixels are aligned in a row. For example, as shown in FIG. 2, a plurality of lines may be formed at regular intervals. For example, the y-axis on which the above-described nb pixels are aligned is one line, and it is possible to include as many lines as the number of pixels (na) included in the x-axis.

According to an exemplary embodiment of the present invention, the organic material layer includes na x nb pixels.

According to an exemplary embodiment of the present invention, a plurality of pixels included in the organic material layer have a uniform thickness.

According to an exemplary embodiment of the present invention, the organic material layer includes n' pixels, the thickness uniformity V of the n' pixels is 0.4 or lower, and the thickness uniformity V satisfies the above-described Equation 1.

According to an exemplary embodiment of the present invention, n' is 2 to $10^4$.

According to an exemplary embodiment of the present invention, the organic material layer is included in an organic light emitting device.

According to an exemplary embodiment of the present invention, the organic material layer is formed by a solution process.

According to an exemplary embodiment of the present invention, the organic material layer is one or more layers selected from the group consisting of a hole injection layer, a hole transport layer, a hole injection and transport layer, an electron blocking layer, a light emitting layer, a hole blocking layer, an electron injection layer, an electron transport layer, and an electron injection and transport layer.

According to an exemplary embodiment of the present invention, the organic material layer is one or more layers selected from the group consisting of a hole injection layer, a hole transport layer, a hole injection and transport layer, an electron blocking layer, and a light emitting layer.

According to an exemplary embodiment of the present invention, the organic material layer is one or more layers selected from the group consisting of a hole injection layer, a hole transport layer, a hole injection and transport layer, and an electron blocking layer.

According to an exemplary embodiment of the present invention, the ink composition is a composition for forming one or more layers selected from the group consisting of a hole injection layer, a hole transport layer, a hole injection and transport layer, and an electron blocking layer.

According to a preferred exemplary embodiment of the present invention, the organic material layer is a hole injection layer, a hole transport layer or an electron blocking layer.

Hereinafter, an organic light emitting device will be specifically described.

An exemplary embodiment of the present invention provides an organic light emitting device including: a first electrode; a second electrode; and an organic material layer having one or more layers provided between the first electrode and the second electrode, in which one or more layers of the organic material layer include the above-described ink composition or a cured product thereof.

According to an exemplary embodiment of the present invention, the organic material layer includes one or more layers selected from the group consisting of a hole injection layer, a hole transport layer, a hole injection and transport layer, an electron blocking layer, a light emitting layer, a hole blocking layer, an electron injection layer, an electron transport layer, and an electron injection and transport layer, and the one or more layers selected from the group consisting of the hole injection layer, the hole transport layer, the hole injection and transport layer, the electron blocking layer, the light emitting layer, the hole blocking layer, the electron injection layer, the electron transport layer, and the electron injection and transport layer include the ink composition or a cured product thereof.

According to an exemplary embodiment of the present invention, the organic material layer includes one or more layers selected from the group consisting of a hole injection layer, a hole transport layer, a hole injection and transport layer, and an electron blocking layer, and the one or more layers selected from the group consisting of the hole injection layer, the hole transport layer, the hole injection and transport layer, and the electron blocking layer include the ink composition or a cured product thereof.

According to a preferred exemplary embodiment of the present invention, the organic material layer includes a hole injection layer, a hole transport layer, or an electron blocking layer, and the hole injection layer, the hole transport layer, or the electron blocking layer includes the ink composition or a cured product thereof.

According to an exemplary embodiment of the present invention, the organic material layer included in the organic light emitting device includes n' of the above-described pixels, the thickness uniformity V of the n' pixels is 0.4 or lower, and the thickness uniformity V satisfies the above-described Equation 1.

Hereinafter, a method for manufacturing an organic light emitting device will be specifically described.

An exemplary embodiment of the present invention provides a method for manufacturing an organic light emitting device, the method including: preparing a first electrode;

forming an organic material layer having one or more layers on the first electrode; and
forming a second electrode on the organic material layer having one or more layers,
in which the forming of the organic material layer includes forming an organic material layer using the above-described ink composition.

According to an exemplary embodiment of the present invention, the forming of the organic material layer using the ink composition may form the organic material layer on a substrate including a line-shaped bank or an organic material layer.

As an example, the above-described ink composition is applied (FIG. 3) on a substrate including a line-shaped bank (FIG. 2), and then a thin film in which the ink composition is dried and/or heat-treated (FIG. 4) may be formed.

According to another exemplary embodiment of the present invention, the forming of the organic material layer using the ink composition uses a spin coating method.

According to still another exemplary embodiment of the present invention, the forming of the organic material layer using the ink composition uses a printing method.

According to an exemplary embodiment of the present invention, examples of the printing method include inkjet printing, nozzle printing, offset printing, transfer printing, screen printing, or the like, but are not limited to the printing

methods listed above.

According to an exemplary embodiment of the present invention, the forming of the organic material layer using the ink composition uses an inkjet printing method.

A solution process is suitable for the ink composition according to an exemplary embodiment of the present invention due to the composition and structural characteristics of materials included, so that the organic material layer may be formed by a printing method, and as a result, there is an economic effect in terms of time and costs when a device is manufactured.

According to an exemplary embodiment of the present invention, the forming of the organic material layer on the first electrode may further include drying after one or more steps of pressure reduction. As an example, the forming of the organic material layer on the first electrode further includes drying after two steps of pressure reduction. In this case, the drying after the first pressure reduction is performed under normal pressure to $2x10^{-2}$ torr, and the pressure reduction time under normal pressure to $2x10^{-2}$ torr may be 30 seconds to 600 seconds. Further, the drying after the second pressure reduction is performed under $2x10^{-2}$ to $2x10^{-6}$ torr, and the pressure reduction time under $2x10^{-2}$ torr to $2x10^{-6}$ torr may also be 30 seconds to 600 seconds.

According to an exemplary embodiment of the present invention, the forming of the organic material layer using the ink composition includes: coating the ink composition; and heat-treating or light-treating the coated ink composition.

According to an exemplary embodiment of the present invention, the forming of the organic material layer using the ink composition includes: coating the first electrode or the organic material layer having one or more layers with the ink composition; and heat-treating or light-treating the coated ink composition.

According to an exemplary embodiment of the present invention, the heat-treating of the ink composition may be performed through a heat treatment, and the heat treatment temperature in the heat-treating of the ink composition may be 85°C to 250°C, may be 100°C to 250°C according to an exemplary embodiment, and may be 150°C to 250°C in another exemplary embodiment.

According to an exemplary embodiment of the present invention, the heat treatment time in the heat-treating of the ink composition may be in a range of 10 minutes to 2 hours, may be in a range of 10 minutes to 1 hour according to an exemplary embodiment, and may be in a range of 20 minutes to 1 hour in another exemplary embodiment.

When the forming of the organic material layer using the ink composition includes the heat-treating or light-treating of the ink composition, a plurality of the compounds included in the ink composition may form a crosslinkage, thereby providing an organic material layer including a thin-filmed structure. In this case, it is possible to prevent the organic material layer from being dissolved, morphologically affected or decomposed by a solvent when another layer is stacked on the surface of the organic material layer formed using the ink composition.

Therefore, when the organic material layer formed using the ink composition is formed by a method including the subjecting of the organic material layer to the heat treatment or the light treatment, resistance to a solvent is increased, so that a plurality of layers may be formed by repeatedly carrying out solution deposition and crosslinking methods, and stability is increased, so that service life characteristics of the device may be increased.

Hereinafter, the types of organic material layers included in the above-described organic light emitting device will be specifically described.

According to an exemplary embodiment of the present invention, the organic light emitting device includes an organic material layer having one layer, and the organic material layer includes the above-described ink composition or a cured product thereof.

According to another exemplary embodiment of the present invention, the organic light emitting device includes an organic material layer having two or more layers, and the organic material layer having two or more layers includes the above-described ink composition or a cured product thereof. For example, among organic material layers having two or more layers, the organic material layer having any one layer includes the ink composition or a cured product thereof, and further includes the organic material layer having the other layer or more. The organic material layer having the other layer or more according to an exemplary embodiment does not include the ink composition or a cured product thereof. The organic material layer having the other layer or more according to another exemplary embodiment further includes the ink composition or a cured product thereof. However, the organic material layer having the other layer or more is not limited to the examples.

The organic material layer having two or more layers includes two or more layers selected from the group consisting of, for example, a hole injection layer, a hole transport layer, a hole injection and transport layer, an electron blocking layer, a light emitting layer, a hole blocking layer, an electron transport layer, an electron injection layer, an electron injection and transport layer, and the like. In this case, the hole injection and transport layer means a layer which simultaneously injects and transports holes, and the electron injection and transport layer means a layer which simultaneously injects and transports electrons. However, the organic material layers forming the group are merely an example, and are not limited to the above example. Further, the organic material layer having two or more layers may include two or more layers that play the same role, if necessary. The organic light emitting device according to an exemplary embodiment includes a first hole injection layer and a second hole injection layer. However, the organic material

layer having two or more layers is not limited to the examples.

According to an exemplary embodiment of the present invention, the organic material layer includes a light emitting layer. As an example, the light emitting layer includes the ink composition or a cured product thereof. As a specific example, the light emitting layer includes the ink composition or a cured product thereof as a host of the light emitting layer. As another specific example, the light emitting layer includes the ink composition or a cured product thereof as a dopant of the light emitting layer.

According to an exemplary embodiment of the present invention, the organic material layer includes a hole injection and transport layer, a hole injection layer, a hole transport layer and an electron blocking layer. As an example, the hole injection and transport layer, the hole injection layer, the hole transport layer or the electron blocking layer includes an ink composition or a cured product thereof.

According to a preferred exemplary embodiment of the present invention, the organic material layer includes a hole transport layer or an electron blocking layer. As an example, the hole transport layer or the electron blocking layer includes the ink composition or a cured product thereof.

According to a preferred exemplary embodiment of the present invention, the organic material layer includes a hole injection layer and a hole transport layer.

According to an exemplary embodiment of the present invention, the organic material layer further includes one or more layers selected from the group consisting of a hole blocking layer, an electron transport layer, an electron injection layer, and an electron injection and transport layer. As an example, one or more layers selected from the group consisting of the hole blocking layer, the electron transport layer, the electron injection layer, and the electron injection and transport layer include the ink composition or a cured product thereof. As another example, one or more layers selected from the group consisting of the hole blocking layer, the electron transport layer, the electron injection layer, and the electron injection and transport layer do not include an ink composition or a cured product thereof.

An exemplary embodiment of the present invention provides an organic light emitting device including: a first electrode; a second electrode; and an organic material layer having one or more layers provided between the first electrode and the second electrode, in which the organic material layer includes a light emitting layer and a hole injection layer, and the hole injection layer includes the ink composition or a cured product thereof.

Hereinafter, the stacking structure of the organic material layer and the organic light emitting device including the organic material layer will be specifically described.

The organic material layer of the organic light emitting device according to an exemplary embodiment of the present invention has a single-layered structure. For example, the organic material layer having the single-layered structure is provided between the first electrode and the second electrode of the organic light emitting device, and includes the ink composition or a cured product thereof. According to a specific exemplary embodiment, the organic material layer having the single-layered structure is a light emitting layer, and in this case, the light emitting layer includes the ink composition or a cured product thereof.

The organic material layer of the organic light emitting device according to another exemplary embodiment of the present invention has a multi-layered structure in which an organic material layer having two or more layers is stacked. For example, the organic material layer having the multi-layered structure is provided between the first electrode and the second electrode of the organic light emitting device.

According to an exemplary embodiment of the present invention, the organic material layer having the multi-layered structure includes a light emitting layer and an organic material layer other than the light emitting layer. As an example, the light emitting layer is provided between the first electrode and the second electrode, and the organic material layer other than the light emitting layer is provided between the first electrode and the light emitting layer. As another example, the light emitting layer is provided between the first electrode and the second electrode, and the organic material layer other than the light emitting layer is provided between the light emitting layer and the second electrode. As still another example, the light emitting layer is provided between the first electrode and the second electrode, any one organic material layer other than the light emitting layer is provided between the first electrode and the light emitting layer, and the other organic material layer other than the light emitting layer is provided between the light emitting layer and the second electrode. However, the above structure is merely an example, and the structure is not limited to the above structure. Further, the organic material layer other than the light emitting layer may be one or more layers selected from the group consisting of, for example, a hole injection and transport layer, a hole injection layer, a hole transport layer, an electron blocking layer, a hole blocking layer, an electron transport layer, an electron injection layer, an electron injection and transport layer, and the like, but is not limited thereto.

In general, a hole injection layer, a hole transport layer or an electron blocking layer in an organic light emitting device is provided between an anode and a light emitting layer. As a specific example, the hole injection layer is provided on the anode, the hole transport layer is provided on the hole injection layer, and the electron blocking layer is provided on the hole injection layer, but the present invention is not limited to the above examples.

Further, in general, an electron injection layer, an electron transport layer or a hole blocking layer in an organic light emitting device is provided between a cathode and a light emitting layer. As a specific example, the hole blocking layer

is provided on the light emitting layer, the electron transport layer is provided on the hole blocking layer, and the electron injection layer is provided on the electron transport layer, but the present invention is not limited to the above examples.

The organic material layer having a multi-layered structure included in the organic light emitting device according to an exemplary embodiment of the present invention includes: one or more layers selected from the group consisting of a hole injection and transport layer, a hole injection layer, a hole transport layer, an electron blocking layer, a hole blocking layer, an electron transport layer, an electron injection layer, and an electron injection and transport layer; and a light emitting layer, the light emitting layer is provided between a first electrode and a second electrode, the one or more layers are provided between the first electrode and the light emitting layer or between the second electrode and the light emitting layer, and the one or more layers include an ink composition or a cured product thereof.

The structure of the organic light emitting device according to an exemplary embodiment of the present invention is illustrated in FIG. 1. FIG. 1 exemplifies a structure of an organic light emitting device in which a first electrode 201, a hole injection layer 301, a hole transport layer 401, a light emitting layer 501, an electron injection and transport layer 601, and a second electrode 701 are sequentially stacked on a substrate 101. The hole injection layer 301 and/or the hole transport layer 401 of FIG. 1 include/includes the above-described ink composition or a cured product thereof, or may be formed using the ink composition. In this case, for the hole injection layer 301 and/or the hole transport layer 401 of FIG. 1, a specific content of the organic material layer which may be formed using the ink composition, a material thereof and a manufacturing method thereof will described below. In addition, FIG. 1 exemplifies the organic light emitting device according to an exemplary embodiment of the present invention, and the organic light emitting device is not limited thereto.

As described above, an organic light emitting device having an organic material layer having a single-layered or multi-layered structure may have, for example, the following stacking structure, but the stacking structure is not limited thereto.

(1) Anode/Hole transport layer/Light emitting layer/Cathode
(2) Anode/Hole injection layer/Hole transport layer/Light emitting layer/Cathode
(3) Anode/Hole injection layer/Hole buffer layer/Hole transport layer/Light emitting layer/Cathode
(4) Anode/Hole transport layer/Light emitting layer/Electron transport layer/Cathode
(5) Anode/Hole transport layer/Light emitting layer/Electron transport layer/Electron injection layer/Cathode
(6) Anode/Hole injection layer/Hole transport layer/Light emitting layer/Electron transport layer/Cathode
(7) Anode/Hole injection layer/Hole transport layer/Light emitting layer/Electron transport layer/Electron injection layer/Cathode
(8) Anode/Hole injection layer/Hole buffer layer/Hole transport layer/Light emitting layer/Electron transport layer/-Cathode
(9) Anode/Hole injection layer/Hole buffer layer/Hole transport layer/Light emitting layer/Electron transport layer/-Electron injection layer/Cathode
(10) Anode/Hole transport layer/Electron blocking layer/Light emitting layer/Electron transport layer/Cathode
(11) Anode/Hole transport layer/Electron blocking layer/Light emitting layer/Electron transport layer/Electron injection layer/Cathode
(12) Anode/Hole injection layer/Hole transport layer/Electron blocking layer/Light emitting layer/Electron transport layer/Cathode
(13) Anode/Hole injection layer/Hole transport layer/Electron blocking layer/Light emitting layer/Electron transport layer/Electron injection layer/Cathode
(14) Anode/Hole transport layer/Light emitting layer/Hole blocking layer/Electron transport layer/Cathode
(15) Anode/Hole transport layer/Light emitting layer/Hole blocking layer/Electron transport layer/Electron injection layer/Cathode
(16) Anode/Hole injection layer/Hole transport layer/Light emitting layer/Hole blocking layer/Electron transport layer/Cathode
(17) Anode/Hole injection layer/Hole transport layer/Light emitting layer/Hole blocking layer/Electron transport layer/Electron injection layer/Cathode
(18) Anode/Hole injection layer/Hole transport layer/Light emitting layer/Hole blocking layer/Electron transport layer/Electron injection layer/Cathode/Capsule
(19) Anode/Hole injection layer/First hole transport layer/Second hole transport layer/Light emitting layer/Hole blocking layer/Electron transport layer/Electron injection layer/Cathode/Capsule

In the structures, the 'Electron transport layer/Electron injection layer' may be replaced with an 'electron injection and transport layer' or a 'layer that simultaneously injects and transports electrons'.

Further, in the structures, the 'Hole injection layer/Hole transport layer' may be replaced with the 'hole injection and transport layer' or the "layer which simultaneously injects and transports holes".

According to an exemplary embodiment of the present invention, the first electrode is an anode, and the second

electrode is a cathode.

According to another exemplary embodiment of the present invention, the first electrode is a cathode, and the second electrode is an anode.

According to an exemplary embodiment of the present invention, the organic light emitting device may be a normal type organic light emitting device in which an anode, an organic material layer having one or more layers, and a cathode are sequentially stacked on a substrate.

According to another exemplary embodiment of the present invention, the organic light emitting device may be an inverted type organic light emitting device in which a cathode, an organic material layer having one or more layers, and an anode are sequentially stacked on a substrate.

Hereinafter, the specific content of the above-described organic material layer, a material thereof, and a manufacturing method thereof will be described. However, the organic light emitting device of the present invention may be manufactured by materials and methods known in the art, except that the organic material layer includes the above-described compound.

In an organic light emitting device according to an exemplary embodiment of the present invention, one or more layers of the organic material layer are formed using the ink composition or a cured product thereof. Except for this, the organic light emitting device may be manufactured by materials and methods known in the art.

For example, the organic light emitting device of the present invention may be manufactured by sequentially stacking an anode, an organic material layer, and a cathode on a substrate. In this case, the organic light emitting device may be manufactured by depositing a metal or a metal oxide having conductivity, or an alloy thereof on a substrate to form an anode, forming an organic material layer including one or more layers of a hole injection layer, a hole transport layer, a light emitting layer, an electron injection layer, an electron transport layer, a hole transport and injection layer, and an electron injection and transport layer thereon through a solution process, a deposition process, the like, and then depositing a material, which may be used as a cathode, thereon, by using a physical vapor deposition (PVD) method such as sputtering or e-beam evaporation. In addition to the method described above, an organic light emitting device may be made by sequentially depositing a cathode material, an organic material layer, and an anode material on a substrate.

As the anode material, materials having a high work function are usually preferred so as to facilitate the injection of holes into an organic material layer. Examples thereof include: a metal, such as vanadium, chromium, copper, zinc, and gold, or an alloy thereof; a metal oxide, such as zinc oxide, indium oxide, indium tin oxide (ITO), and indium zinc oxide (IZO); a combination of a metal and an oxide, such as ZnO:Al or $SnO_2$:Sb; a conductive polymer, such as poly(3-methylthiophene), poly[3,4-(ethylene-1,2-dioxy)thiophene] (PEDOT), polypyrrole, and polyaniline; and the like, but are not limited thereto.

As the cathode material, materials having a low work function are usually preferred so as to facilitate the injection of electrons into an organic material layer. Examples thereof include: a metal, such as magnesium, calcium, sodium, potassium, titanium, indium, yttrium, lithium, gadolinium, aluminum, silver, tin, and lead, or an alloy thereof; a multi-layer structured material, such as LiF/Al or $LiO_2$/Al; and the like, but are not limited thereto.

The light emitting layer may include a host material and/or a dopant material.

Examples of the host material include a fused aromatic ring derivative, a hetero ring-containing compound, or the like. Specific examples of the fused aromatic ring derivative include anthracene derivatives, pyrene derivatives, naphthalene derivatives, pentacene derivatives, phenanthrene compounds, fluoranthene compounds, and the like, and specific examples of the hetero ring-containing compound include dibenzofuran derivatives, ladder-type furan compounds, pyrimidine derivatives, and the like, but the examples are not limited thereto.

Examples of the dopant material include an aromatic amine derivative, a styrylamine compound, a boron complex, a fluoranthene compound, a metal complex, and the like. Specifically, the aromatic amine derivative is a fused aromatic ring derivative having a substituted or unsubstituted arylamine group, and examples thereof include pyrene, anthracene, chrysene, periflanthene, and the like having an arylamine group. Further, the styrylamine compound is a compound in which a substituted or unsubstituted arylamine is substituted with at least one arylvinyl group, and one or two or more substituents selected from the group consisting of an aryl group, a silyl group, an alkyl group, a cycloalkyl group, and an arylamine group are substituted or unsubstituted. Specific examples thereof include styrylamine, styryldiamine, styryltriamine, styryltetramine, and the like, but are not limited thereto. Further, examples of the metal complex include an iridium complex, a platinum complex, and the like, but are not limited thereto.

The hole injection layer is a layer which accepts holes from an electrode. It is preferred that hole injection material has an ability to transport holes, and has an effect of accepting holes from an anode and an excellent hole injection effect for a light emitting layer or a light emitting material. Further, the hole injection material is preferably a material which is excellent in ability to prevent excitons produced from a light emitting layer from moving to an electron injection layer or an electron injection material. In addition, the hole injection material is preferably a material which is excellent in ability to form a thin film. In addition, the HOMO of the hole injection material is preferably a value between the work function of the anode material and the HOMO of the neighboring organic material layer. Specific examples of the hole injection material include: metal porphyrin, oligothiophene, and arylamine-based organic materials; hexanitrile hexaazatriphenylene-based organic

materials; quinacridone-based organic materials; perylene-based organic materials; polythiophene-based conductive polymers such as anthraquinone and polyaniline; and the like, but are not limited thereto.

The hole transport layer is a layer which accepts holes from a hole injection layer and transports the holes to a light emitting layer, and may have a single-layered structure or a multi-layered structure having two or more layers. A hole transport material is preferably a material having high hole mobility which may accept holes from an anode or a hole injection layer and transfer the holes to a light emitting layer. In the present invention, the compound represented by the above-described Chemical Formula 1 may be included as a hole transport material. Furthermore, it is possible to further include other hole transport materials in addition to the compound represented by Chemical Formula 1, if necessary. Specific examples thereof include arylamine-based organic materials, carbazole-based compounds, conductive polymers, block copolymers having both conjugated portions and nonconjugated portions, and the like, but are not limited thereto. According to an exemplary embodiment of the present invention, the hole transport material is the ink composition or a cured product thereof.

The electron transport layer is a layer which accepts electrons from an electron injection layer and transports the electrons to a light emitting layer. An electron transport material is preferably a material having high electron mobility which may proficiently accept electrons from a cathode and transfer the electrons to a light emitting layer. Specific examples thereof include: Al complexes of 8-hydroxyquinoline; complexes including $Alq_3$; organic radical compounds; hydroxyflavone-metal complexes; and the like, but are not limited thereto. The electron transport layer may be used with any desired cathode material, as used according to the related art. In particular, an appropriate cathode material is a typical material which has a low work function, followed by an aluminum layer or a silver layer. Specific examples thereof include cesium, barium, calcium, ytterbium, and samarium, in each case followed by an aluminum layer or a silver layer.

The electron injection layer is a layer which accepts electrons from an electrode. It is preferred that an electron injection material is excellent in ability to transport electrons and has an effect of accepting electrons from the cathode and an excellent electron injection effect for a light emitting layer or a light emitting material. Further, the electron injection material is preferably a material which prevents excitons produced from a light emitting layer from moving to a hole injection layer and is excellent in ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, metal complex compounds, nitrogen-containing 5-membered ring derivatives, and the like, but are not limited thereto. Examples of the metal complex compounds include 8-hydroxyquinolinato lithium, bis(8-hydroxyquinolinato) zinc, bis(8-hydroxyquinolinato) copper, bis(8-hydroxyquinolinato) manganese, tris(8-hydroxyquinolinato) aluminum, tris(2-methyl-8-hydroxyquinolinato) aluminum, tris(8-hydroxyquinolinato) gallium, bis(10-hydroxybenzo[h]quinolinato) beryllium, bis(10-hydroxybenzo[h]quinolinato) zinc, bis(2-methyl-8-quinolinato) chlorogallium, bis(2-methyl-8-quinolinato) (o-cresolato) gallium, bis(2-methyl-8-quinolinato) (1-naphtholato) aluminum, bis(2-methyl-8-quinolinato) (2-naphtholato) gallium, and the like, but are not limited thereto.

The electron blocking layer is a layer which may improve the service life and efficiency of a device by preventing electrons injected from an electron injection layer from passing through a light emitting layer and entering a hole injection layer. The electron-blocking layer may be formed between the light emitting layer and the hole injection layer, or between the light emitting layer and the layer which simultaneously injects and transports holes using the compound of the above-described Chemical Formula 2.

The hole blocking layer is a layer which blocks holes from reaching a cathode, and may be generally formed under the same conditions as those of the electron injection layer. Specific examples of the hole blocking layer material include oxadiazole derivatives or triazole derivatives, phenanthroline derivatives, aluminum complexes, and the like, but are not limited thereto.

The hole injection and transport layer may include materials for the above-described hole injection layer and hole transport layer.

The electron injection and transport layer may include materials for the above-described electron injection layer and electron transport layer.

When the organic light emitting device includes a plurality of organic material layers, the organic material layers may be formed of the same material or different materials.

The organic light emitting device according to the present invention may be a top emission type, a bottom emission type, or a dual emission type according to the material to be used.

An exemplary embodiment of the present invention provides an electronic device including an organic light emitting device including the above-described ink composition or a cured product thereof, or including an organic material layer formed using the ink composition.

The electronic device may include all of the interlayer insulation film of a semiconductor device, a color filter, a black matrix, an overcoat, a column spacer, a passivation film, a buffer coating film, a multilayer printed board insulation film, a cover coat of a flexible copper-clad board, a solder resist film, an insulation film of OLED, a protection film of a thin-film transistor of a liquid crystal display device, an electrode protection film and a semiconductor protection film of an organic EL device, an OLED insulation film, an LCD insulation film, a semiconductor insulation film, a solar light module, a

touch panel, a display device such as a display panel, and the like, but is not limited thereto.

[Mode for Invention]

Hereinafter, the present invention will be described in detail with reference to Examples for specifically describing the present invention. However, the Examples according to the present invention may be modified into various different forms, and it should not be interpreted that the scope of the present invention is limited to the Examples to be described below. The Examples of the present invention are provided for more completely explaining the present invention to the person with ordinary skill in the art.

**<Preparation Examples of Compound>**

**Synthesis Example A-1. Preparation of Compound 1**

(1) Preparation of Intermediate 1-1

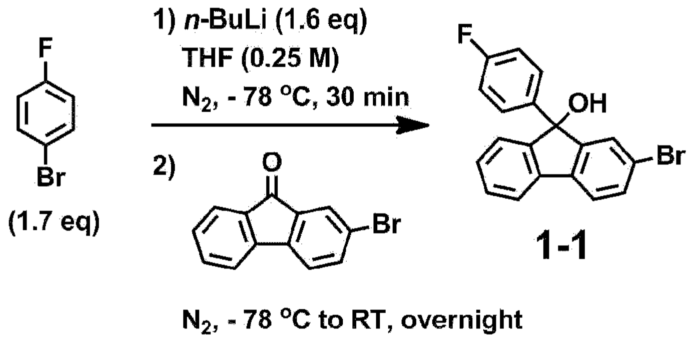


1-1

1-Bromo-4-fluorobenzene (27.9 mL, 255 mmol) was put into tetrahydrofuran (THF) (500 mL). After substitution with nitrogen, the mixture was cooled to -78°C. n-BuLi (2.5 M Hex) (96 mL, 240 mmol) was put into a dropping funnel and then slowly introduced into the reaction mixture. The resulting mixture was stirred at -78°C for 30 minutes. 2-Bromofluorenone (38.9 g, 150 mmol) was added thereto. The mixture was stirred overnight while being slowly warmed to room temperature. After the reaction was terminated by adding distilled water thereto, extraction was performed with ethyl acetate and water. After an organic layer was collected, the organic layer was dried using $MgSO_4$ and filtered. The filtrate was dried using a vacuum rotary concentrator to remove the organic solvent, and Intermediate 1-1 was secured and used in the next reaction.

(2) Preparation of Intermediate 1-2

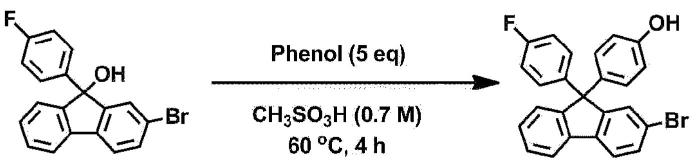


1-1 1-2

Intermediate 1-1 (53 g, 150 mmol) and phenol (70.6 g, 750 mmol) were put into a round bottom flask (RBF). After $CH_3SO_3H$ (214 mL) was added thereto, the resulting mixture was stirred at 60°C for 4 hours. After ice water was added thereto, extraction was performed with ethyl acetate and water. After an organic layer was collected, the organic layer was dried using $MgSO_4$ and filtered. The filtrate was dried using a vacuum rotary concentrator to remove the organic solvent. After column purification, crystallization was performed under dichloromethane (DCM)/heptane conditions to secure 40.6 g of Intermediate 1-2.

(3) Preparation of Intermediate 1-3

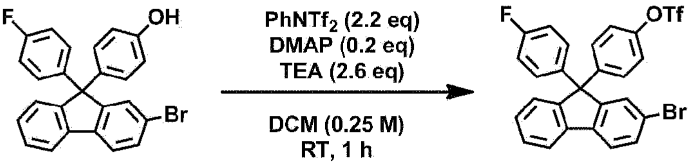


1-2 1-3

Intermediate 1-2 (45 g, 104 mmol), phenyl triflimide ($PhNTf_2$) (81.8 g, 229 mmol) and 4-dimethylaminopyridine (DMAP) (2.54 g, 21 mmol) were put into a round bottom flask. Dichloromethane (416 mL) and triethylamine (TEA) (37.7 mL, 270 mmol) were added thereto, and the resulting mixture was stirred at room temperature (RT) for 1 hour. Extraction was performed with dichloromethane and a 3% aqueous HCl solution, an organic layer was collected, and then the organic layer was dried using $MgSO_4$ and then filtered. The filtrate was dried using a vacuum rotary concentrator to remove the organic solvent. Crystallization was performed under dichloromethane/heptane conditions to prepare Intermediate 1-3 (52.5 g).

(4) Preparation of Intermediate 1-4

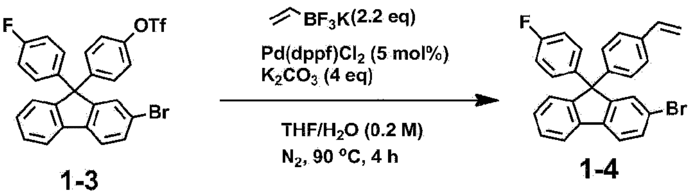


1-3 1-4

Intermediate 1-3 (52.2 g, 92.7 mmol), potassium vinyltrifluoroborate (27.3 g, 204 mmol), $Pd(dppf)Cl_2$ (3.4 g, 4.6 mmol) and $K_2CO_3$ (51.2 g, 971 mmol) were put into a round bottom flask. After substitution with nitrogen, THF (371 mL) and $H_2O$ (93 mL) were added thereto, and the resulting mixture was stirred at 90°C for 4 hours. Extraction was performed with ethyl acetate and water, an organic layer was collected, and then the organic layer was dried using $MgSO_4$ and then filtered. The filtrate was dried using a vacuum rotary concentrator to remove the organic solvent. After column purification, crystallization was performed under DCM/EtOH conditions to prepare Intermediate 1-4 (34 g).

(5) Preparation of Compound 1

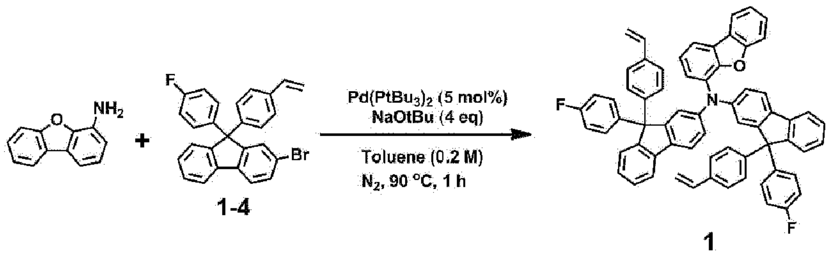


1

4-Aminodibenzofuran (513 mg, 2.8 mmol), Intermediate 1-4 (2.53 g, 5.74 mmol), $Pd(PtBu_3)_2$ (72 mg, 0.14 mmol) and NaOtBu (1.08 g, 11.2 mmol) were put into RBF. After substitution with nitrogen, toluene (14 mL) was added thereto, and the resulting mixture was stirred at 90°C for 1 hour. Extraction was performed with ethyl acetate and water, an organic layer was collected, and then the organic layer was dried using $MgSO_4$ and then filtered. The filtrate was dried using a vacuum rotary concentrator to remove the organic solvent. After column purification, crystallization was performed under DCM/EtOH conditions to obtain 2.0 g of Compound 1. The synthesis of the compound was confirmed through LC-MS and NMR. MS: [M+H]+ = 904

NMR measurement value of Compound 1: $^1$H NMR (500 MHz, DMSO-d6) δ 8.07 (dd, 1H), 7.92 (d, 1H), 7.63 (dd, 4H), 7.39 - 7.24 (m, 10H), 7.21 (dd, 1H), 7.10 (m, 2H), 6.97 - 6.83 (m, 18H), 6.72 (dd, 2H), 5.75 (d, 2H), 5.21 (d, 2H)

**Synthesis Example A-2. Preparation of Compound 2**

(1) Preparation of Intermediate 2-3

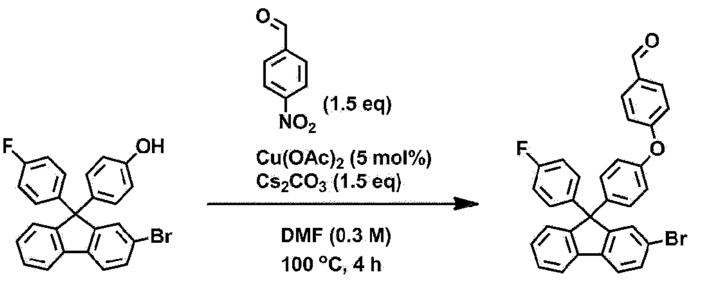


1-2 2-3

Intermediate 1-2 (40 g, 92.7 mmol), 4-nitrobenzaldehyde (21.2 g, 139 mmol), $Cu(OAc)_2$ (842 mg, 4.64 mmol) and $Cs_2CO_3$ (45.3 g, 139 mmol) were put into RBF. DMF (310 mL) was added thereto, and the resulting mixture was stirred at 100°C for 4 hours. Extraction was performed with ethyl acetate and water, an organic layer was collected, and then the organic layer was dried using $MgSO_4$ and then filtered. The filtrate was dried using a vacuum rotary concentrator to remove the organic solvent. After column purification, crystallization was performed under DCM/heptane conditions to secure 38.7 g of Intermediate 2-3.

(2) Preparation of Intermediate 2-4

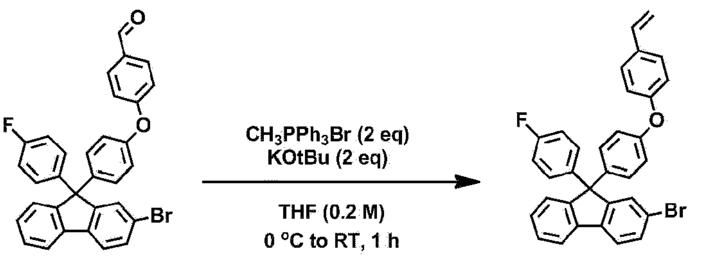


2-3 2-4

After $CH_3PPh_3Br$ (51.6 g, 144.6 mmol) , KOtBu (16.2 g, 144.6 mmol) and THF (217 mL) were put into RBF, the resulting mixture was cooled to 0°C. A solution of Intermediate 2-3 (38.7 g, 72.3 mmol) dissolved in THF (144 mL) was added to the reaction mixture. The resulting mixture was stirred for 1 hour while being warmed to room temperature. Extraction was performed with ethyl acetate and water, an organic layer was collected, and then the organic layer was dried using $MgSO_4$ and then filtered. The filtrate was dried using a vacuum rotary concentrator to remove the organic solvent. After column purification, crystallization was performed under DCM/EtOH conditions to secure 33.7 g of Intermediate 2-4.

(3) Preparation of Compound 2

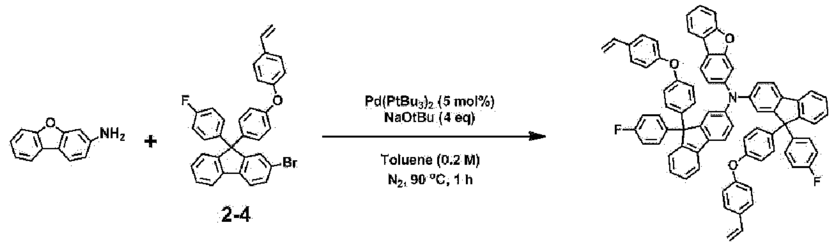


2-4

2

3-Aminodibenzofuran (513 mg, 2.8 mmol), Intermediate 2-4 (3.1 g, 5.74 mmol) , $Pd(PtBu_3)_2$ (72 mg, 0.14 mmol) and NaOtBu (1.08 g, 11.2 mmol) were put into RBF. After substitution with nitrogen, toluene (14 mL) was added thereto, and the resulting mixture was stirred at 90°C for 1 hour. Extraction was performed with ethyl acetate and water, an organic layer was collected, and then the organic layer was dried using $MgSO_4$ and then filtered. The filtrate was dried using a vacuum rotary concentrator to remove the organic solvent. After column purification, crystallization was performed under DCM/EtOH conditions to secure 2.9 g of Compound 2. The synthesis of the compound was confirmed through LC-MS and NMR. MS: [M+H]+ = 1088

NMR measurement value of Compound 2: $^1$H NMR (500 MHz, DMSO-d6) δ 8.11 (s, 1H), 8.07 (dd, 1H), 7.85 (d, 1H), 7.82 (dd, 4H), 7.46 (dd, 4H), 7.39 - 7.20 (m, 10H), 7.08 (m, 2H), 6.97 - 6.83 (m, 18H), 6.72 (dd, 2H), 6.65 (d, 4H), 5.75 (d, 2H), 5.21 (d, 2H)

**Synthesis Example A-3. Preparation of Compound 3**

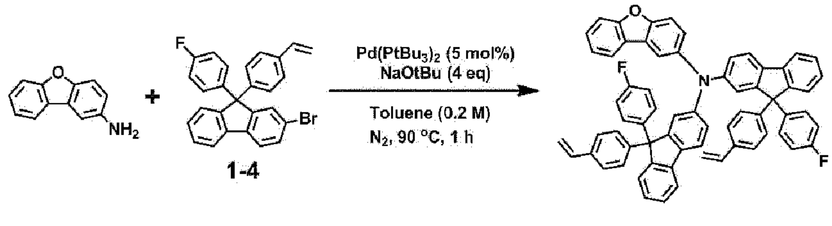


1-4

3

Compound 3 was prepared in the same manner as in Synthesis Example A-1, except that 2-aminodibenzofuran was used instead of 4-aminodibenzofuran in (5) of Synthesis Example A-1. The synthesis of the compound was confirmed through LC-MS and NMR. MS: [M+H]+ = 904

NMR measurement value of Compound 3: $^1$H NMR (500 MHz, DMSO-d6) δ 8.08 (dd, 1H), 7.92 (d, 1H), 7.63 (dd, 4H), 7.39 - 7.24 (m, 10H), 7.21 (dd, 1H), 7.10 (m, 2H), 6.97 - 6.83 (m, 18H), 6.72 (dd, 2H), 5.75 (d, 2H), 5.21 (d, 2H)

Synthesis Example A-4. Preparation of Compound 4

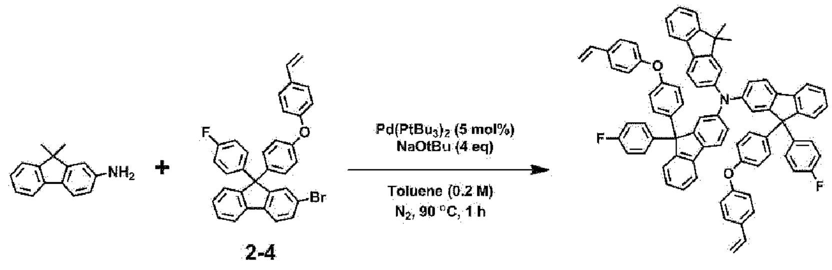


2-4

4

2-Amino-9,9-dimethylfluorene (586 mg, 2.8 mmol), Intermediate 2-4 (3.1 g, 5.74 mmol), $Pd(PtBu_3)_2$ (72 mg, 0.14 mmol) and NaOtBu (1.08 g, 11.2 mmol) were put into RBF. After substitution with nitrogen, toluene (14 mL) was added thereto, and the resulting mixture was stirred at 90°C for 1 hour. Extraction was performed with ethyl acetate and water, an

organic layer was collected, and then the organic layer was dried using $MgSO_4$ and then filtered. The filtrate was dried using a vacuum rotary concentrator to remove the organic solvent. After column purification, crystallization was performed under DCM/EtOH conditions to secure 2.6 g of Compound 4. The synthesis of the compound was confirmed through LC-MS and NMR. MS: [M+H]+ = 1114

NMR measurement value of Compound 4: $^1$H NMR (500 MHz, DMSO-d6) δ 7.95 (dd, 1H), 7.90 (dd, 1H), 7.84 (dd, 4H), 7.46 (dd, 4H), 7.39 - 7.20 (m, 11H), 7.08 (m, 2H), 6.97 - 6.83 (m, 18H), 6.72 (dd, 2H), 6.65 (d, 4H), 5.75 (d, 2H), 5.21 (d, 2H), 1.68 (s, 6H)

**Synthesis Example A-5. Preparation of Compound 5**

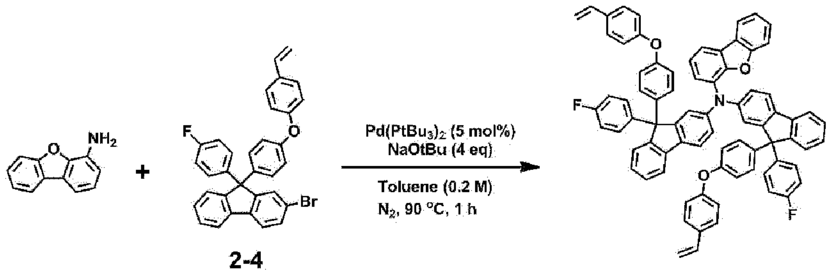


2-4

5

Compound 5 was prepared in the same manner as in Synthesis Example A-2, except that 4-aminodibenzofuran was used instead of 3-aminodibenzofuran in (3) of Synthesis Example A-2. The synthesis of the compound was confirmed through LC-MS and NMR. MS: [M+H]+ = 1088
NMR measurement value of Compound 5: $^1$H NMR (500 MHz, DMSO-d6) δ 8.07 (dd, 1H), 7.92 (d, 1H), 7.82 (dd, 4H), 7.46 (dd, 4H), 7.39 - 7.24 (m, 10H), 7.21 (dd, 1H), 7.10 (m, 2H), 6.97 - 6.83 (m, 18H), 6.72 (dd, 2H), 6.65 (d, 4H), 5.75 (d, 2H), 5.21 (d, 2H)

<Preparation Examples of Ionic Compound>

Ionic Compound Preparation Example 1. Preparation Example of Compound D-1

(1) Preparation of Intermediate D-1-1

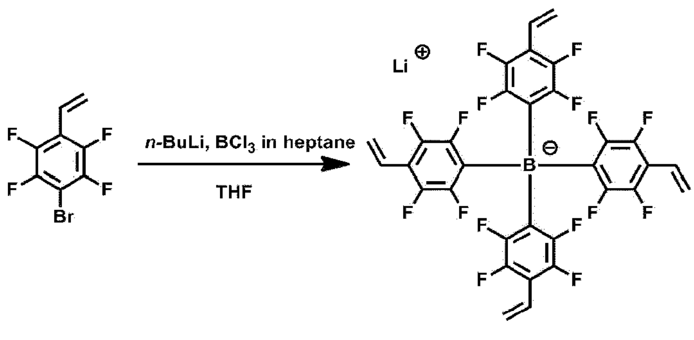


1-Bromo-2,3,5,6-tetrafluoro-4-vinylbenzene (2 g, 7.84 mmol) was put into THF (20 mL) in a 50-mL round bottom flask, and the resulting solution was stirred at -78°C for 30 minutes. n-BuLi in hexane (3.45 mL, 8.63 mmol, 2.5 M) was slowly added to the solution, and the resulting mixture was stirred at -78°C for 30 minutes. $BCl_3$ (2.6 mL, 2.61 mmol, 1 M in heptane solution) was added to the reaction solution at -78°C over 15 minutes. The resulting solution was slowly warmed to room temperature, the reaction solution was stirred overnight, and then water (30 mL) was added thereto. After the synthetic material was extracted with ethyl acetate (three times with 10 mL), the solvent was thoroughly removed. Water was completely removed by means of a Dean-stark using benzene, and the solid was filtered to prepare Intermediate D-1-1 (800 mg, yield 43%).

(2) Preparation of Compound D-1

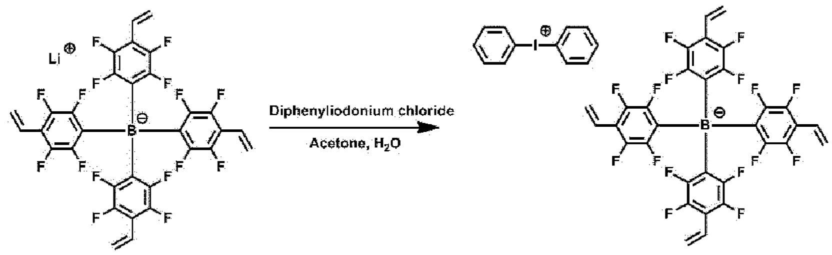


Intermediate D-1-1 (400 mg, 0.56 mmol), diphenyliodonium chloride (176 mg, 0.56 mmol), water (10 mL), and acetone (10 mL) were put into a 25-mL round bottom flask and stirred vigorously for 30 minutes. Extraction was performed using dichloromethane (three times with 10 mL) to remove the solvent, and the residue was dried to prepare Compound D-1 (Dopant 1) (552 mg, yield 100%).

MS: $[M-H]^-$ = 711 (negative mode)
MS: $[M+H]^+$ = 281 (positive mode)

**Ionic Compound Preparation Example 2. Preparation Example of Compound D-2**

(1) Preparation of Intermediate D-2-1

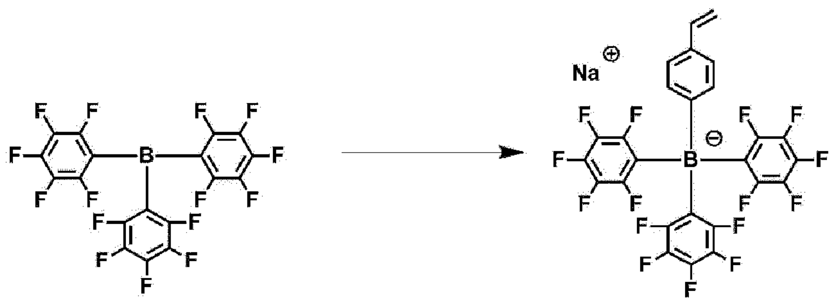

Mg (193 mg, 7.92 mmol), $I_2$ (4 mg), and THF (10 mL) were put into a 100-mL round bottom flask under a nitrogen atmosphere, and stirred for 30 minutes. 4-bromostyrene (1.04 mL, 7.92 mmol) was put thereinto, a water bath at 30 °C was placed under the round bottom flask, and the resulting mixture was stirred overnight. It was confirmed that the reaction solution had become black and Mg had been dissolved therein. The reaction solution was diluted by adding ether (5 mL) thereto. Tris(pentafluorophenyl)borane (1 g, 3.96 mmol) was dissolved in ether (5 mL), and the resulting solution was slowly added to the reaction solution for 30 minutes. The solution was stirred overnight. $Na_2CO_3$ (0.1 M, 80 mL, 8.0 mmol) was slowly added to the reaction solution. The organic solvent was extracted using ethyl acetate (three times with 20 mL), and the remaining water was removed over $MgSO_4$. Additionally, distillation was conducted with benzene by using a Dean-stark in order to remove the remaining water and impurities. When about 10 mL of the solvent remained, the solution was cooled and filtered to prepare Intermediate D-2-1 (1.6 g, yield 64%).

(2) Preparation of Compound D-2

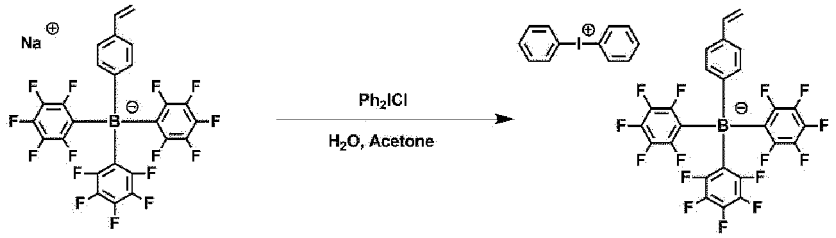


Intermediate D-2-1 (100 mg, 0.16 mmol), distilled water (10 mL) and $Ph_2ICl$ (60 mg, 0.19 mmol) were put into a 25-mL round bottom flask and stirred for 1 hour. A precipitate was produced by adding acetone (15 mL) to the reaction solution, and the precipitate was filtered and dried to prepare Compound D-2 (Dopant 2) (140 mg, yield 100%).

MS: $[M-H]^-$ = 615 (negative mode)
MS: $[M+H]^+$ = 281 (positive mode)

**<Preparation Examples of Ink Composition>**

**Ink Composition Preparation Example 1.**

Hole injection layer host 1 and hole injection layer dopant 1 were mixed at a weight ratio of 8:2. Thereafter, a mixture of the hole injection layer host 1 and the hole injection layer dopant 1 was added at a concentration of 2.0 wt% to a solvent in which 3-isopropylbiphenyl that is a solvent represented by Chemical Formula A, benzyl butyrate that is a first additional solvent, and dipropylene glycol monomethyl ether that is a second additional solvent were included and mixed in an amount of 10 wt%, 15 wt% and 75 wt%, respectively. The mixed and prepared solution was stirred for 24 hours and completely dissolved to prepare Ink Composition 1.

**Ink Composition Preparation Examples 2 to 63.**

Ink Compositions 2 to 63 were prepared in the same manner as in Ink Composition Preparation Example 1, except that materials shown in the following Table 1 were used as the hole injection layer host 1, the hole injection layer dopant 1, the solvent represented by Chemical Formula A, the first additional solvent and the second additional solvent.

**Ink Composition Preparation Example 64.**

Hole injection layer host 1 and hole injection layer dopant 1 were mixed at a weight ratio of 8:2. Thereafter, a mixture of the hole injection layer host 1 and the hole injection layer dopant 1 was added at a concentration of 2.0 wt% to a solvent in which 3-isopropylbiphenyl that is a solvent represented by Chemical Formula A and benzyl butyrate that is a first additional solvent were included and mixed in an amount of 20 wt% and 80 wt%, respectively. The mixed and prepared solution was stirred for 24 hours and completely dissolved to prepare Ink Composition 64.

**Ink Composition Preparation Examples 65 to 75.**

Ink Compositions 65 to 75 were prepared in the same manner as in Ink Composition Preparation Example 64, except that materials shown in the following Table 1 were used as the hole injection layer host 1, the hole injection layer dopant 1, the solvent, and the first additional solvent.

**HIL HOST (Hole injection layer host)**

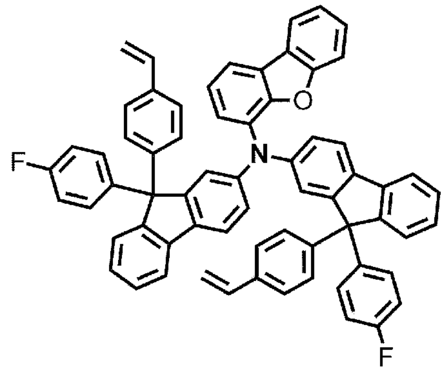

Host 1

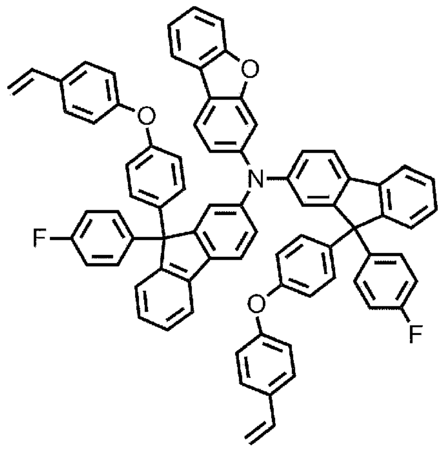

Host 2

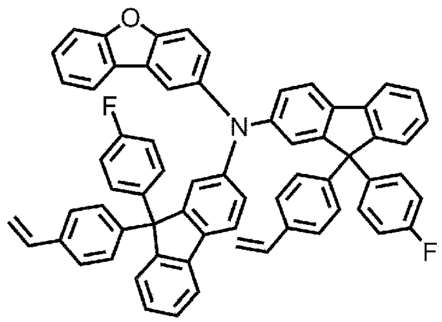

Host 3

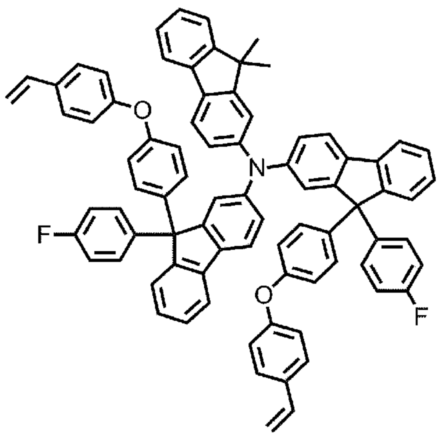

Host 4

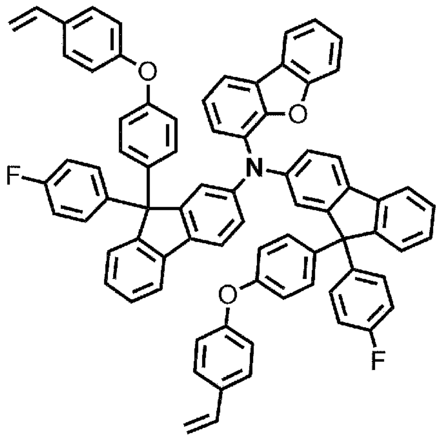

Host 5

HIL DOPANT (Hole injection layer dopant)

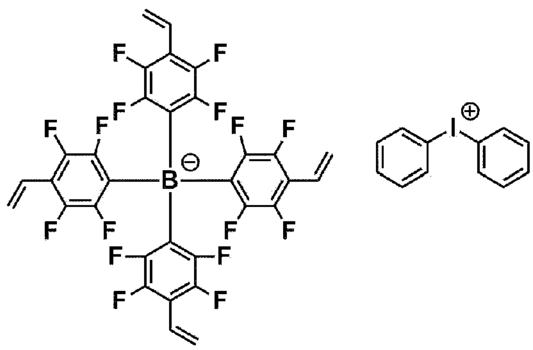

Dopant 1

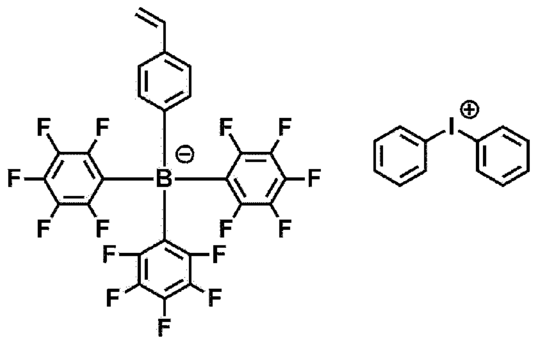

Dopant 2

[Table 1]

| Ink composition | HIL HOST | HIL DOPANT | Solvent | Second additional solvent | First additional solvent |
|---|---|---|---|---|---|
| 1 | Host 1 | Dopant 1 | 3-Isopropylbiphenyl | Dipropylene glycol monomethyl ether | Benzyl butyrate |
| 2 | Host 2 | Dopant 1 | 3-Isopropylbiphenyl | Dipropylene glycol monomethyl ether | Benzyl butyrate |
| 3 | Host 2 | Dopant 2 | 3-Isopropylbiphenyl | Dipropylene glycol monomethyl ether | Benzyl butyrate |
| 4 | Host 3 | Dopant 1 | 3-Isopropylbiphenyl | Dipropylene glycol monomethyl ether | Benzyl butyrate |
| 5 | Host 4 | Dopant 2 | 3-Isopropylbiphenyl | Dipropylene glycol monomethyl ether | Benzyl butyrate |
| 6 | Host 5 | Dopant 1 | 3-Isopropylbiphenyl | Dipropylene glycol monomethyl ether | Benzyl butyrate |
| 7 | Host 5 | Dopant 2 | 3-Isopropylbiphenyl | Dipropylene glycol monomethyl ether | Benzyl butyrate |
| 8 | Host 1 | Dopant 1 | 3-Ethylbiphenyl | Dipropylene glycol monomethyl ether | Benzyl butyrate |
| 9 | Host 2 | Dopant 1 | 3-Ethylbiphenyl | Dipropylene glycol monomethyl ether | Benzyl butyrate |
| 10 | Host 3 | Dopant 2 | 3-Ethylbiphenyl | Dipropylene glycol monomethyl ether | Benzyl butyrate |
| 11 | Host 5 | Dopant 1 | 3-Ethylbiphenyl | Dipropylene glycol monomethyl ether | Benzyl butyrate |

(continued)

| Ink composition | HIL HOST | HIL DOPANT | Solvent | Second additional solvent | First additional solvent |
|---|---|---|---|---|---|
| 12 | Host 5 | Dopant 2 | 3-Ethylbiphenyl | Dipropylene glycol monomethyl ether | Benzyl butyrate |
| 13 | Host 1 | Dopant 1 | 3-Methoxybiphenyl | Dipropylene glycol monomethyl ether | Benzyl butyrate |
| 14 | Host 2 | Dopant 1 | 3-Methoxybiphenyl | Dipropylene glycol monomethyl ether | Benzyl butyrate |
| 15 | Host 4 | Dopant 2 | 3-Methoxybiphenyl | Dipropylene glycol monomethyl ether | Benzyl butyrate |
| 16 | Host 5 | Dopant 1 | 3-Methoxybiphenyl | Dipropylene glycol monomethyl ether | Benzyl butyrate |
| 17 | Host 5 | Dopant 2 | 3-Methoxybiphenyl | Dipropylene glycol monomethyl ether | Benzyl butyrate |
| 18 | Host 1 | Dopant 1 | 4-Butylbiphenyl | Dipropylene glycol monomethyl ether | Benzyl butyrate |
| 19 | Host 1 | Dopant 2 | 4-Butylbiphenyl | Dipropylene glycol monomethyl ether | Benzyl butyrate |
| 20 | Host 2 | Dopant 1 | 4-Butylbiphenyl | Dipropylene glycol monomethyl ether | Benzyl butyrate |
| 21 | Host 2 | Dopant 2 | 4-Butylbiphenyl | Dipropylene glycol monomethyl ether | Benzyl butyrate |
| 22 | Host 3 | Dopant 1 | 4-Butylbiphenyl | Dipropylene glycol monomethyl ether | Benzyl butyrate |
| 23 | Host 4 | Dopant 1 | 4-Butylbiphenyl | Dipropylene glycol monomethyl ether | Benzyl butyrate |
| 24 | Host 5 | Dopant 1 | 4-Butylbiphenyl | Dipropylene glycol monomethyl ether | Benzyl butyrate |
| 25 | Host 5 | Dopant 2 | 4-Butylbiphenyl | Dipropylene glycol monomethyl ether | Benzyl butyrate |
| 26 | Host 1 | Dopant 1 | 4-Pentylbiphenyl | Dipropylene glycol monomethyl ether | Benzyl butyrate |
| 27 | Host 2 | Dopant 1 | 4-Pentylbiphenyl | Dipropylene glycol monomethyl ether | Benzyl butyrate |

(continued)

| Ink composition | HIL HOST | HIL DOPANT | Solvent | Second additional solvent | First additional solvent |
|---|---|---|---|---|---|
| 28 | Host 5 | Dopant 1 | 4-Pentylbiphenyl | Dipropylene glycol monomethyl ether | Benzyl butyrate |
| 29 | Host 1 | Dopant 1 | Tastromine | Dipropylene glycol monomethyl ether | Benzyl butyrate |
| 30 | Host 2 | Dopant 2 | Tastromine | Dipropylene glycol monomethyl ether | Benzyl butyrate |
| 31 | Host 3 | Dopant 2 | Tastromine | Dipropylene glycol monomethyl ether | Benzyl butyrate |
| 32 | Host 4 | Dopant 2 | Tastromine | Dipropylene glycol monomethyl ether | Benzyl butyrate |
| 33 | Host 5 | Dopant 1 | Tastromine | Dipropylene glycol monomethyl ether | Benzyl butyrate |
| 34 | Host 2 | Dopant 1 | DELTA-TRIDECANOLACTONE | Dipropylene glycol monomethyl ether | Benzyl butyrate |
| 35 | Host 3 | Dopant 1 | DELTA-TRIDECANOLACTONE | Dipropylene glycol monomethyl ether | Benzyl butyrate |
| 36 | Host 3 | Dopant 2 | DELTA-TRIDECANOLACTONE | Dipropylene glycol monomethyl ether | Benzyl butyrate |
| 37 | Host 4 | Dopant 1 | DELTA-TRIDECANOLACTONE | Dipropylene glycol monomethyl ether | Benzyl butyrate |
| 38 | Host 5 | Dopant 1 | DELTA-TRIDECANOLACTONE | Dipropylene glycol monomethyl ether | Benzyl butyrate |
| 39 | Host 5 | Dopant 2 | DELTA-TRIDECANOLACTONE | Dipropylene glycol monomethyl ether | Benzyl butyrate |
| 40 | Host 1 | Dopant 1 | 1-Nonylimidazole | Dipropylene glycol monomethyl ether | Benzyl butyrate |
| 41 | Host 2 | Dopant 1 | 1-Nonylimidazole | Dipropylene glycol monomethyl ether | Benzyl butyrate |
| 42 | Host 2 | Dopant 2 | 1-Nonylimidazole | Dipropylene glycol monomethyl ether | Benzyl butyrate |
| 43 | Host 3 | Dopant 1 | 1-Nonylimidazole | Dipropylene glycol monomethyl ether | Benzyl butyrate |

(continued)

| Ink composition | HIL HOST | HIL DOPANT | Solvent | Second additional solvent | First additional solvent |
|---|---|---|---|---|---|
| 44 | Host 4 | Dopant 1 | 1-Nonylimidazole | Dipropylene glycol monomethyl ether | Benzyl butyrate |
| 45 | Host 4 | Dopant 2 | 1-Nonylimidazole | Dipropylene glycol monomethyl ether | Benzyl butyrate |
| 46 | Host 5 | Dopant 1 | 1-Nonylimidazole | Dipropylene glycol monomethyl ether | Benzyl butyrate |
| 47 | Host 5 | Dopant 2 | 1-Nonylimidazole | Dipropylene glycol monomethyl ether | Benzyl butyrate |
| 48 | Host 1 | Dopant 1 | N-Isopentyl-N-phenylpro-pionamid e | Dipropylene gly-col monomethyl ether | Benzyl butyrate |
| 49 | Host 2 | Dopant 1 | N-Isopentyl-N-phenylpro-pionamid e | Dipropylene gly-col monomethyl ether | Benzyl butyrate |
| 50 | Host 2 | Dopant 2 | N-Isopentyl-N-phenylpro-pionamid e | Dipropylene gly-col monomethyl ether | Benzyl butyrate |
| 51 | Host 3 | Dopant 1 | N-Isopentyl-N-phenylpro-pionamid e | Dipropylene gly-col monomethyl ether | Benzyl butyrate |
| 52 | Host 3 | Dopant 2 | N-Isopentyl-N-phenylpro-pionamid e | Dipropylene gly-col monomethyl ether | Benzyl butyrate |
| 53 | Host 4 | Dopant 1 | N-Isopentyl-N-phenylpro-pionamid e | Dipropylene gly-col monomethyl ether | Benzyl butyrate |
| 54 | Host 4 | Dopant 2 | N-Isopentyl-N-phenylpro-pionamid e | Dipropylene gly-col monomethyl ether | Benzyl butyrate |
| 55 | Host 5 | Dopant 1 | N-Isopentyl-N-phenylpro-pionamid e | Dipropylene gly-col monomethyl ether | Benzyl butyrate |
| 56 | Host 5 | Dopant 2 | N-Isopentyl-N-phenylpro-pionamid e | Dipropylene gly-col monomethyl ether | Benzyl butyrate |
| 57 | Host 2 | Dopant 1 | 3-Isopropylbiphenyl | Dipropylene glycol monomethyl ether | Butyl benzoate |
| 58 | Host 3 | Dopant 2 | 3-Methoxybiphenyl | Dipropylene glycol monomethyl ether | Butyl benzoate |
| 59 | Host 2 | Dopant 1 | 4-Butylbiphenyl | Dipropylene glycol monomethyl ether | Butyl benzoate |

(continued)

| Ink composition | HIL HOST | HIL DOPANT | Solvent | Second additional solvent | First additional solvent |
|---|---|---|---|---|---|
| 60 | Host 5 | Dopant 1 | 4-Butylbiphenyl | Dipropylene glycol monomethyl ether | Butyl benzoate |
| 61 | Host 5 | Dopant 1 | 4-Pentylbiphenyl | Dipropylene glycol monomethyl ether | Butyl benzoate |
| 62 | Host 3 | Dopant 1 | Benzyl benzoate | Dipropylene glycol monomethyl ether | Butyl benzoate |
| 63 | Host 5 | Dopant 2 | 1-Methoxynaphthalen e | Dipropylene glycol monomethyl ether | Butyl benzoate |
| 64 | Host 1 | Dopant 1 | 3-Ethylbiphenyl | - | Benzyl butyrate |
| 65 | Host 3 | Dopant 2 | 3-Ethylbiphenyl | - | Butyl benzoate |
| 66 | Host 5 | Dopant 1 | 3-Ethylbiphenyl | - | Butyl benzoate |
| 67 | Host 2 | Dopant 1 | 4-Butylbiphenyl | - | Butyl benzoate |
| 68 | Host 4 | Dopant 1 | 4-Butylbiphenyl | - | Benzyl butyrate |
| 69 | Host 5 | Dopant 1 | 4-Butylbiphenyl | - | Butyl benzoate |
| 70 | Host 2 | Dopant 1 | 4-Butylbiphenyl | - | Butyl benzoate |
| 71 | Host 3 | Dopant 2 | 4-Pentylbiphenyl | - | Ethyl 4-methyl benzoate |
| 72 | Host 4 | Dopant 2 | 4-Pentylbiphenyl | - | Butyl benzoate |
| 73 | Host 2 | Dopant 1 | 4-Pentylbiphenyl | - | Butyl benzoate |
| 74 | Host 5 | Dopant 1 | Benzyl benzoate | - | Butyl benzoate |
| 75 | Host 2 | Dopant 1 | Benzyl benzoate | - | Butyl benzoate |

As the solvents used in Ink Compositions 29 to 56, tastromine (CAS# 91-46-3), DELTA-TRIDECANOLACTONE (CAS# 7370-92-5), 1-Nonylimidazole (CAS# 53657-08-2) and N-Isopentyl-N-phenylpropionamide (CAS# 63916-02-9) were each used.

**<Experimental Example A>**

**Example 1.**

After Ink Composition 1 was printed on an ITO substrate having banks formed thereon by an inkjet method, the printed substrate was put into a vacuum chamber, and the pressure was reduced from normal pressure to $2\times10^{-2}$ torr for 60 seconds and from $2\times10^{-2}$ torr to $2x10^{-6}$ torr for 180 seconds, and drying was performed at room temperature. After drying, the thin film was cured by performing a heat treatment under the conditions of $N_2$ atmosphere and hot plate 230°C for 1 hour. The thicknesses of pixels included in the cured thin film was measured using an optical profiler (model name: ContourGT-I) manufactured by Bruker, which is an optical thickness measuring device, and based on the measured thicknesses of the pixels, the thickness uniformity V between pixels was derived through the following Equation 1'. The derived values are shown in the following Table 2.

[Equation 1']

$$V = \frac{\sum_{i=2}^{n'}\left(X(i)-Xm'\right)^2}{n' \times Xm'}$$

In Equation 1',

X(i) is the thickness of the i-th pixel,
Xm' is the average value of X(i) in which i is 2 to 8, and
n' is an integer from 2 to 8.

The ITO substrate on which the bank is formed in Example 1 corresponds to FIG. 2, the printing on the ITO substrate on which the bank is formed corresponds to FIG. 3, and the cured thin film corresponds to FIG. 4.

**Examples 2 to 43 and Comparative Examples 1 to 32.**

Except that the ink compositions shown in the following Table 2 were used instead of Ink Composition 1, preparation was performed in the same manner as in Example 1, and the thickness uniformity V was derived and shown in the following Table 2.

[Table 2]

| | Ink composition | Thickness uniformity V |
|---|---|---|
| Example 1 | 1 | 0.20 |
| Example 2 | 2 | 0.39 |
| Example 3 | 3 | 0.26 |
| Example 4 | 4 | 0.04 |
| Example 5 | 5 | 0.15 |
| Example 6 | 6 | 0.13 |
| Example 7 | 7 | 0.10 |
| Example 8 | 8 | 0.18 |
| Example 9 | 9 | 0.19 |
| Example 10 | 10 | 0.40 |
| Example 11 | 11 | 0.10 |
| Example 12 | 12 | 0.37 |
| Example 13 | 13 | 0.07 |
| Example 14 | 14 | 0.09 |
| Example 15 | 15 | 0.19 |
| Example 16 | 16 | 0.21 |
| Example 17 | 17 | 0.39 |
| Example 18 | 18 | 0.31 |
| Example 19 | 19 | 0.27 |
| Example 20 | 20 | 0.18 |
| Example 21 | 21 | 0.23 |
| Example 22 | 22 | 0.39 |

(continued)

| | Ink composition | Thickness uniformity V |
|---|---|---|
| Example 23 | 23 | 0.11 |
| Example 24 | 24 | 0.13 |
| Example 25 | 25 | 0.20 |
| Example 26 | 26 | 0.27 |
| Example 27 | 27 | 0.15 |
| Example 28 | 28 | 0.17 |
| Example 29 | 57 | 0.13 |
| Example 30 | 58 | 0.17 |
| Example 31 | 59 | 0.15 |
| Example 32 | 60 | 0.20 |
| Example 33 | 61 | 0.11 |
| Example 34 | 64 | 0.37 |
| Example 35 | 65 | 0.23 |
| Example 36 | 66 | 0.26 |
| Example 37 | 67 | 0.18 |
| Example 38 | 68 | 0.29 |
| Example 39 | 69 | 0.15 |
| Example 40 | 70 | 0.07 |
| Example 41 | 71 | 0.24 |
| Example 42 | 72 | 0.31 |
| Example 43 | 73 | 0.21 |
| Comparative Example 1 | 29 | 1.15 |
| Comparative Example 2 | 30 | 0.73 |
| Comparative Example 3 | 31 | 1.54 |
| Comparative Example 4 | 32 | 1.66 |
| Comparative Example 5 | 33 | 0.84 |
| Comparative Example 6 | 34 | 1.26 |
| Comparative Example 7 | 35 | 0.75 |
| Comparative Example 8 | 36 | 1.10 |
| Comparative Example 9 | 37 | 1.71 |
| Comparative Example 10 | 38 | 0.67 |
| Comparative Example 11 | 39 | 0.89 |
| Comparative Example 12 | 40 | 0.81 |
| Comparative Example 13 | 41 | 1.13 |
| Comparative Example 14 | 42 | 1.48 |
| Comparative Example 15 | 43 | 0.70 |
| Comparative Example 16 | 44 | 0.73 |
| Comparative Example 17 | 45 | 1.23 |
| Comparative Example 18 | 46 | 1.30 |

(continued)

| | Ink composition | Thickness uniformity V |
|---|---|---|
| Comparative Example 19 | 47 | 1.09 |
| Comparative Example 20 | 48 | 0.82 |
| Comparative Example 21 | 49 | 0.62 |
| Comparative Example 22 | 50 | 0.71 |
| Comparative Example 23 | 51 | 0.89 |
| Comparative Example 24 | 52 | 0.82 |
| Comparative Example 25 | 53 | 1.36 |
| Comparative Example 26 | 54 | 1.15 |
| Comparative Example 27 | 55 | 0.62 |
| Comparative Example 28 | 56 | 0.67 |
| Comparative Example 29 | 62 | 0.57 |
| Comparative Example 30 | 63 | 0.71 |
| Comparative Example 31 | 74 | 0.89 |
| Comparative Example 32 | 75 | 0.48 |

As shown in the contents of Table 2, it can be confirmed that Examples using the ink composition according to an exemplary embodiment of the present invention have a small value of thickness uniformity V of 0.4 or lower, Examples using particularly the solvent (specifically, butyl benzoate) of Chemical Formula C-1 as a first additional solvent while using two additional solvents have a very small value of thickness uniformity V of 0.2 or less, and Comparative Examples using ink compositions not corresponding to the present invention have a large value of thickness uniformity V of more than 0.4.

In this regard, FIGS. 5 to 16 illustrate thickness uniformity characteristics of pixels according to the Experimental Examples of the present application. Specifically, FIGS. 5, 6, 11 and 12 illustrate the thickness uniformity of pixels according to Examples 6, 16, 36 and 40 of the present application, respectively, FIGS. 7 to 10 and 13 to 16 illustrate the thickness uniformity of pixels according to Comparative Examples 5, 10, 15, 27, and 29 to 32 of the present application, respectively, the horizontal axis means the distance between pixels (um), and the vertical axis means the thickness (nm) of the pixel. As shown in the contents of FIGS. 5, 6, 11 and 12, it can be confirmed that Examples 6, 16, 36 and 40 using the ink composition according to an exemplary embodiment of the present invention have a pixel thickness uniformity V of 0.13, 0.21, 0.26, and 0.07, respectively, which are small values of 0.4 or lower, and thus have even thickness uniformity. Accordingly, it is possible to expect excellent light emitting and/or coloring effects by reducing factors that affect the interference condition of light emitted from the light emitting device according to the thickness because the ink composition is used in the organic material layer and the like in the organic light emitting device. Conversely, it can be confirmed that Comparative Examples 5, 10, 15, 27, and 29 to 32 using ink compositions not corresponding to the present invention have pixel thickness uniformity V of 0.84, 0.67, 0.70, 0.62, 0.57, 0.71, 0.89 and 0.48, respectively, which are values more than 0.4, the difference in thickness between pixels is large, and the thickness uniformity is unbalanced.

**<Experimental Example B>**

**Example B-1.**

A glass substrate which was thin-film coated to have a thickness of 700 Å of ITO (indiumtinoxide) and patterned with hydrophobic banks was washed with distilled water for 30 minutes and then dried on a hot plate at 230°C for 10 minutes. Ink Composition 1 in Table 1 was inkjet printed on a washed substrate patterned with banks, and heat-treated at 230°C for 30 minutes, thereby forming a hole injection layer having a thickness of 30 nm. A hole transport layer having a thickness of 40 nm was formed on the hole injection layer by inkjet printing an ink prepared by dissolving the following $\alpha$-NPD compound in cyclohexyl benzene at a concentration of 2 wt %. Thereafter, the glass substrate was transferred to a vacuum deposition machine, and then the following ADN compound and the following DPAVBi compound were vacuum-deposited on the hole transport layer at a weight ratio of 20:1 (ADN:DPAVBi) to have a thickness of 20 nm, thereby forming a light emitting layer. The following BCP compound was vacuum-deposited to have a thickness of 35 nm on the light emitting layer, thereby forming an electron injection and transport layer. A cathode was formed by depositing LiF and

aluminum to have a thickness of 1 nm and 100 nm, respectively, on the electron injection and transport layer, thereby manufacturing an organic light emitting device.

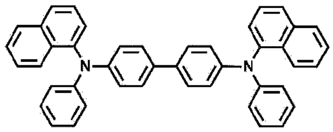

a-NPD

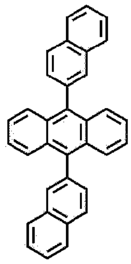

ADN

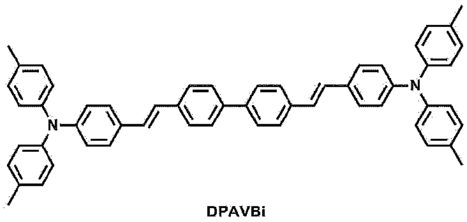

DPAVBi

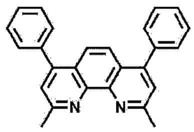

BCP

**Examples B-2 to B-43.**

Organic light emitting devices were manufactured using the ink composition in the following Table 3 instead of Ink Composition 1 in Example B-1.

[Table 3]

| | Ink composition |
|---|---|
| Example B-1 | 1 |
| Example B-2 | 2 |
| Example B-3 | 3 |
| Example B-4 | 4 |
| Example B-5 | 5 |
| Example B-6 | 6 |
| Example B-7 | 7 |
| Example B-8 | 8 |
| Example B-9 | 9 |
| Example B-10 | 10 |
| Example B-11 | 11 |
| Example B-12 | 12 |
| Example B-13 | 13 |
| Example B-14 | 14 |
| Example B-15 | 15 |
| Example B-16 | 16 |
| Example B-17 | 17 |
| Example B-18 | 18 |
| Example B-19 | 19 |
| Example B-20 | 20 |

(continued)

| | Ink composition |
|---|---|
| Example B-21 | 21 |
| Example B-22 | 22 |
| Example B-23 | 23 |
| Example B-24 | 24 |
| Example B-25 | 25 |
| Example B-26 | 26 |
| Example B-27 | 27 |
| Example B-28 | 28 |
| Example B-29 | 57 |
| Example B-30 | 58 |
| Example B-31 | 59 |
| Example B-32 | 60 |
| Example B-33 | 61 |
| Example B-34 | 64 |
| Example B-35 | 65 |
| Example B-36 | 66 |
| Example B-37 | 67 |
| Example B-38 | 68 |
| Example B-39 | 69 |
| Example B-40 | 70 |
| Example B-41 | 71 |
| Example B-42 | 72 |
| Example B-43 | 73 |

It was confirmed that the organic light emitting devices manufactured in Examples B-1 to B-43 were driven. Through this, it was confirmed that the ink compositions according to the exemplary embodiments of the present invention can be applied to an organic light emitting device.

## Claims

1. An ink composition comprising a compound represented by the following Chemical Formula 1 and a solvent represented by the following Chemical Formula A:

[Chemical Formula 1]

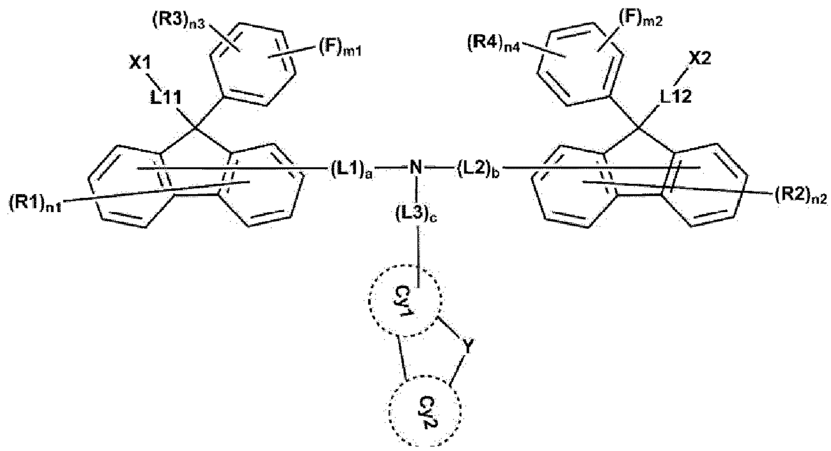

[Chemical Formula A]

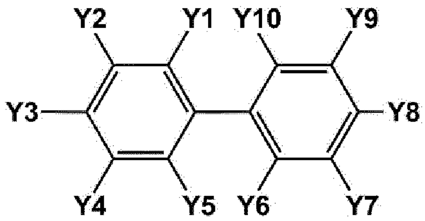

wherein, in Chemical Formulae 1 and A,
Y is O, S, CRaRb or SiRcRd,
Cy1 and Cy2 are the same as or different from each other, and are each independently a substituted or unsubstituted benzene ring; or a substituted or unsubstituted naphthalene ring,
Ra, Rb, Rc and Rd are the same as or different from each other, and are each independently hydrogen; deuterium; a substituted or unsubstituted alkyl group; a substituted or unsubstituted aryl group; or a substituted or unsubstituted heteroaryl group, or are bonded to an adjacent group to form a substituted or unsubstituted ring,
L1 to L3 are the same as or different from each other, and are each independently a direct bond; or a substituted or unsubstituted arylene group,
L11 and L12 are the same as or different from each other, and are each independently a direct bond; a substituted or unsubstituted alkylene group; or a substituted or unsubstituted arylene group,
X1 and X2 are the same as or different from each other, and are each independently a curable group,
R1 and R2 are the same as or different from each other, and are each independently deuterium; a halogen group; a substituted or unsubstituted alkyl group; a substituted or unsubstituted alkoxy group; a substituted or unsubstituted aryl group; or a substituted or unsubstituted heteroaryl group,
R3 and R4 are the same as or different from each other, and are each independently deuterium; a substituted or unsubstituted alkyl group; a substituted or unsubstituted alkoxy group; a substituted or unsubstituted aryl group; or a substituted or unsubstituted heteroaryl group,
a, b and c are each 0 or 1,
n1 and n2 are each an integer from 0 to 7, and when n1 and n2 are each 2 or higher, two or more substituents in the parenthesis are the same as or different from each other,
m1 and m2 are each an integer from 1 to 5, n3 and n4 are each an integer from 0 to 4, m1+n3 is 5 or less, and m2+n4 is 5 or less, and
Y1 to Y10 are the same as or different from each other, and are each independently hydrogen; deuterium; a hydroxyl group; an ether group; a carbonyl group; an ester group; a substituted or unsubstituted alkyl group; a substituted or unsubstituted cycloalkyl group; a substituted or unsubstituted cycloalkenyl group; a substituted or unsubstituted alkoxy group; a substituted or unsubstituted aryl group; or a substituted or unsubstituted amine group.

2. The ink composition of claim 1, wherein the curable group is any one selected from the group consisting of the

following structures:

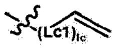
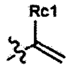
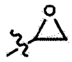
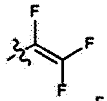
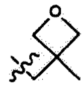
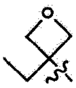
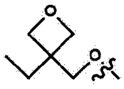
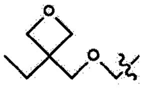
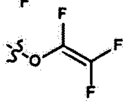
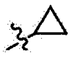
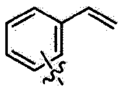
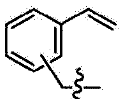
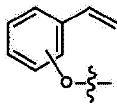
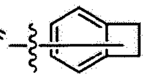
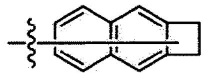
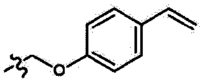
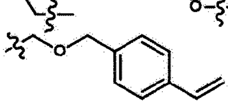
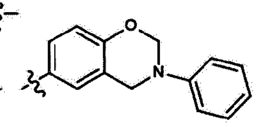
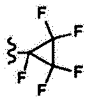
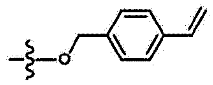
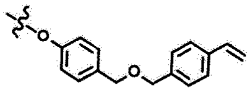
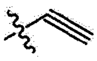
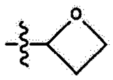
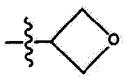
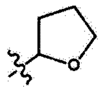
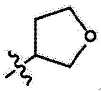
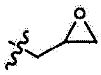
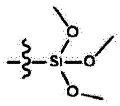
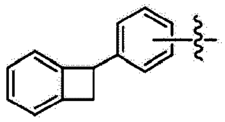
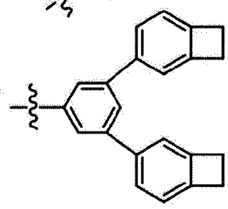
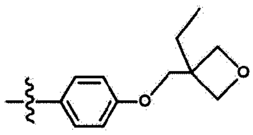
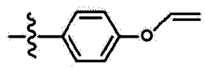
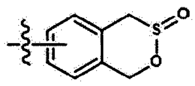
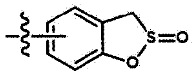

in the structures,
Lc1 is a direct bond; -O-; -S-; a substituted or unsubstituted alkylene group; a substituted or unsubstituted arylene group; or a substituted or unsubstituted heteroarylene group,
1c is 1 or 2,
when 1c is 2, Lc1's are the same as or different from each other,
Rc1 is a substituted or unsubstituted alkyl group, and

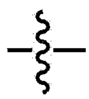

is a moiety bonded to Chemical Formula 1.

**3.** The ink composition of claim 1, wherein the solvent represented by Chemical Formula A is any one selected from the group consisting of the following compounds:

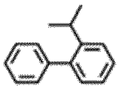 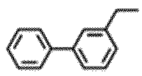 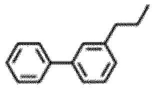 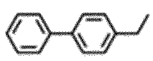

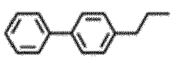 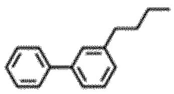 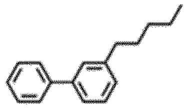 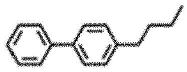

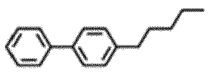 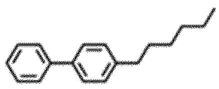 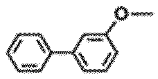 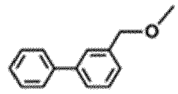

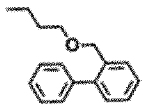 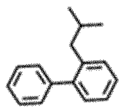 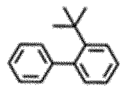

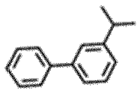 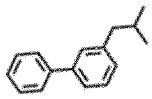 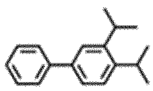 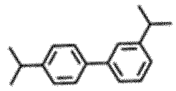

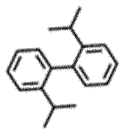 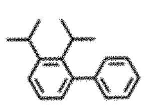 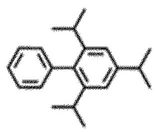 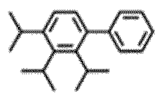

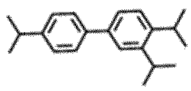 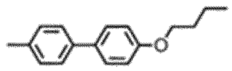

**4.** The ink composition of claim 1, wherein Chemical Formula 1 is represented by the following Chemical Formula 11:

[Chemical Formula 11]

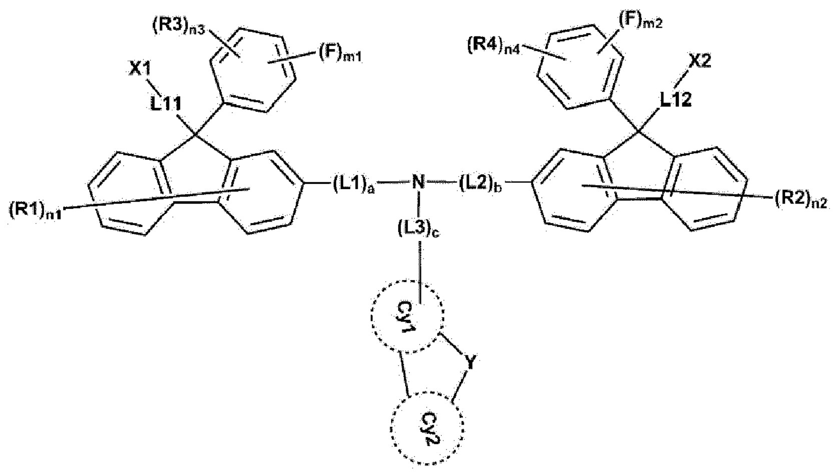

the definitions of L1 to L3, L11, L12, X1, X2, R1 to R4, Y, Cy1, Cy2, a, b, c, n1 to n4, m1 and m2 are the same as those in Chemical Formula 1.

**5.** The ink composition of claim 1, wherein Chemical Formula 1 is any one selected from the group consisting of the following compounds:

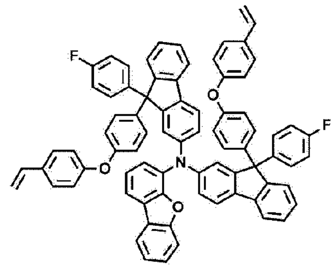
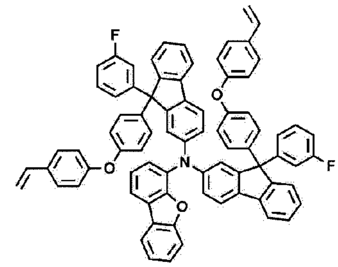
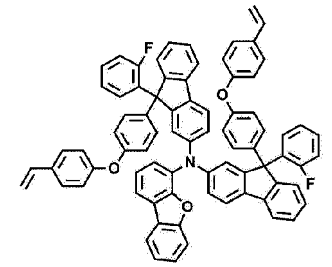

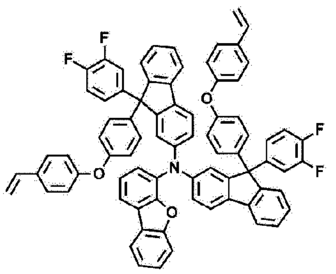
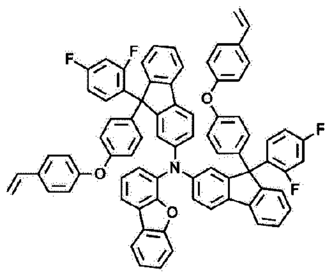
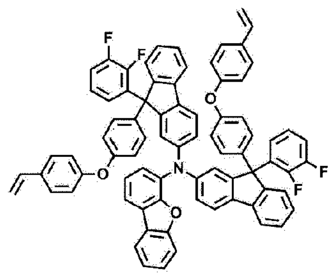

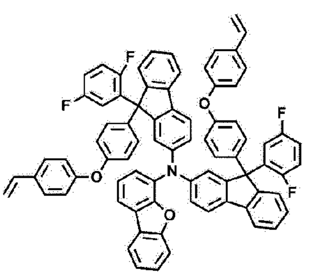
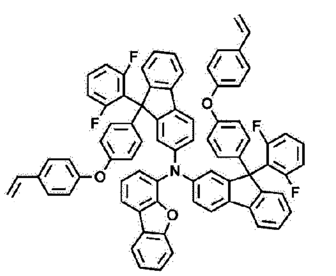
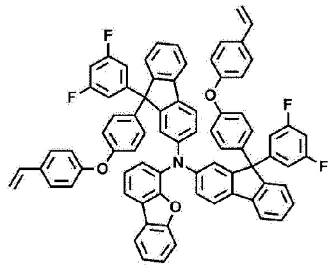

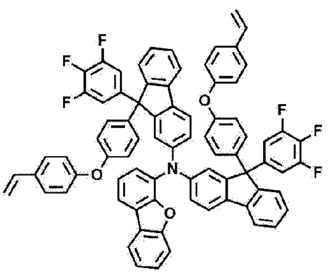
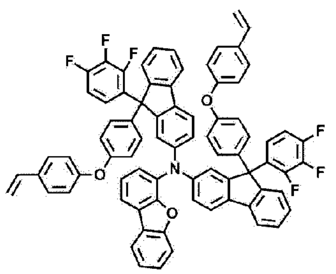
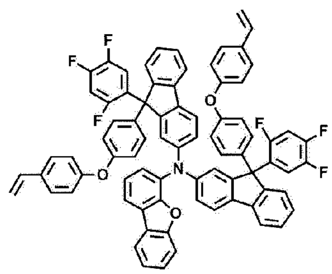

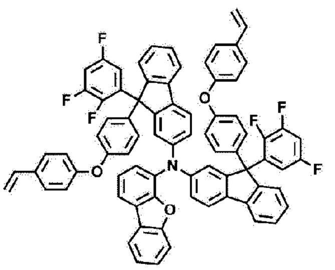
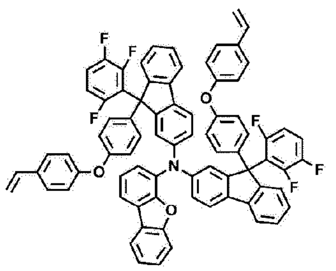
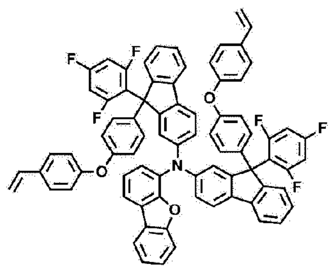

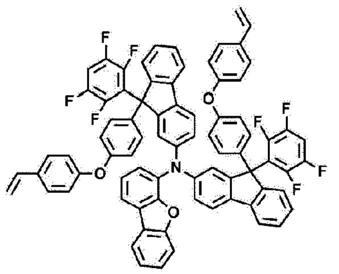
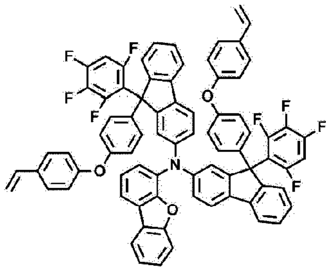
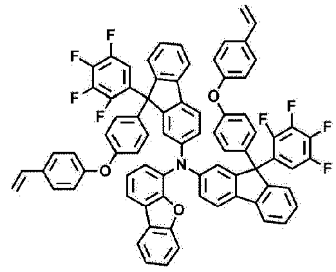

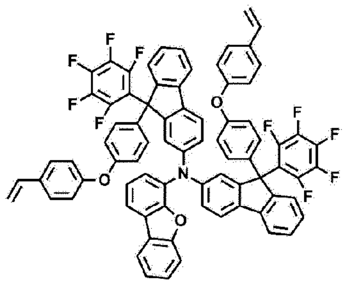
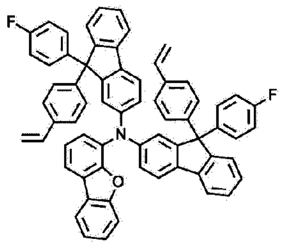
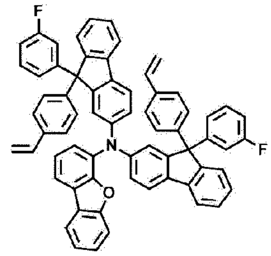

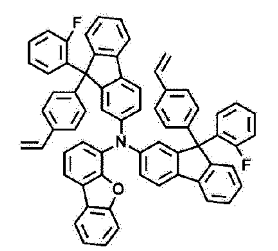
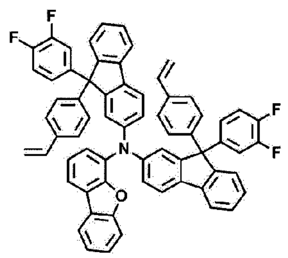
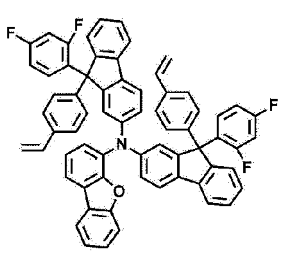

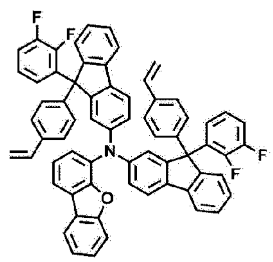
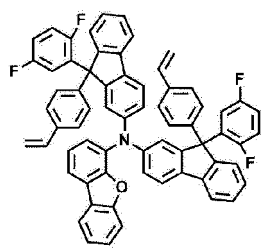
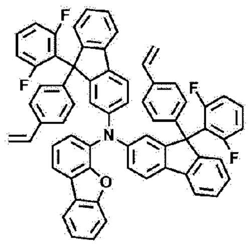

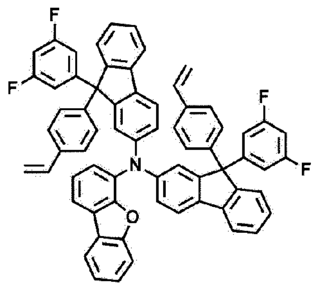
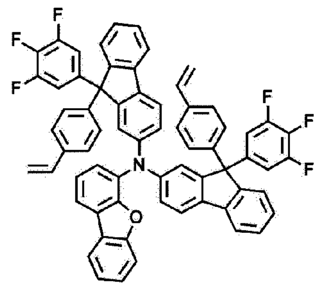
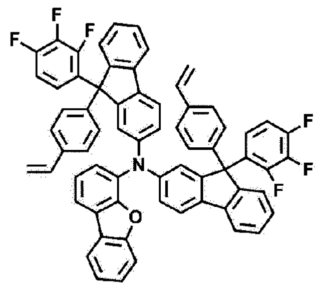

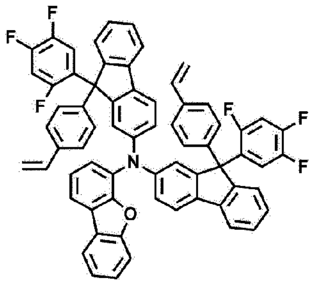
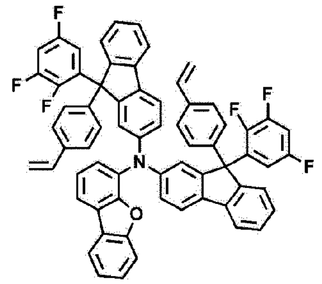
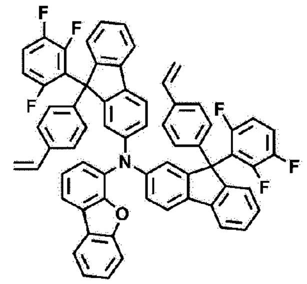

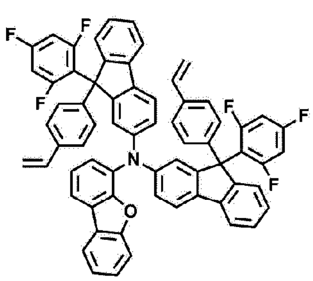
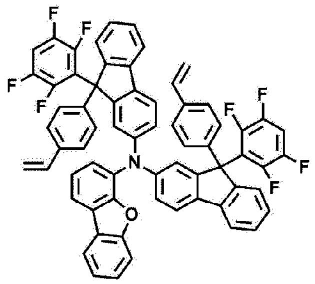
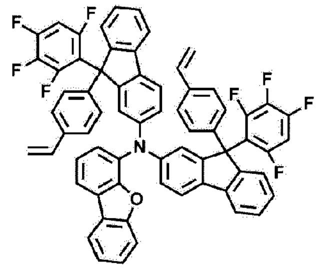

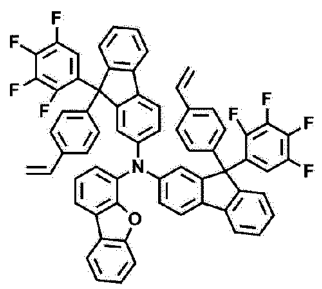
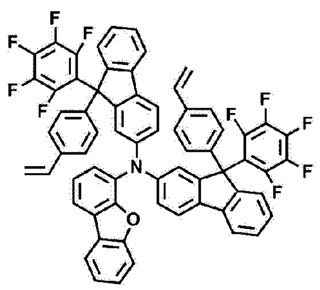

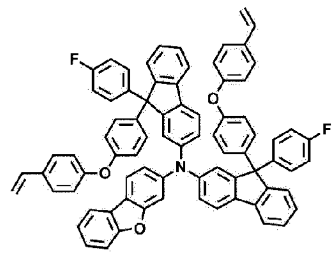
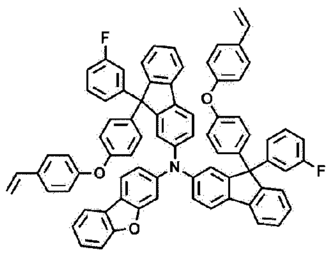
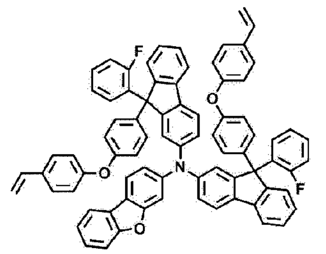

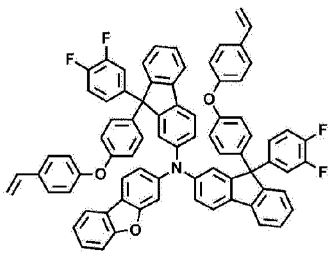
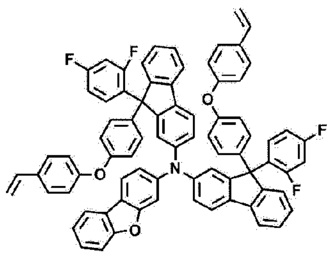
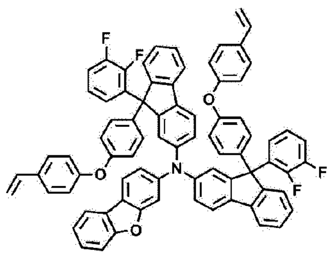

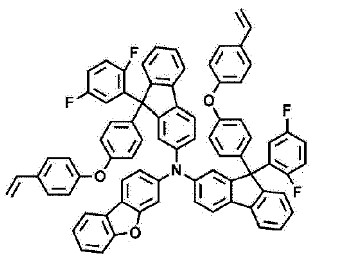
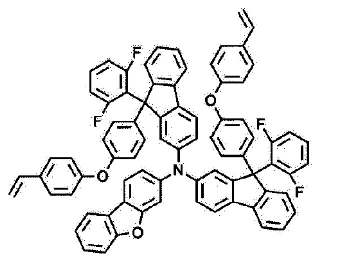
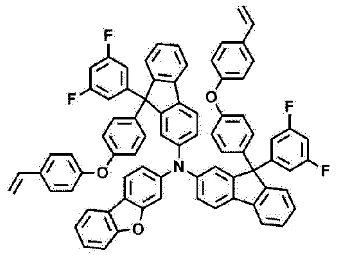

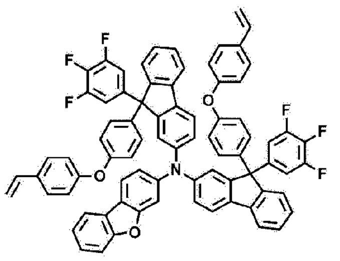
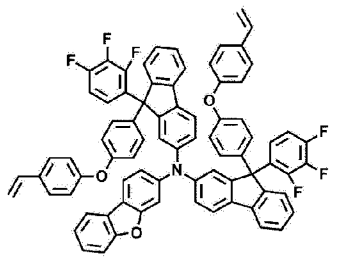
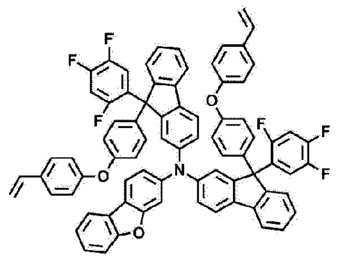

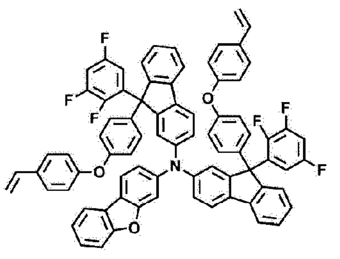
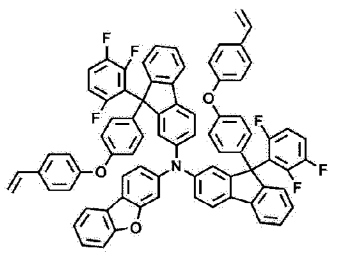
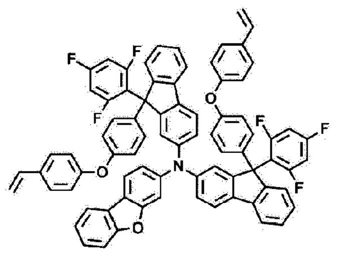

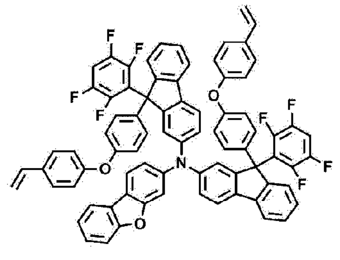
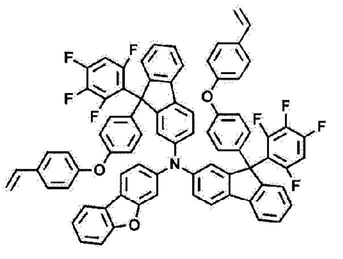
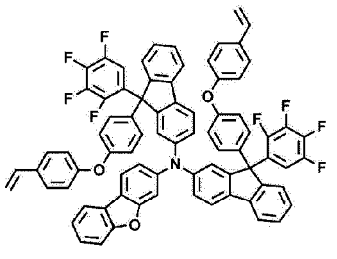

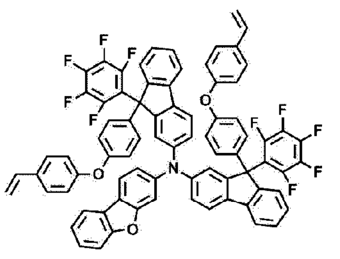
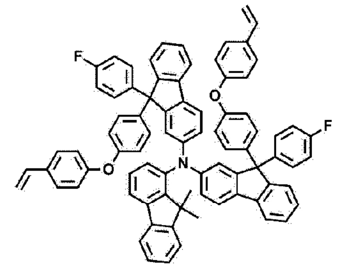
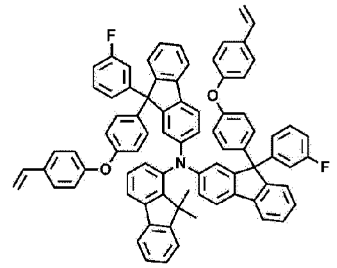

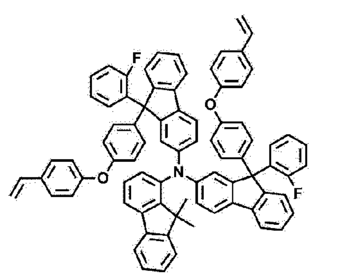
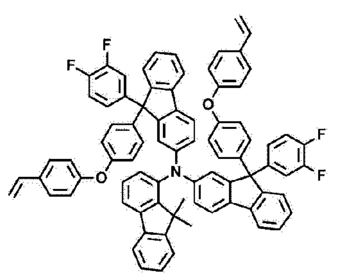
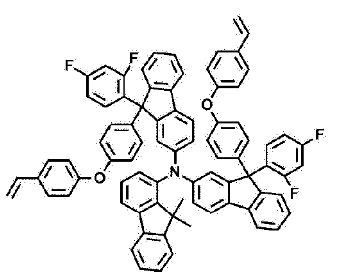

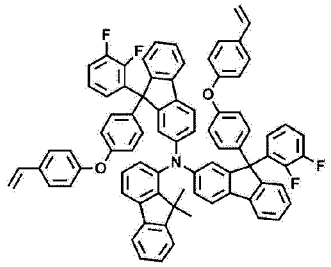
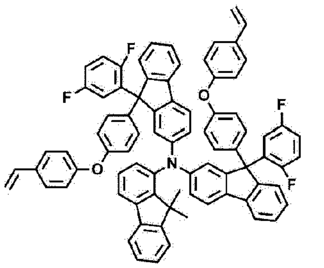
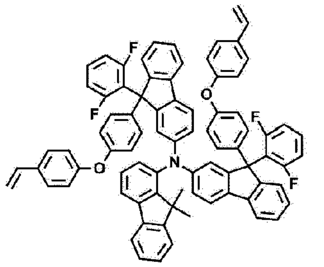

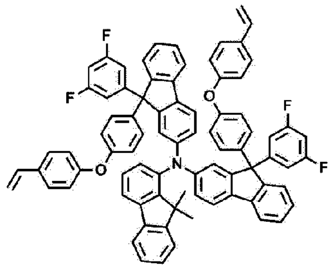
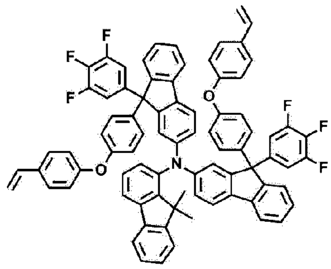
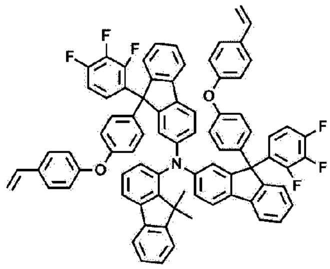

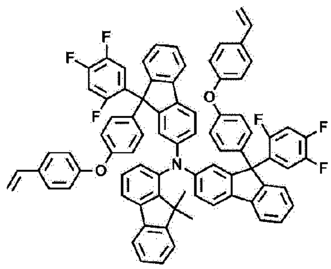
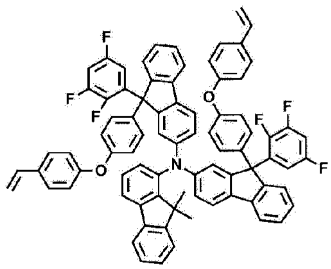
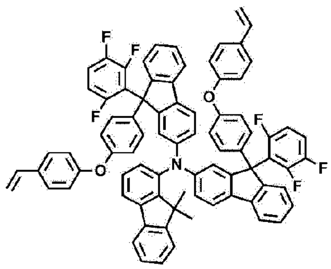

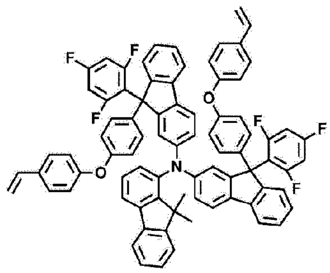
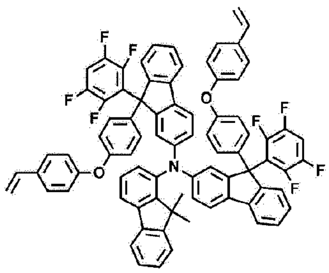
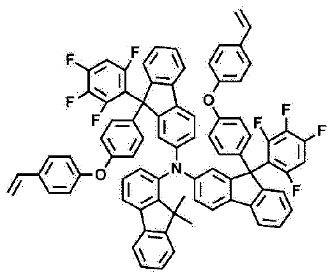

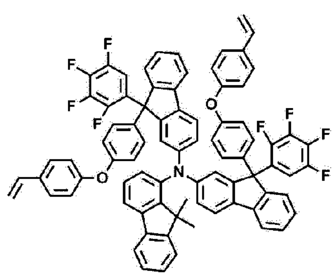
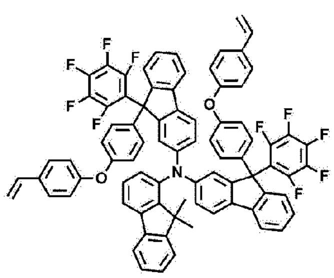

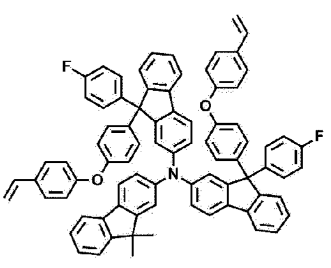
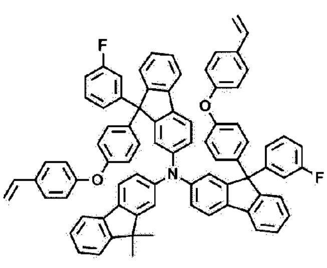
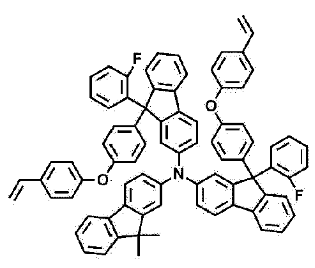

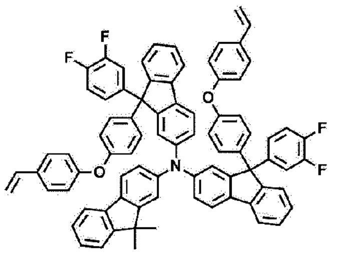
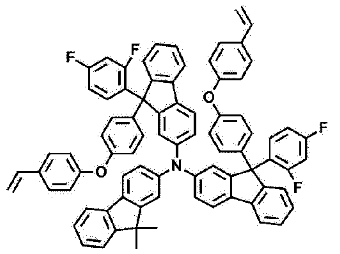
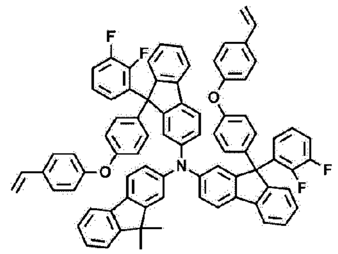
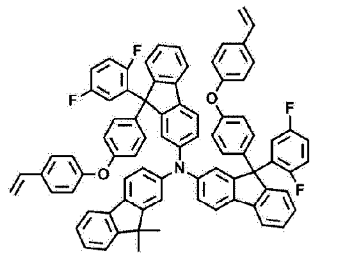
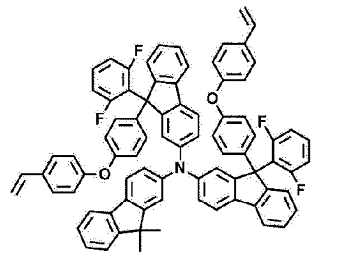
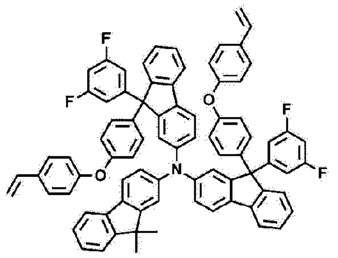
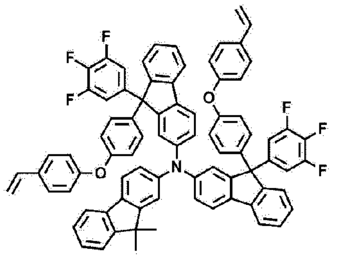
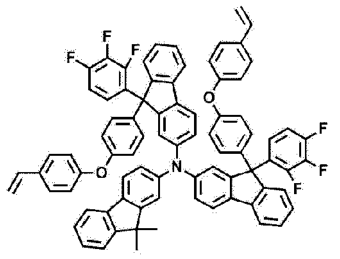
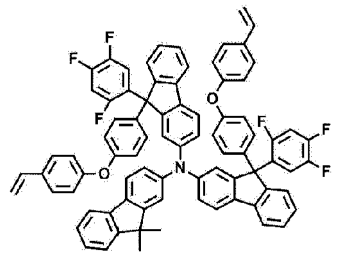
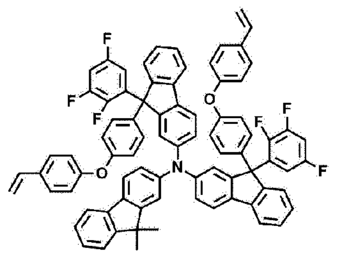
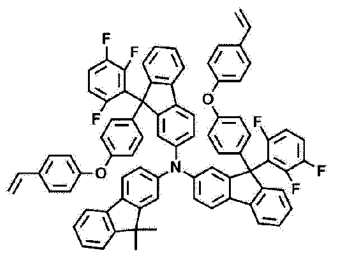
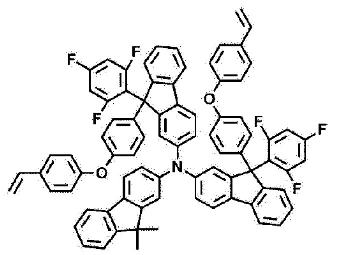
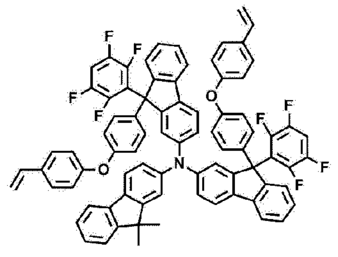
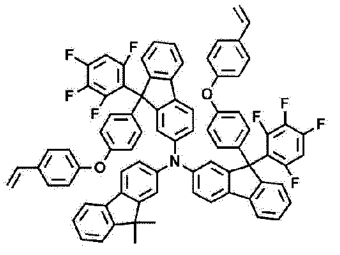
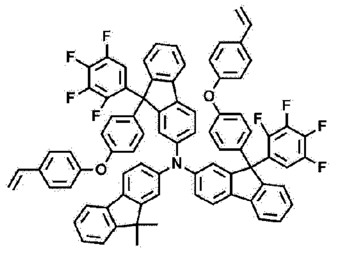
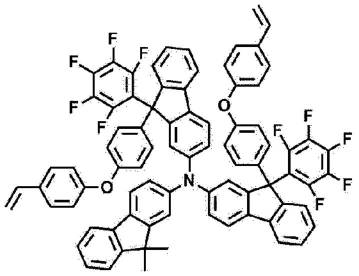
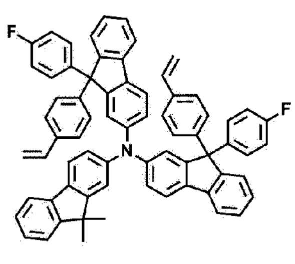
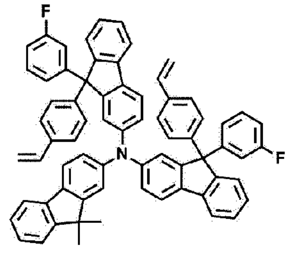

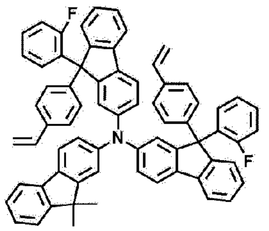
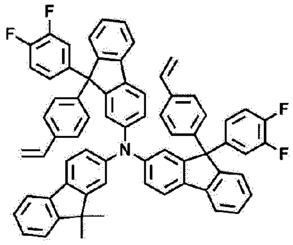
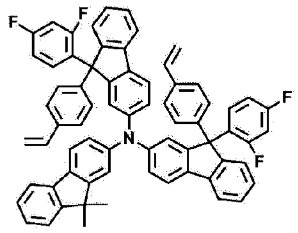
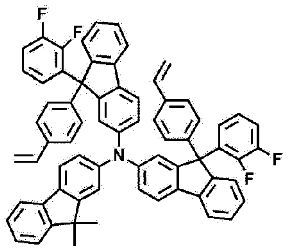
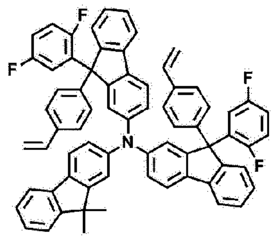
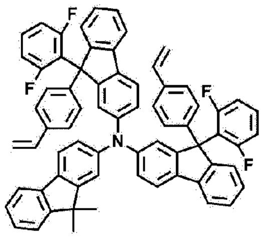
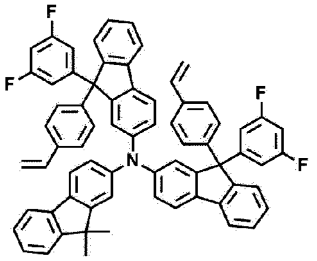
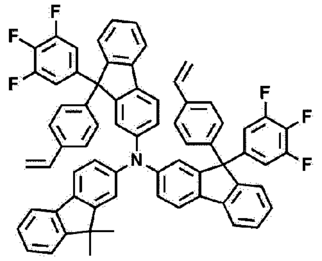
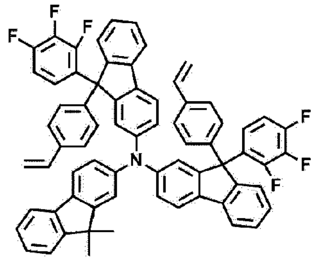
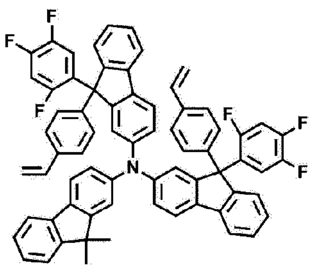
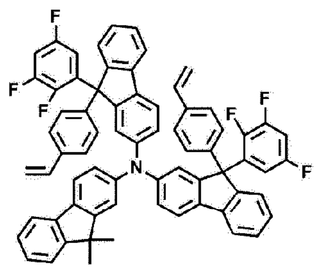
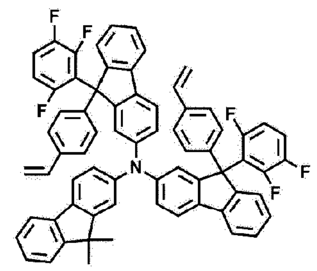
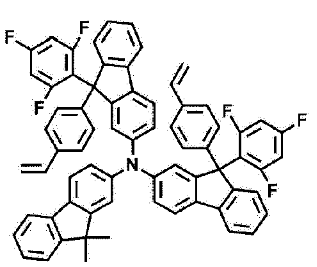
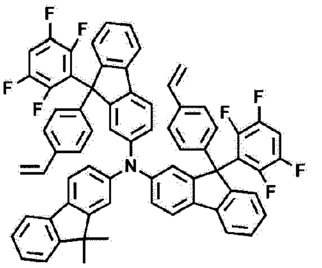
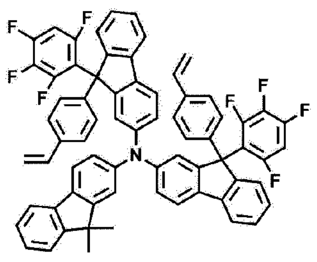
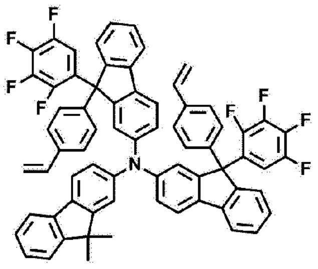
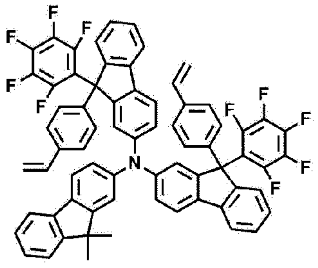

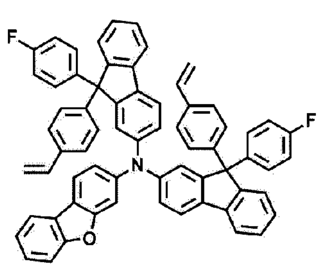
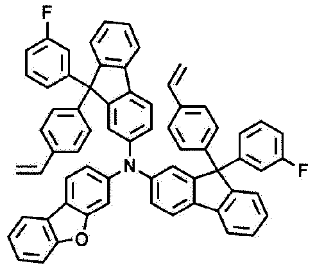
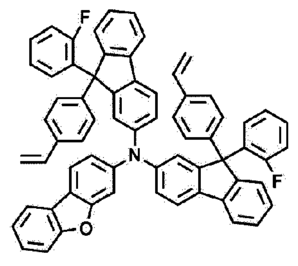

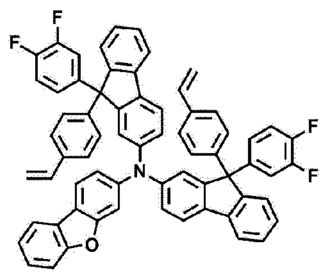
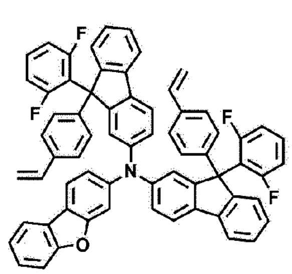
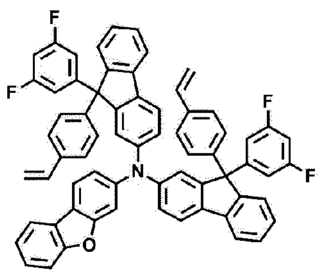

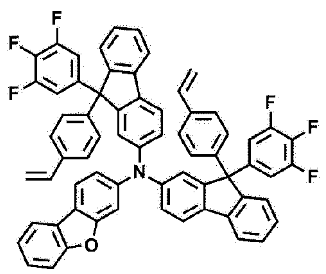
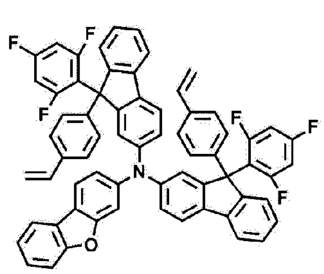
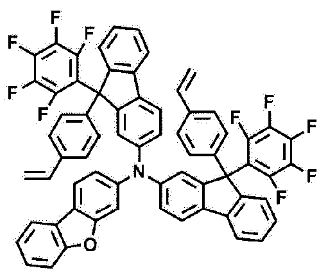

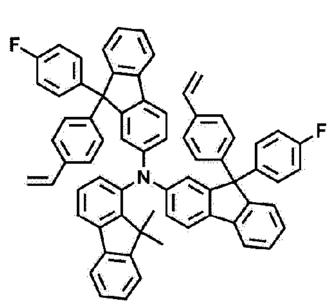
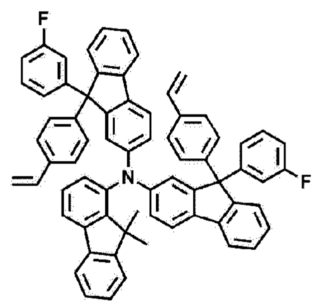
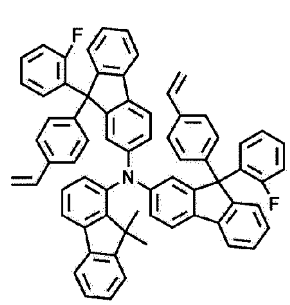

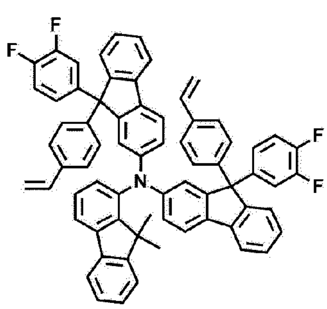
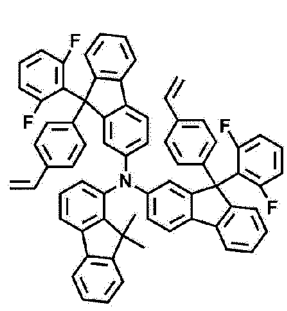
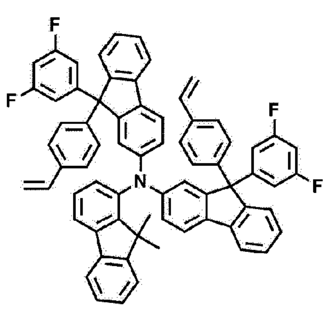

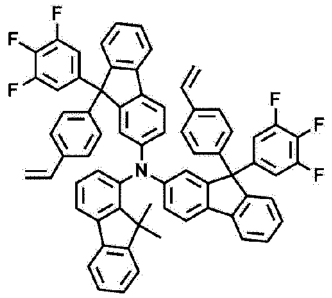
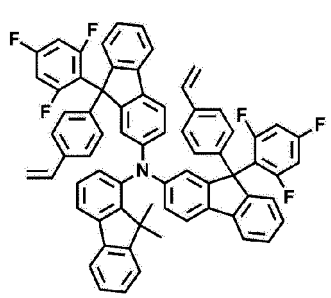
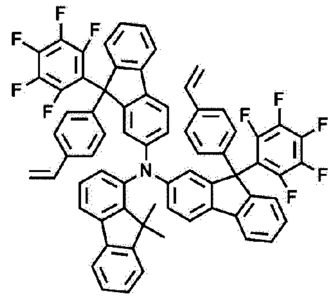

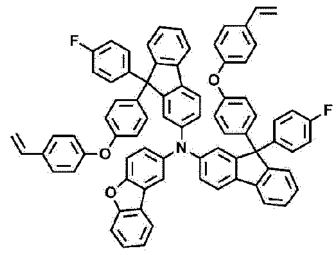
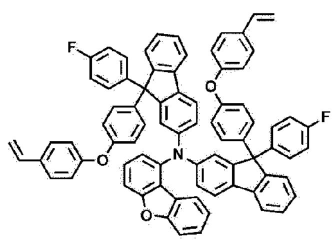
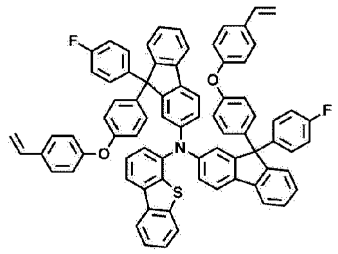

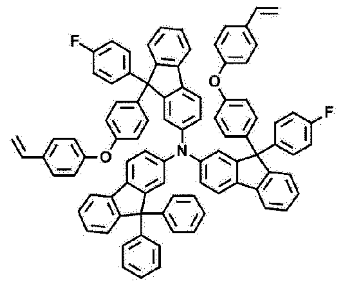
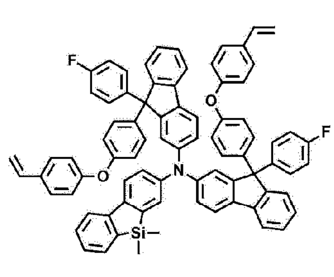
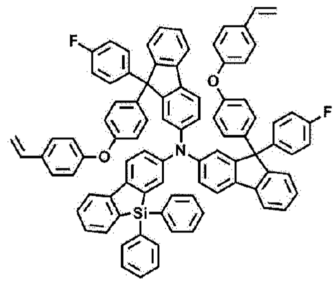

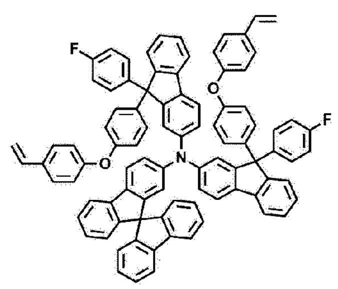
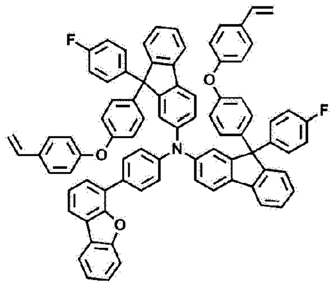
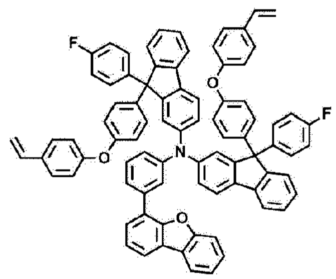

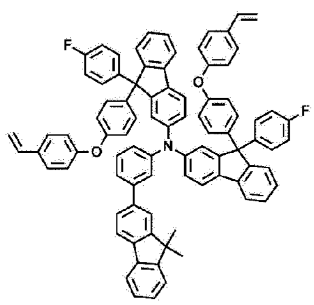
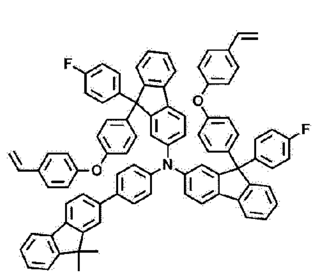
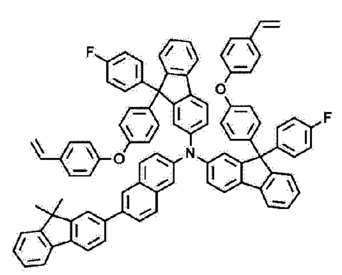

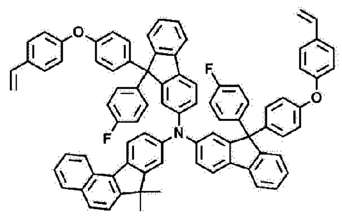
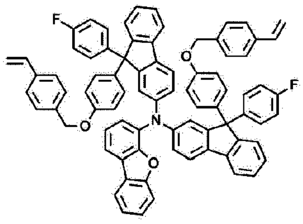
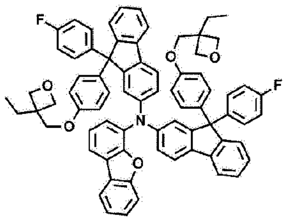

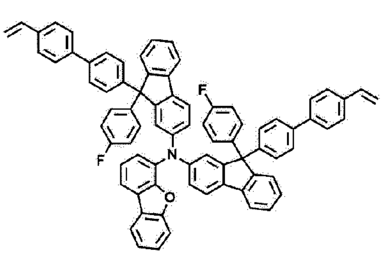
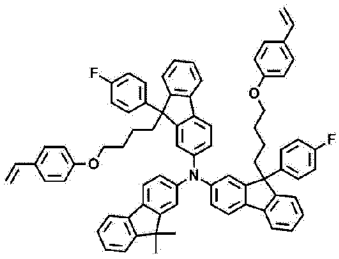
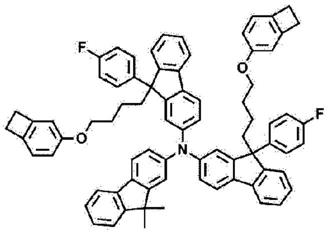

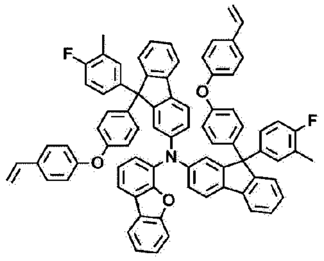
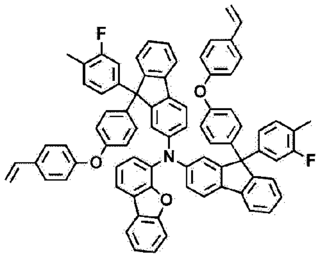
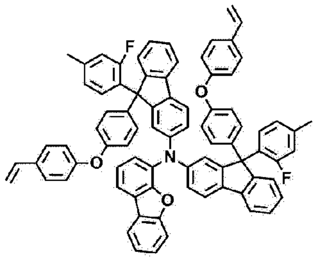

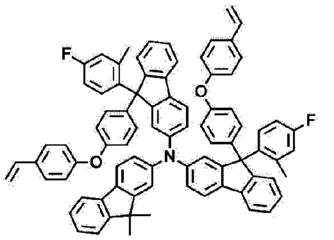
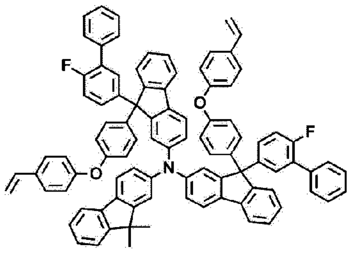
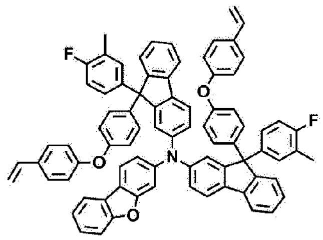

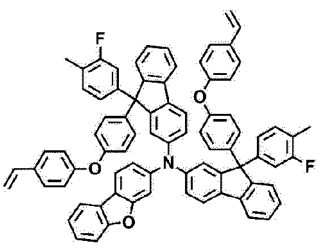
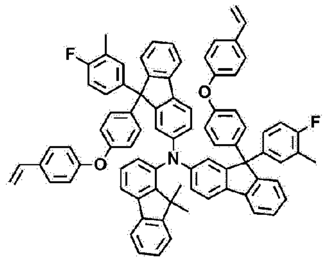
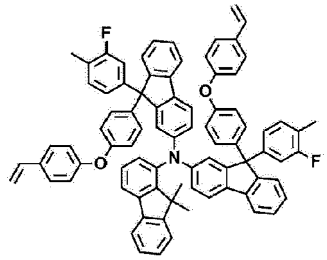

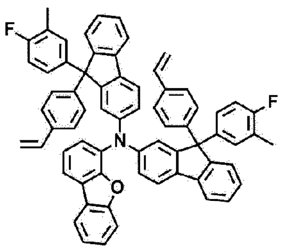
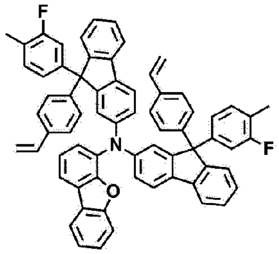
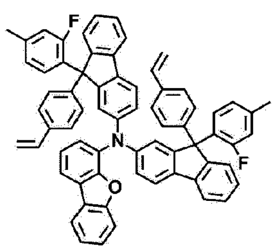

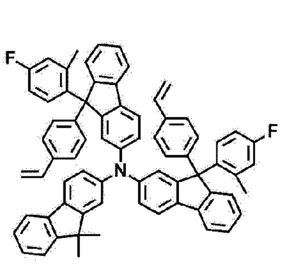
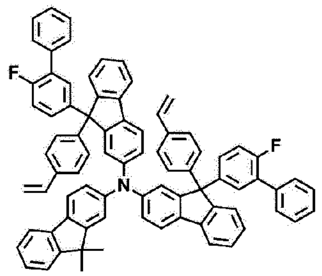
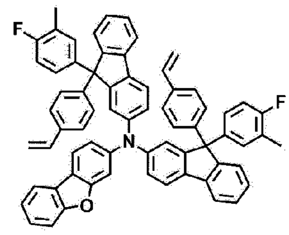

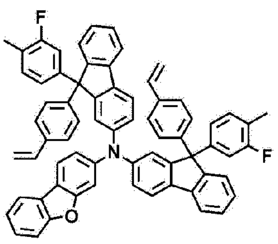
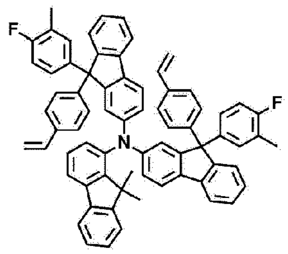
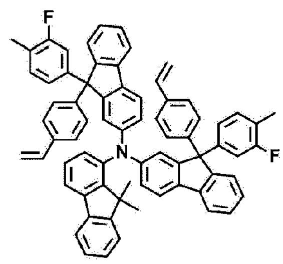

6. The ink composition of claim 1, further comprising an ionic compound comprising an anionic group represented by the following Chemical Formula 2:

[Chemical Formula 2]

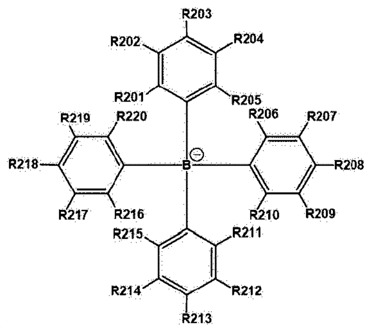

in Chemical Formula 2,
at least one of R201 to R220 is F; a cyano group; or a substituted or unsubstituted fluoroalkyl group,
at least one of the other R201 to R220 is a curable group,
the remaining R201 to R220 are the same as or different from each other, and are each independently hydrogen; deuterium; a nitro group; -C(O)R220'; -OR221; -SR222; -$SO_3$R223; -COOR224; -OC(O)R225; -C(O)NR226R227; a substituted or unsubstituted alkyl group; a substituted or unsubstituted fluoroalkyl group; a substituted or unsubstituted alkenyl group; a substituted or unsubstituted alkynyl group; a substituted or unsubstituted amine group; a substituted or unsubstituted aryl group; or a substituted or unsubstituted heterocyclic group, and
R220' and R221 to R227 are the same as or different from each other, and are each independently hydrogen; deuterium; or a substituted or unsubstituted alkyl group.

7. The ink composition of claim 6, wherein Chemical Formula 2 is any one selected from the group consisting of the following compounds:

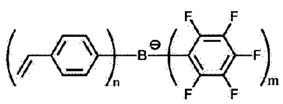
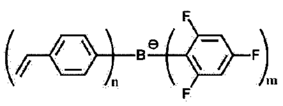
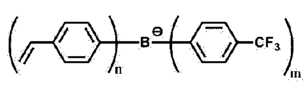

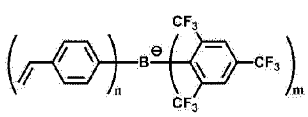
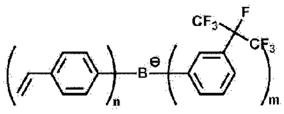
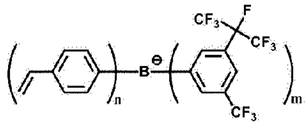

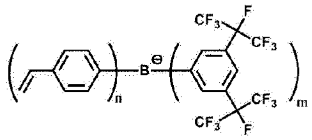
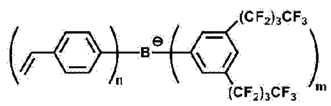
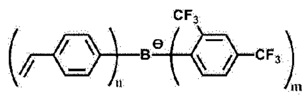

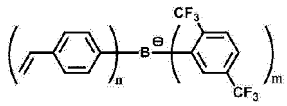
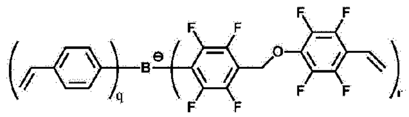

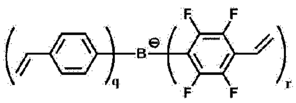
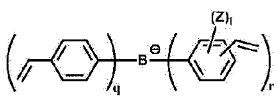
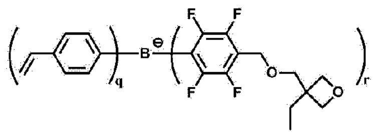

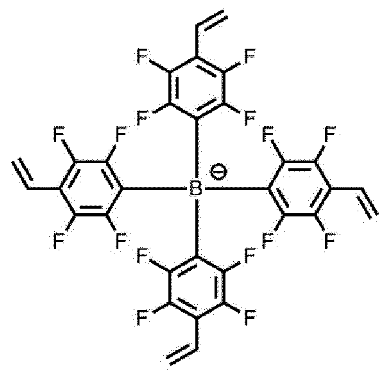
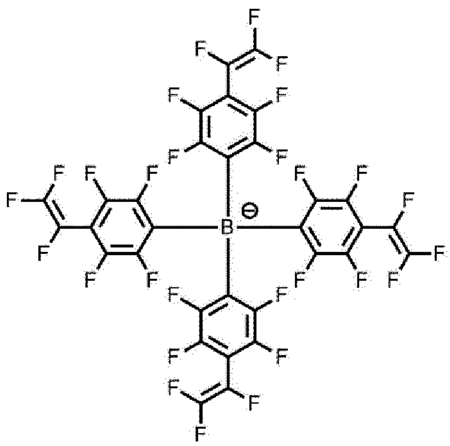

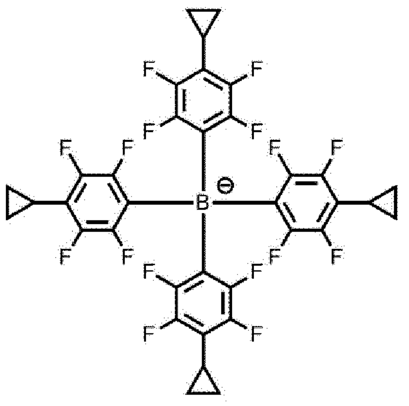
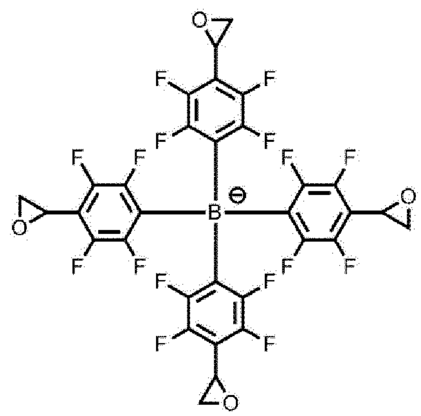

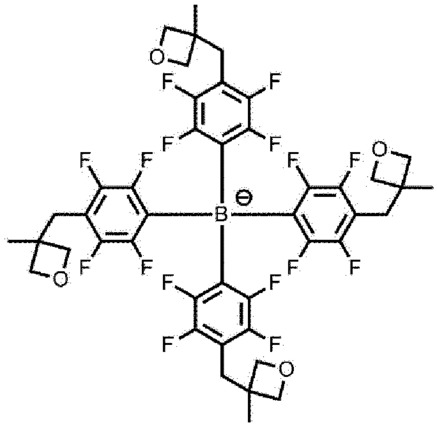

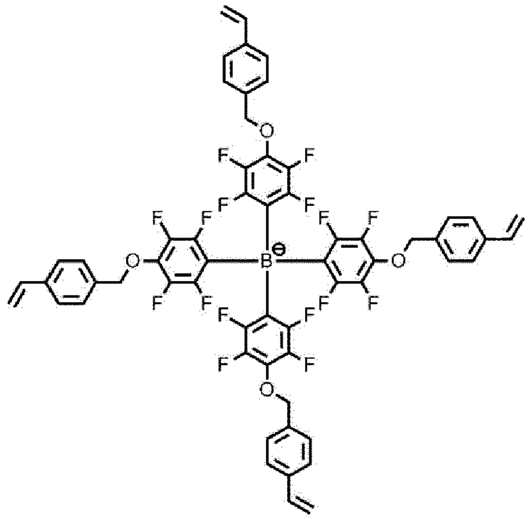

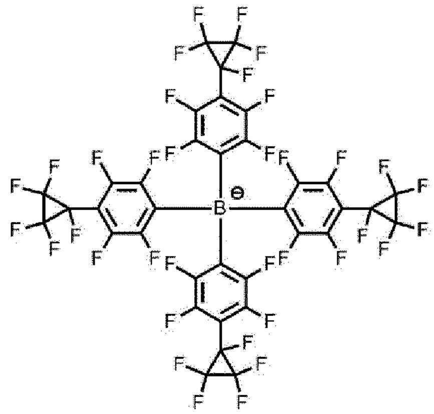

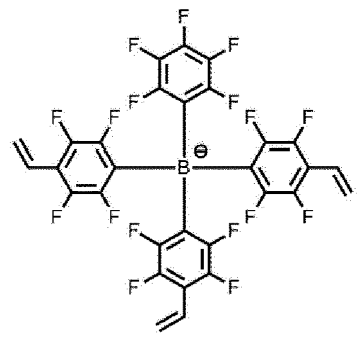

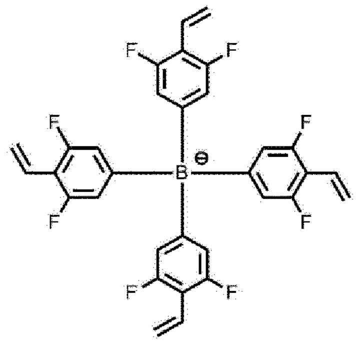

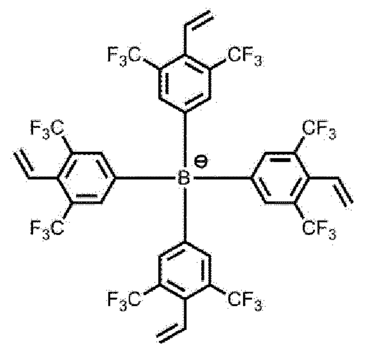

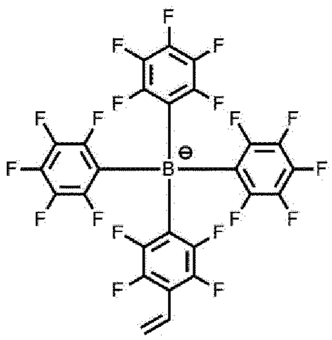

in the compounds,

n is an integer from 1 to 3, m is an integer from 1 to 3, and m+n=4,

q is an integer from 0 to 3, r is an integer from 1 to 4, and q+r=4,

Z is deuterium; a halogen group; a nitro group; a cyano group; an amino group; - C(O)R220'; -OR221; -SR222; $-SO_3R223$; -COOR224; -OC(O)R225; -C(O)NR226R227; a substituted or unsubstituted alkyl group; a substituted or unsubstituted alkenyl group; a substituted or unsubstituted alkynyl group; a substituted or unsubstituted amine group; a

1 is an integer from 1 to 4, and when 1 is 2 or higher, Z's are the same as or different from each other, and

R220' and R221 to R227 are the same as or different from each other, and are each independently hydrogen; deuterium; or a substituted or unsubstituted alkyl group.

**8.** The ink composition of claim 6, wherein the ionic compound further comprises a cationic group,

the cationic group is a monovalent cationic group; an onium compound; or any one selected from the following structural formulae:

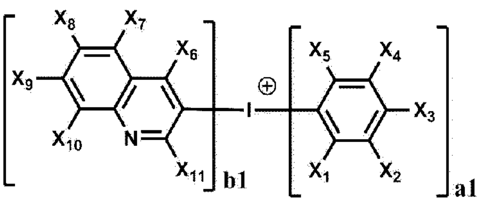

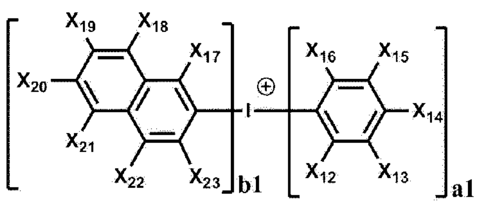

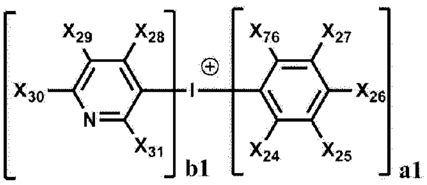

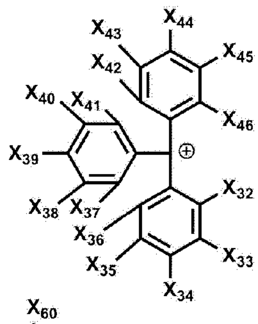

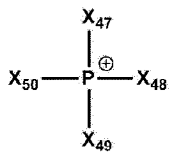

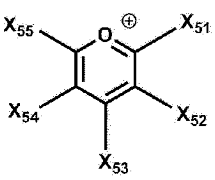

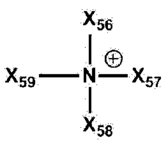

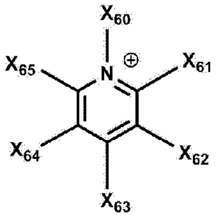

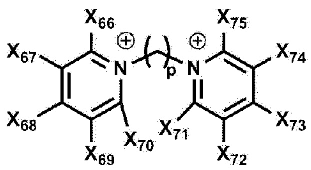

in the structural formulae,

$X_1$ to $X_{76}$ are the same as or different from each other, and are each independently hydrogen; deuterium; a cyano group; a nitro group; a halogen group; -COOR224; a substituted or unsubstituted alkyl group; a substituted or unsubstituted alkoxy group; a substituted or unsubstituted cycloalkyl group; a substituted or unsubstituted fluoroalkyl group; a substituted or unsubstituted aryl group; or a curable group,

R224 is hydrogen; deuterium; or a substituted or unsubstituted alkyl group,

p is an integer from 0 to 10, and

a1 is 1 or 2, b1 is 0 or 1, and a1+b1=2.

**9.** The ink composition of claim 8, wherein the ink composition further comprises one or more additional solvents selected from the group consisting of tetralin, 4-methoxytoluene, tetrahydrofuran, dioxane, dipropylene glycol monomethyl ether, tripropylene glycol monomethyl ether, a solvent represented by the following Chemical Formula C-1 and a solvent represented by the following Chemical Formula C-2:

[Chemical Formula C-1]

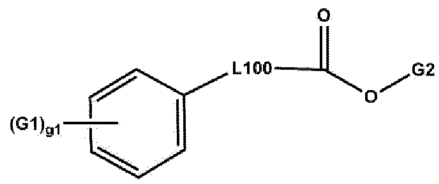

[Chemical Formula C-2]

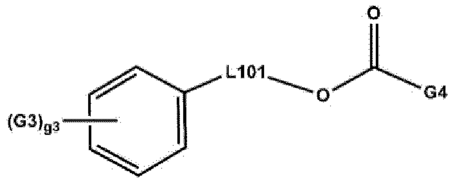

in Chemical Formulae C-1 and C-2,
L100 and L101 are the same as or different from each other, and are each independently a direct bond; or a substituted or unsubstituted alkylene group,
G1 and G3 are the same as or different from each other, and are each independently hydrogen; deuterium; a substituted or unsubstituted alkyl group; a substituted or unsubstituted alkoxy group; a substituted or unsubstituted aryl group; or a substituted or unsubstituted heteroaryl group,
G2 is a straight-chained alkyl group,
G4 is a substituted or unsubstituted alkyl group, and
g1 and g3 are each an integer from 1 to 5, and when g1 and g3 are each 2 or higher, two or more substituents in the parenthesis are the same as or different from each other.

**10.** The ink composition of claim 9, wherein based on 100 wt% of the ink composition,

the compound represented by Chemical Formula 1 is comprised in an amount of 0.1 wt% and 15 wt%;
the solvent represented by Chemical Formula A is comprised in an amount of 1 wt% to 70 wt%;
the ionic compound comprising the anionic group represented by Chemical Formula 2 is comprised in an amount of 0.1 wt% to 15 wt%; and
the additional solvent is comprised in an amount of 10 wt% to 85 wt%.

**11.** A pixel comprising the ink composition of any one of claims 1, 2, 4 and 7 to 10 or a cured product thereof.

**12.** An organic material layer comprising the pixel of claim 11.

**13.** The organic material layer of claim 12, wherein the organic material layer comprises n' pixels,

the n' pixels have a thickness uniformity V of 0.4 or lower, and
the thickness uniformity V satisfies the following Equation 1:

[Equation 1]

$$V = \frac{\sum_{i=2}^{n'} \left( X(i) - Xm' \right)^2}{n' \times Xm'}$$

in Equation 1,
X(i) is the thickness of the i-th pixel,
Xm' is the average value of X(i) in which i is 2 to n', and
n' is an integer from 2 to $10^4$.

**14.** An organic light emitting device comprising:

a first electrode;
a second electrode; and
an organic material layer having one or more layers provided between the first electrode and the second electrode,
wherein one or more layers of the organic material layer are the organic material layer of claim 12.

**15.** The organic light emitting device of claim 14, wherein the organic material layer comprises one or more layers selected from the group consisting of a hole injection layer, a hole transport layer, a hole injection and transport layer, and an electron blocking layer, and
the one or more layers selected from the group consisting of the hole injection layer, the hole transport layer, the hole injection and transport layer, and the electron blocking layer comprise the ink composition or a cured product thereof.

## Patentansprüche

**1.** Tintenzusammensetzung, umfassend eine durch die folgende chemische Formel 1 dargestellte Verbindung und ein durch die folgende chemische Formel A dargestelltes Lösungsmittel:

[Chemische Formel 1]

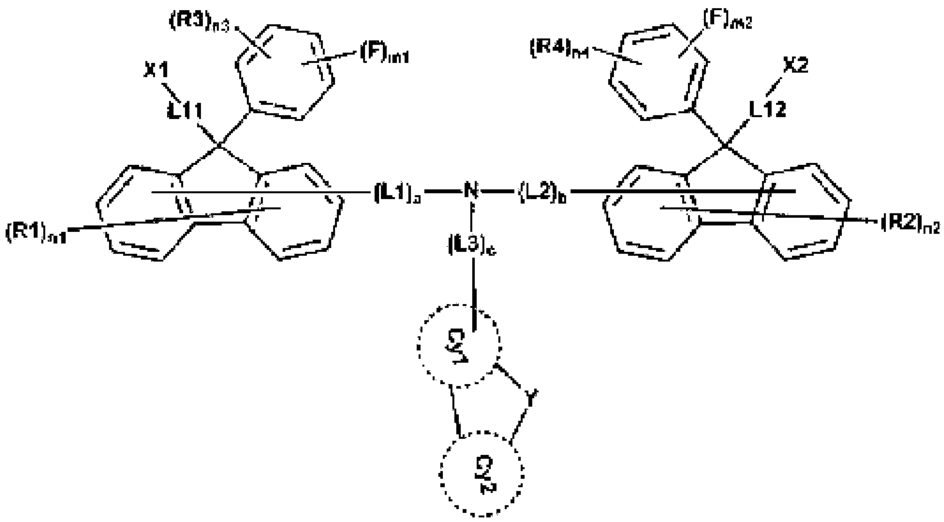

[Chemische Formel A]

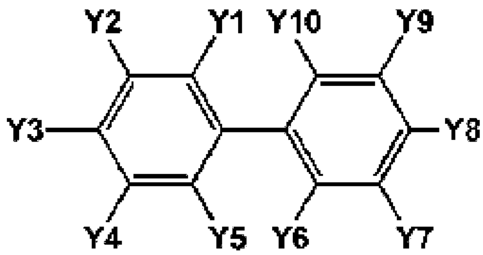

wobei in den chemischen Formeln 1 und A
Y O, S, CRaRb oder SiRcRd ist,
Cy1 und Cy2 gleich oder verschieden voneinander sind und jeweils unabhängig ein substituierter oder unsubstituierter Benzolring oder ein substituierter oder unsubstituierter Naphthalinring sind,
Ra, Rb, Rc und Rd gleich oder verschieden voneinander sind und jeweils unabhängig Wasserstoff, Deuterium,

eine substituierte oder unsubstituierte Alkylgruppe, eine substituierte oder unsubstituierte Arylgruppe oder eine substituierte oder unsubstituierte Heteroarylgruppe sind oder an eine benachbarte Gruppe gebunden sind, um einen substituierten oder unsubstituierten Ring zu bilden,

L1 bis L3 gleich oder verschieden voneinander sind und jeweils unabhängig eine direkte Bindung oder eine substituierte oder unsubstituierte Arylengruppe sind,

L11 und L12 gleich oder verschieden voneinander sind und jeweils unabhängig eine direkte Bindung, eine substituierte oder unsubstituierte Alkylengruppe oder eine substituierte oder unsubstituierte Arylengruppe sind,

X1 und X2 gleich oder verschieden voneinander sind und jeweils unabhängig eine härtbare Gruppe sind,

R1 und R2 gleich oder verschieden voneinander sind und jeweils unabhängig Deuterium, eine Halogengruppe, eine substituierte oder unsubstituierte Alkylgruppe, eine substituierte oder unsubstituierte Alkoxygruppe, eine substituierte oder unsubstituierte Arylgruppe oder eine substituierte oder unsubstituierte Heteroarylgruppe sind,

R3 und R4 gleich oder verschieden voneinander sind und jeweils unabhängig Deuterium, eine substituierte oder unsubstituierte Alkylgruppe, eine substituierte oder unsubstituierte Alkoxygruppe, eine substituierte oder unsubstituierte Arylgruppe oder substituierte oder unsubstituierte Heteroarylgruppe sind,

a, b und c jeweils 0 oder 1 sind,

n1 und n2 jeweils eine ganze Zahl von 0 bis 7 sind, und wenn n1 und n2 jeweils 2 oder größer sind, zwei oder mehr Substituenten in der Klammer gleich oder verschieden voneinander sind,

m1 und m2 jeweils eine ganze Zahl von 1 bis 5 sind, n3 und n4 jeweils eine ganze Zahl von 0 bis 4 sind, m1+n3 5 oder weniger ist und m2+n4 5 oder weniger ist und

Y1 bis Y10 gleich oder verschieden voneinander sind und jeweils unabhängig Wasserstoff, Deuterium, eine Hydroxylgruppe, eine Ethergruppe, eine Carbonylgruppe, eine Estergruppe, eine substituierte oder unsubstituierte Alkylgruppe, eine substituierte oder unsubstituierte Cycloalkylgruppe, eine substituierte oder unsubstituierte Cycloalkenylgruppe, eine substituierte oder unsubstituierte Alkoxygruppe, eine substituierte oder unsubstituierte Arylgruppe oder eine substituierte oder unsubstituierte Amingruppe sind.

2. Tintenzusammensetzung nach Anspruch 1, wobei die härtbare Gruppe eine, ausgewählt aus der Gruppe, bestehend aus den folgenden Strukturen, ist:

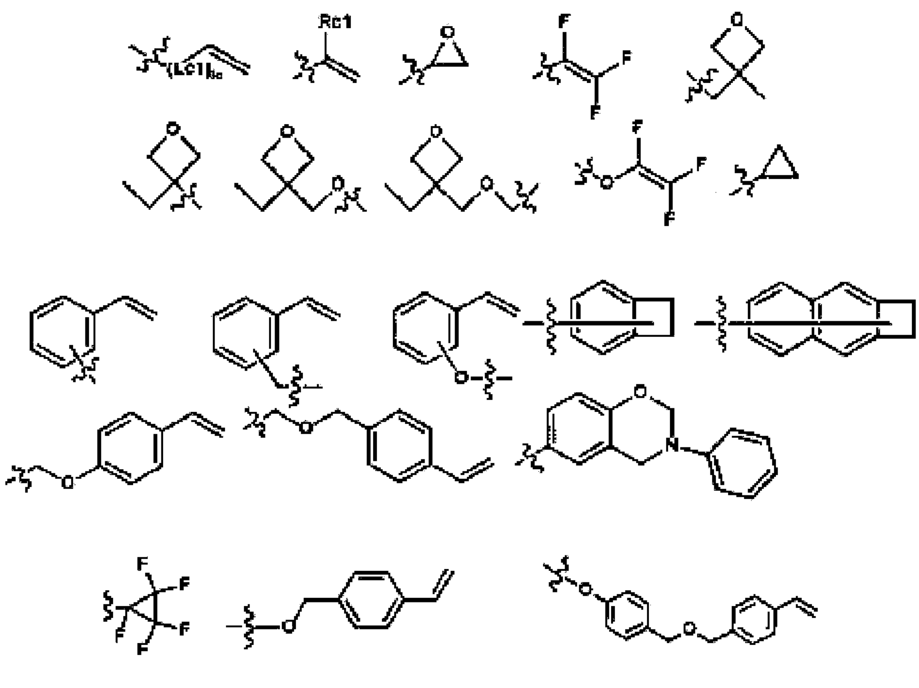

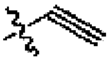 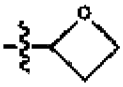 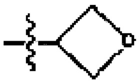 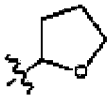 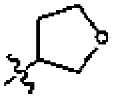

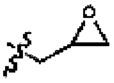 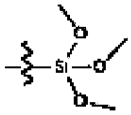 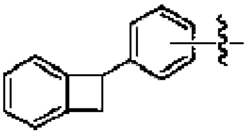 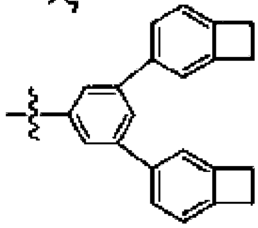

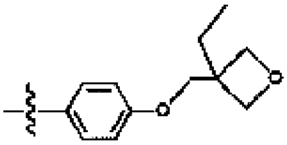 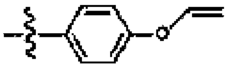 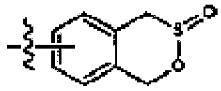

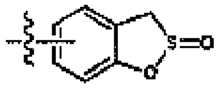

in den Strukturen,
Lc1 eine direkte Bindung, -O-, -S-, eine substituierte oder unsubstituierte Alkylengruppe, eine substituierte oder unsubstituierte Arylengruppe oder eine substituierte oder unsubstituierte Heteroarylengruppe ist,
lc 1 oder 2 ist,
wenn lc 2 ist, die Lc1 gleich oder verschieden voneinander sind,
Rc1 eine substituierte oder unsubstituierte Alkylgruppe ist und

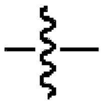

ein an die chemische Formel 1 gebundener Rest ist.

3. Tintenzusammensetzung nach Anspruch 1, wobei das durch die chemische Formel A dargestellte Lösungsmittel eine, ausgewählt aus der Gruppe, bestehend aus den folgenden Verbindungen, ist:

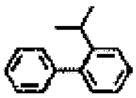 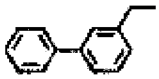 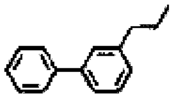 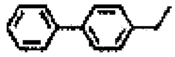

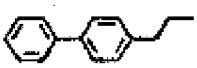 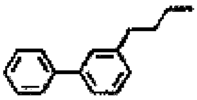 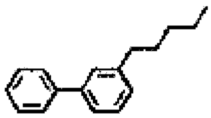 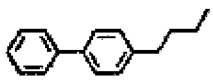

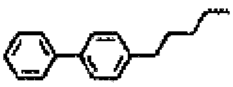 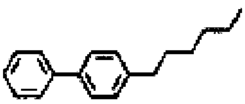 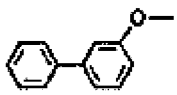 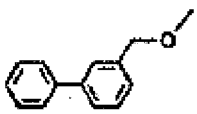

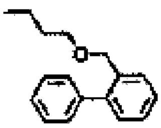 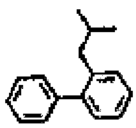 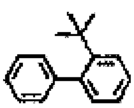

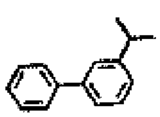
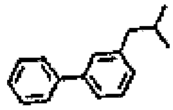
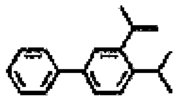
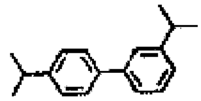
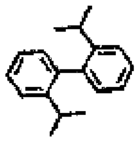
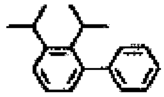
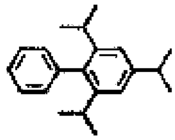
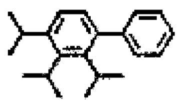
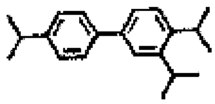
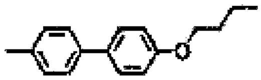

4. Tintenzusammensetzung nach Anspruch 1, wobei die chemische Formel durch die folgende chemische Formel 11 dargestellt ist:

[Chemische Formel 11]

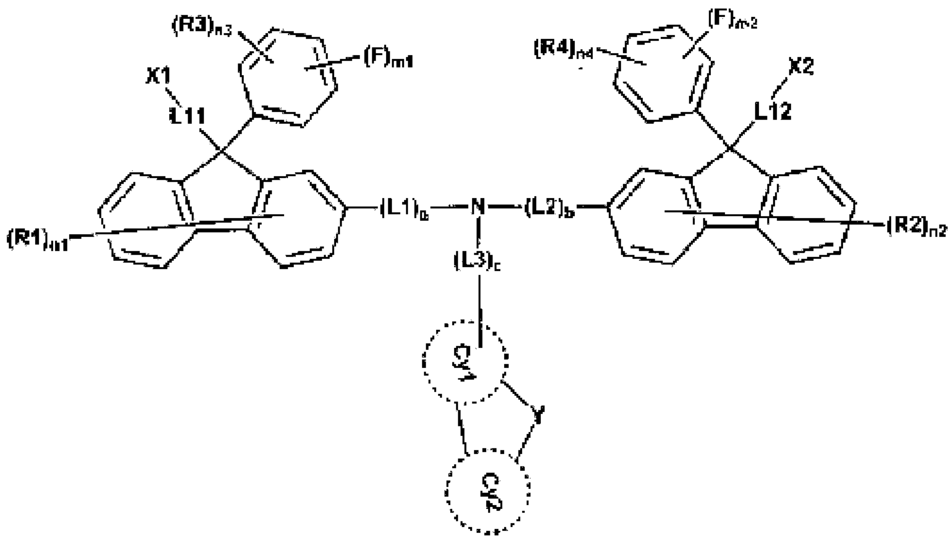

in der chemischen Formel 11
die Definitionen von L1 bis L3, L11, L12, X1, X2, R1 bis R4, Y, Cy1, Cy2, a, b, c, n1 bis n4, m1 und m2 die gleichen wie jene in der chemischen Formel 1 sind.

5. Tintenzusammensetzung nach Anspruch 1, wobei die chemische Formel 1 eine, ausgewählt aus der Gruppe, bestehend aus den folgenden Verbindungen, ist:

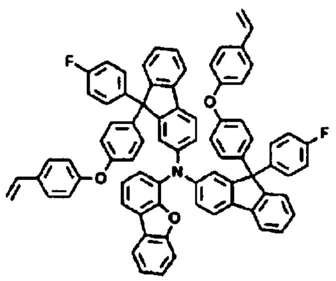
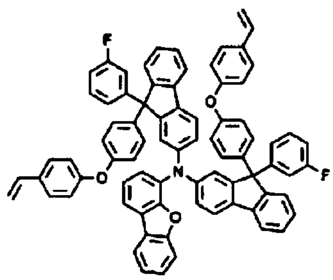
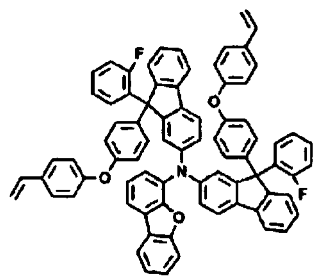

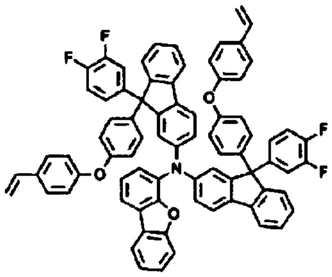 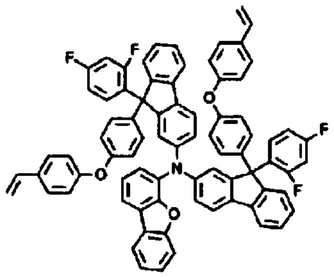 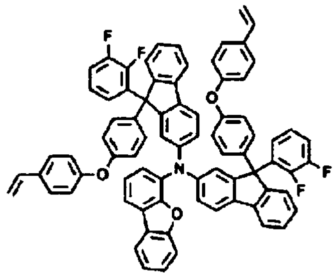

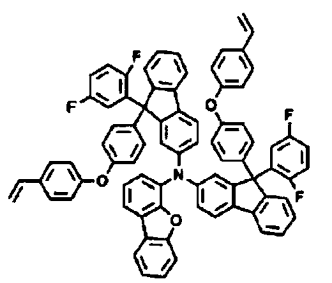 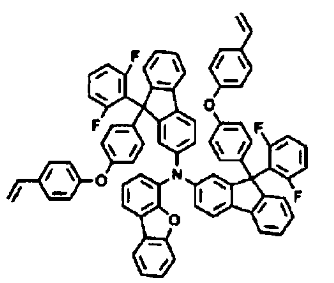 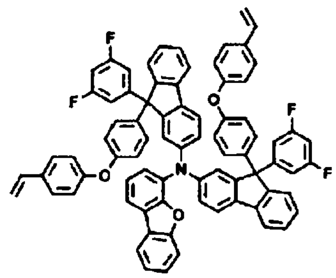

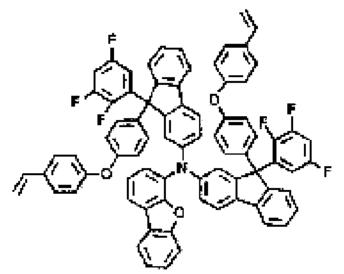 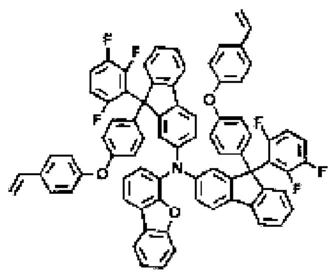 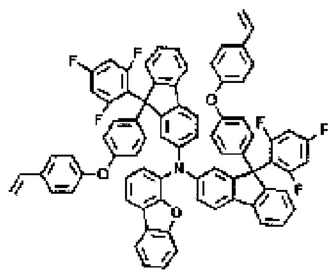

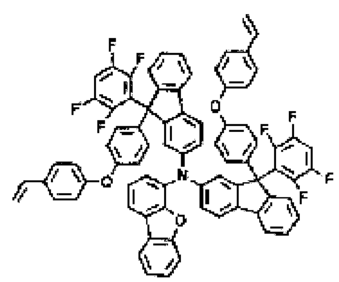 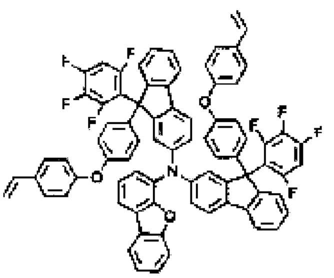 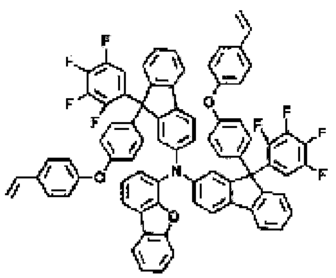

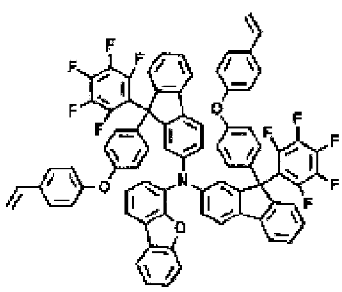 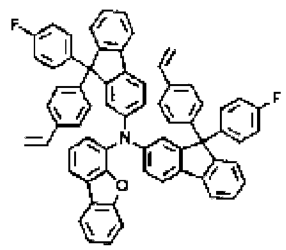 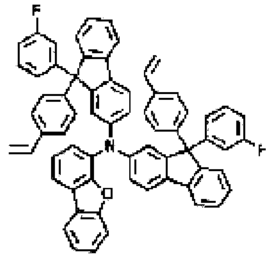

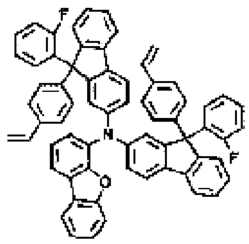
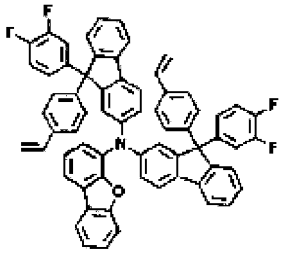
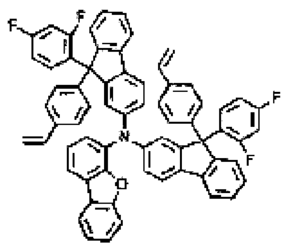

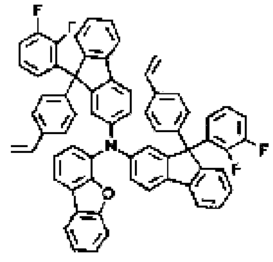
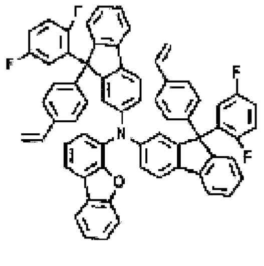
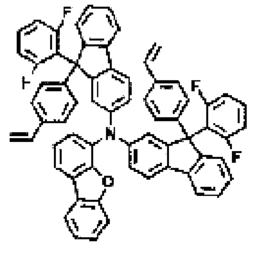

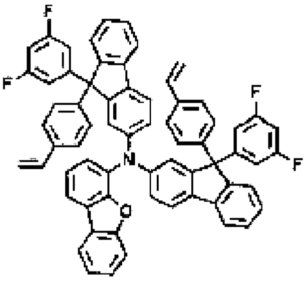
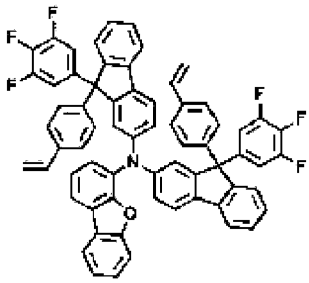
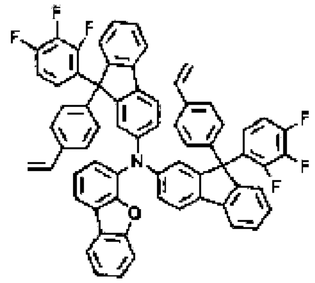

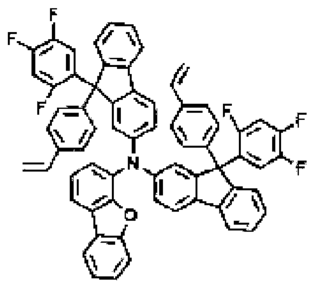
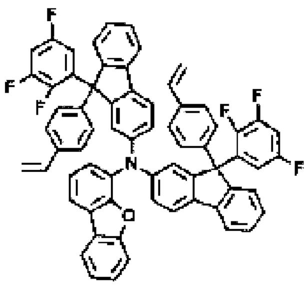
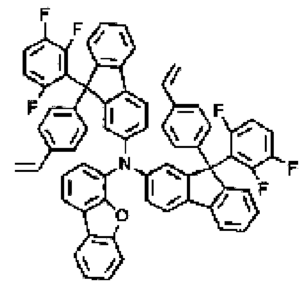

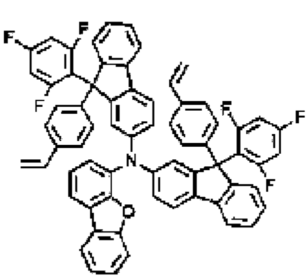
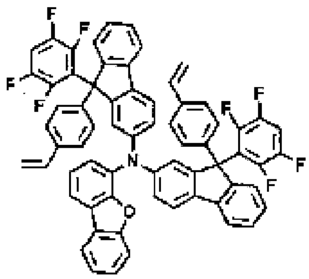
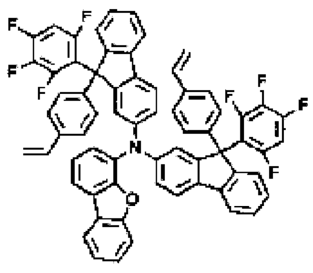

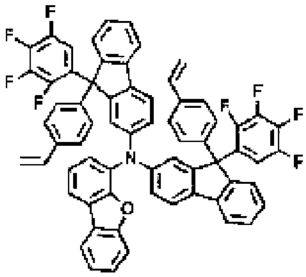
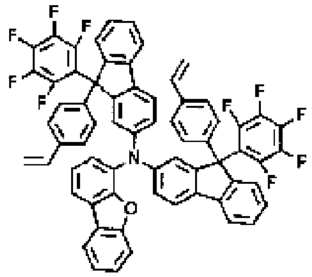

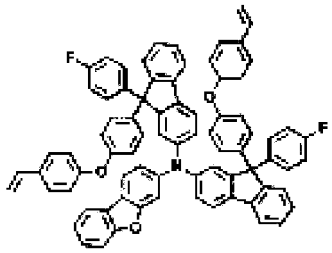
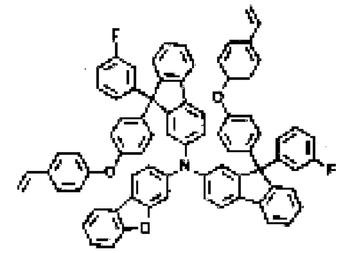
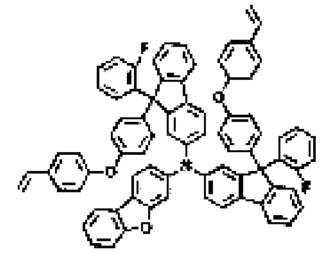

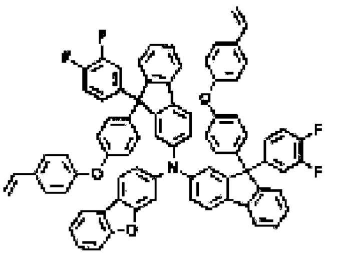
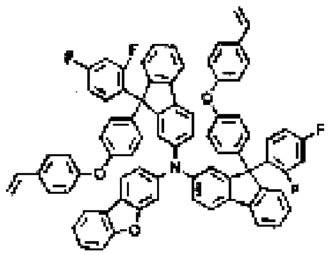
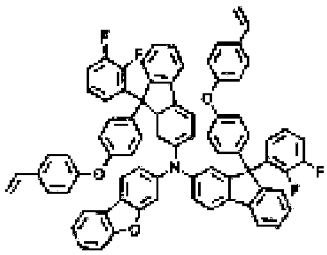

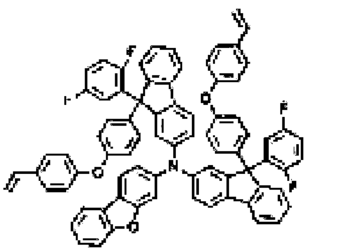
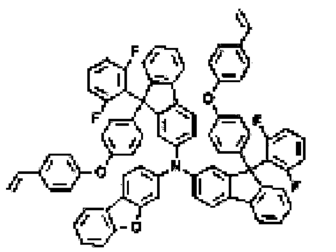
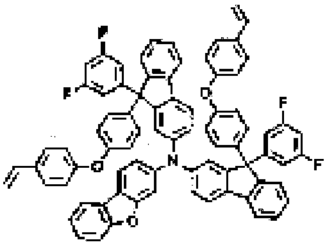

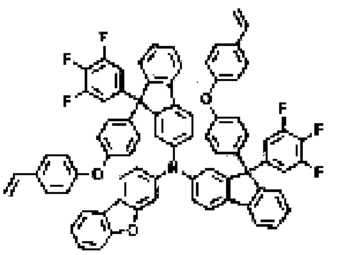
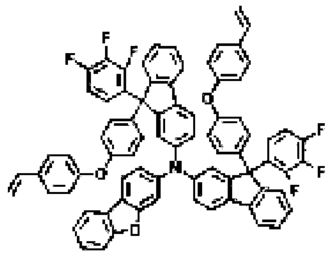
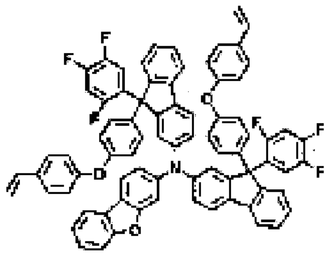

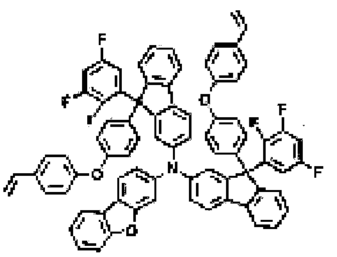
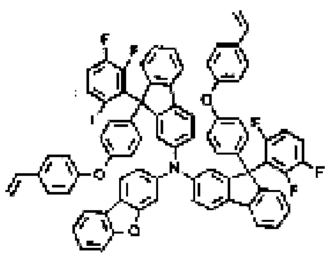
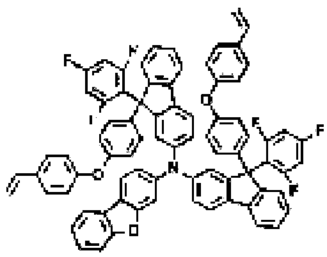

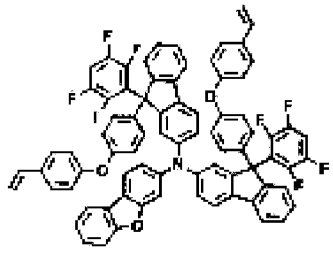
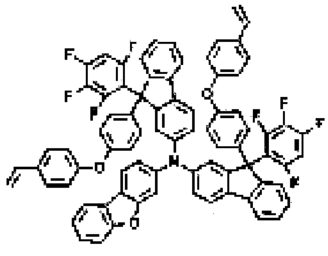
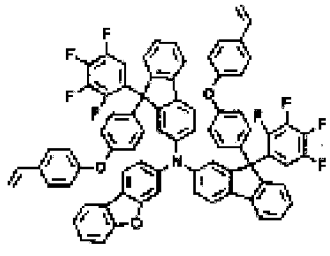

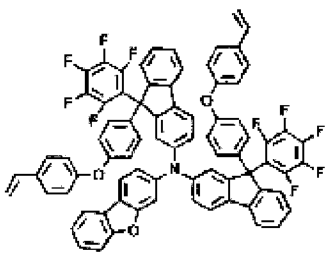
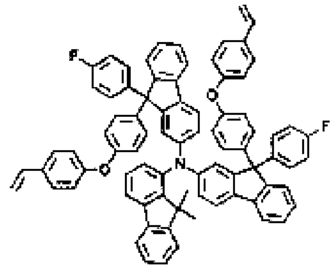
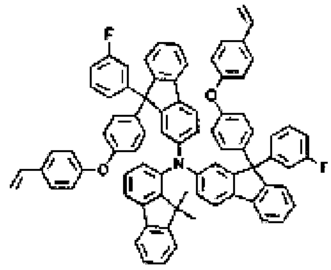

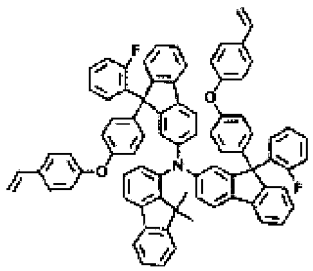
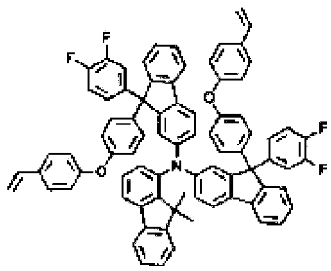
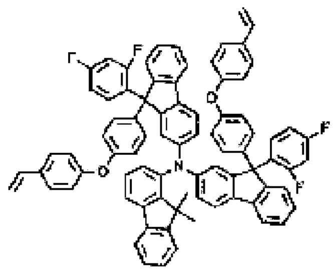

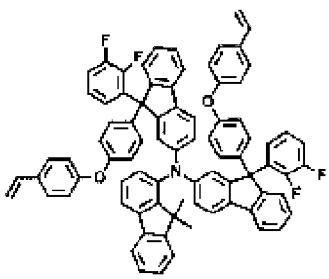
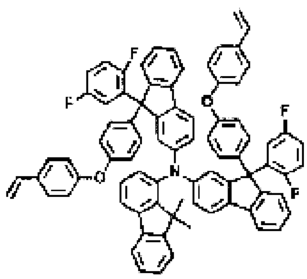
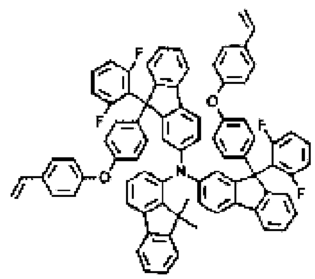

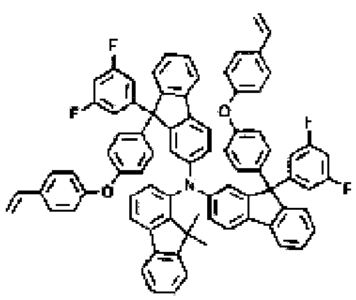
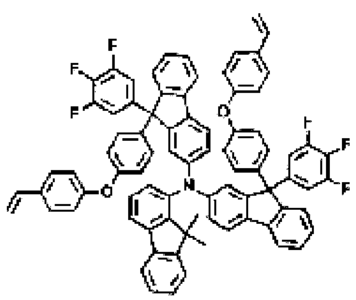
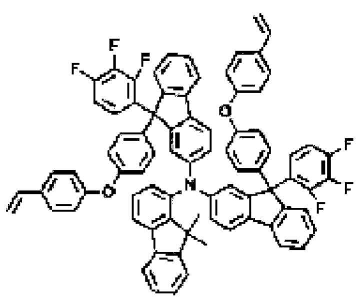

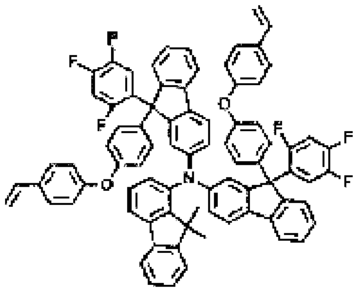
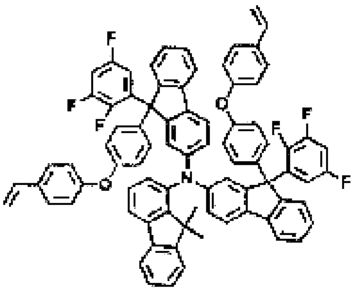
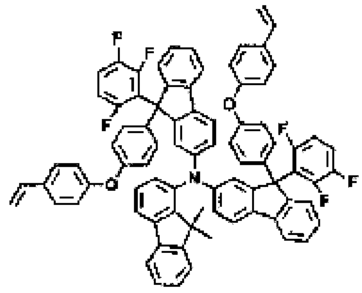

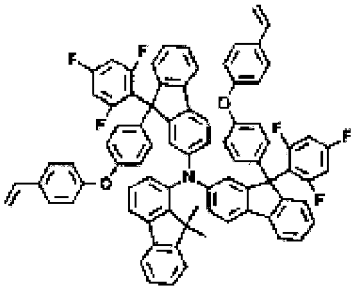
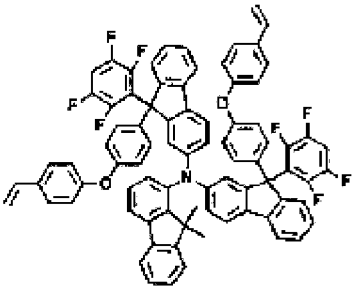
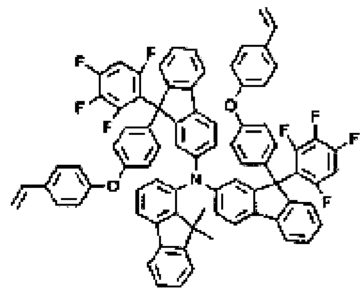

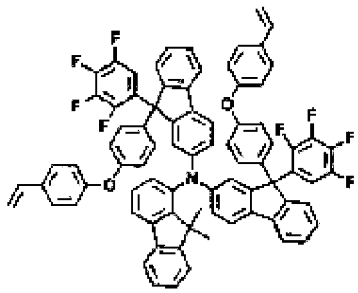
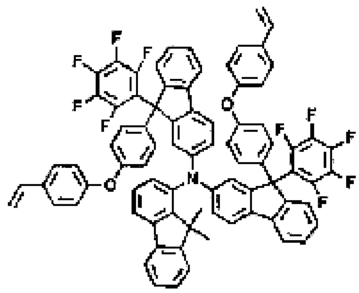

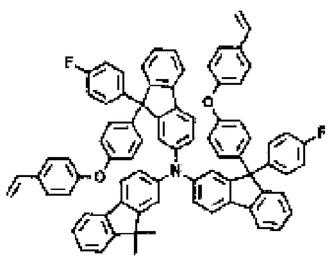
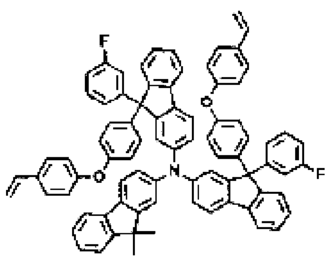
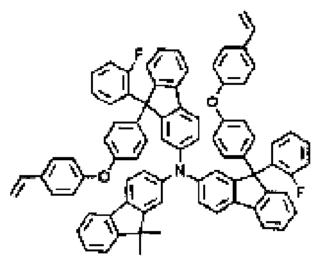

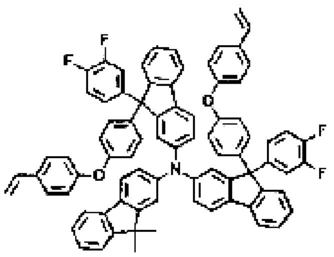
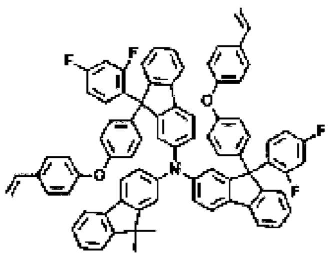
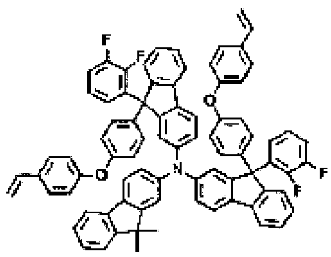

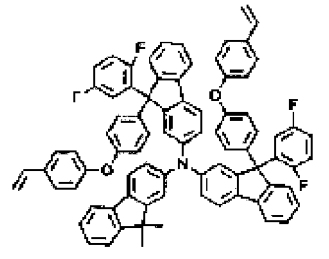
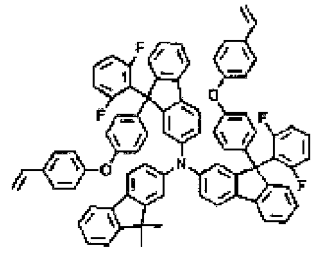
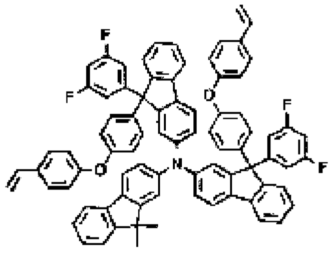
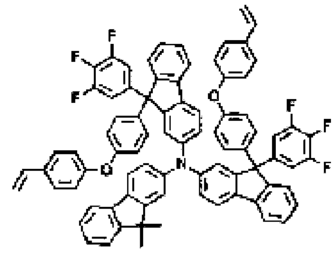
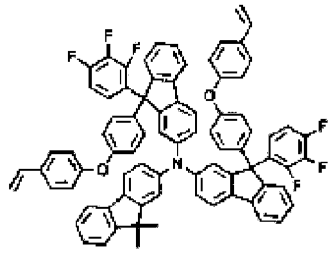
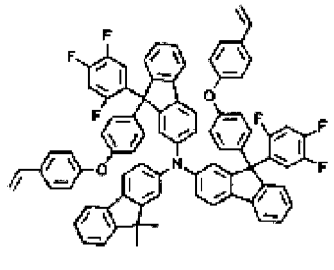
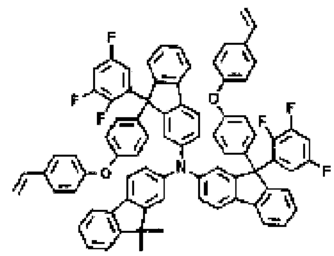
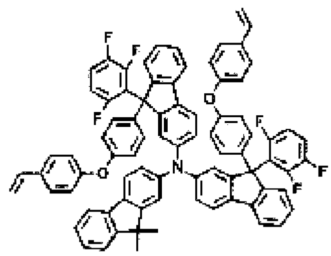
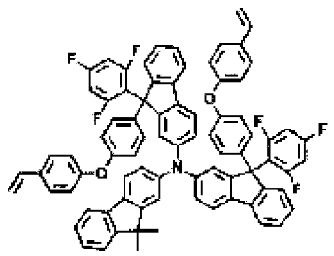
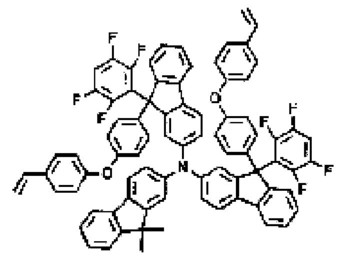
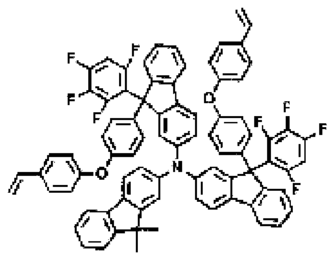
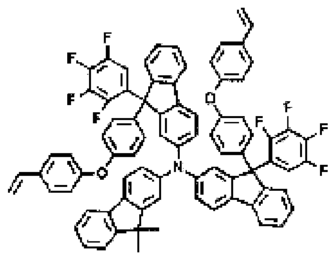
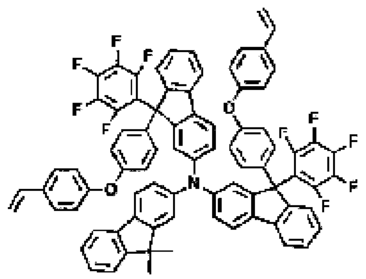
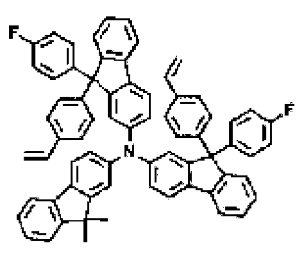
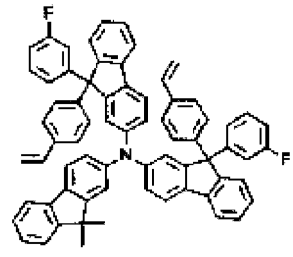
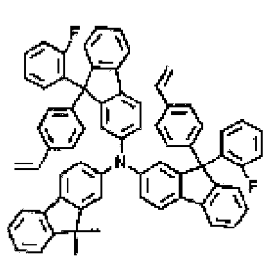
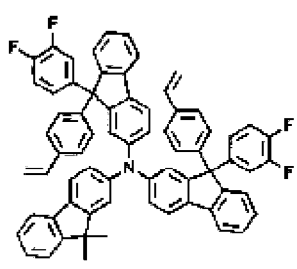
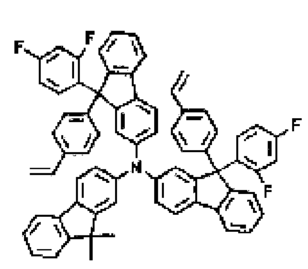

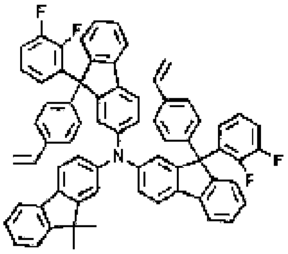
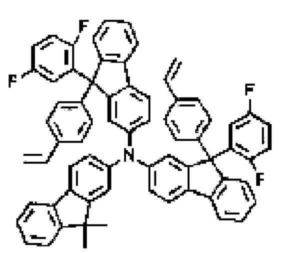
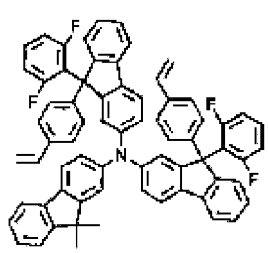
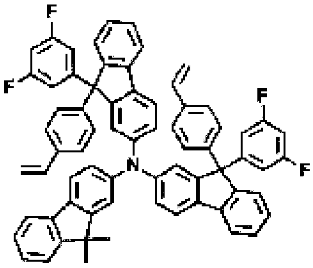
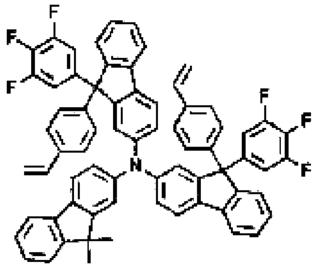
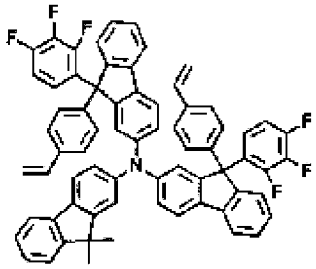
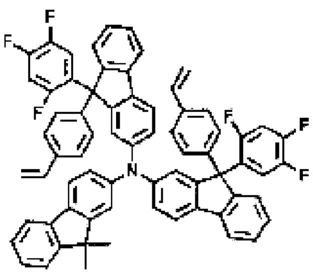
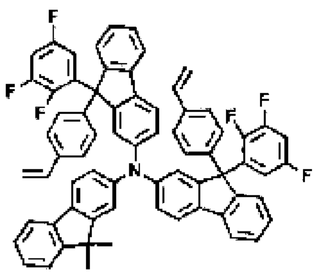
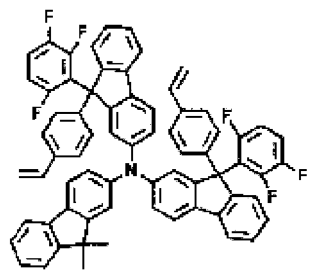
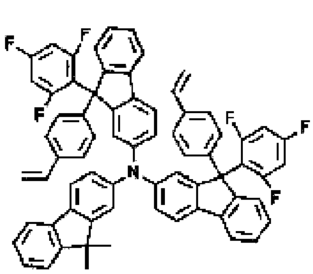
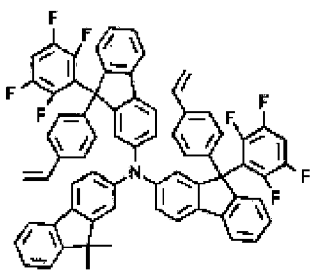
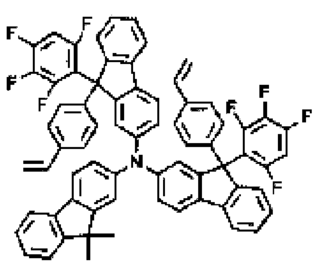
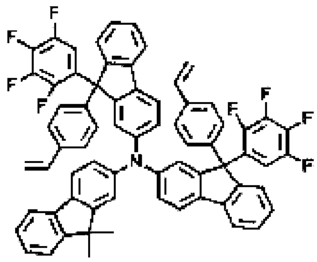
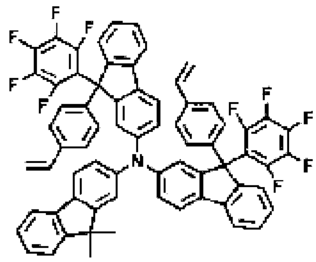
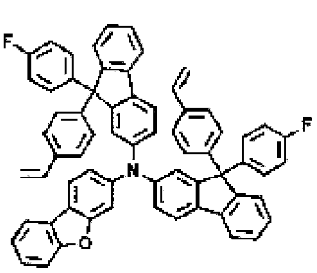
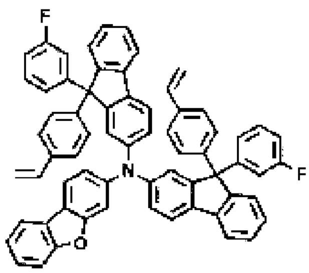
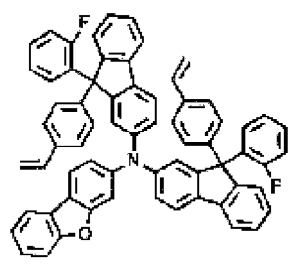

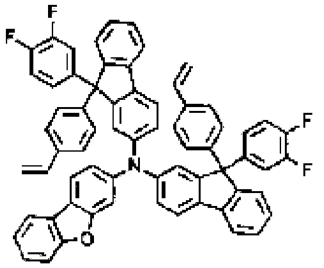
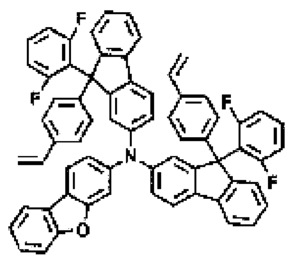
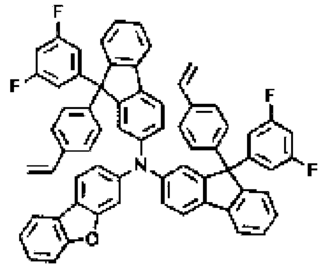

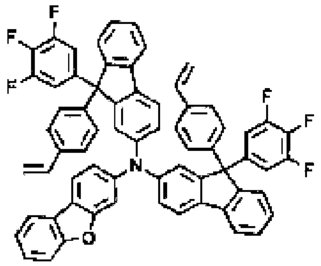
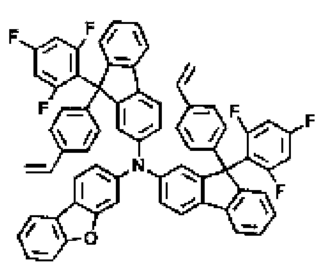
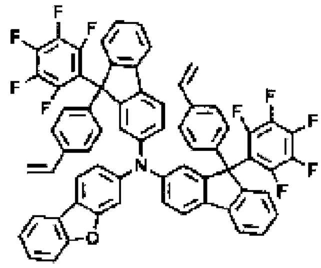

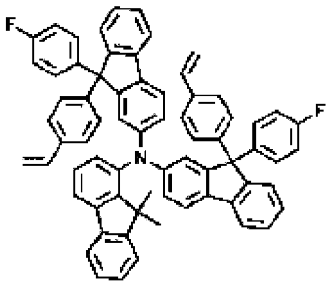
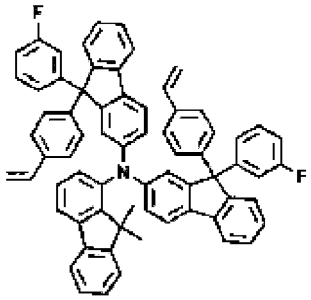
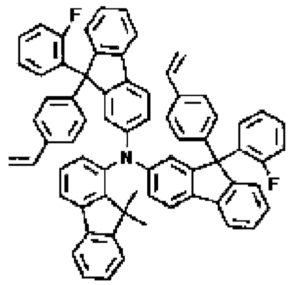

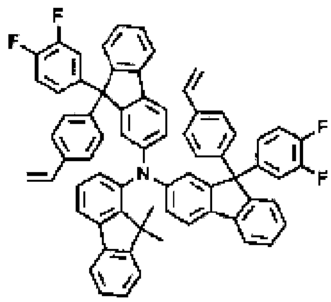
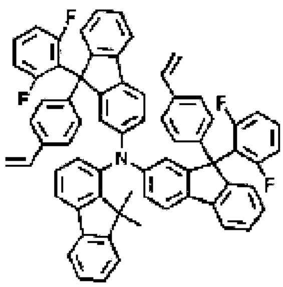
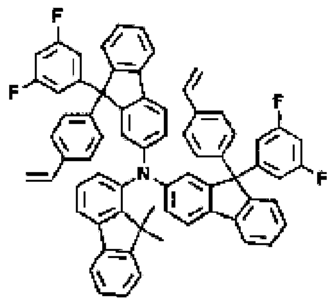

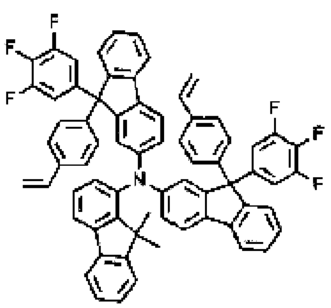
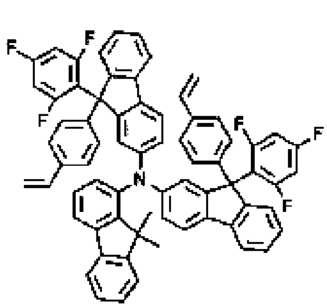
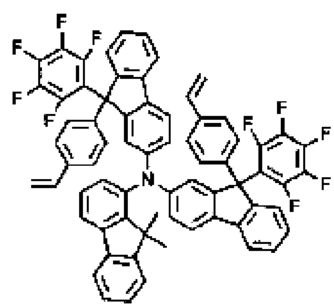

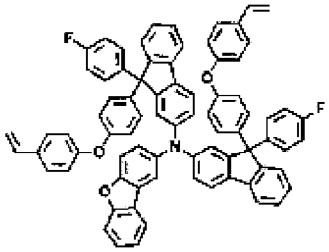
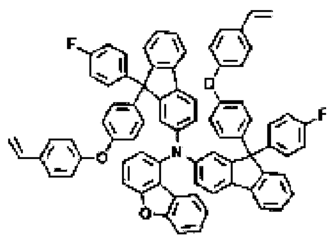
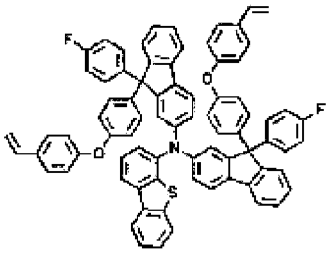
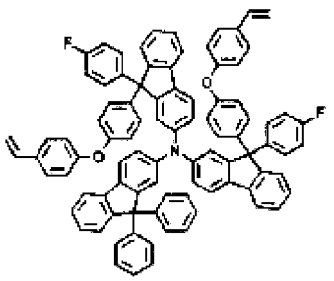
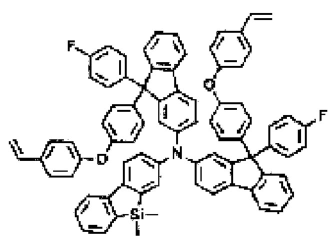
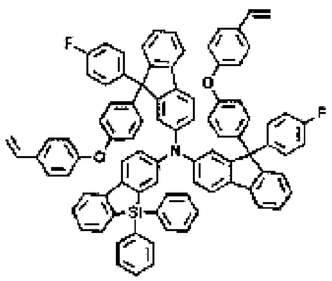
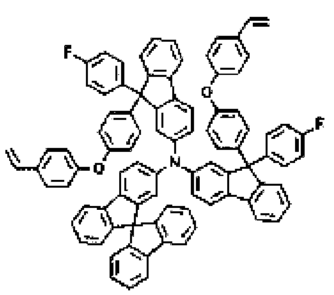
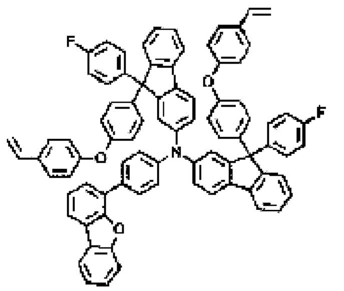
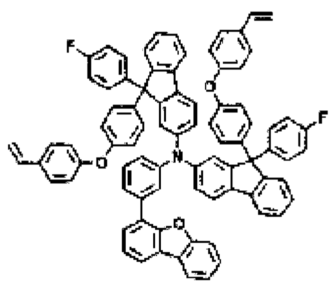
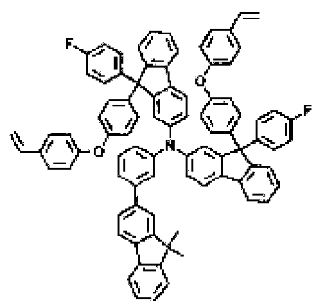
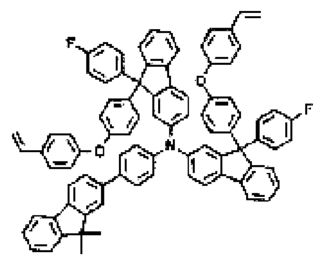
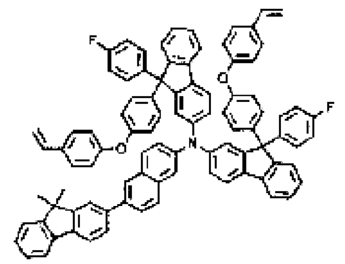
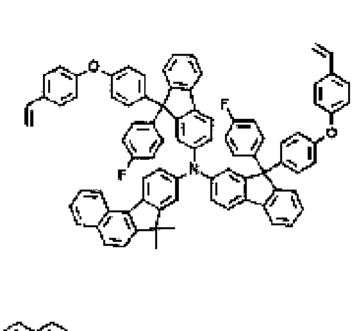
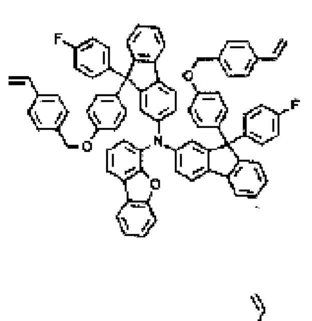
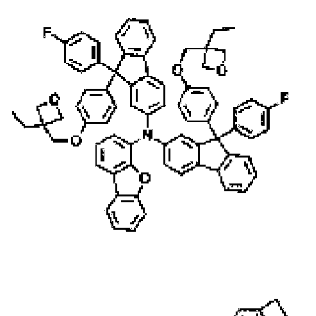
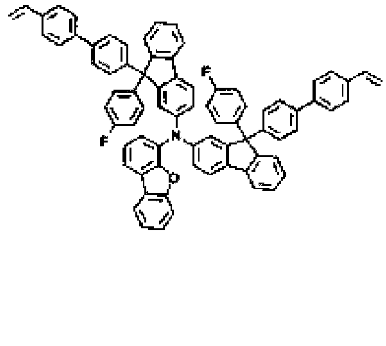
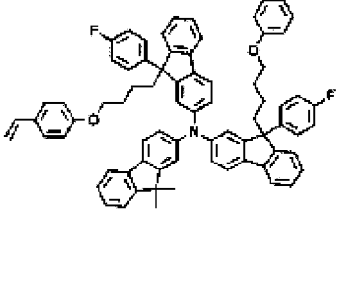
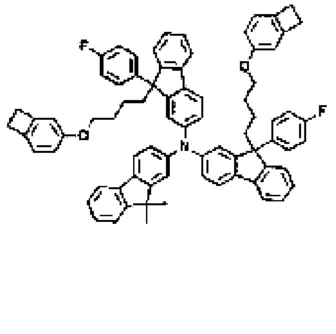

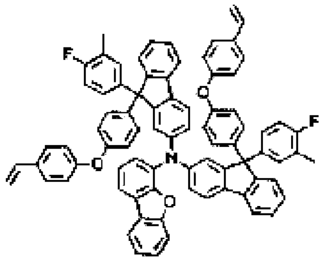 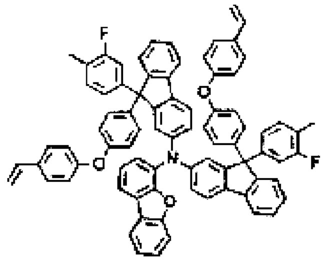 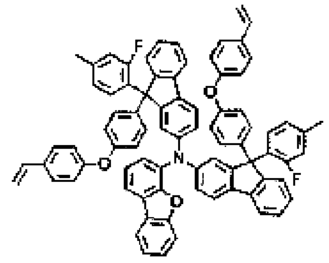

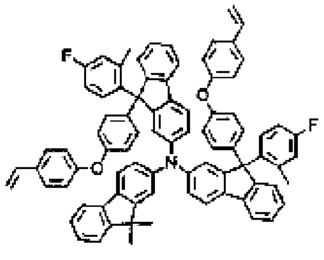 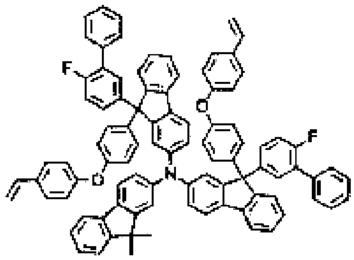 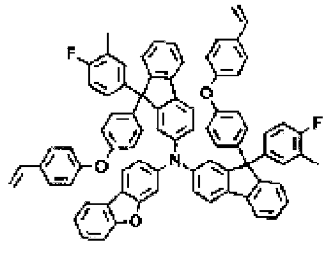

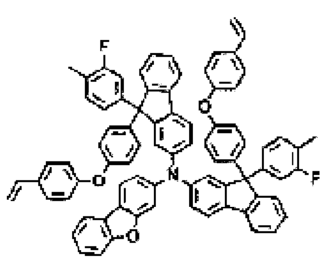 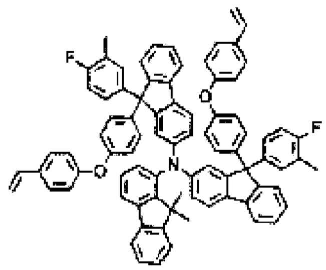 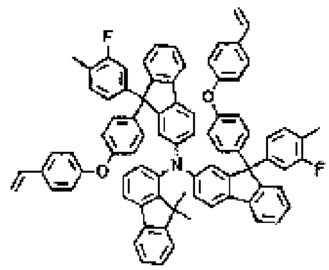

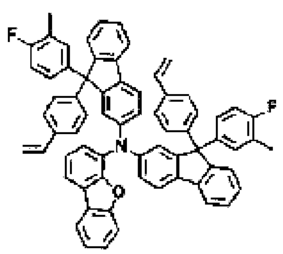 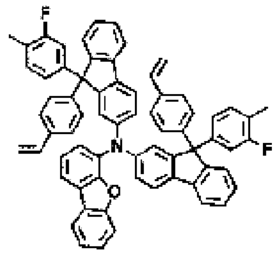 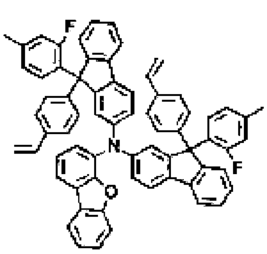

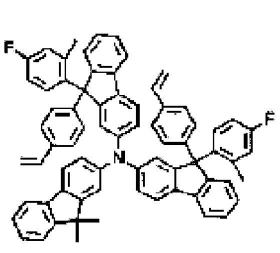 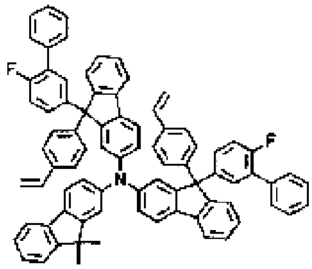 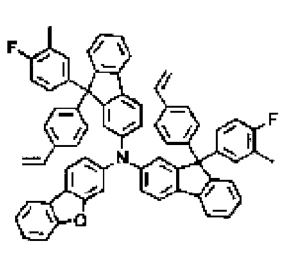

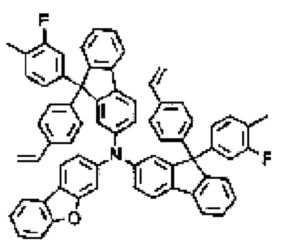 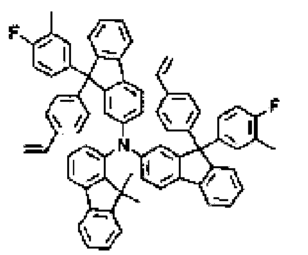 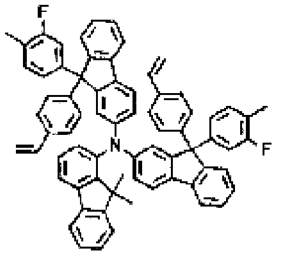

.

**6.** Tintenzusammensetzung nach Anspruch 1, ferner umfassend eine ionische Verbindung, die eine durch die folgende chemische Formel 2 dargestellte anionische Gruppe umfasst:

[Chemische Formel 2]

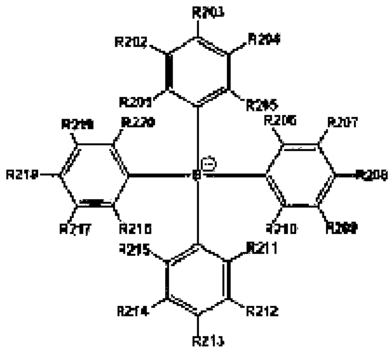

in der chemischen Formel 2
ist mindestens eines von R201 bis R220 F, eine Cyanogruppe oder eine substituierte oder unsubstituierte Fluoralkylgruppe,
mindestens eines der anderen R201 bis R220 ist eine härtbare Gruppe,
die restlichen R201 bis R220 sind gleich oder verschieden voneinander und sind jeweils unabhängig Wasserstoff, Deuterium, eine Nitrogruppe, -C(=)R220', -OR221, -SR222, $-SO_3R223$, -COOR224, -OC(O)R225, -C(O)NR226R227, eine substituierte oder unsubstituierte Alkylgruppe, eine substituierte oder unsubstituierte Fluoralkylgruppe, eine substituierte oder unsubstituierte Alkenylgruppe, eine substituierte oder unsubstituierte Alkinylgruppe, eine substituierte oder unsubstituierte Amingruppe, eine substituierte oder unsubstituierte Arylgruppe oder eine substituierte oder unsubstituierte heterocyclische Gruppe und
R220' und R221 bis R227 sind gleich oder verschieden voneinander und sind jeweils unabhängig Wasserstoff, Deuterium oder eine substituierte oder unsubstituierte Alkylgruppe.

**7.** Tintenzusammensetzung nach Anspruch 6, wobei die chemische Formel 2 eine, ausgewählt aus der Gruppe, bestehend aus den folgenden Verbindungen, ist:

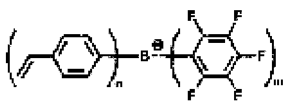
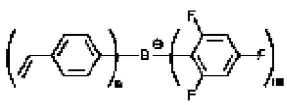
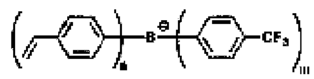

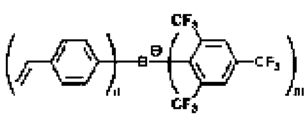
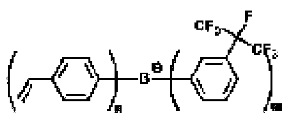
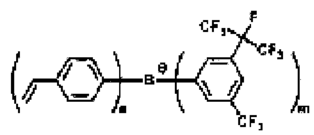

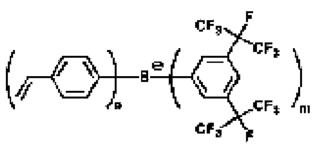
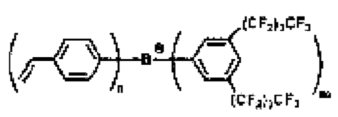
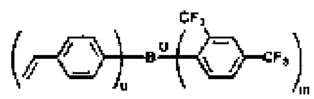

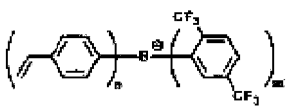
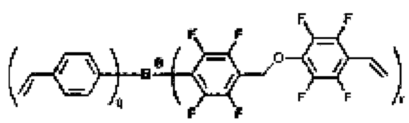

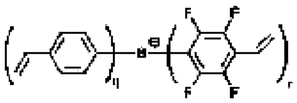

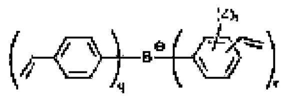

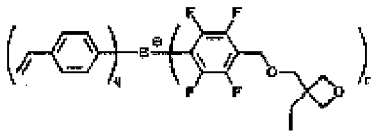

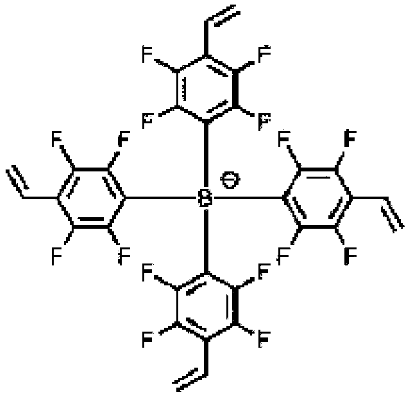

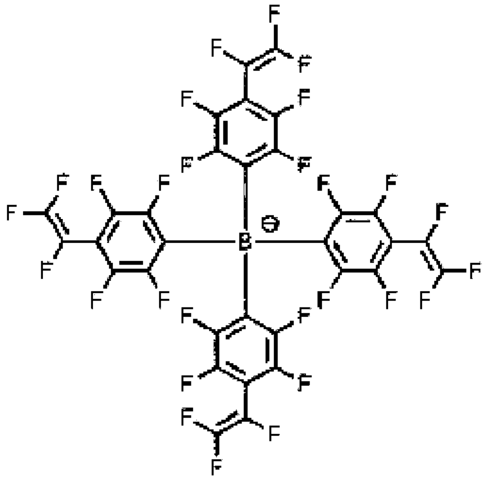

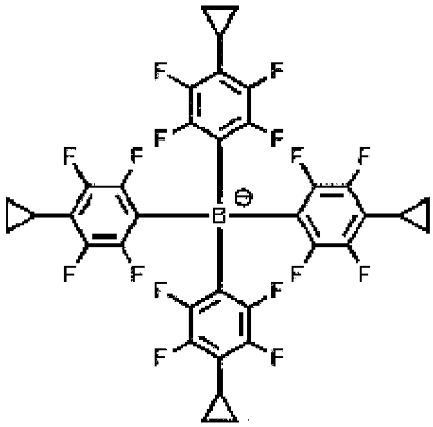

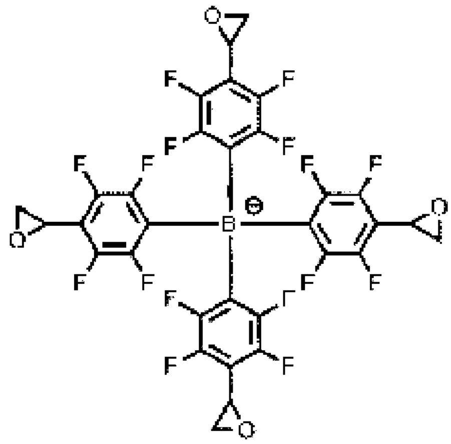

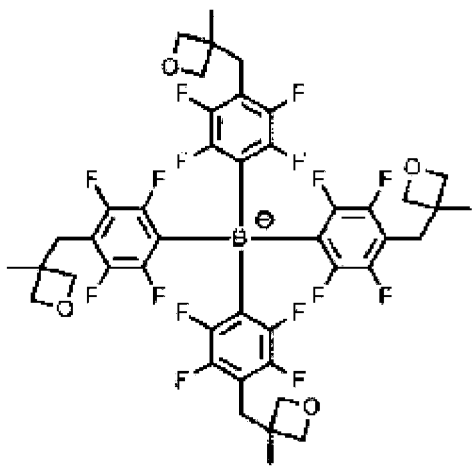

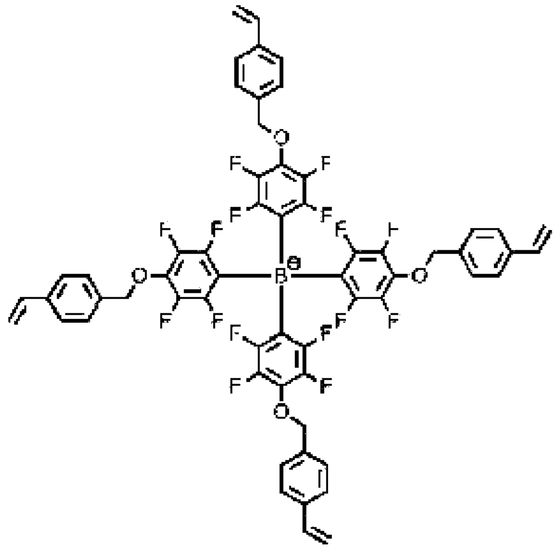

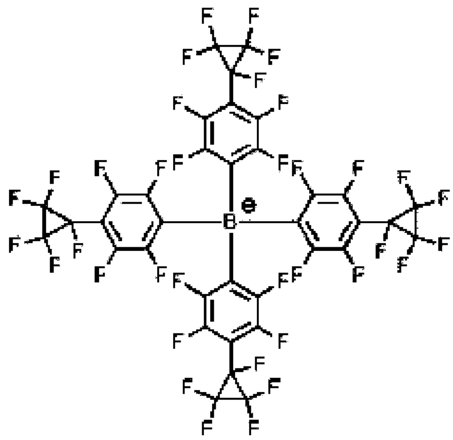

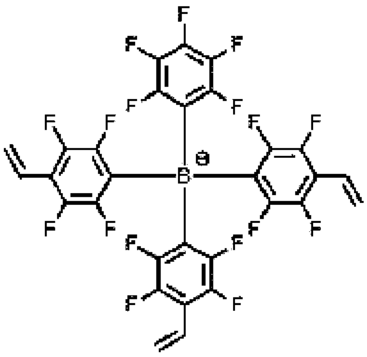

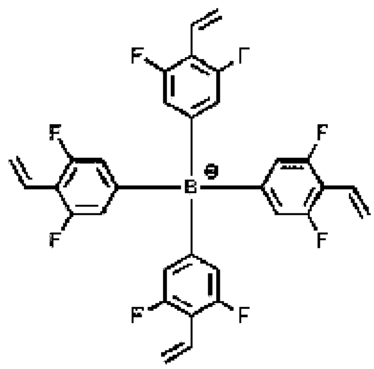

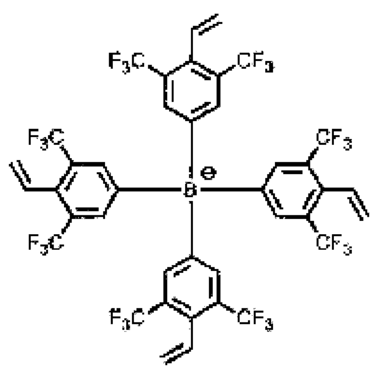

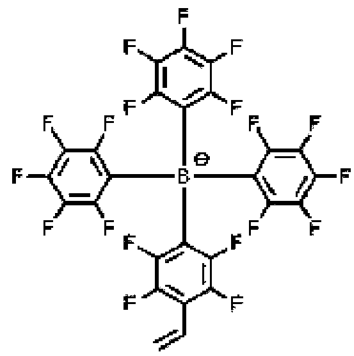

in den Verbindungen
n eine ganze Zahl von 1 bis 3 ist, m eine ganze Zahl von 1 bis 3 ist und m+n=4 ist,
q eine ganze Zahl von 0 bis 3 ist, r eine ganze Zahl von 1 bis 4 ist und q+r=4 ist,
Z Deuterium, eine Halogengruppe, eine Nitrogruppe, eine Cyanogruppe, eine Aminogruppe, -C(O)R220', -OR221, -SR222, -$SO_3$R223, -COOR224, -OC(O)R225, -C(O)NR226R227, eine substituierte oder unsubstituierte Alkylgruppe, eine substituierte oder unsubstituierte Alkenylgruppe, eine substituierte oder unsubstituierte Alkinylgruppe, eine substituierte oder unsubstituierte Amingruppe, eine substituierte oder unsubstituierte Arylgruppe oder eine substituierte oder unsubstituierte heterocyclische Gruppe ist,
l eine ganze Zahl von 1 bis 4 ist, und wenn l 2 oder größer ist, die Z gleich oder verschieden voneinander sind, und R220' und R221 bis R227 gleich oder verschieden voneinander sind und jeweils unabhängig Wasserstoff, Deuterium oder eine substituierte oder unsubstituierte Alkylgruppe sind.

**8.** Tintenzusammensetzung nach Anspruch 6, wobei die ionische Verbindung ferner eine kationische Gruppe umfasst,

die kationische Gruppe eine einwertige kationische Gruppe, eine Oniumverbindung oder eine, ausgewählt aus den folgenden Strukturformeln, ist:

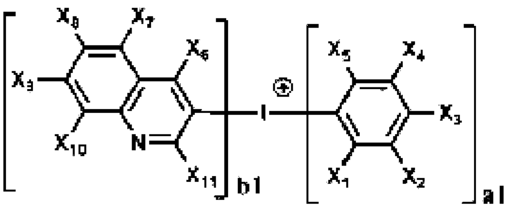

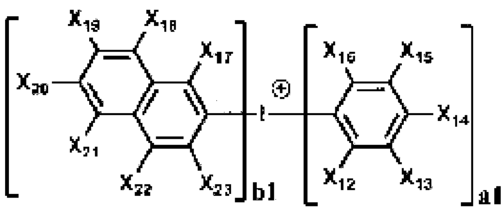

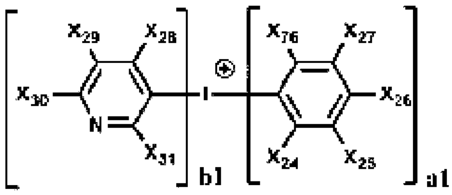

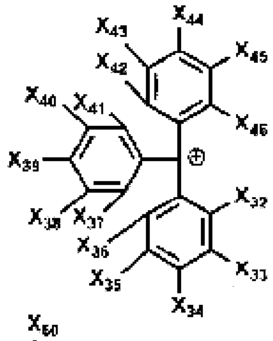

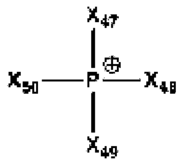

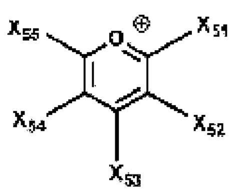

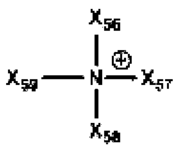

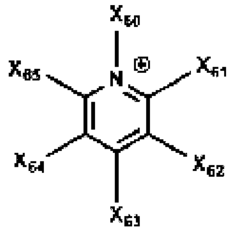

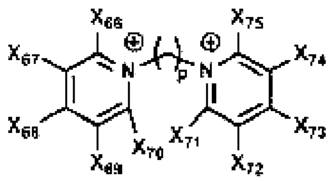

in den Strukturformeln
$X_1$ bis $X_{76}$ gleich oder verschieden voneinander sind und jeweils unabhängig Wasserstoff, Deuterium, eine Cyanogruppe, eine Nitrogruppe, eine Halogengruppe, -COOR224, eine substituierte oder unsubstituierte Alkylgruppe, eine substituierte oder unsubstituierte Alkoxygruppe, eine substituierte oder unsubstituierte Cycloalkylgruppe, eine substituierte oder unsubstituierte Fluoralkylgruppe, eine substituierte oder unsubstituierte Arylgruppe oder eine härtbare Gruppe sind,
R224 Wasserstoff, Deuterium oder eine substituierte oder unsubstituierte Alkylgruppe ist,
p eine ganze Zahl von 0 bis 10 ist und
a1 1 oder 2 ist, b1 0 oder 1 ist und a1+b1=2 ist.

**9.** Tintenzusammensetzung nach Anspruch 8, wobei die Tintenzusammensetzung ferner ein oder mehrere zusätzliche Lösungsmittel, ausgewählt aus der Gruppe, bestehend aus Tetralin, 4-Methoxytoluol, Tetrahydrofuran, Dioxan, Dipropylenglykolmonomethylether, Tripropylenglykolmonomethylether, einem durch die folgende chemische Formel C-1 dargestellten Lösungsmittel und einem durch die folgende chemische Formel C-2 dargestellten Lösungsmittel, umfasst:

[Chemische Formel C-1]

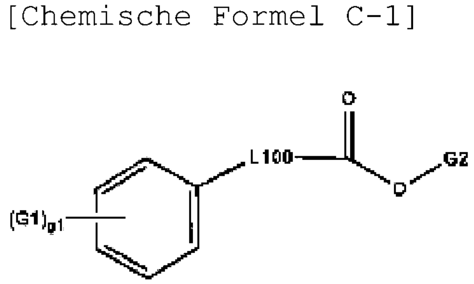

[Chemische Formel C-2]

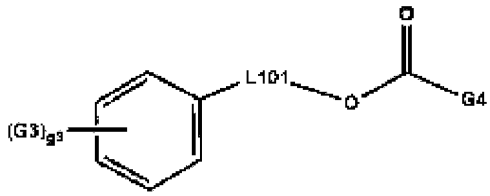

in den chemischen Formeln C-1 und C-2
L100 und L101 gleich oder verschieden voneinander sind und jeweils unabhängig eine direkte Bindung oder eine substituierte oder unsubstituierte Alkylengruppe sind,
G1 und G3 gleich oder verschieden voneinander sind und jeweils unabhängig Wasserstoff, Deuterium, eine substituierte oder unsubstituierte Alkylgruppe eine substituierte oder unsubstituierte Alkoxygruppe, eine substituierte oder unsubstituierte Arylgruppe oder eine substituierte oder unsubstituierte Heteroarylgruppe sind,
G2 eine geradkettige Alkylgruppe ist,
G4 eine substituierte oder unsubstituierte Alkylgruppe ist und
g1 und g3 jeweils eine ganze Zahl von 1 bis 5 sind, und wenn g1 und g3 jeweils 2 oder größer sind, zwei oder mehr Substituenten in der Klammer gleich oder verschieden voneinander sind.

**10.** Tintenzusammensetzung nach Anspruch 9, wobei, bezogen auf 100 Gew.-% der Tintenzusammensetzung,

die durch die chemische Formel 1 dargestellte Verbindung in einer Menge von 0,1 Gew.-% und 15 Gew.-% umfasst ist,
das durch die chemische Formel A dargestellte Lösungsmittel in einer Menge von 1 Gew.-% bis 70 Gew.-% umfasst ist,
die ionische Verbindung, die die durch die chemische Formel 2 dargestellte anionische Gruppe umfasst, in einer Menge von 0,1 Gew.-% bis 15 Gew.-% umfasst ist und
das zusätzliche Lösungsmittel in einer Menge von 10 Gew.-% bis 85 Gew.-% umfasst ist.

**11.** Pixel, umfassend die Tintenzusammensetzung nach einem der Ansprüche 1, 2, 4 und 7 bis 10 oder ein gehärtetes Produkt davon.

**12.** Organische Materialschicht, umfassend das Pixel nach Anspruch 11.

**13.** Organische Materialschicht nach Anspruch 12, wobei die organische Materialschicht n' Pixel umfasst,

die n' Pixel eine Dickengleichmäßigkeit V von 0,4 oder niedriger aufweisen und
die Dickengleichmäßigkeit V die folgende Gleichung 1 erfüllt:

[Gleichung 1]

$$V = \frac{\sum_{i=2}^{n'} \left(X(i) - Xm'\right)^2}{n' \times Xm'}$$

in Gleichung 1
X(i) die Dicke des i-ten Pixel ist,
Xm' der Durchschnittswert von X(i) ist, wobei i 2 bis n' ist, und
n' eine ganze Zahl von 2 bis $10^4$ ist.

**14.** Organische lichtemittierende Vorrichtung, umfassend:

eine erste Elektrode,
eine zweite Elektrode und
eine organische Materialschicht mit einer oder mehreren Schichten, die zwischen der ersten Elektrode und der zweiten Elektrode vorhanden ist,
wobei eine oder mehrere Schichten der organischen Materialschicht die organische Materialschicht nach Anspruch 12 sind.

**15.** Organische lichtemittierende Vorrichtung nach Anspruch 14, wobei die organische Materialschicht eine oder mehrere Schichten, ausgewählt aus der Gruppe, bestehend aus einer Lochinjektionsschicht, einer Lochtransportschicht, einer Lochinjektions- und -transportschicht und einer Elektronenblockierschicht, umfasst, und
die eine oder mehreren Schichten, ausgewählt aus der Gruppe, bestehend aus der Lockinjektionsschicht, der Locktransportschicht, der Lochinjektions- und -transportschicht und der Elektronenblockierschicht, die Tintenzusammensetzung oder ein gehärtetes Produkt davon umfasst.

## Revendications

**1.** Composition d'encre comprenant un composé représenté par la formule chimique 1 suivante et un solvant représenté par la formule chimique A suivante :

[Formule chimique 1]

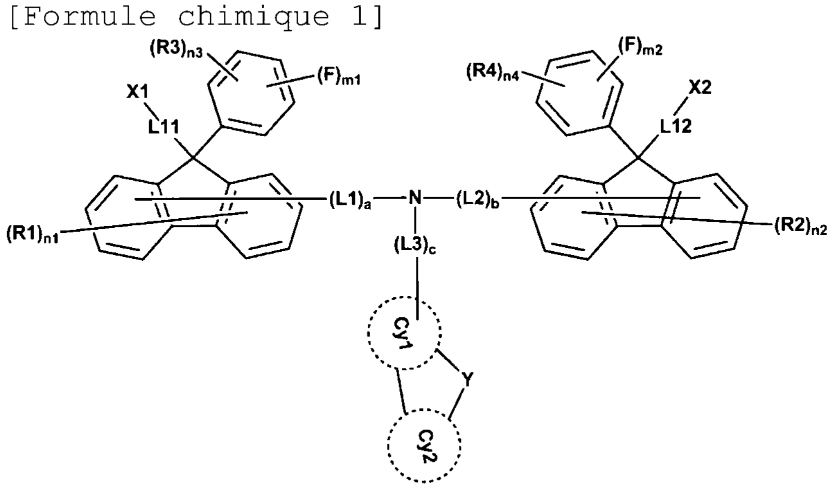

[Formule chimique A]

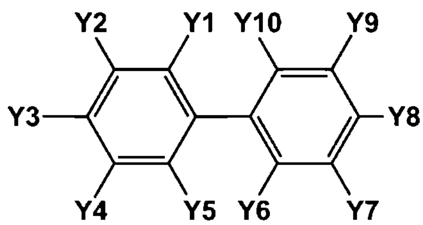

dans laquelle, dans les formules chimiques 1 et A,
Y représente O, S, CRaRb ou SiRcRd,
Cy1 et Cy2 sont identiques ou différents l'un de l'autre et représentent chacun indépendamment un cycle benzène substitué ou non substitué ; ou un cycle naphtalène substitué ou non substitué,
Ra, Rb, Rc et Rd sont identiques ou différents les uns des autres, et représentent chacun indépendamment un atome d'hydrogène ; un atome de deutérium ; un groupe alkyle substitué ou non substitué ; un groupe aryle substitué ou non substitué ; ou un groupe hétéroaryle substitué ou non substitué, ou sont liés à un groupe adjacent pour former un cycle substitué ou non substitué,
L1 à L3 sont identiques ou différents les uns des autres, et représentent chacun indépendamment une liaison directe ; ou un groupe arylène substitué ou non substitué,

L11 et L12 sont identiques ou différents l'un de l'autre, et représentent chacun indépendamment une liaison directe ; un groupe alkylène substitué ou non substitué ; ou un groupe arylène substitué ou non substitué,
X1 et X2 sont identiques ou différents l'un de l'autre, et sont chacun indépendamment un groupe durcissable,
R1 et R2 sont identiques ou différents l'un de l'autre, et représentent chacun indépendamment un deutérium ; un groupe halogène ; un groupe alkyle substitué ou non substitué ; un groupe alcoxy substitué ou non substitué ; un groupe aryle substitué ou non substitué ; ou un groupe hétéroaryle substitué ou non substitué,
R3 et R4 sont identiques ou différents l'un de l'autre, et représentent chacun indépendamment le deutérium ; un groupe alkyle substitué ou non substitué ; un groupe alcoxy substitué ou non substitué ; un groupe aryle substitué ou non substitué ; ou un groupe hétéroaryle substitué ou non substitué,
a, b et c sont chacun égaux à 0 ou 1,
n1 et n2 sont chacun un nombre entier de 0 à 7, et lorsque n1 et n2 sont chacun 2 ou plus, deux ou plusieurs substituants entre parenthèses sont identiques ou différents les uns des autres,
m1 et m2 sont chacun un nombre entier compris entre 1 et 5, n3 et n4 sont chacun un nombre entier compris entre 0 et 4, m1+n3 est inférieur ou égal à 5, et m2+n4 est inférieur ou égal à 5, et
Y1 à Y10 sont identiques ou différents les uns des autres, et sont chacun indépendamment un atome d'hydrogène ; un atome de deutérium ; un groupe hydroxyle ; un groupe éther ; un groupe carbonyle ; un groupe ester ; un groupe alkyle substitué ou non substitué ; un groupe cycloalkyle substitué ou non substitué ; un groupe cycloalcényle substitué ou non substitué ; un groupe alcoxy substitué ou non substitué ; un groupe aryle substitué ou non substitué ; ou un groupe amine substitué ou non substitué.

2. La composition de la revendication 1, dans laquelle le groupe durcissable est choisi parmi le groupe constitué des structures suivantes :

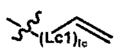 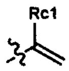 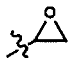 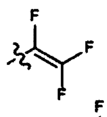 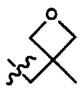

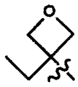 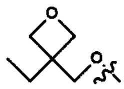 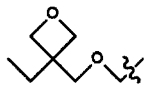 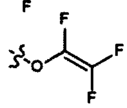 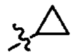

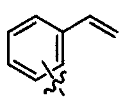 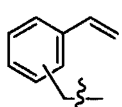 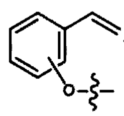 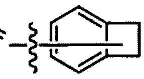 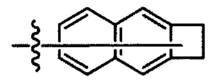

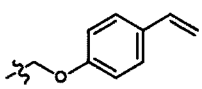 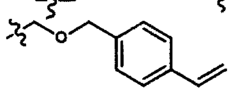 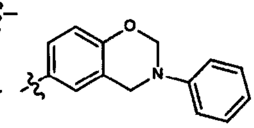

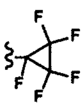 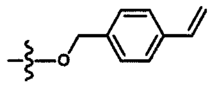 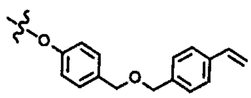

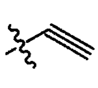 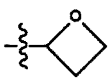 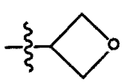 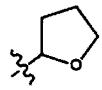 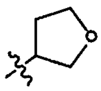

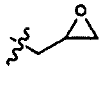 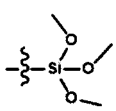 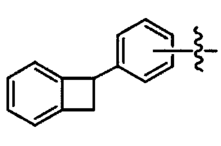 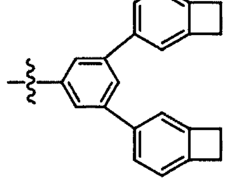

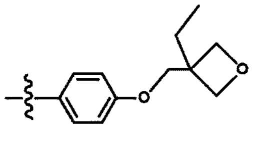 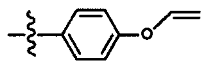 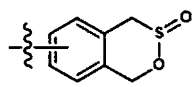

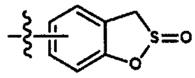

dans les structures,

Lc1 est une liaison directe ; -O- ; -S- ; un groupe alkylène substitué ou non substitué ; un groupe arylène substitué ou non substitué ; ou un groupe hétéroarylène substitué ou non substitué,

lc vaut 1 ou 2,

lorsque lc vaut 2, les Lc1 sont identiques ou différents les uns des autres,

Rc1 est un groupe alkyle substitué ou non substitué, et

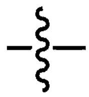

est un groupement lié à la formule chimique 1.

**3.** La composition d'encre de la revendication 1, dans laquelle le solvant représenté par la formule chimique A est choisi parmi le groupe constitué des composés suivants :

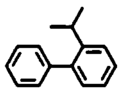 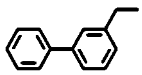 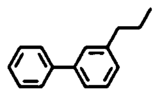 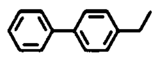

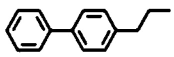 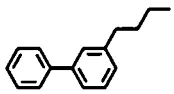 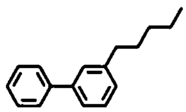 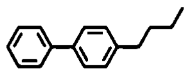

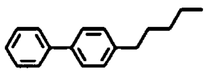 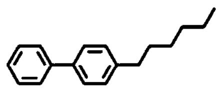 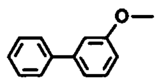 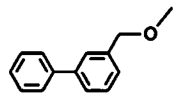

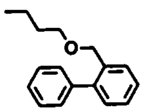 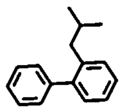 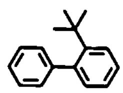

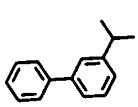 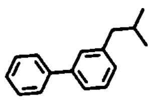 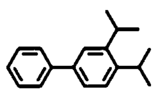 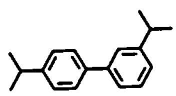

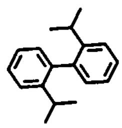 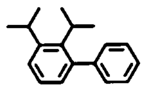 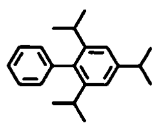 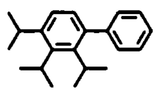

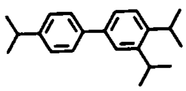 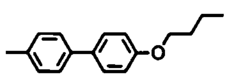

**4.** La composition d'encre de la revendication 1, dans laquelle la formule chimique 1 est représentée par la formule chimique 11 suivante :

[Formule chimique 11]

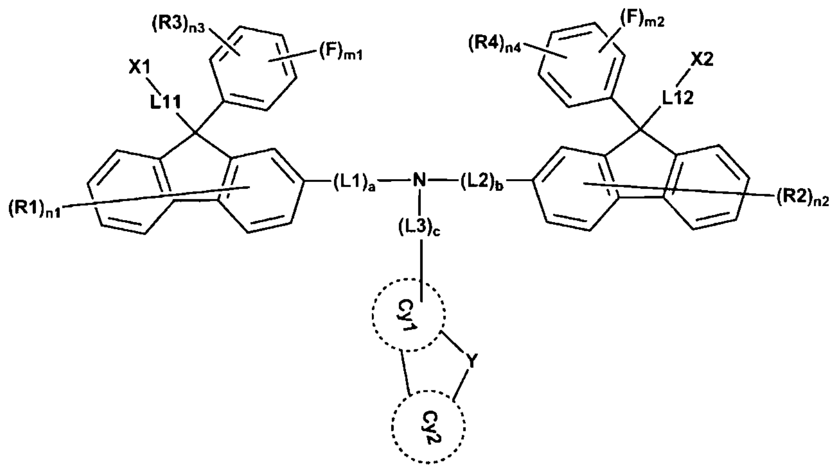

dans la formule chimique 11,
les définitions de L1 à L3, L11, L12, X1, X2, R1 à R4, Y, Cy1, Cy2, a, b, c, n1 à n4, m1 et m2 sont les mêmes que celles de la formule chimique 1.

5. La composition d'encre de la revendication 1, dans laquelle la formule chimique 1 est l'une quelconque choisie parmi le groupe constitué des composés suivants :

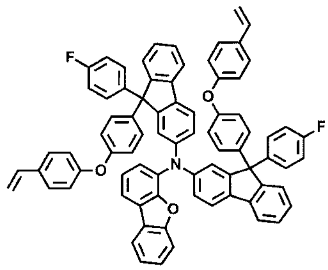
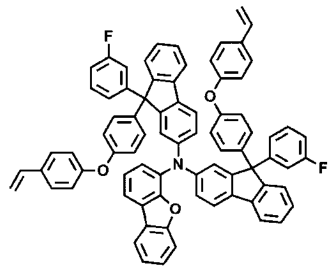
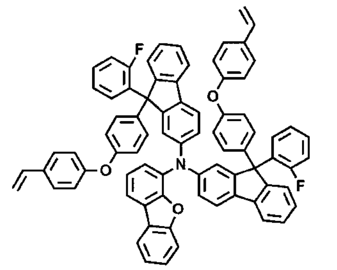
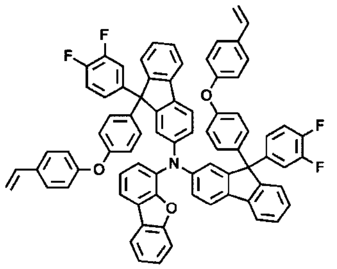
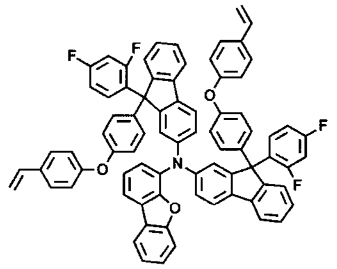
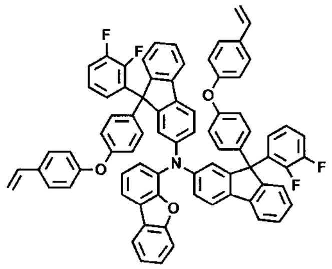
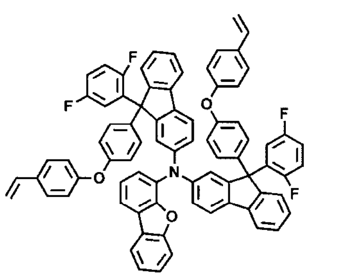
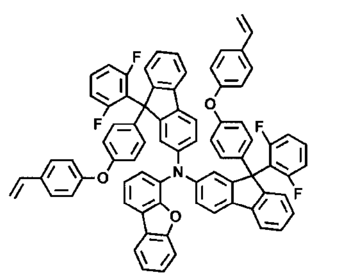
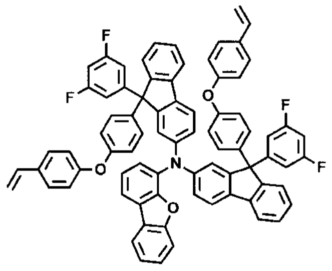

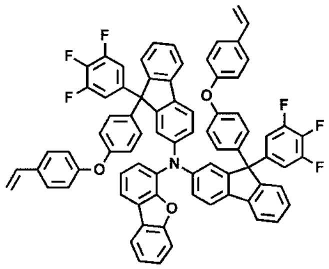
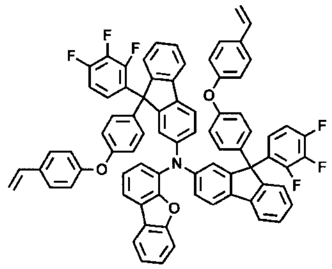
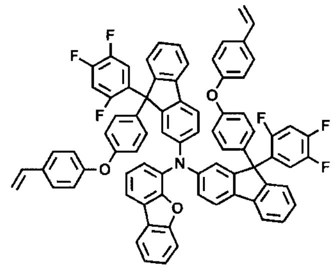
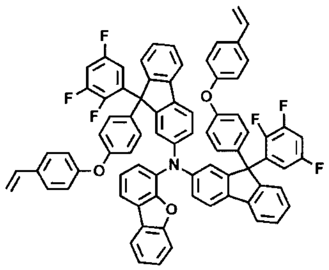
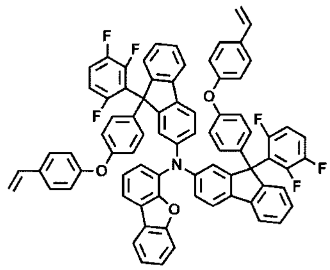
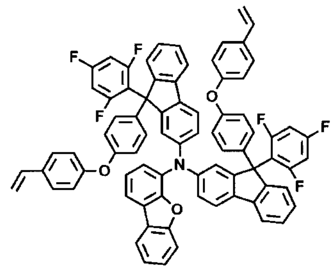
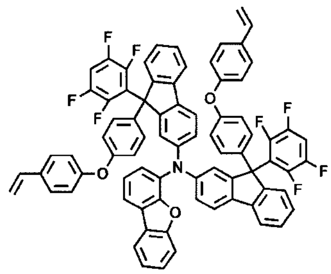
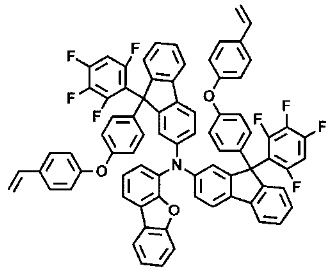
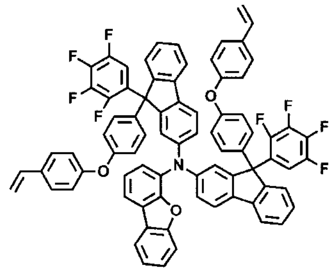
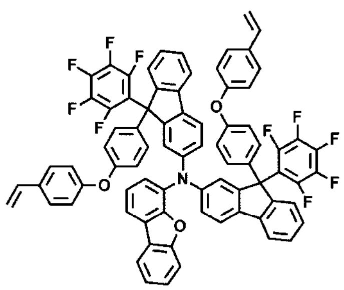
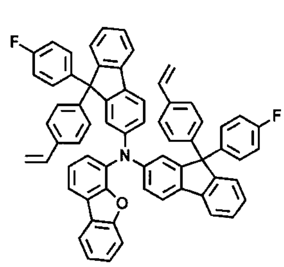
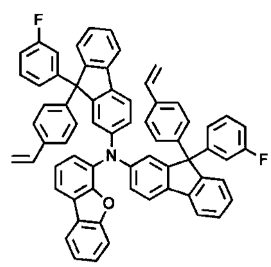
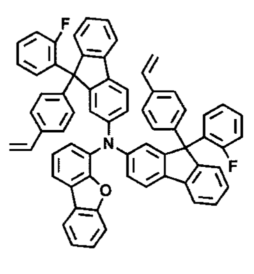
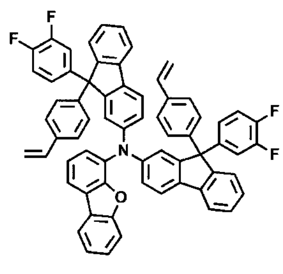
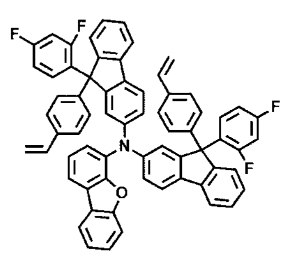
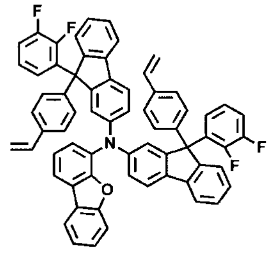
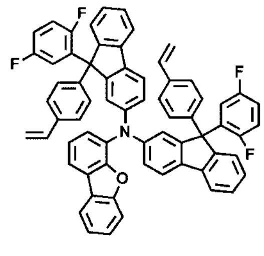
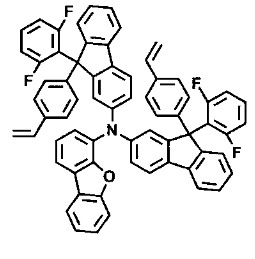

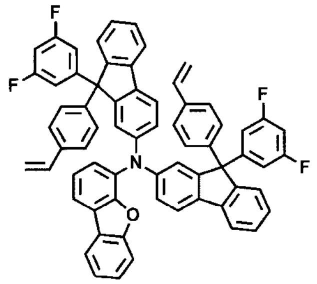
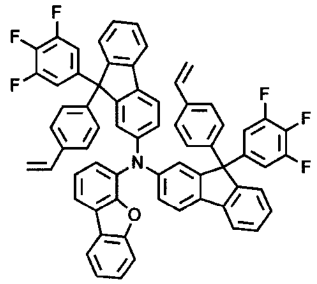
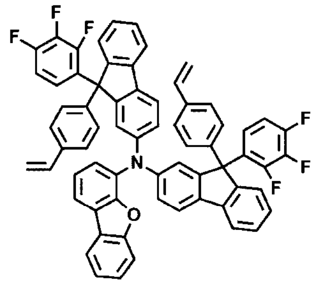

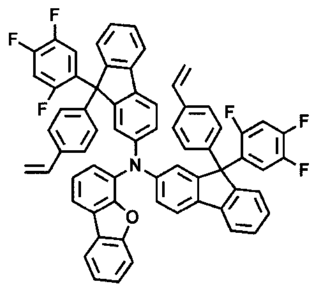
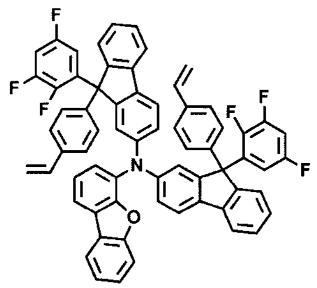
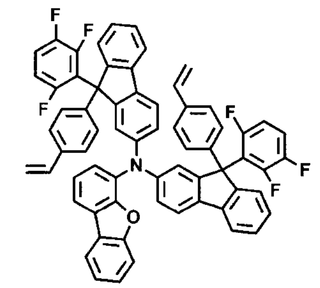

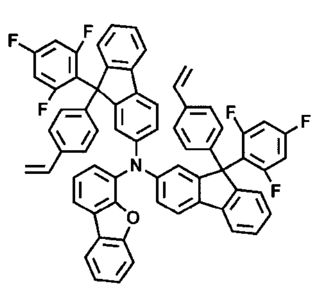
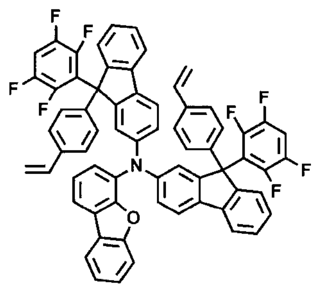
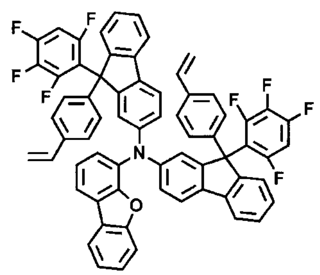

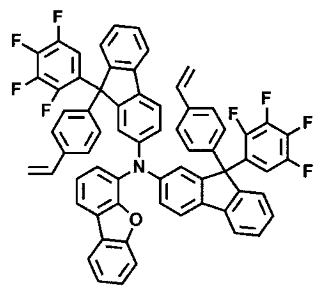
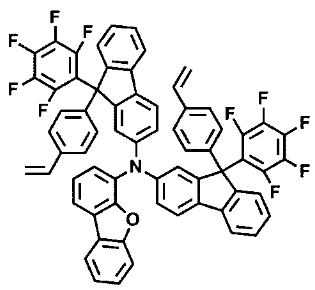

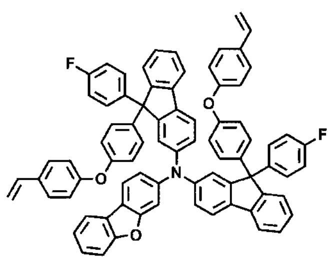
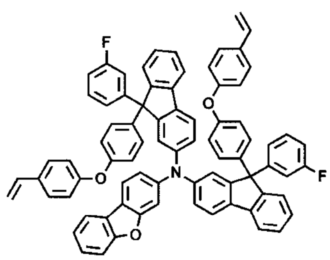
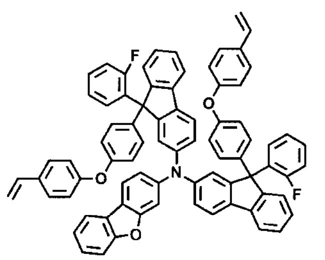

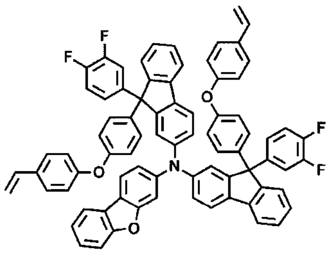
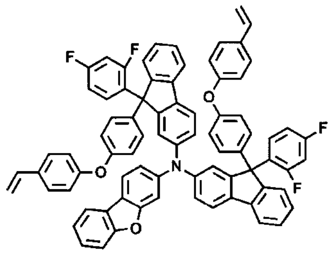
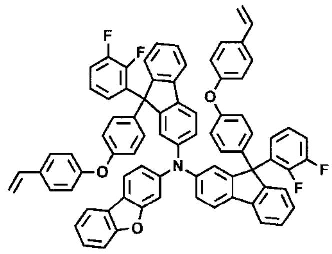

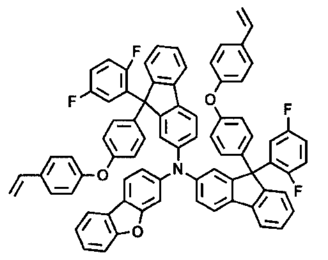
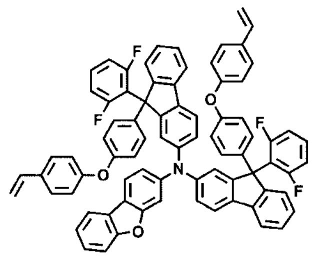
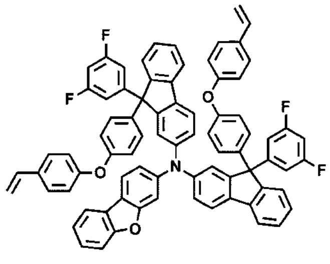

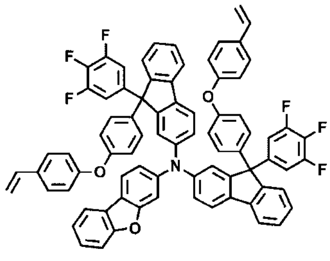
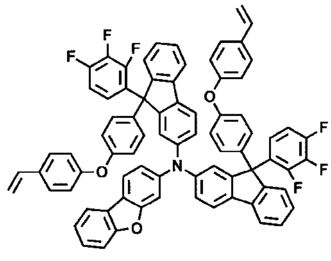
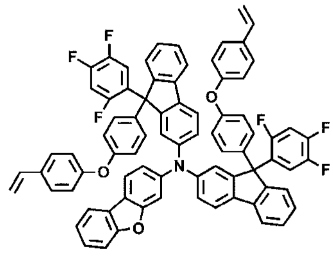

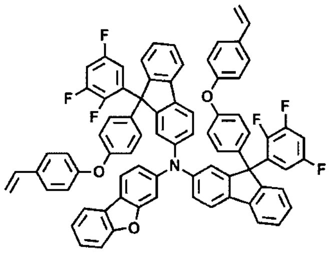
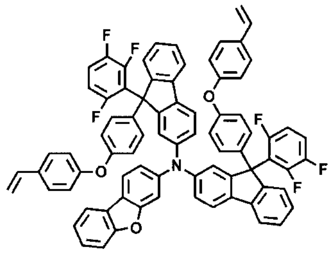
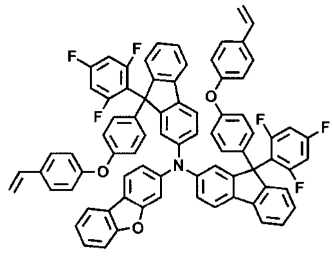

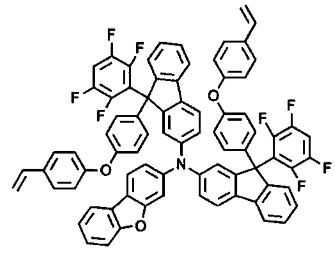
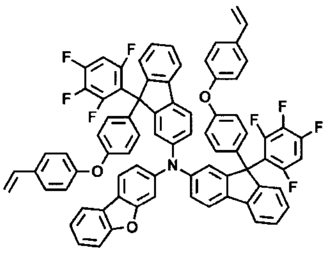
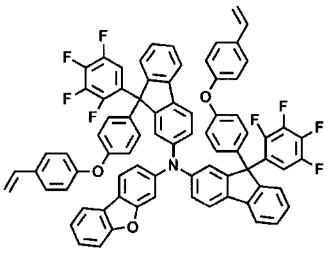

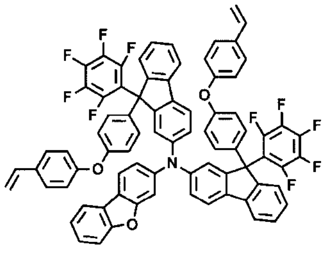
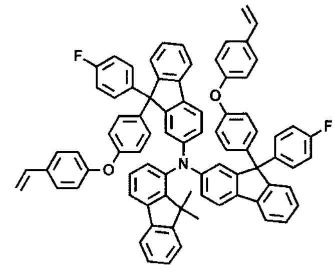
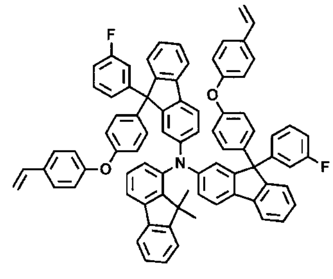

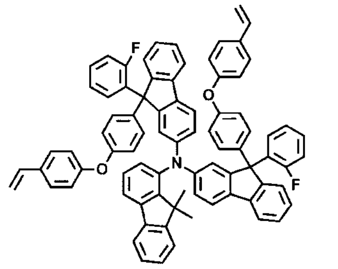

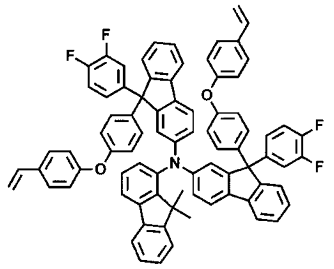

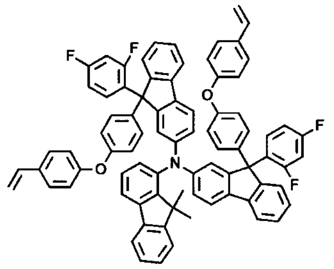

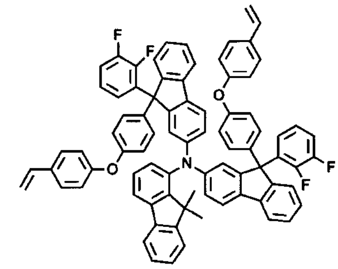

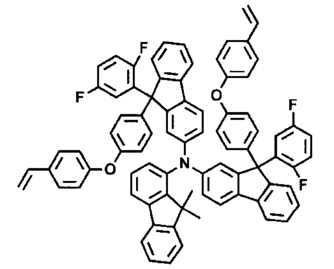

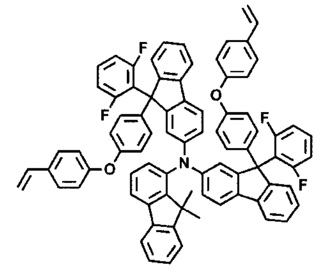

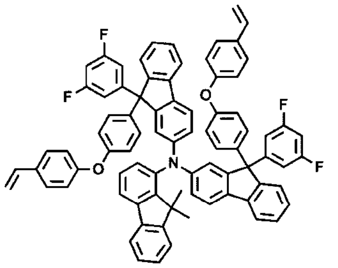

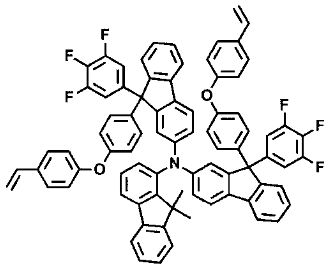

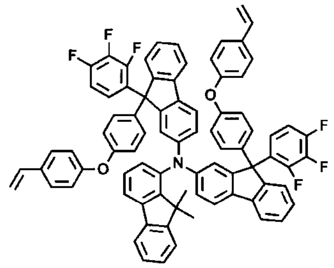

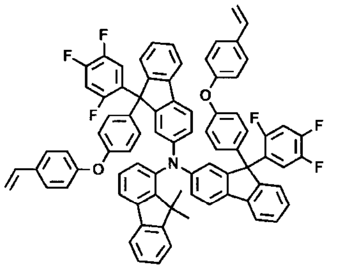

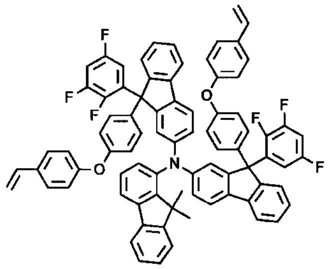

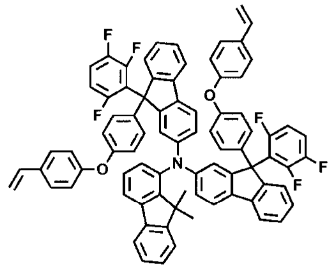

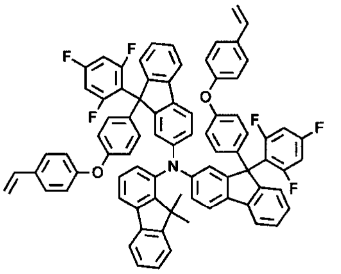

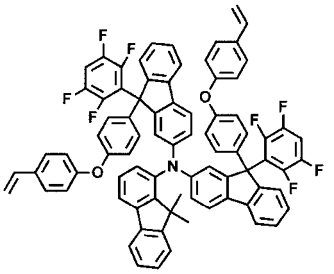

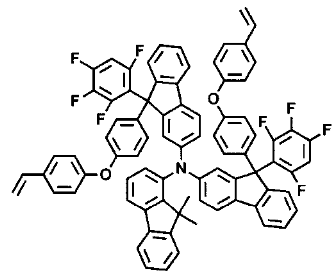

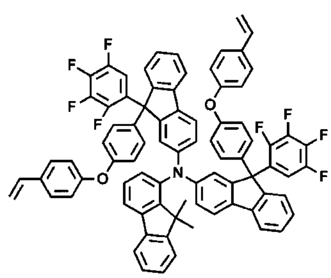
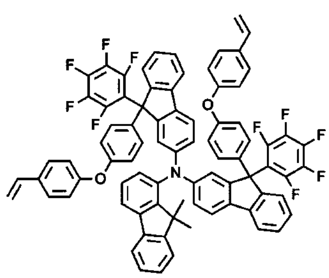

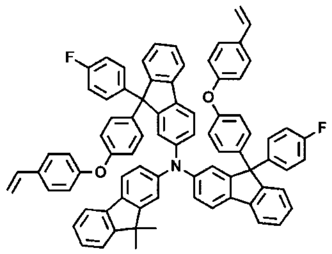
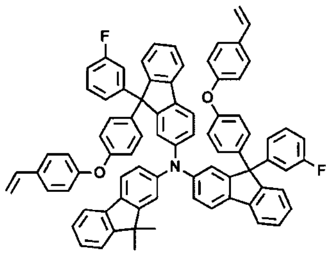
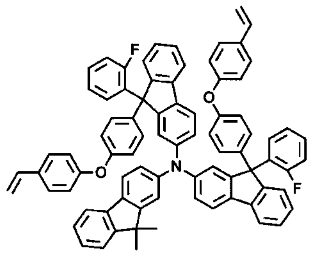

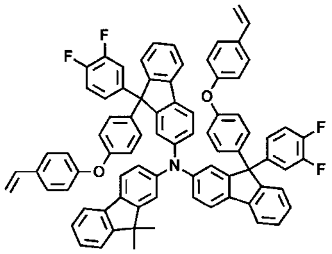
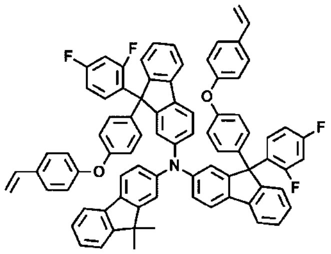
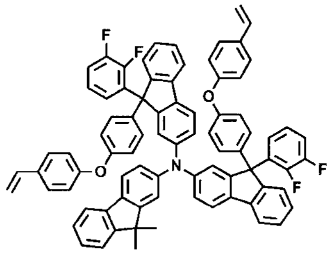

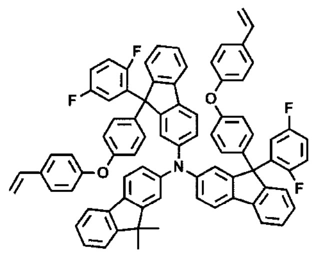
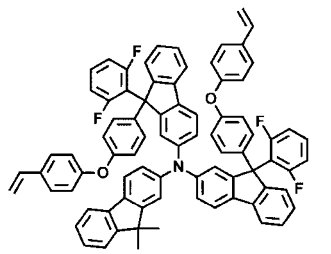
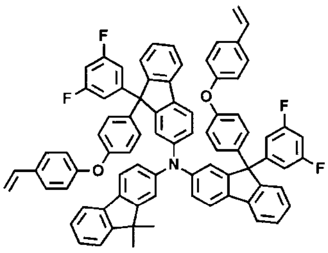

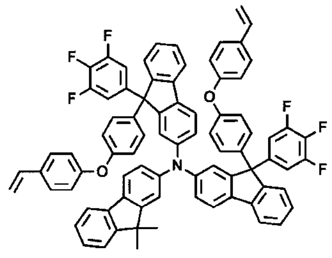
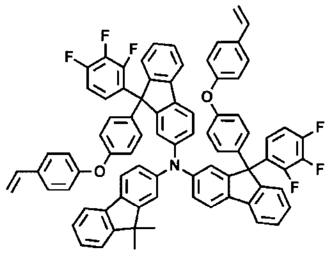
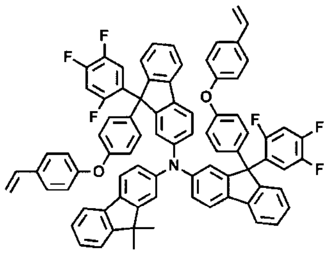

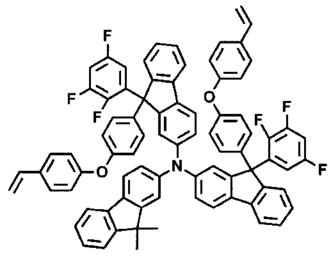
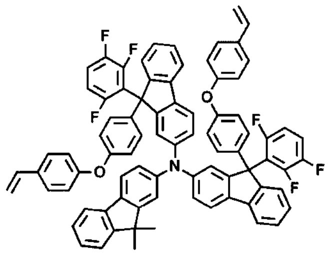
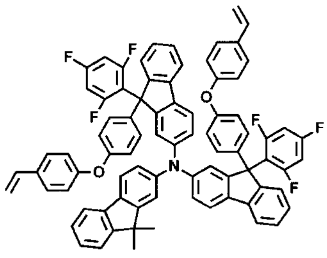

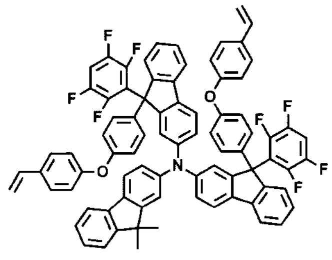
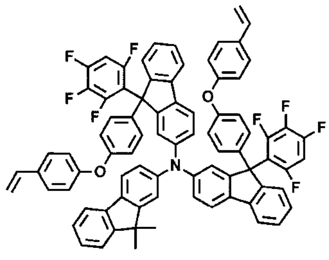
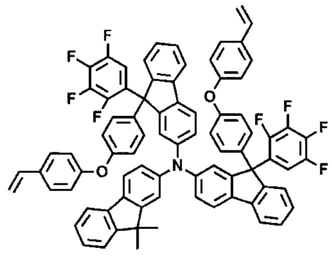

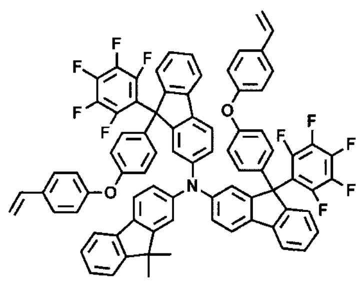
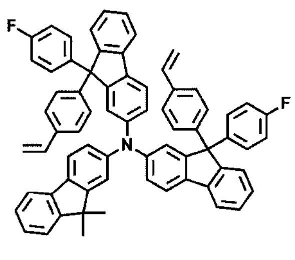
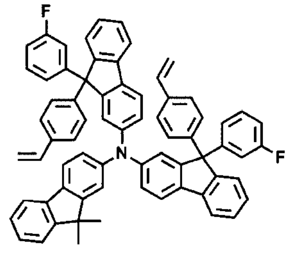

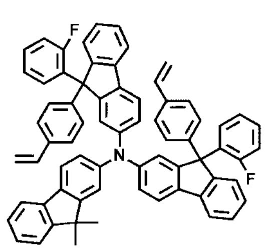
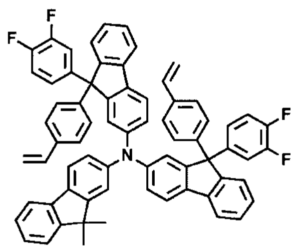
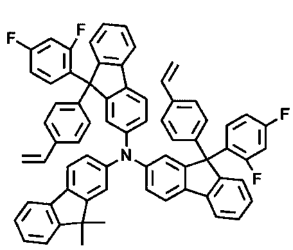

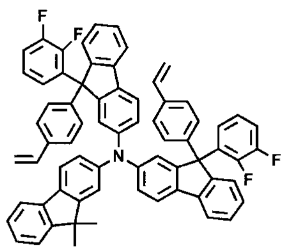
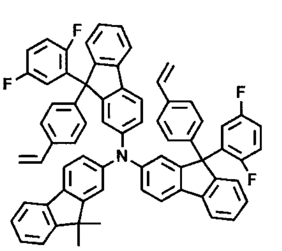
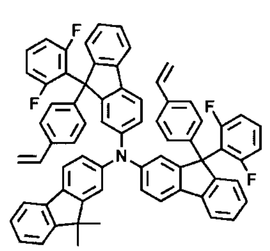

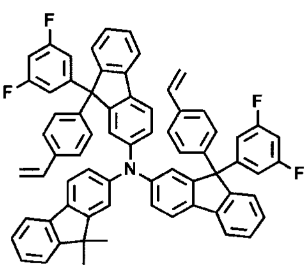
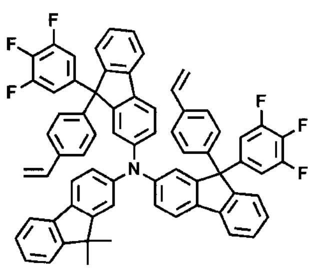
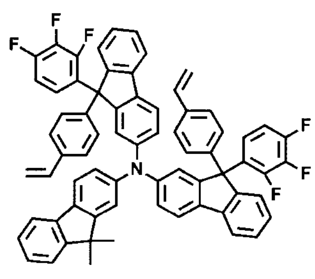

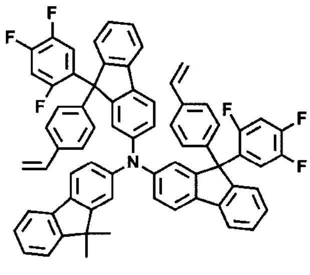
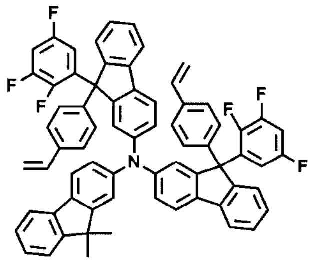
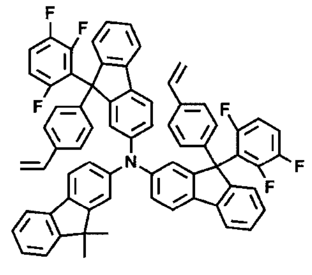

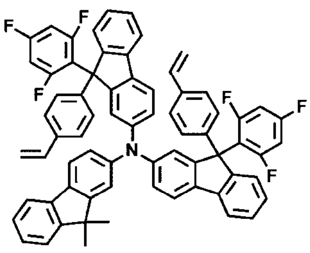
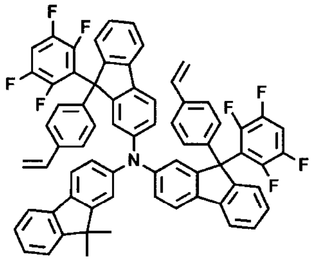
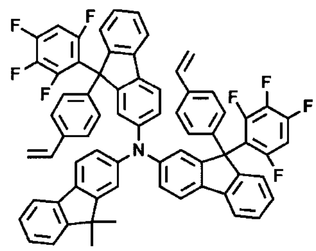

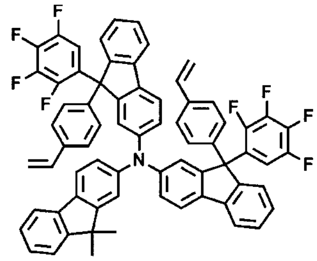
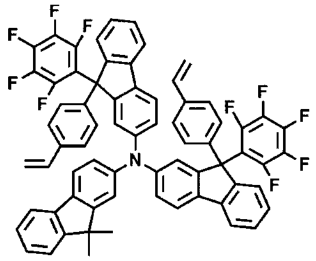

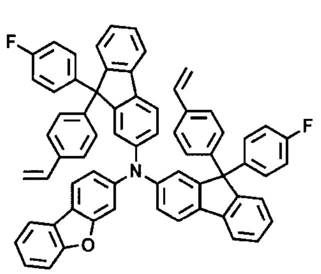
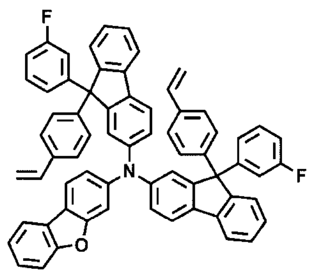
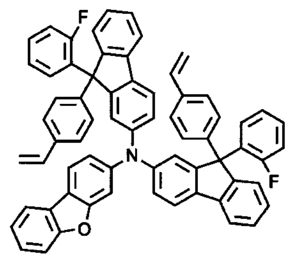

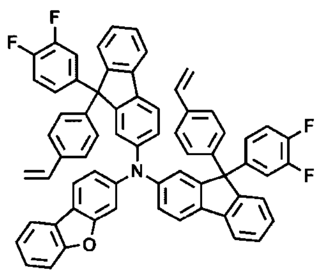
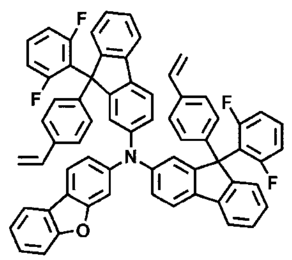
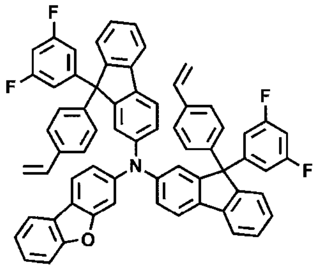

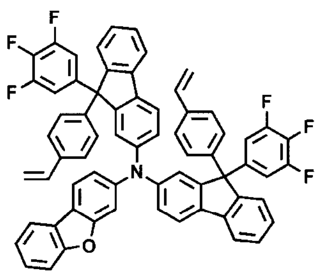
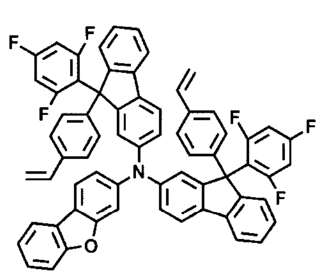
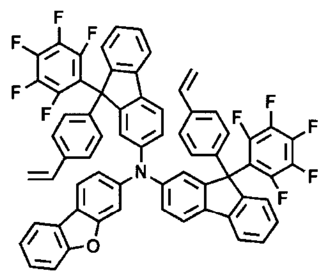

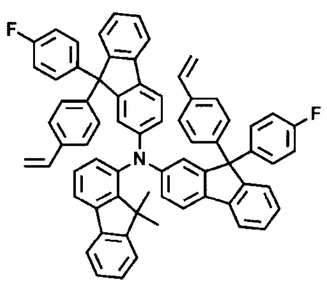
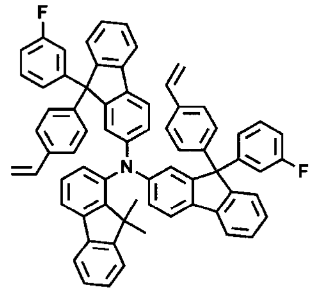
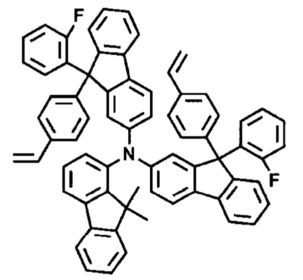
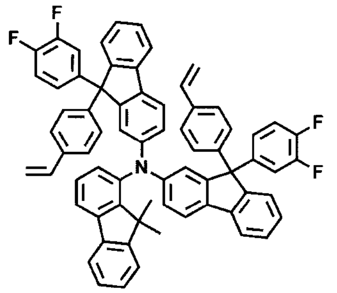
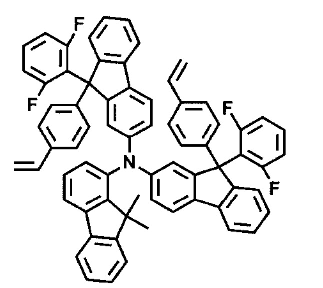
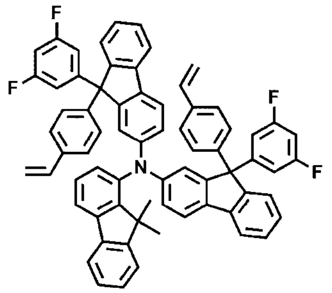
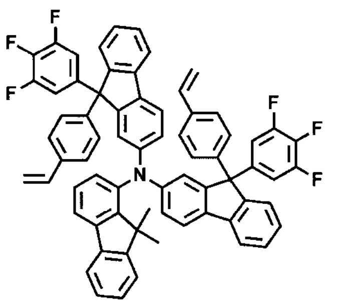
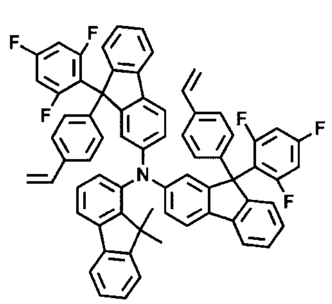
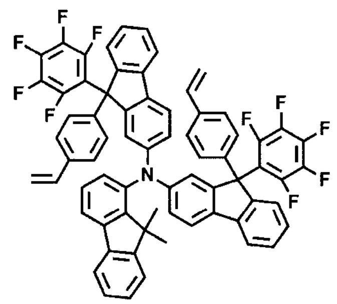
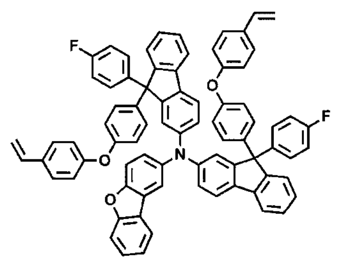
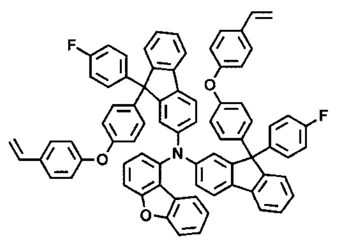
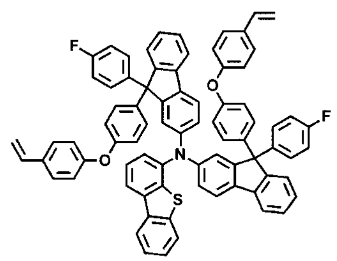
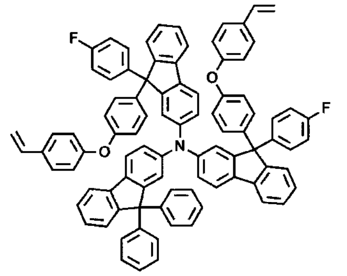
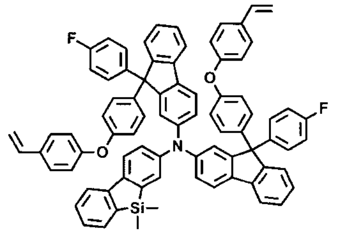
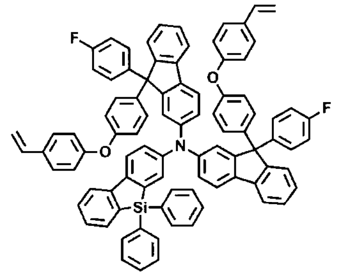

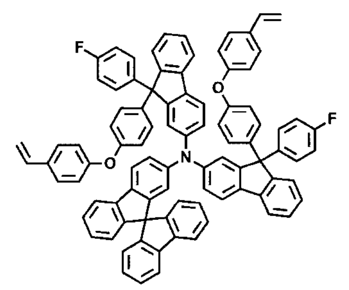 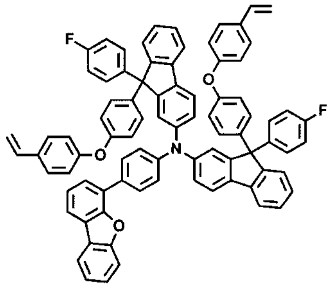 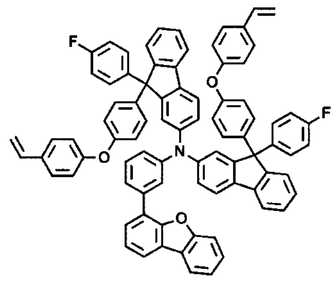

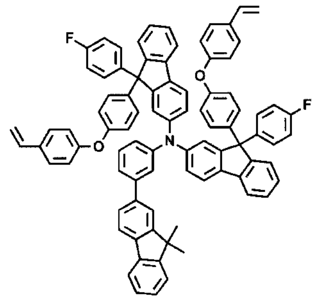 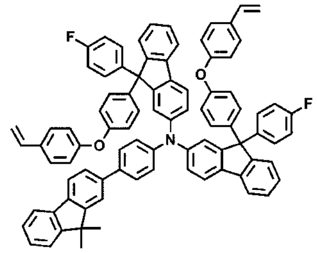 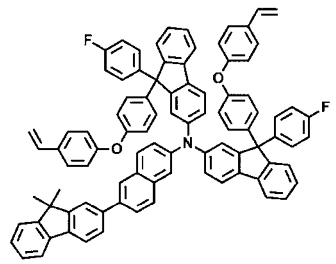

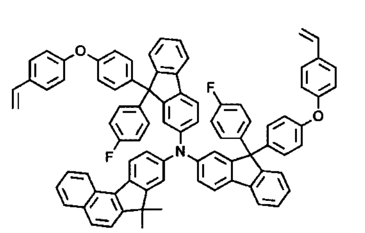 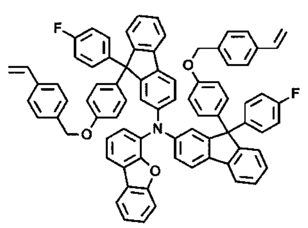 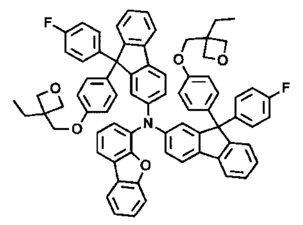

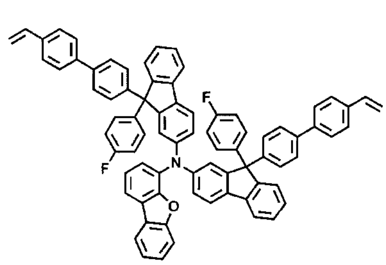 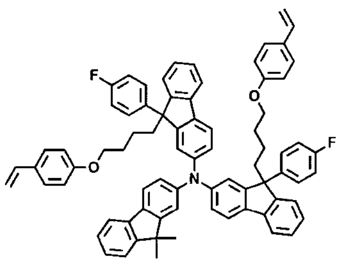 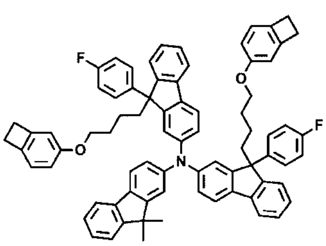

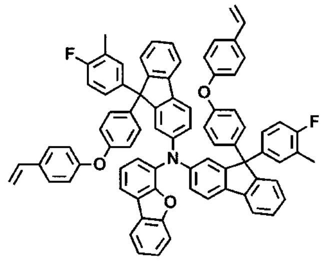 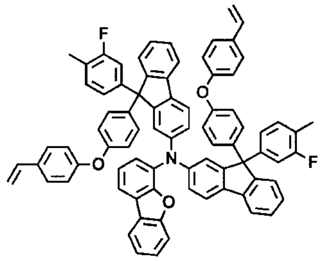 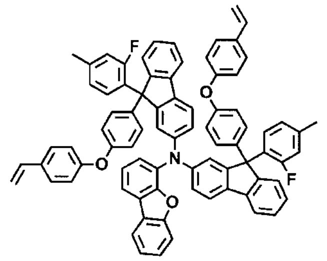

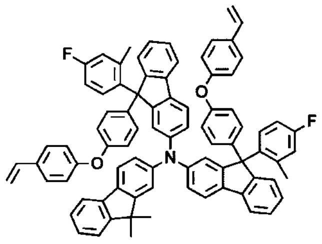 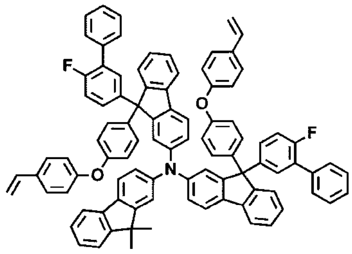 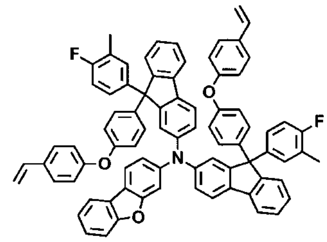

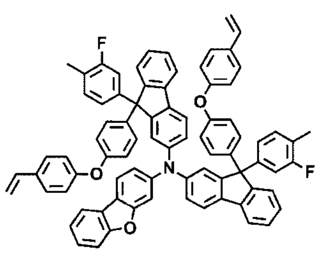
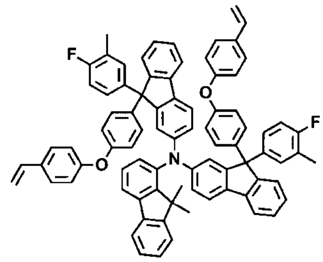
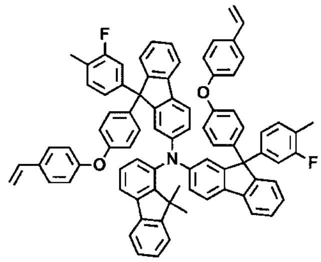

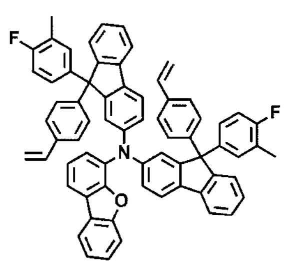
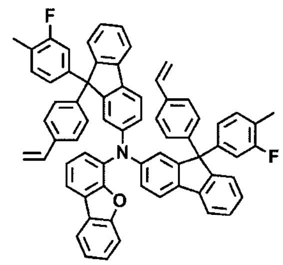
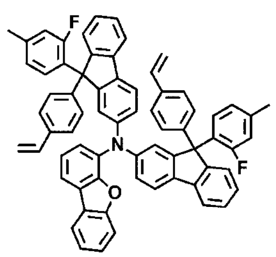

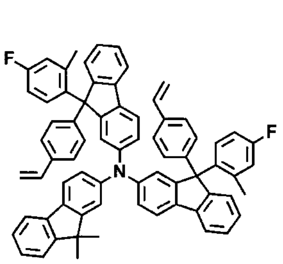
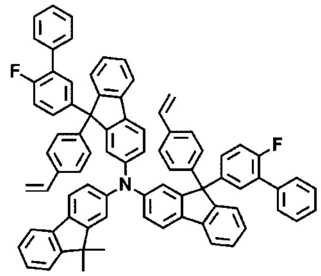
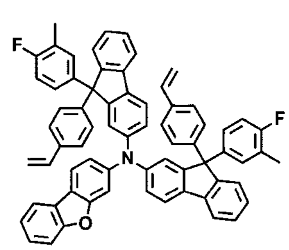

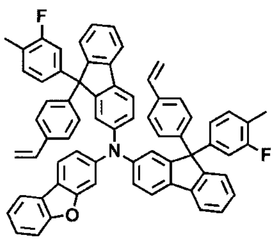
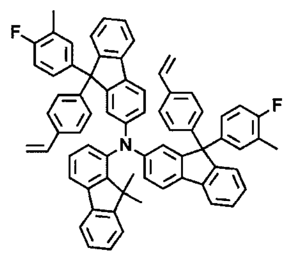
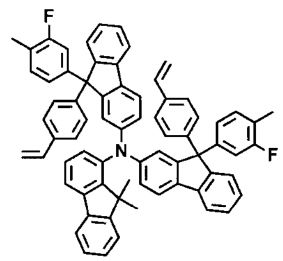

**6.** La composition d'encre de la revendication 1, comprenant en outre un composé ionique comprenant un groupe anionique représenté par la formule chimique 2 suivante :

[Formule chimique 2]

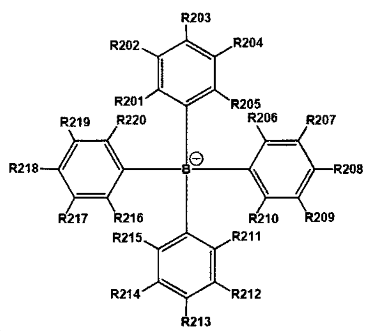

dans la formule chimique 2,
au moins l'un des R201 à R220 est F ; un groupe cyano ; ou un groupe fluoroalkyle substitué ou non substitué,

au moins l'un des autres R201 à R220 est un groupe durcissable,
les R201 à R220 restants sont identiques ou différents les uns des autres, et sont chacun indépendamment un atome d'hydrogène ; du deutérium ; un groupe nitro ; - C(O)R220' ; -OR221 ; -SR222 ; -SO3R223 ; -COOR224 ; -OC(O)R225 ; -C(O)NR226R227 ; un groupe alkyle substitué ou non substitué ; un groupe fluoroalkyle substitué ou non substitué ; un groupe alcényle substitué ou non substitué ; un groupe alcynyle substitué ou non substitué ; un groupe amine substitué ou non substitué ; un groupe aryle substitué ou non substitué ; ou un groupe hétérocyclique substitué ou non substitué, et R220' et R221 à R227 sont identiques ou différents les uns des autres, et sont chacun indépendamment un atome d'hydrogène ; un atome de deutérium ; ou un groupe alkyle substitué ou non substitué.

**7.** La composition d'encre de la revendication 6, dans laquelle la formule chimique 2 est l'une quelconque choisie parmi le groupe constitué des composés suivants :

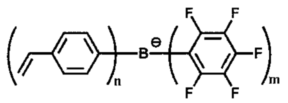
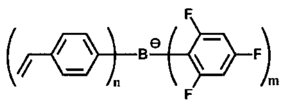
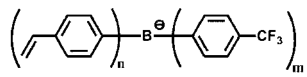

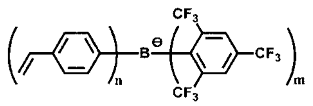
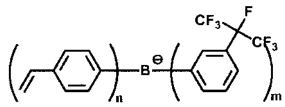
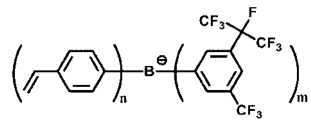

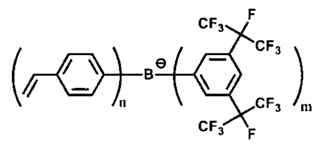
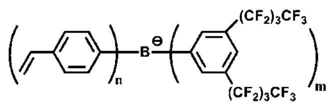
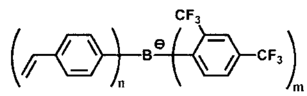

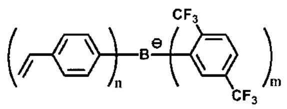
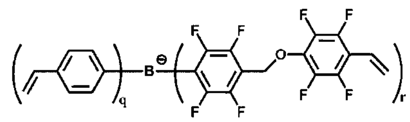

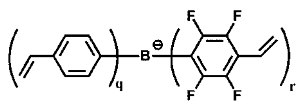
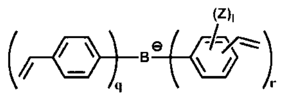
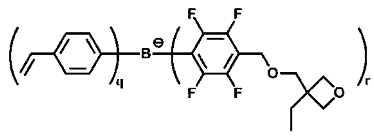

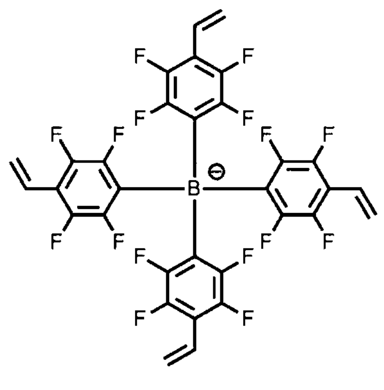
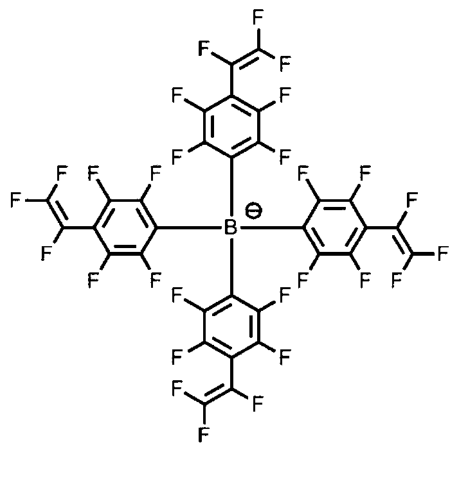

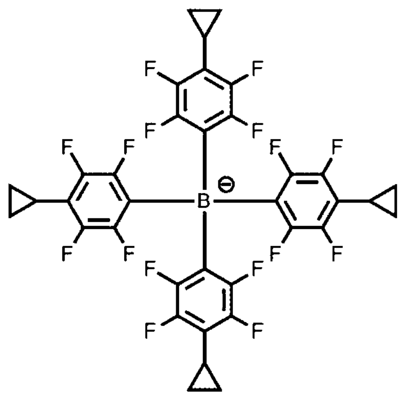

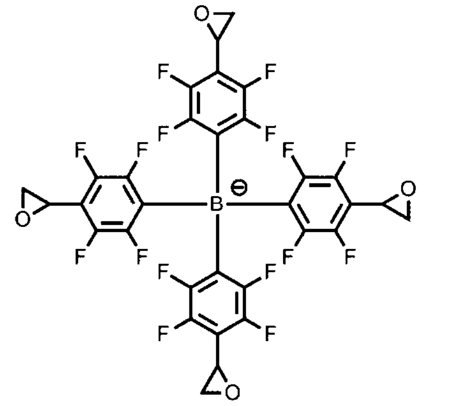

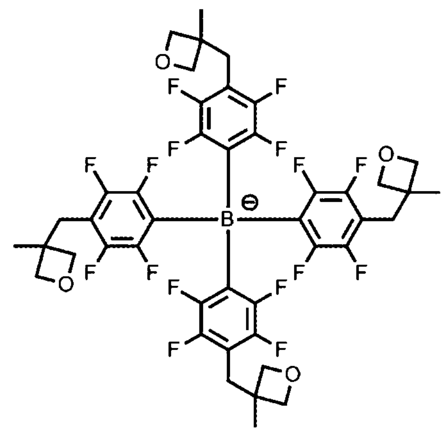

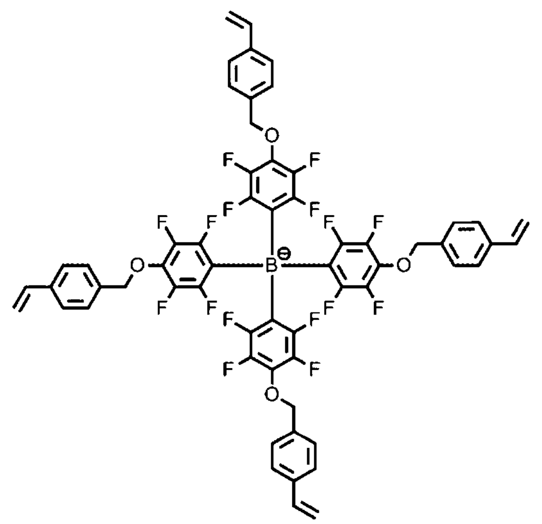

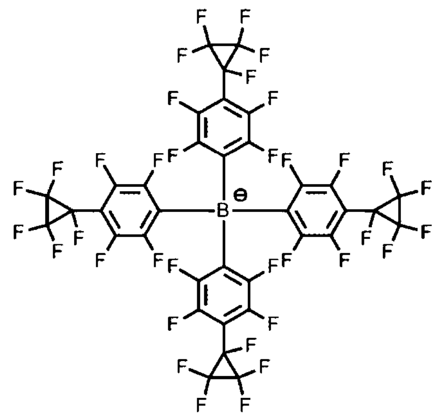

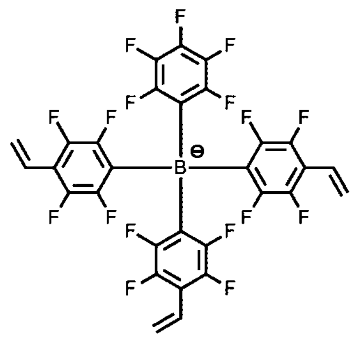

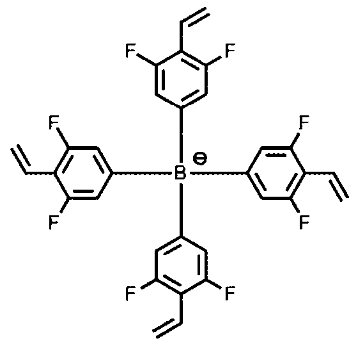

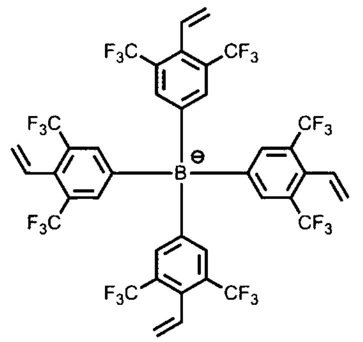

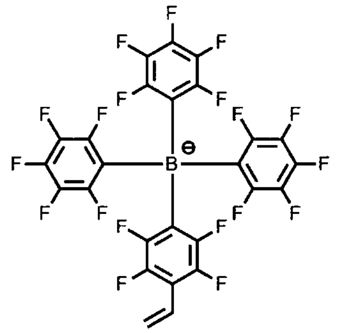

dans les composés,

n est un nombre entier compris entre 1 et 3, m est un nombre entier compris entre 1 et 3, et m+n=4,

q est un nombre entier compris entre 0 et 3, r est un nombre entier compris entre 1 et 4, et q+r=4,

Z est un deutérium ; un groupe halogène ; un groupe nitro ; un groupe cyano ; un groupe amino ; -C(O)R220' ; -OR221 ; -SR222 ; -SO3R223 ; -COOR224 ; -OC(O)R225 ; - C(O)NR226R227 ; un groupe alkyle substitué ou non substitué ; un groupe alcényle substitué ou non substitué ; un groupe alcynyle substitué ou non substitué ; un groupe amine substitué ou non substitué ;

un groupe aryle substitué ou non substitué ; ou un groupe hétérocyclique substitué ou non substitué,

l est un nombre entier compris entre 1 et 4, et lorsque l est égal ou supérieur à 2, les Z sont identiques ou différents les uns des autres, et

R220' et R221 à R227 sont identiques ou différents les uns des autres, et sont chacun indépendamment un atome d'hydrogène ; un atome de deutérium ; ou un groupe alkyle substitué ou non substitué.

**8.** La composition d'encre de la revendication 6, dans laquelle le composé ionique comprend en outre un groupe cationique,

le groupe cationique étant un groupe cationique monovalent ; un composé onium ; ou l'un quelconque choisi parmi les formules structurales suivantes :

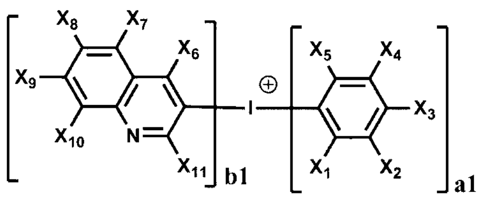

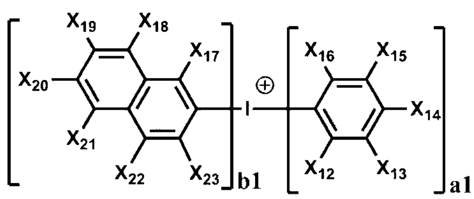

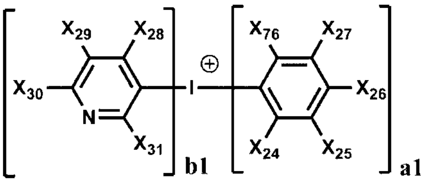
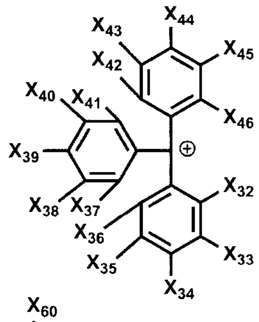
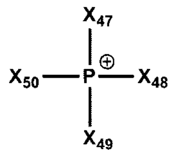
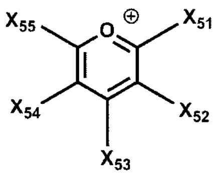
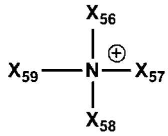
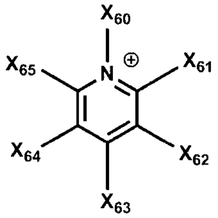
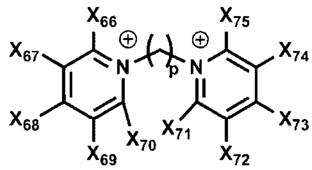

dans les formules structurales,
$X_1$ à $X_{76}$ sont identiques ou différents les uns des autres, et représentent chacun indépendamment un atome d'hydrogène ; un atome de deutérium ; un groupe cyano ; un groupe nitro ; un groupe halogène ; -COOR224 ; un groupe alkyle substitué ou non substitué ; un groupe alcoxy substitué ou non substitué ; un groupe cycloalkyle substitué ou non substitué ; un groupe fluoroalkyle substitué ou non substitué ; un groupe aryle substitué ou non substitué ; ou un groupe durcissable,
R224 est un atome d'hydrogène, un atome de deutérium ou un groupe alkyle substitué ou non substitué,
p est un nombre entier compris entre 0 et 10, et a1 vaut 1 ou 2, b1 vaut 0 ou 1, et a1+b1=2.

**9.** La composition d'encre de la revendication 8, dans laquelle la composition d'encre comprend en outre un ou plusieurs solvants supplémentaires choisis dans le groupe constitué par la tétraline, le 4-méthoxytoluène, le tétrahydrofurane, le dioxane, le dipropylène glycol monométhyl éther, le tripropylène glycol monométhyl éther, un solvant représenté par la formule chimique C-1 suivante et un solvant représenté par la formule chimique C-2 suivante :

[Formule chimique C-1]

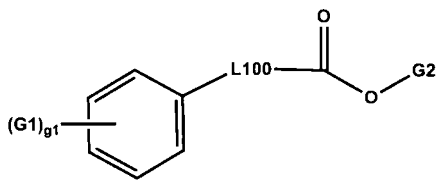

[Formule chimique C-2]

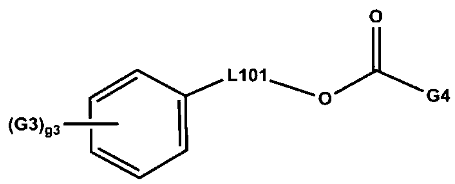

dans les formules chimiques C-1 et C-2,
L100 et L101 sont identiques ou différents l'un de l'autre, et représentent chacun indépendamment une liaison directe ; ou un groupe alkylène substitué ou non substitué,
G1 et G3 sont identiques ou différents l'un de l'autre, et représentent chacun indépendamment un atome d'hydrogène ; un atome de deutérium ; un groupe alkyle substitué ou non substitué ; un groupe alcoxy substitué ou non substitué ; un groupe aryle substitué ou non substitué ; ou un groupe hétéroaryle substitué ou non substitué,
G2 est un groupe alkyle à chaîne droite,
G4 est un groupe alkyle substitué ou non substitué, et

g1 et g3 sont chacun un nombre entier de 1 à 5, et lorsque g1 et g3 sont chacun 2 ou plus, deux ou plusieurs substituants entre parenthèses sont identiques ou différents les uns des autres.

**10.** La composition d'encre de la revendication 9, dans laquelle, sur la base de 100 % en poids de la composition d'encre,

le composé représenté par la formule chimique 1 est présent en une quantité de 0,1 % à 15 % en poids ;
le solvant représenté par la formule chimique A est présent en une quantité de 1 % à 70 % en poids ;
le composé ionique comprenant le groupe anionique représenté par la formule chimique 2 est présent en une quantité de 0,1 % à 15 % en poids ; et
le solvant supplémentaire est présent en une quantité de 10 % à 85 % en poids.

**11.** Pixel comprenant la composition d'encre de l'une quelconque des revendications 1, 2, 4 et 7 à 10 ou un produit durci de celle-ci.

**12.** Couche de matériau organique comprenant le pixel de la revendication 11.

**13.** La couche de matériau organique de la revendication 12, dans laquelle la couche de matériau organique comprend n' pixels,

les n' pixels ont une uniformité d'épaisseur V de 0,4 ou moins, et
l'uniformité d'épaisseur V satisfait à l'équation 1 suivante :

[Équation 1]

$$V = \frac{\sum_{i=2}^{n'} \left(X(i) - Xm'\right)^2}{n' \times Xm'}$$

dans l'équation 1,
X(i) est l'épaisseur du i-ième pixel,
Xm' est la valeur moyenne de X(i) dans laquelle i est 2 à n', et
n'est un entier de 2 à $10^4$.

**14.** Dispositif électroluminescent organique comprenant :

une première électrode ;
une deuxième électrode ; et
une couche de matériau organique comportant une ou
plusieurs couches disposées entre la première électrode et la deuxième électrode,
dans lequel une ou plusieurs couches de la couche de matériau organique sont la couche de matériau organique de la revendication 12.

**15.** Le dispositif électroluminescent organique selon la revendication 14, dans lequel la couche de matériau organique comprend une ou plusieurs couches choisies dans le groupe constitué d'une couche d'injection de trous, d'une couche de transport de trous, d'une couche d'injection et de transport de trous, et d'une couche de blocage d'électrons, et
la ou les couches choisies dans le groupe constitué de la couche d'injection de trous, de la couche de transport de trous, de la couche d'injection et de transport de trous, et de la couche de blocage d'électrons comprennent la composition d'encre ou un produit durci de celle-ci.

[Figure 1]

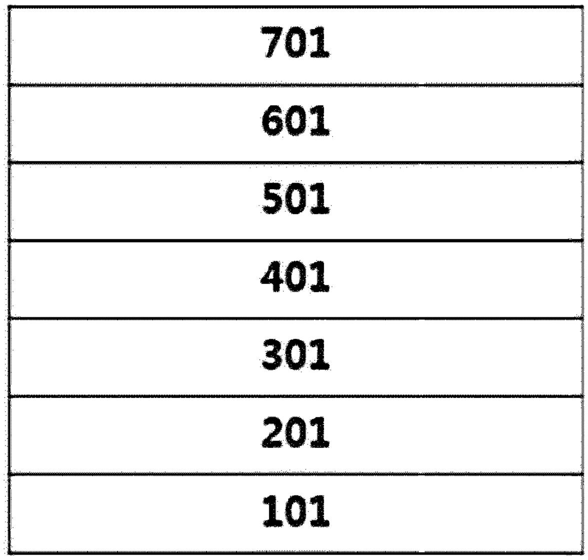


[Figure 2]

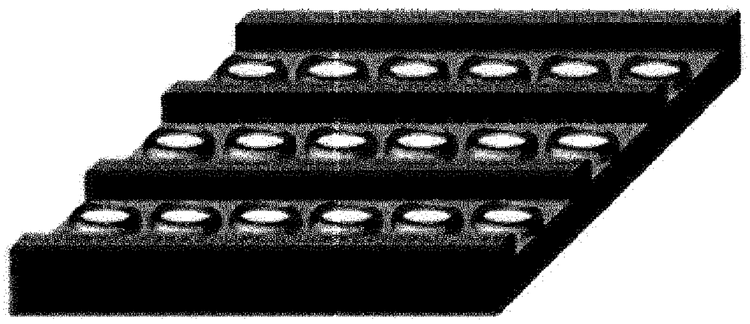

[Figure 3]

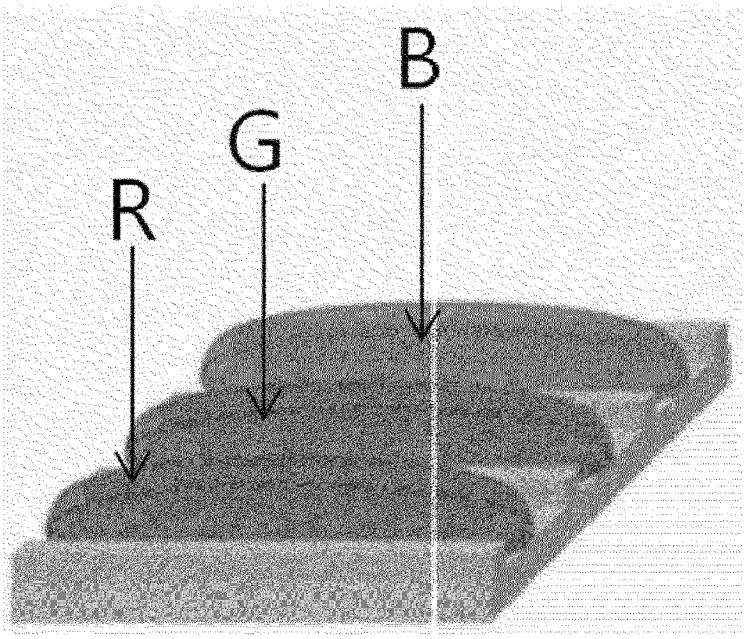


[Figure 4]

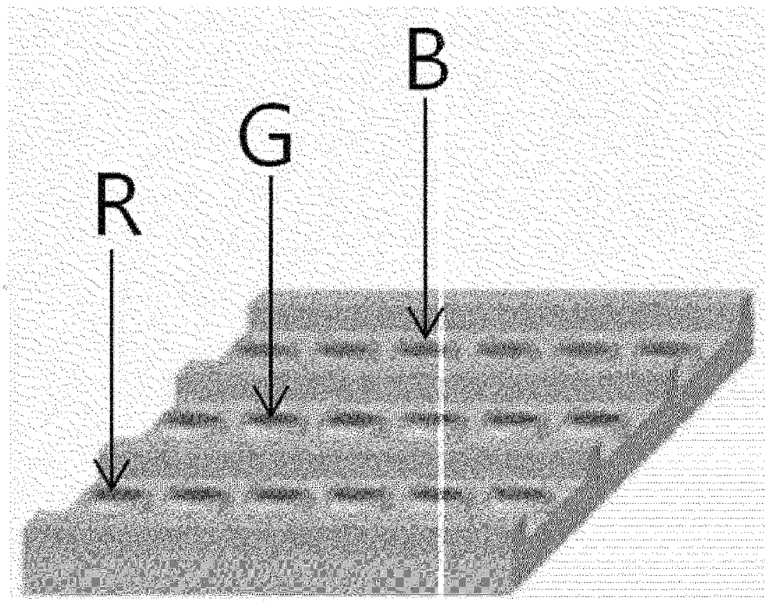

[Figure 5]

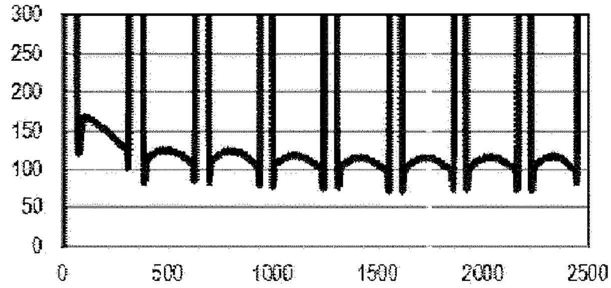


[Figure 6]

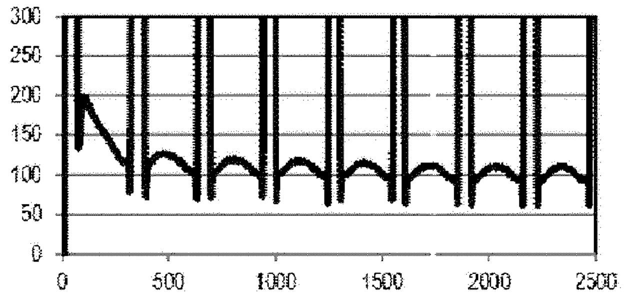


[Figure 7]

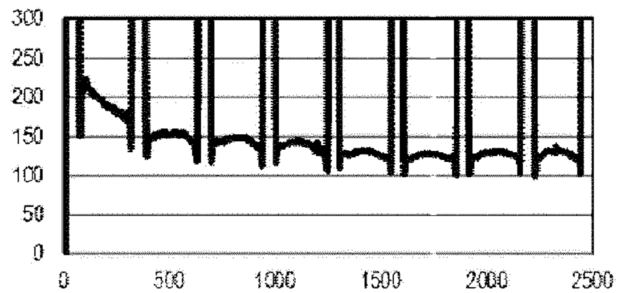


[Figure 8]

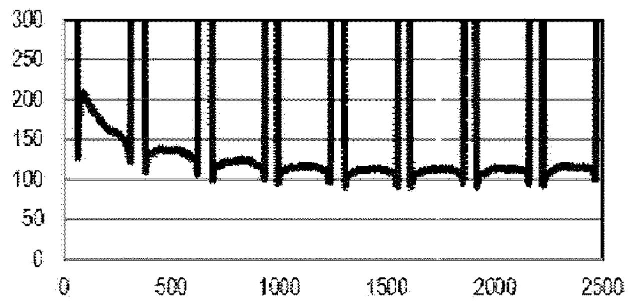

[Figure 9]

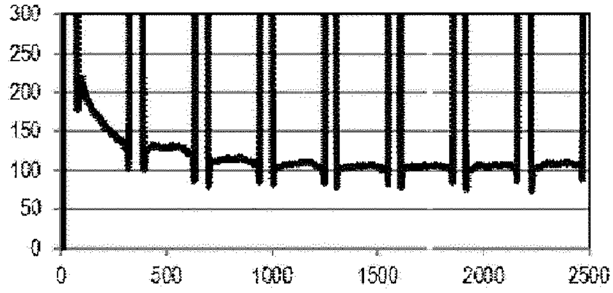


[Figure 10]

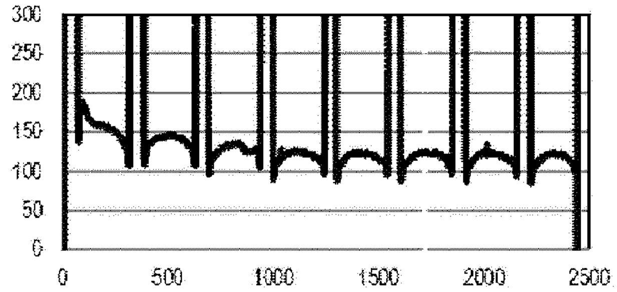


[Figure 11]

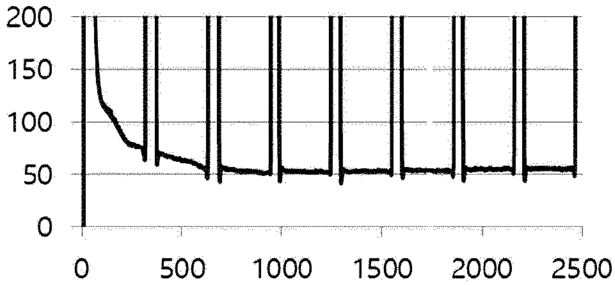


[Figure 12]

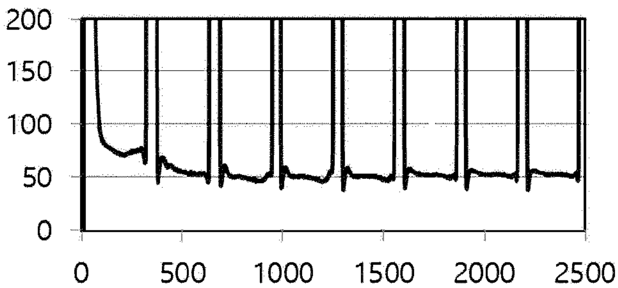

[Figure 13]

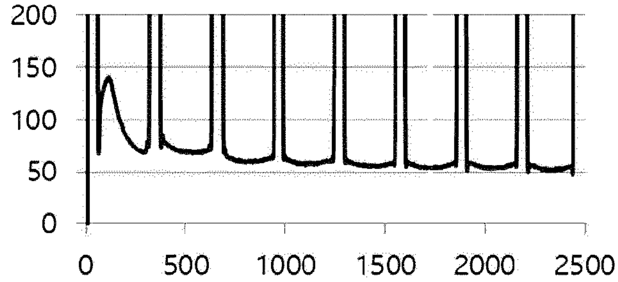


[Figure 14]

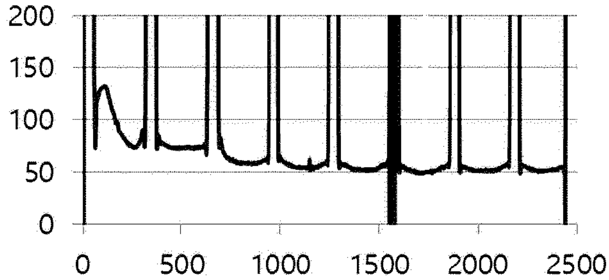


[Figure 15]

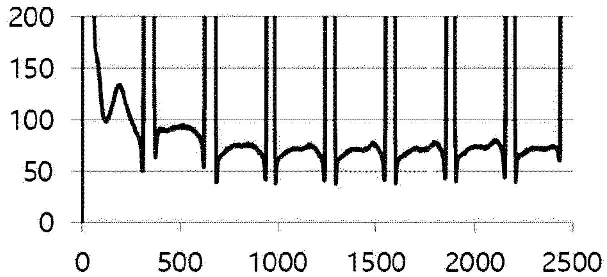


[Figure 16]

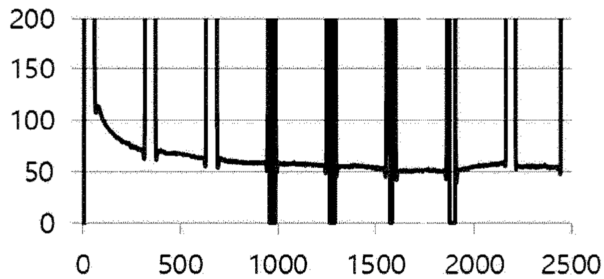

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210122564
- WO 2021154041 A1


### Non-patent literature cited in the description


- *CHEMICAL ABSTRACTS*, 91-46-3
- *CHEMICAL ABSTRACTS*, 7370-92-5
- *CHEMICAL ABSTRACTS*, 53657-08-2
- *CHEMICAL ABSTRACTS*, 63916-02-9